# EUROPEAN PATENT APPLICATION

(11) **EP 2 216 023 A1**
(43) Date of publication of application: **11.08.2010**
(21) Application number: 08851020.1
(22) Date of filing: 14.11.2008
(51) Int. Cl.: A61K 31/4545, A61K 31/5377, A61K 31/55, A61K 45/00, A61P 1/00, A61P 3/04, A61P 13/02, A61P 13/10, A61P 43/00, C07D 471/04, G01N 33/15, G01N 33/50

(54) **CONDENSED PYRIDINE DERIVATIVE AND USE THEREOF**

(30) Priority: 15.11.2007 JP 2007297170
(71) Applicant: Takeda Pharmaceutical Company Limited, Osaka-shi, Osaka 541-0045 (JP)
(72) Inventor: MATSUMOTO, Takahiro, Osaka-shi Osaka 532-8686 (JP); NAGAMIYA, Hiroyuki, Osaka-shi Osaka 532-8686 (JP); MAEZAKI, Hironobu, Osaka-shi Osaka 532-8686 (JP); KUSUMOTO, Tomokazu, Osaka-shi Osaka 532-8686 (JP); YOSHIKAWA, Kyoko, Osaka-shi Osaka 532-8686 (JP); FURUKAWA, Hideki, Osaka-shi Osaka 532-8686 (JP); KAMO, Izumi, Osaka-shi Osaka 532-8686 (JP)
(74) Representative: Held, Stephan
(86) International application number: PCT/JP2008/070809
(87) International publication number: WO 2009/063992

(57) **Abstract**

Provided are a condensed pyridine derivative having a serotonin 5-HT_{2C} receptor activation action, a prophylactic or therapeutic agent for a lower urinary tract symptom, obesity and/or organ prolapse and the like containing the condensed pyridine derivative, a screening method for a substance that increases leak point pressure upon a rise in the intravesical pressure or a prophylactic or therapeutic drug for stress urinary incontinence, a prophylactic or therapeutic drug for cystoceles or enteroceles, containing a substance that activates serotonin 5-HT_{2C} receptor, and a method of screening for a therapeutic drug for cystoceles or enteroceles, including increasing an intravesical pressure after bilateral transection of the hypogastric nerve and pudendal nerve of an animal, and measuring a closure response in the urethra, the rectum or the vagina observed at that time. A serotonin 5-HT_{2C} receptor activator containing a compound represented by the formula: 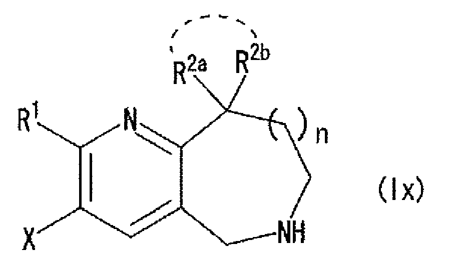 wherein each symbol is as defined in the specification, or a salt thereof.

## Description

### Technical Field

The present invention relates to a condensed pyridine derivative having an excellent serotonin 5-HT_{2C} receptor activation action, and useful as a therapeutic or prophylactic agent and the like for a lower urinary tract symptom, obesity and/or organ prolapse and the like, a screening method for a substance that increases leak point pressure upon a rise in the intravesical pressure or a prophylactic or therapeutic drug for stress urinary incontinence, a prophylactic or therapeutic drug for cystoceles and enteroceles, comprising a serotonin 5-HT_{2C} receptor activator and a screening method therefor, and a screening method for a prophylactic or therapeutic drug for cystoceles and enteroceles, which improves functional decline of pelvic floor muscles and the like.

### (Background of the Invention)

The serotonin 5-HT_{2C} receptor, which is one of the receptor of the biological transmitter, serotonin, is distributed mainly in the central nervous system and controls many physiological functions in vivo. A representative example is the control of appetite; it has been demonstrated in a study with rodents that when the central serotonin 5-HT_{2C} receptor is stimulated, eating behavior lessens and body weight is lost. In humans as well, it has been reported that when a serotonin 5-HT_{2C} receptor activator is administered, appetite is suppressed and body weight is lost (see non-patent document 1). In addition, stimulation of the central serotonin 5-HT_{2C} receptor has been shown to suppress depression-related behavior in a rat study using a serotonin 5-HT_{2C} receptor activator (see non-patent document 2), and has also been reported to be effective on many central nervous diseases such as anxiety (see non-patent document 3). The serotonin 5-HT_{2C} receptor is also highly expressed in the parasympathetic nucleus and motor neurons in the sacral spinal cord, and is thought to control peripheral nervous functions (see non-patent document 4). It has been reported that when a serotonin 5-HT_{2C} receptor activator is administered to rats, penile erection is induced (see non-patent document 5), and urethral resistance is increased (see patent document 1); all these actions are attributed to stimulation of the serotonin 5-HT_{2C} receptor in the sacral spinal cord. For serotonin 5-HT_{2C} receptor activators, many clinical applications are likely, with particular expectations for anti-obesity drugs, antidepressants, anti-anxiety drugs, therapeutic drugs for male erectile dysfunction, and therapeutic drugs for stress urinary incontinence and the like.

As the condensed pyridine derivative, the following compounds have been reported.
Patent document 2:

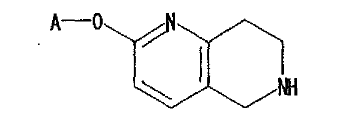

wherein A is

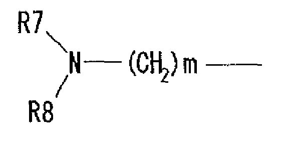

wherein m is 2-6, R7 and R8 are each hydrogen, C₁₋₆ alkyl and the like, or

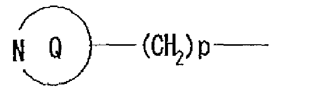

wherein Q is a 4- to 6-membered saturated heterocyclic group and p is 0 to 2;
patent document 3:

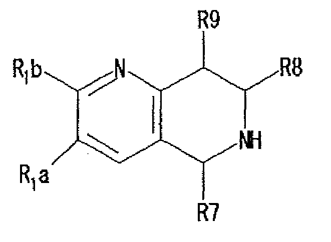

wherein R₁a is a hydrogen atom, CO₂C₁₋₆ alkyl; R₁b is CF₃, C₁₋₆ alkoxy; R7 to R9 are each a hydrogen atom and the like;
patent document 4:

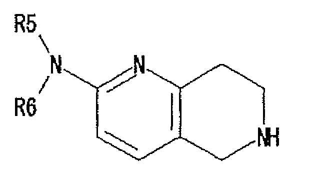

wherein R5 and R6 are each hydrogen, C₃₋₆ cycloalkyl, C₃-₆ cycloalkyl-C₁₋₄ alkylene, -SO₂(C₁₋₄ alkyl) and C₁₋₄ alkyl (optionally substituted by -OR⁸ wherein R⁸ is hydrogen or C₁₋₄ alkyl, -NR¹⁰R¹¹ (R¹⁰ and R¹¹ are each hydrogen or C₁₋₄ alkyl), Het¹ (4- to 7-membered saturated heterocyclic group) or Het⁴ (5- or 6-membered unsaturated heterocyclic group));
patent document 5:

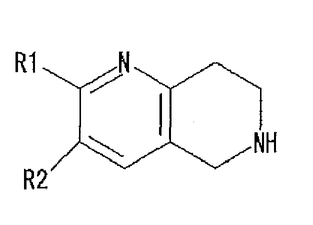

wherein R1 is hydrogen, lower alkyl; R2 is hydrogen or R1 and R2 are bonded to form a C₂₋₅ alkylene group.
However, no reference is made to the serotonin 5-HT_{2C} receptor-activating action of these compounds.

"Stress urinary incontinence" is a disease characterized by a symptom of urine leakage when abdominal pressure rises transiently as a result of coughing, sneezing, straining, light exercise such as ascending or descending of stairs and the like, carrying heavy baggage and the like, and is a disorder of urinary continence mechanism. This disease is often found in female, and considered to be developed because pelvic floor muscles are weakened due to childbirth and aging, and the anatomical positions of the organs in the pelvic floor including the bladder and the urethra have changed (see, for example, non-patent document 6). When intravesical pressure rises due to an increase in abdominal pressure, while the abdominal pressure is considered to be passively transmitted to the bladder and the urethra, the pelvic floor muscles and the external urethral sphincter actively contract via the nerve system to maintain urinary continence (see, for example, non-patent document 7). The weakening of the pelvic floor muscles and the external urethral sphincter due to childbirth and aging is one of the factors causing stress urinary incontinence, and the possibility of disordered reflex urinary continence mechanism in patients with stress urinary incontinence has been reported (see, for example, non-patent document 8).

In mammal inclusive of human, when the urinary continence mechanism is intact, the reflex elevation of urethral resistande prevents urinary leakage by an increase in the intravesical pressure irrelevant to detrusor contraction. On the other hand, for example, when even one part related to the urinary continence mechanism is disordered, for example, a disorder of a neural circuit involved in the reflex, decrease in contractile strength of muscles involved in intraurethral pressure increase and the like, reflex increase in the intraurethral pressure cannot antagonize an increase in the intravesicular pressure and urine leakage occurs. To screen for a therapeutic drug for stress urinary incontinence, therefore, an evaluation system having such pathology is important.

Since human patients with stress urinary incontinence include many parous women, and the rate thereof increases after menopause, trauma associated with childbirth and decrease of female sex hormone are presumed to cause the onset of stress urinary incontinence, and parous rats (or virginal female rats with vaginal expansion), rats with ovariectomy, or a combination of these is reported as a rat model for stress urinary incontinence (see, for example, non-patent documents 9 and 10). In clinical setting, one of the evaluation items used for diagnosing stress urinary incontinence is a leak point pressure showing the urethral resistance in the urinary storage phase. In animal experiments, reported methods for the measurement of the leak point pressure upon rise in the intravesicular pressure include induction of sneezing by mechanical stimulation of intranasal mucous membrane, electric stimulation of abdominal muscle to cause abrupt rise of the abdominal pressure, connecting a syringe filled with saline to the bladder and increasing the height thereof until urine leakage is induced, pressing the bladder filled with a small amount of saline or abdomen until induction of urine leakage, and the like (see, for example, non-patent documents 11 to 13). By the method inducing sneezing, however, the level of increase in the intravesicular pressure depends on the size of sneezing, which is difficult to control. The method including electric stimulation of abdominal muscle of rats and the method including connecting a container filled with saline to the bladder and increasing the height thereof until induction of urine leakage require many trials of intravesicular pressure increase by raising the increase level of intravesicular pressure so as to determine the leak point pressure, and therefore, are not entirely efficient screening methods. In addition, the method including pressing bladder filled with a small amount of saline or abdomen of the test animal until induction of urine leakage does not ensure an increase in the intravesicular pressure by a stable procedure.

A method including injecting saline into the bladder of a rat under anesthesia at a predetermined rate with an infusion pump and determining the leak point pressure has also been reported (see, for example, non-patent document 10). However, this method uses an animal showing micturition reflex, in which injection was performed at a rate of 6 ml/hr as in general cystometry, and the intravesicular pressure at a moment when urination began was taken as the leak point pressure (modified leak point pressure). The intravesicular pressure at the time point of the start of voiding in an animal showing micturition reflex may reflect urethral resistance after relaxation of the urethra due to micturition reflex, and evaluation of the urethral resistance in the urinary storage phase is hardly achieved. In addition, injection at a rate of 6 ml/h requires a long time before urine leakage is observed, and is considered to not accompany an abrupt increase in the intravesicular pressure like human pathology.

Cystoceles and enteroceles are diseases wherein bladder and small intestine descend and protrude beyond the vaginal orifice (see, for example, non-patent document 14 and non-patent document 15). This descent becomes conspicuous when abdominal pressure rises transiently as a result of straining or bearing a heavy load and the like. These diseases are prevalent in females, with childbirth, aging, and obesity being known as risk factors, where one of the causes is considered to be the weakening of pelvic floor muscles, fascia and connective tissues surrounding the organs, which support the intrapelvic organs and the like including bladder. The pelvic floor muscles are skeletal muscles that unite with the pelvis in a hammock-like way, serving constantly to maintain some contraction and support the organs in the pelvis from below. In cystoceles and enteroceles, organ weights reportedly become unendurable because of the weakening of these pelvic floor muscles, resulting in the descent of bladder and the like (see, for example, non-patent document 14 and non-patent document 15), it is thought that when abdominal pressure rises particularly, the increased pressure becomes unendurable and the protrusion becomes more conspicuous. On the other hand, it has been reported that when abdominal pressure rises, the urinary bladder is compressed, and the pelvic floor muscles contract by the reflex via bladder-spinal cord-pelvic floor muscles (see, for example, non-patent document 16). For this reason, it is thought that upon a rise in abdominal pressure, the pelvic floor muscles contract reflexly to prevent the descent of bladder, small intestine and the like. If there is a disorder in this reflex pathway or the pelvic floor muscles, sufficient contraction of the pelvic floor muscles cannot be obtained and support for the bladder, small intestine and the like becomes inadequate. Therefore, in screening for a therapeutic drug for cystoceles and enteroceles, a test system for evaluating the contractile responses of the pelvic floor muscles is useful. However, almost no basic studies have been conducted on these diseases, and currently there is no evaluation system.

Many compounds are known to bind to the serotonin 5-HT_{2C} receptor; patent documents 6 to 11 state that compounds that bind to the serotonin 5-HT_{2C} receptor are useful in the treatment of stress urinary incontinence and the like, but there is no statement on their therapeutic effects on cystoceles and enteroceles.
patent document 1: WO04/09619
patent document 2: WO2005/111
patent document 3: WO2004/06916
patent document 4: WO03/07642
patent document 5: JP-A-58-57379
patent document 5: WO02/040457
patent document 7: WO02/08386
patent document 8: WO03/09763
patent document 9: WO04/00082
patent document 10: WO04/000830
patent document 11: WO02/0081
non-patent document 1: Expert Opinion on Investigational Drugs, 2006, vol. 15, p. 257-266
non-patent document 2: J. Pharmacol. Exp. Ther., 1998, vol. 286, p. 913-924
non-patent document 3: Pharmacology Biochemistry Behavior, 2002, vol. 71, p. 533-554
non-patent document 4: Neuroscience, 1999, vol. 92, p. 1523-1537
non-patent document 5: Eur. J. Pharmacol., 2004, vol. 483, p. 37-43
non-patent document 6: The Journal of Family Practice, 1982, vol. 14, p. 935-936
non-patent document 7: The Journal of Urology, 1982, vol. 127, p. 1202-1206
non-patent document 8: British Journal of Urology, 1994, vol. 73, p. 413-417
non-patent document 9: Urology, 1998, vol. 52, p. 143-151 non-patent document 10: The Journal of Urology, 2001, vol. 166, p. 311-317
non-patent document 11: American Journal of Physiology-Regulatory, Integrative and Comparative Physiology, 2003, vol. 285, p. R356-R365
non-patent document 12: American Journal of Physiology-Renal Physiology, 2007, vol. 293, p. F920-F926
non-patent document 13: International Urogynecology Journal Including Pelvic Floor Dysfunction, 2005, vol. 16, p. 359-363
non-patent document 14: Lancet 2007, vol. 369, p. 1027-38 non-patent document 15: European Urology 2007, vol. 51, p. 884-886
non-patent document 16: American Journal of Physiology Renal Physiology 2004, vol. 287, p. F434-441

### Disclosure of the Invention

### Problems to be Solved by the Invention

There is a demand for the development of a compound that possesses serotonin 5-HT_{2C} receptor activation action, that is useful as an agent for the treatment or prophylaxis and the like of a lower urinary tract symptom, obesity and/or organ prolapse and the like, and that has excellent profiles in terms of receptor selectivity, pharmacological efficacy, duration of action, specificity, low toxicity and the like.

The present invention aims to provide a condensed pyridine derivative having a serotonin 5-HT_{2C} receptor activating action and the like, which has a chemical structure different from that of known compounds (including the aforementioned compounds), and an agent for the prophylaxis or treatment of diseases such as a lower urinary tract symptom, obesity and/or organ prolapse and the like, containing the condensed pyridine derivative.

When screening for a new therapeutic drug for stress urinary incontinence, construction of an in vivo evaluation system, which is convenient, useful and operable by a stable procedure is an important problem. As an efficacy evaluation system by the measurement of leak point pressure due to a transient increase in the intravesicular pressure in animals, measurement of the leak point pressure by induction of sneezing, electric stimulation of abdominal muscles, changing the height of a container containing saline, which is connected to the bladder, directly pressing the bladder or abdomen and the like, and the like have been employed so far. Moreover, since the methods based on sneezing, electric stimulation, control of intravesicular pressure by adjusting the height of a container and the like require many times of increase in the intravesicular pressure, and the measurement takes time since the minimum intravesical pressure inducing urine incontinence is taken as the leak point pressure. In addition, the method including pressing the bladder or abdomen does not ensure operation by a stable procedure, and is not a convenient method suitable for screening.
The present invention aims to construct a new evaluation system capable of efficiently screening for a therapeutic drug for stress urinary incontinence by a stable procedure, and provide a superior therapeutic drug for stress urinary incontinence.

Furthermore, a medicament for cystoceles or enteroceles has not been developed. Therefore, once a medicament that improves functional disorders including decline in the reflex contractile force of the pelvic floor muscles and prevents cystoceles or enteroceles is developed, it can improve quality of life of many patients.
The present invention aims to provide an agent for the prophylaxis or treatment of cystoceles or enteroceles, for which no effective therapeutic drug exists. Moreover, the present invention aims to provide a method of screening for such drugs.

### Means of Solving the Problems

The present inventors have conducted intensive studies in an attempt to solve the aforementioned problems and found that a compound represented by the following formula and a salt thereof have a superior serotonin 5-HT_{2C} receptor activation action.
Moreover, the present inventors have enabled measurement of a leak point pressure by injecting saline rapidly and transiently with an infusion pump and the like into the bladder of an animal showing no micturition reflex due to an operation such as spinal cord transection and the like but showing functional bladder-spinal cord-urethra reflex.
Furthermore, the present inventors have found that a substance that activates serotonin 5-HT_{2C} receptor can increase reflex contraction reaction of the pelvic floor muscles and prevent or treat cystoceles or enteroceles. In a state where the spinal cord is cut at the thoracic spinal cord, and where the hypogastric nerve and the pudendal nerve are cut bilaterally, the urethral closure responses observed upon a rise in the intravesical pressure in an anesthetized female rat are remarkably decreased by cutting the nerves that innervates iliococcygeus muscle and the pubococcygeus muscle bilaterally, and are reactions by the iliococcygeus muscle and the pubococcygeus muscle, which are the pelvic floor muscles. That is, it is possible to evaluate the contractile responses of the pelvic floor muscles in vivo by raising intravesical pressure in a condition wherein the hypogastric nerve and the pudendal nerve are cut bilaterally, and monitoring the urethral closure responses. Thus, the present inventors found that a new method of in vivo evaluation of pharmacological efficacy for seeking a therapeutic drug for cystoceles or enteroceles is provided by introducing this evaluation item. On the basis of the same principle as this method, by raising the intravesical pressure in a state wherein the hypogastric nerve and the pudendal nerve are cut bilaterally, and measuring the closure responses of rectal internal pressure or vaginal internal pressure, it is also possible to evaluate the contractile responses of the iliococcygeus muscle and the pubococcygeus muscle, which are the pelvic floor muscles. Urethral resistance in the urine storage phase can also be evaluated instead of urethral closure responses. As an index of urethral resistance, leak point pressure, which is an intravesical pressure that causes urine leakage, can be used. By raising the intravesical pressure in a condition wherein the hypogastric nerve and the pudendal nerve are cut bilaterally, and evaluating leak point pressure, it is possible to evaluate the contractile responses of the pelvic floor muscles. According to these methods, a new in vivo efficacy evaluation method for searching a therapeutic drug for cystoceles or enteroceles is provided.
Furthermore, the present inventors have found that a substance that activates a serotonin 5-HT_{2C} receptor increases the reflex contractile responses of the pelvic floor muscles by using these in vivo efficacy evaluation methods, and newly found that a substance that activates the serotonin 5-HT_{2C} receptor can prevent or treat cystoceles or enteroceles. The present inventors have conducted further investigations based on these findings, and completed the present invention.
Accordingly, the present invention relates to the following:
[1] A serotonin 5-HT_{2c} receptor activator comprising a compound represented by the formula

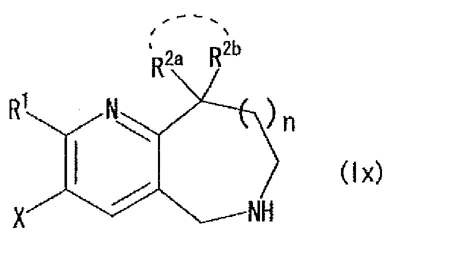

wherein
X is a hydrogen atom or a halogen atom,
R¹ is
(1) a group represented by the formula

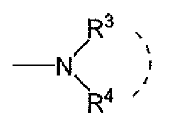

wherein
R³ and R⁴ are the same or different and each is a hydrogen atom, a cycloalkyl group or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,

(2) a group represented by -OR⁵ wherein R⁵ is a hydrogen atom, a cycloalkyl group, an alkyl group optionally substituted by a cycloalkyl group, an aryl group or a heteroaryl group,
(3) a group represented by -SR⁶ wherein R⁶ is an alkyl group, an aryl group or a heteroaryl group,
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof (hereinafter to be sometimes abbreviated as "compound (Ix)") .
[2] A serotonin 5-HT_{2c} receptor activator comprising a compound represented by the formula

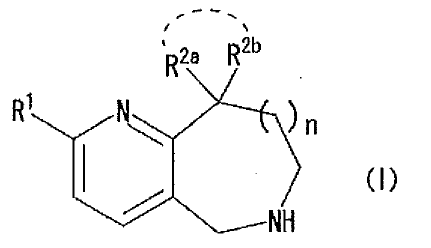

wherein
R¹ is
(1) a group represented by the formula

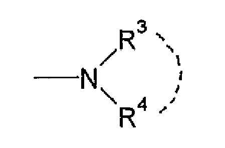

wherein
R³ and R⁴ are the same or different and each is a hydrogen atom or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ wherein R⁵ is an alkyl group optionally substituted by a cycloalkyl group, an aryl group or a heteroaryl group,
(3) a group represented by -SR⁶ wherein R⁶ is an alkyl group, an aryl group or a heteroaryl group,
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof (hereinafter to be sometimes abbreviated as "compound (I)").
[3] The agent of the above-mentioned [1], wherein R¹ is a group represented by the formula

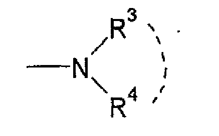

wherein
R³ and R⁴ are the same or different and each is a hydrogen atom, a cycloalkyl group or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group.
[4] The agent of the above-mentioned [1], wherein n is 0.
[5] The agent of the above-mentioned [1], which is an agent for the prophylaxis or treatment of a lower urinary tract symptom, obesity and/or organ prolapse.
[6] A method for the prophylaxis or treatment of a lower urinary tract symptom, obesity and/or organ prolapse, comprising administering, to a mammal, an effective amount of a compound represented by the formula

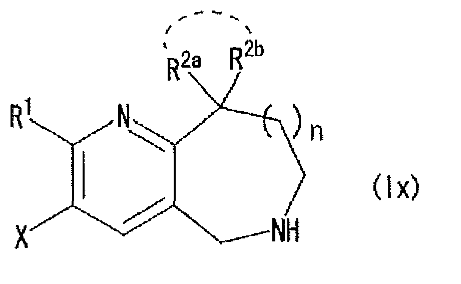

wherein
X is a hydrogen atom or a halogen atom,
R¹ is
(1) a group represented by the formula

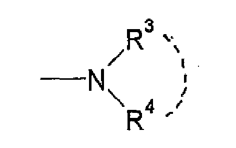

wherein
R³ and R⁴ are the same or different and each is a hydrogen atom, a cycloalkyl group or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ wherein R⁵ is a hydrogen atom, a cycloalkyl group, an alkyl group optionally substituted by a cycloalkyl group, an aryl group or a heteroaryl group,
(3) a group represented by -SR⁶ wherein R⁶ is an alkyl group, an aryl group or a heteroaryl group,
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof.
[7] A method for the prophylaxis or treatment of a lower urinary tract symptom, obesity and/or organ prolapse, comprising administering, to a mammal, an effective amount of a compound represented by the formula

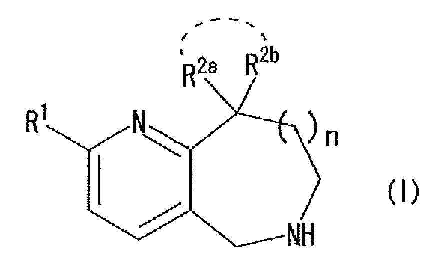

wherein
R¹ is
(1) a group represented by the formula

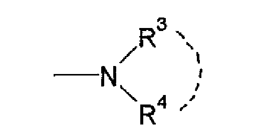

wherein
R³ and R⁴ are the same or different and each is a hydrogen atom or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,

(2) a group represented by -OR⁵ (R⁵ is an alkyl group optionally substituted by a cycloalkyl group, an aryl group or a heteroaryl group),
(3) a group represented by -SR⁶ (R⁶ is an alkyl group, an aryl group or a heteroaryl group,
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof.
[8] Use of a compound represented by the formula

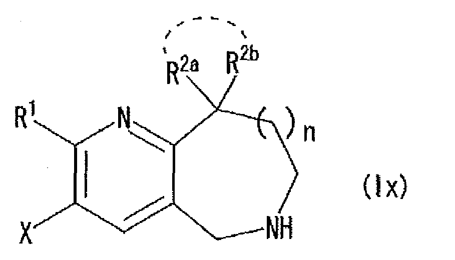

wherein
X is a hydrogen atom or a halogen atom,
R¹ is
(1) a group represented by the formula

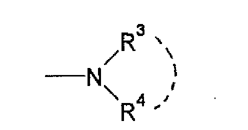

wherein
R³ and R⁴ are the same or different and each is a hydrogen atom, a cycloalkyl group or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ (R⁵ is a hydrogen atom, a cycloalkyl group, an alkyl group optionally substituted by a cycloalkyl group, an aryl group or a heteroaryl group,
(3) a group represented by -SR⁶ (R⁶ is an alkyl group, an aryl group or a heteroaryl group),
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof for the production of a prophylactic or therapeutic agent for a lower urinary tract symptom, obesity and/or organ prolapse.
[9] Use of a compound represented by the formula

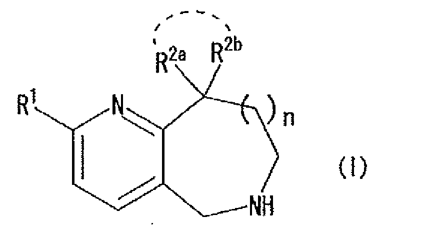

wherein
R¹ is
(1) a group represented by the formula

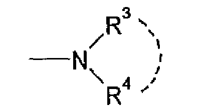

wherein
R³ and R⁴ are the same or different and each is a hydrogen atom or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,

(2) a group represented by -OR⁵ (R⁵ is an alkyl group optionally substituted by a cycloalkyl group, an aryl group or a heteroaryl group),
(3) a group represented by -SR⁶ (R⁶ is an alkyl group, an aryl group or a heteroaryl group),
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof for the production of a prophylactic or therapeutic agent for a lower urinary tract symptom, obesity and/or organ prolapse.
[10] A compound represented by the formula

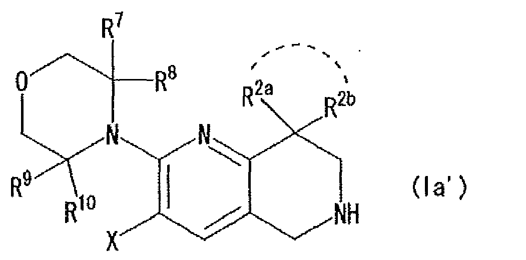

wherein
X is a hydrogen atom or a halogen atom,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
R⁷, R⁸, R⁹ and R¹⁰ are the same or different and each is a hydrogen atom or a substituent,
provided that
(1) one or each of R^{2a} and R^{2b} is an optionally substituted alkyl group,
(2) R⁷, R⁸, R⁹ and R¹⁰ are the same or different and each is a hydrogen atom or a substituent, and R^{2a} and R^{2b} form, together with the adjacent carbon atom, an alicyclic hydrocarbon group optionally substituted by substituent(s) selected from a halogen atom, an alkoxy group and an alkyl group, and
(3) at least one group of R⁷, R⁸, R⁹ and R¹⁰ is a substituent, or a salt thereof (hereinafter sometimes to be abbreviated as "compound (a')").
[11] A compound represented by the formula

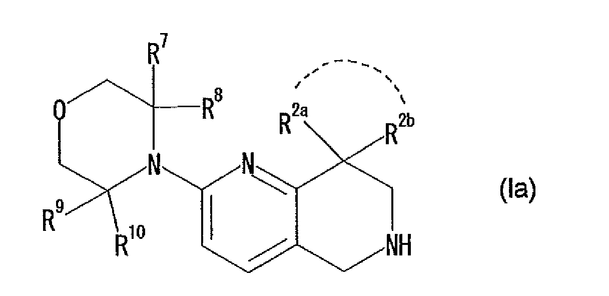

wherein
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
R⁷, R⁸, R⁹ and R¹⁰ are the same or different and each is a hydrogen atom or a substituent,
provided that
(1) one or each of R^{2a} and R^{2b} is an optionally substituted alkyl group,
(2) R⁷, R⁸, R⁹ and R¹⁰ are the same or different and each is a hydrogen atom or a substituent, and R^{2a} and R^{2b} form, together with the adjacent carbon atom, an alicyclic hydrocarbon group optionally substituted by substituent(s) selected from a halogen atom, an alkoxy group and an alkyl group, or
(3) at least one group of R⁷, R⁸, R⁹ and R¹⁰ is a substituent, or a salt thereof (hereinafter sometimes to be abbreviated as "compound (Ia)").
[12] The compound of the above-mentioned [10], wherein at least one group of R^{2a}, R^{2b}, R⁷, R⁸, R⁹ and R¹⁰ is an optionally substituted alkyl group.
[13] A compound represented by the formula

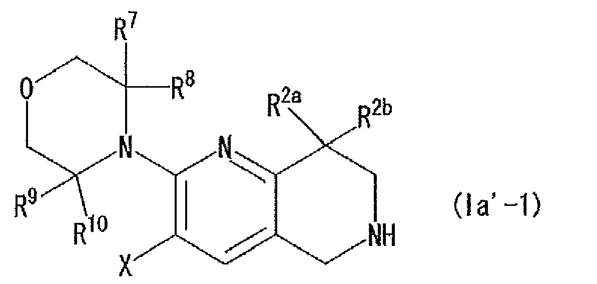

wherein
(1)
   X is a hydrogen atom,
   R^{2a} and R^{2b} are the same or different and each is a hydrogen atom or a C₁₋₆ alkyl group (provided that one or each of R^{2a} and R^{2b} is a C₁₋₆ alkyl group),
   R⁷ and R⁸ are the same or different and each is a hydrogen atom or a C₁₋₆ alkyl group (provided that at least one group of R⁷ and R⁸ is a hydrogen atom), and
   R⁹ and R¹⁰ are each a hydrogen atom, or
(2)
   X is a hydrogen atom or a halogen atom,
   R^{2a} and R^{2b} are each a hydrogen atom,
   R⁷, R⁹ and R¹⁰ are each a hydrogen atom, and
   R⁸ is a C₁₋₆ alkyl group,
or a salt thereof (hereinafter sometimes to be abbreviated as "compound (Ia'-1)").
[14] A compound represented by the formula

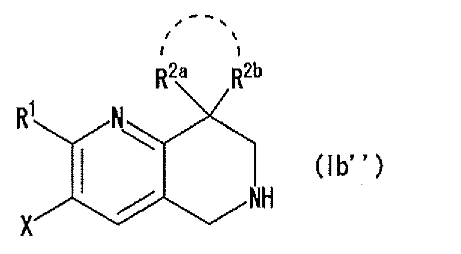

wherein
X is a hydrogen atom or a halogen atom,
R¹ is a group represented by the formula

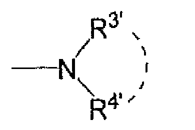

wherein
R³' is a hydrogen atom or an alkyl group optionally substituted by a cycloalkyl group,
R^{4'} is a cycloalkyl group or an alkyl group optionally substituted by a cycloalkyl group, or
R^{3'} and R^{4'} are crosslinked to each other via a carbon-carbon bond to form, together with the adjacent nitrogen atom, a non-aromatic cyclic amino group optionally substituted by substituent(s) selected from a halogen atom, an alkyl group, an alkoxy group and an aryl group, excluding a methylamino group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group,
or a salt thereof (hereinafter sometimes to be abbreviated as "compound (Ib")").
[15] A compound represented by the formula

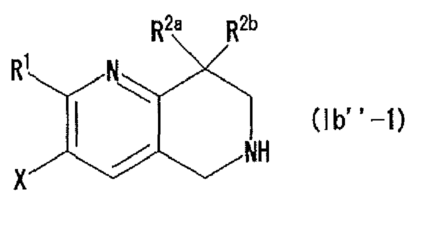

wherein
X is a hydrogen atom or a halogen atom,
R¹ is
(1) a group represented by the formula

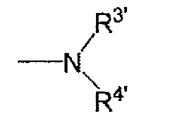

wherein
R^{3'} is a hydrogen atom or a C₁₋₆ alkyl group, and
R^{4'} is a C₃₋₆ cycloalkyl group or a C₁₋₆ alkyl group optionally substituted by a C₃₋₆ cycloalkyl group, excluding a methylamino group, or
(2) a group represented by the formula 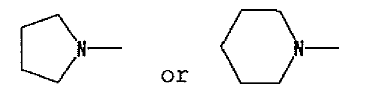 optionally substituted by 1 to 3 C₁₋₆ alkyl group, and
R^{2a} and R^{2b} are the same or different and each is a hydrogen atom or a C₁₋₆ alkyl group,
or a salt thereof (hereinafter sometimes to be abbreviated as "compound (Ib"-1)").
[16] A compound represented by the formula 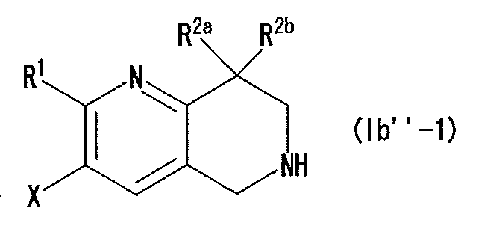 wherein
X is a hydrogen atom or a halogen atom,
R¹ is
(1) a group represented by the formula

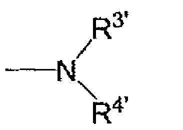

wherein
R^{3'} is a hydrogen atom or a C₁₋₆ alkyl group, and
R^{4'} is C₃₋₆ cycloalkyl or a C₁₋₆ alkyl group optionally substituted by a C₃₋₆ cycloalkyl group, excluding a methylamino group, or
(2) a group represented by the formula

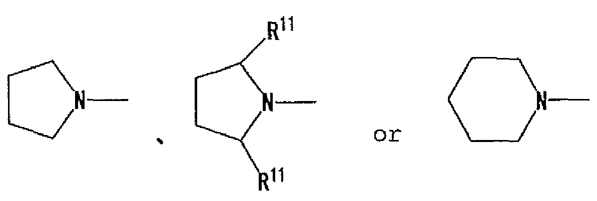

wherein R¹¹ is a hydrogen atom or a C₁₋₆ alkyl group, and
R^{2a} and R^{2b} are the same or different and each is a hydrogen atom or a C₁₋₆ alkyl group (provided that at least one group of R^{2a} and R^{2b} is a hydrogen atom),
or a salt thereof.
[17] A compound represented by the formula

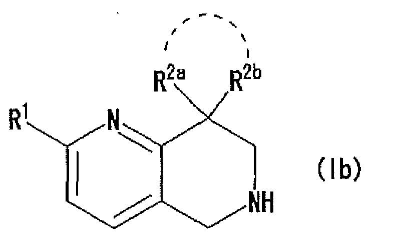

wherein
R¹ is a group represented by the formula

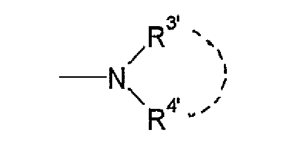

wherein
R^{3'} is a hydrogen atom or an alkyl group optionally substituted by a cycloalkyl group,
R^{4'} is an alkyl ,group optionally substituted by a cycloalkyl group, or
R^{3'} and R^{4'} are crosslinked to each other via a carbon-carbon bond to form, together with the adjacent nitrogen atom, a non-aromatic cyclic amino group optionally substituted by substituent(s) selected from a halogen atom, an alkyl group, an alkoxy group and an aryl group,
excluding a methylamino group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group,
or a salt thereof (hereinafter sometimes to be abbreviated as "compound (Ib)").
[18] A compound represented by the formula

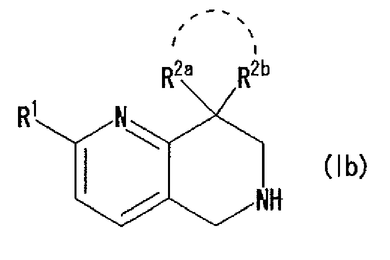

wherein
R¹ is
(1) a group represented by -OR⁵ (R⁵ is a hydrogen atom, a cycloalkyl group, an alkyl group optionally substituted by a cycloalkyl group, an aryl group or a heteroaryl group),
(2) a C₃₋₅ alkyl group (alkyl group having a carbon number of 3 to 5),
(3) an aryl group optionally substituted by substituent(s) selected from a halogen atom, an alkyl group optionally substituted by a halogen atom, an alkoxy group, an alkylthio group and an aryl group,
(4) a heteroaryl group, or
(5) a cycloalkyl group,
   R^{2a} and R^{2b} are the same or different and each is a hydrogen atom or an optionally substituted alkyl group, or
   R^{2a} and R^{2b} form, together with the adjacent carbon atom, an alicyclic hydrocarbon group optionally substituted by substituent(s) selected from a halogen atom, an alkyl group and an alkoxy group,
(excluding a compound wherein R¹ is a methoxy group and R^{2a} and R^{2b} are each a hydrogen atom),
or a salt thereof.
[19] A compound represented by the formula

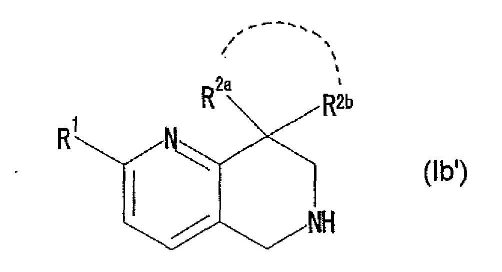

wherein
R¹ is
(1) a group represented by -OR⁵ (R⁵ is an alkyl group optionally substituted by a cycloalkyl group, an aryl group or a heteroaryl group),
(2) a C₃ alkyl group (alkyl group having a carbon number of 3),
(3) an aryl group optionally substituted by substituent(s) selected from a halogen atom, an alkyl group optionally substituted by a halogen atom, an alkoxy group, an alkylthio group and an aryl group, or
(4) a heteroaryl group,
   R^{2a} and R^{2b} are the same or different and each is a hydrogen atom or an optionally substituted alkyl group, or
   R^{2a} and R^{2b} form, together with the adjacent carbon atom, an alicyclic hydrocarbon group optionally substituted by substituent(s) selected from a halogen atom, an alkyl group and an alkoxy group,
(excluding a compound wherein R¹ is a methoxy group and R^{2a} and R^{2b} are each a hydrogen atom),
or a salt thereof (hereinafter to be sometimes abbreviated as "compound (Ib')").
[20] A compound represented by the formula

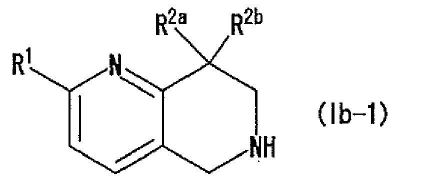

wherein
R¹ is
(1) a group represented by -OR⁵ (R⁵ is a hydrogen atom, a C₃₋₆ cycloalkyl group, or a C₁₋₆ alkyl group optionally substituted by a C₃₋₆ cycloalkyl group),
(2) a C₃₋₅ alkyl group,
(3) a phenyl group optionally substituted by substituent(s) selected from a halogen atom, a C₁₋₆ alkyl group optionally substituted by a halogen atom, and a C₁₋₆ alkylthio group,
(4) a group represented by the formula 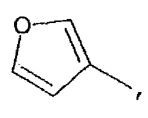
or

(5) a C₃₋₆ cycloalkyl group,
R^{2a} and R^{2b} are the same or different and each is a hydrogen atom or an optionally substituted alkyl group, (excluding a compound wherein R¹ is a methoxy group and R^{2a} and R^{2b} are each a hydrogen atom),
or a salt thereof (hereinafter to be sometimes abbreviated as "compound (Ib-1)").
[21] The compound of the above-mentioned [18], wherein R¹ is a phenyl group substituted by substituent(s) selected from a halogen atom, a C₁₋₆ alkyl group substituted by a halogen atom and a C₁₋₆ alkylthio group.
[22] A compound represented by the formula

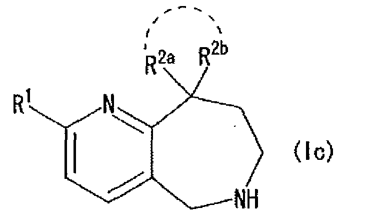

wherein
R¹ is
(1) a group represented by the formula

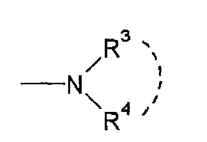

wherein
R³ and R⁴ are the same or different and each is a hydrogen atom or an alkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,

(2) a group represented by -OR⁵ (R⁵ is an alkyl group, an aryl group or a heteroaryl group),
(3) a group represented by -SR⁶ (R⁶ is an alkyl group, an aryl group or a heteroaryl group,
(4) an alkyl group,
(5) a cycloalkyl group,
(6) an aryl group optionally substituted by substituent(s) selected from a halogen atom, an alkyl group, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group,
or a salt thereof (hereinafter sometimes to be abbreviated as "compound (Ic)").
[23] A compound represented by the formula

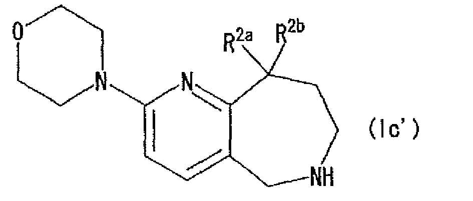

wherein one of R^{2a} and R^{2b} is a hydrogen atom, and the other is a C₁₋₆ alkyl group,
or a salt thereof (hereinafter to be sometimes abbreviated as "compound (Ic')").
[24] 2-Isopropyl-5,6,7,8-tetrahydro-1,6-naphthyridine or a salt thereof.
[25] 2-Isopropoxy-5,6,7,8-tetrahydro-1,6-naphthyridine or a salt thereof.
[26] 2-Cyclopropyl-5,6,7,8-tetrahydro-1,6-naphthyridine or a salt thereof.
[27] 8-Methyl-2-morpholin-4-yl-5,6,7,8-tetrahydro-1,6-naphthyridine or a salt thereof.
[28] 8-Methyl-2-[(3R)-3-methylmorpholin-4-yl]-5,6,7,8-tetrahydro-1,6-naphthyridine or a salt thereof.
[29] 9-Methyl-2-morpholin-4-yl-6,7,8,9-tetrahydro-5H-pyrido[3,2-c]azepine or a salt thereof.
[30] N-Isopropyl-N,8-dimethyl-5,6,7,8-tetrahydro-1,6-naphthyridin-2-amine or a salt thereof.
[31] A prodrug of the compound of any of the above-mentioned [10] - [30].
[32] A medicament comprising the compound of any of the above-mentioned [10] - [30] or a prodrug thereof.
[33] A method of screening for a substance that increases a leak point pressure upon a rise in the intravesical pressure, which comprises measuring a leak point pressure when an intravesical pressure is increased by injection of saline rapidly and transiently into the bladder of an animal showing no urination reflection but showing a functional bladder-spinal cord- urethra reflex.
[34] A method of screening for a prophylactic or therapeutic drug for stress urinary incontinence, which comprises measuring a leak point pressure when an intravesical pressure is increased by injection of saline rapidly and transiently into the bladder of an animal showing no micturition reflex but showing a functional bladder-spinal cord-urethra reflex.
[35] The method of the above-mentioned [33] or [34], wherein the animal used shows a decreased leak point pressure upon a rise in the intravesical pressure.
[36] The method of the above-mentioned [35], wherein the decreased leak point pressure upon a rise in the intravesical pressure is based on the transection or injury of the nerve involved in the reflex contraction of pelvic floor muscles or external urethral sphincter, childbirth, ovariectomy, a mechanical vaginal expansion treatment, diabetes, a drug administration or a combination of these.
[37] The method of the above-mentioned [33] or [34], wherein the animal is female.
[38] A drug for the prophylaxis or treatment of cystoceles or enteroceles, comprising a serotonin 5-HT_{2C} receptor activator.
[39] A method for the prophylaxis or treatment of cystoceles or enteroceles, comprising administering an effective amount of a serotonin 5-HT_{2C} receptor activator to a mammal.
[40] Use of a serotonin 5-HT_{2C} receptor activator for the production of an agent for the prophylaxis or treatment of cystoceles or enteroceles.
[41] A method of screening for a drug for the prophylaxis or treatment of cystoceles or enteroceles, comprising increasing an intravesical pressure after bilateral transection of the hypogastric nerve and pudendal nerve of an animal, and measuring a closure response in the urethra, the rectum or the vagina observed at that time.
[42] A method of screening for a drug for the prophylaxis or treatment of cystoceles or enteroceles, comprising increasing an intravesical pressure after bilateral transection of the hypogastric nerve and pudendal nerve of an animal, and measuring the leak point pressure at that time.
[43] The method of the above-mentioned [41] or [42], wherein the closure response in the urethra, the rectum or the vagina or urethral resistance (decreased leak point pressure) upon increase in the intravesical pressure after bilateral transection of the hypogastric nerve and pudendal nerve of an animal is based on the unilateral transection or injury of the nerve involved in the reflex contraction of pelvic floor muscles, childbirth, ovariectomy, a mechanical vaginal expansion treatment, diabetes, a drug administration or a combination of these.
[44] The method of any of the above-mentioned [41] - [43], wherein the animal is female.

The present invention also relates to the following.
[1] A serotonin 5-HT_{2c} receptor activator comprising a compound represented by the formula

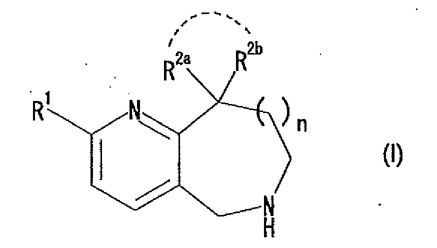

wherein R¹ is
(1) a group represented by the formula

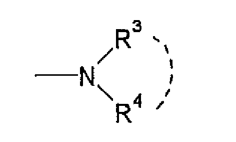

wherein R³ and R⁴ are each a hydrogen atom or an alkyl group optionally substituted by a cycloalkyl group, or R³ and R⁴ optionally form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ (R⁵ is an alkyl group optionally substituted by a cycloalkyl group, an aryl group or a heteroaryl group),
(3) a group represented by -SR⁶ (R⁶ is an alkyl group, an aryl group or a heteroaryl group),
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group or
(7) a heteroaryl group,
R^{2a} and R^{2b} are each a hydrogen atom or an optionally substituted alkyl group, or R^{2a} and R^{2b} optionally form, together with the adjacent carbon atom, an alicyclic hydrocarbon group optionally substituted by substituent(s) selected from a halogen atom, an alkyl group and an alkoxy group, and
n is 0 or 1,
or a salt thereof (hereinafter sometimes to be abbreviated as compound (I)).
[2] The activator of the above-mentioned [1], wherein R¹ is a group represented by the formula

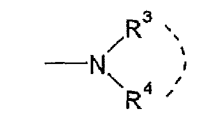

wherein R³ and R⁴ are each a hydrogen atom or an alkyl group optionally substituted by a cycloalkyl group, or R³ and R⁴ optionally form, together with the adjacent nitrogen atom, a non-aromatic cyclic amino group optionally substituted by substituent(s) selected from a halogen atom, an alkyl group, an alkoxy group and an aryl group.
[3] The activator of the above-mentioned [1], wherein n is 0.
[4] The activator of the above-mentioned [1], which is a prophylactic or therapeutic agent for a lower urinary tract symptom, obesity and/or organ prolapse.
[5] A method for the prophylaxis or treatment of a lower urinary tract symptom, obesity and/or organ prolapse, comprising administering, to a mammal, an effective amount of a compound represented by the formula

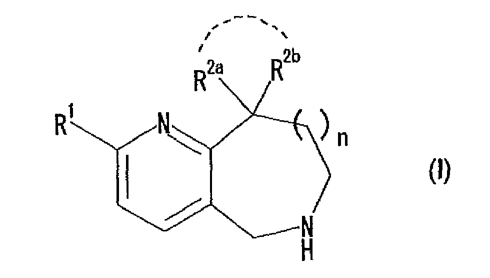

wherein R¹ is
(1) a group represented by the formula

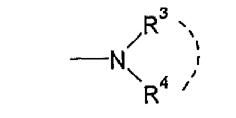

wherein R³ and R⁴ are each a hydrogen atom or an alkyl group optionally substituted by a cycloalkyl group, or R³ and R⁴ optionally form, together with the adjacent nitrogen atom, a non-aromatic cyclic amino group optionally substituted by substituent(s) selected from a halogen atom, an alkyl group, an alkoxy group and an aryl group,

(2) a group represented by -OR⁵ (R⁵ is an alkyl group optionally substituted by a cycloalkyl group, an aryl group or a heteroaryl group),
(3) a group represented by -SR⁶ (R⁶ is an alkyl group, an aryl group or a heteroaryl group),
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are each a hydrogen atom or an optionally substituted alkyl group, or R^{2a} and R^{2b} optionally form, together with the adjacent carbon atom, an alicyclic hydrocarbon group optionally substituted by substituent(s) selected from a halogen atom, an alkyl group and an alkoxy group, and
n is 0 or 1,
or a salt thereof.
[6] Use of a compound represented by the formula

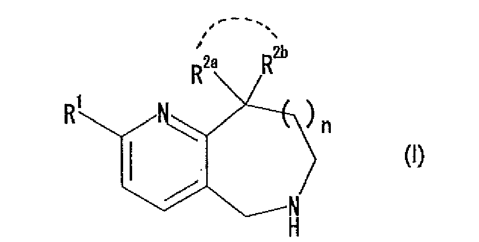

wherein R¹ is
(1) a group represented by the formula

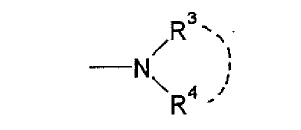

wherein R³ and R⁴ are each a hydrogen atom or an alkyl group optionally substituted by a cycloalkyl group, or R³ and R⁴ optionally form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ (R⁵ is an alkyl group optionally substituted by a cycloalkyl group, an aryl group or a heteroaryl group),
(3) a group represented by -SR⁶ (R⁶ is an alkyl group, an aryl group or a heteroaryl group),
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are each a hydrogen atom or an optionally substituted alkyl group, or R^{2a} and R^{2b} optionally form, together with the adjacent carbon atom, an alicyclic hydrocarbon group optionally substituted by substituent(s) selected from a halogen atom, an alkyl group and an alkoxy group, and
n is 0 or 1,
or a salt thereof for the production of a prophylactic or therapeutic agent for a lower urinary tract symptom, obesity and/or organ prolapse.
[7] A compound represented by the formula

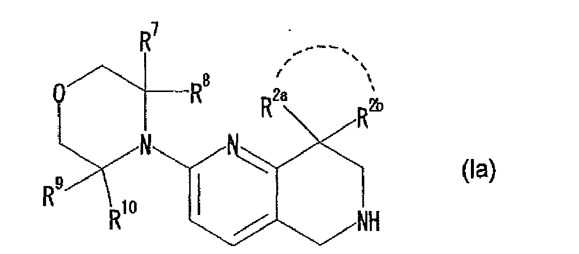

wherein R^{2a} and R^{2b} are each a hydrogen atom or an optionally substituted alkyl group, or R^{2a} and R^{2b} optionally form, together with the adjacent carbon atom, an alicyclic hydrocarbon group optionally substituted by substituent(s) selected from a halogen atom, an alkyl group and an alkoxy group,
R⁷, R⁸, R⁹ and R¹⁰ are each a hydrogen atom or a substituent, provided that (1) one or each of R^{2a} and R^{2b} is an optionally substituted alkyl group, or (2) R⁷, R⁸, R⁹ and R¹⁰ are each a hydrogen atom or a substituent, and R^{2a} and R^{2b} form, together with the adjacent carbon atom, an alicyclic hydrocarbon group optionally substituted by substituent(s) selected from a halogen atom, an alkoxy group and an alkyl group, or (3) at least one group of R⁷ , R⁸, R⁹ and R¹⁰ is a substituent,
or a salt thereof (hereinafter sometimes to be abbreviated as compound (Ia)).
[8] The compound of the above-mentioned [7], wherein at least one group of R^{2a}, R^{2b}, R⁷, R⁸, R⁹ and R¹⁰ is an optionally substituted alkyl group.
[9] A compound represented by the formula

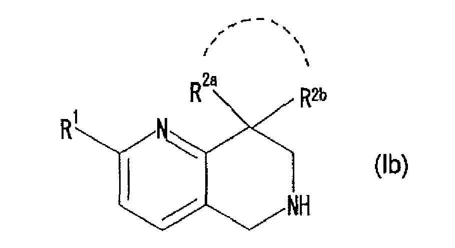

wherein R¹ is a group represented by the formula

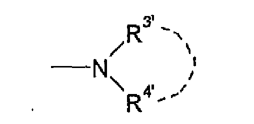

wherein R^{3'} is a hydrogen atom or an alkyl group optionally substituted by a cycloalkyl group, R^{4'} is an alkyl group optionally substituted by a cycloalkyl group, or R^{3'} and R^{4'} are optionally crosslinked to each other via a carbon-carbon bond to form, together with the adjacent nitrogen atom, a non-aromatic cyclic amino group optionally substituted by substituent(s) selected from a halogen atom, an alkyl group, an alkoxy group and an aryl group, excluding a methylamino group, and
R^{2a} and R^{2b} are each a hydrogen atom or an optionally substituted alkyl group, or R^{2a} and R^{2b} optionally form, together with the adjacent carbon atom, an alicyclic hydrocarbon group optionally substituted by substituent(s) selected from a halogen atom, an alkyl group and an alkoxy group,
or a salt thereof (hereinafter sometimes to be abbreviated as compound (Ib)).
[10] A compound represented by the formula

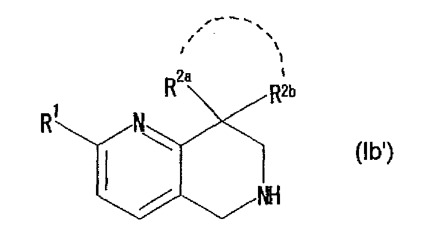

wherein R¹ is
(1) a group represented by -OR⁵ (R⁵ is an alkyl group optionally substituted by a cycloalkyl group, an aryl group or a heteroaryl group),
(2) a C₃ alkyl group (alkyl group having a carbon number of 3),
(3) an aryl group optionally substituted by substituent(s) selected from a halogen atom, an alkyl group optionally substituted by a halogen atom, an alkoxy group, an alkylthio group and an aryl group, or
(4) a heteroaryl group,
   R^{2a} and R^{2b} are each a hydrogen atom or an optionally substituted alkyl group, or R^{2a} and R^{2b} optionally form, together with the adjacent carbon atom, an alicyclic hydrocarbon group optionally substituted by substituent(s) selected from a halogen atom, an alkyl group and an alkoxy group (excluding a compound wherein R¹ is a methoxy group and
   R^{2a} and R^{2b} are each a hydrogen atom),
   or a salt thereof (hereinafter sometimes to be abbreviated as compound (Ib')).
[11] A compound represented by the formula

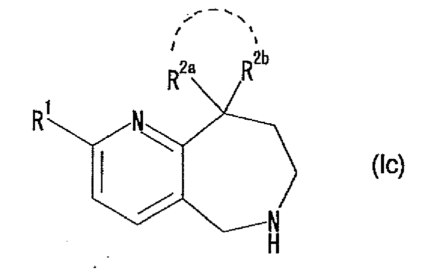

wherein R¹ is
(1) a group represented by the formula

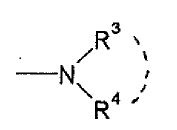

wherein R³ and R⁴ are each a hydrogen atom or an alkyl group, or R³ and R⁴ optionally form, together with the adjacent nitrogen atom, a non-aromatic cyclic amino group optionally substituted by substituent(s) selected from a halogen atom, an alkyl group, an alkoxy group and an aryl group,

(2) a group represented by -OR⁵ (R⁵ is an alkyl group, an aryl group or a heteroaryl group),
(3) a group represented by -SR⁶ (R⁶ is an alkyl group, an aryl group or a heteroaryl group),
(4) an alkyl group,
(5) a cycloalkyl group,
(6) an aryl group optionally substituted by substituent(s) selected from a halogen atom, an alkyl group, an alkoxy group, an alkylthio group and an aryl group, or
(7) a heteroaryl group,
R^{2a} and R^{2b} are each a hydrogen atom or an optionally substituted alkyl group, or R^{2a} and R^{2b} optionally form, together with the adjacent carbon atom, an alicyclic hydrocarbon group optionally substituted by substituent(s) selected from a halogen atom, an alkyl group and an alkoxy group,
or a salt thereof (hereinafter sometimes to be abbreviated as compound (Ic)).
[12] A prodrug of the compound of any of the above-mentioned [7]-[11]. [13] A medicament comprising the compound of any of the above-mentioned [7]-[11] or a salt thereof or a prodrug thereof.

[14] A method of screening for a substance that increases a leak point pressure upon a rise in the intravesical pressure, which comprises measuring a leak point pressure when an intravesical pressure is increased by injection of saline rapidly and transiently into the bladder of an animal showing no micturition reflex but showing a functional bladder-spinal cord-urethra reflex.
[15] A method of screening for a prophylactic or therapeutic drug for stress urinary incontinence, which comprises measuring a leak point pressure when an intravesical pressure is increased by injection of saline rapidly and transiently into the bladder of an animal showing no micturition reflex but showing a functional bladder-spinal cord-urethra reflex.
[16] The method of the above-mentioned [14] or [15], wherein the animal used shows a decreased leak point pressure upon a rise in the intravesical pressure.
[17] The method of the above-mentioned [16], wherein the decreased leak point pressure upon a rise in the intravesical pressure is based on the transection or injury of the nerve involved in the reflex contraction of pelvic floor muscles or external urethral sphincter, childbirth, ovariectomy, a mechanical vaginal expansion treatment, diabetes, a drug administration or a combination of these.
[18] The method of the above-mentioned [14] or [15], wherein the animal is female.
[19] A drug for the prophylaxis or treatment of cystoceles or enteroceles, comprising a serotonin 5-HT_{2C} receptor activator.
[20] A method for the prophylaxis or treatment of cystoceles or enteroceles, comprising administering an effective amount of a serotonin 5-HT_{2C} receptor activator to a mammal.
[21] Use of a serotonin 5-HT_{2C} receptor activator for the production of an agent for the prophylaxis or treatment of cystoceles or enteroceles.
[22] A method of screening for a drug for the prophylaxis or treatment of cystoceles or enteroceles, comprising increasing an intravesical pressure after bilateral transection of the hypogastric nerve and pudendal nerve of an animal, and measuring a closure response in the urethra, the rectum or the vagina observed at that time.
[23] A method of screening for a drug for the prophylaxis or treatment of cystoceles or enteroceles, comprising increasing an intravesical pressure after bilateral transection of the hypogastric nerve and pudendal nerve of an animal, and measuring the leak point pressure at that time.
[24] The method of the above-mentioned [22] or [23], wherein the closure response in the urethra, the rectum or the vagina or urethral resistance (decreased leak point pressure) upon increase in the intravesical pressure after bilateral transection of the hypogastric nerve and pudendal nerve of an animal is based on the unilateral transection or injury of the nerve involved in the reflex contraction of pelvic floor muscles, childbirth, ovariectomy, a mechanical vaginal expansion treatment, diabetes, a drug administration or a combination of these.
[25] The method of any of the above-mentioned [22] - [24], wherein the animal is female.

### Effect of the Invention

Since the compound of the present invention or a prodrug thereof has a superior serotonin 5-HT_{2C} receptor activation action, it is useful as a safe prophylactic or therapeutic drug for any serotonin 5-HT_{2C} receptor associated disease, for example, a lower urinary tract symptom, obesity and/or organ prolapse and the like.
In the screening method for a prophylactic or therapeutic drug for stress urinary incontinence of the present invention, since the urethral resistance upon a rapid and transient rise in the intravesical pressure is measured as a leak point pressure conveniently by a stable procedure, it is superior as an in vivo evaluation system, and can be applied beneficially and efficiently to screening for a substance used for the prophylaxis or treatment of stress urinary incontinence. On the other hand, it is also useful as an evaluation system to verify that a substance used for the prophylaxis or treatment of other diseases does not induce stress urinary incontinence. Moreover, using the screening method of the present invention, various pathological and physiological studies aiming at elucidation of the pathology mechanism of stress urinary incontinence, such as identification of a gene with expression that varies along with pathology and elucidation of behavior thereof, analysis of protein expression variation, study of treatment effect of gene introduction on stress urinary incontinence and the like, can be efficiently conducted with high precision.
Moreover, a substance usable for the screening method of the present invention, for example, a substance that increases leak point pressure upon a rise in the intravesical pressure can be used as a prophylactic or therapeutic agent for stress urinary incontinence.
In the screening method for a prophylactic or therapeutic drug for cystoceles or enteroceles of the present invention, since contraction force of pelvic floor muscles is measured, the method is superior as an in vivo evaluation system for pathology, and can be applied beneficially and efficiently to screening for a substance used for the prophylaxis or treatment of cystoceles or enteroceles. On the other hand, it is also useful as an evaluation system to verify that a substance used for the prophylaxis or treatment of other diseases does not induce cystoceles and enteroceles. Moreover, using the screening method of the present invention, various pathological and physiological studies aiming at elucidation of the pathology mechanism of cystoceles and enteroceles, such as identification of a gene with expression that varies along with pathology and elucidation of behavior thereof, analysis of protein expression variation, study of treatment effect of gene introduction on cystoceles and enteroceles and the like, can be efficiently conducted with high precision. Furthermore, a substance obtained by the screening method of the present invention, for example, a medicament comprising a serotonin 5-HT_{2C} receptor activator can be used as a prophylactic or therapeutic drug for cystoceles or enteroceles.

### Brief Description of the Drawings

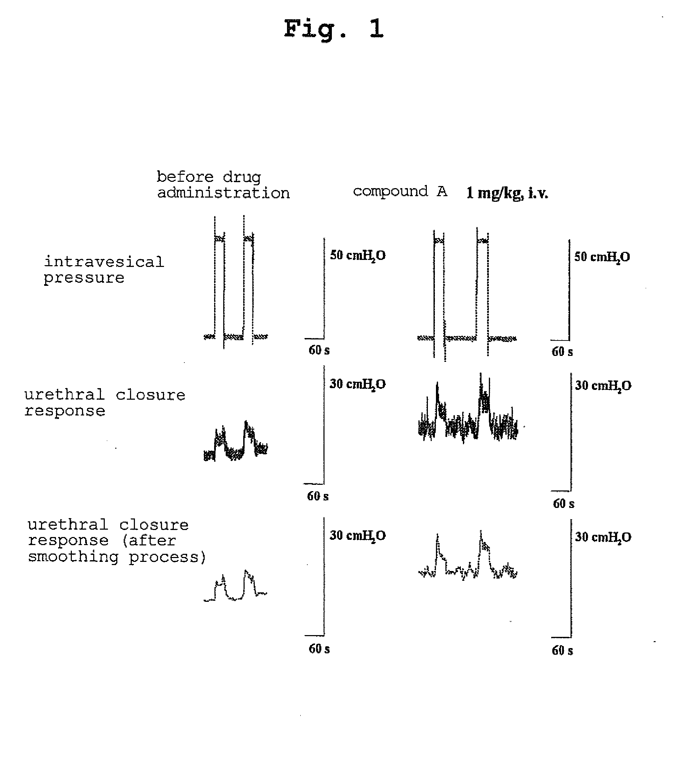

Fig. 1 shows a typical example of the action of a 5-HT_{2C} receptor agonist, 8-morpholin-4-yl-2,3,4,5-tetrahydropyrido[3,2-f][1,4]oxazepine (to be referred to as compound A) on a urethral closure response induced by an increased intravesical pressure after bilateral transection of the hypogastric nerve and pudendal nerve of a female rat under urethane anesthesia.

### (Detailed Description of the Invention)

The present invention is explained in detail in the following.
In the present specification, examples of the "halogen atom" include a fluorine atom, a chlorine atom, a bromine atom and an iodine atom.

In the present specification, examples of the "alkyl group" include a C₁₋₆ alkyl group (e.g., methyl, ethyl, n-propyl, isopropyl, n-butyl, isobutyl, sec-butyl, tert-butyl, pentyl, hexyl etc.).
In the present specification, examples of the "C₁₋₆ alkyl group" include an alkyl group having a carbon number of 1 to 6 (e.g., methyl, ethyl, n-propyl, isopropyl, n-butyl, isobutyl, sec-butyl, tert-butyl, pentyl, hexyl etc.).
In the present specification, examples of the "C₃₋₅ alkyl group" include an alkyl group having a carbon number of 3 to 5 (e.g., n-propyl, isopropyl, n-butyl, isobutyl, sec-butyl, tert-butyl, pentyl etc.).
In the present specification, moreover, examples of the "C₃ alkyl group" include an alkyl group having a carbon number of 3, and n-propyl or isopropyl can be mentioned.

In the present specification, examples of the "alkyl group optionally substituted by a cycloalkyl group" include the above-mentioned "alkyl group" optionally substituted by 1 to 4, preferably 1 to 3, C₃₋₆ cycloalkyl groups (e.g., cyclopropyl, cyclobutyl, cyclopentyl, cyclohexyl).

In the present specification, examples of the "alkyl group optionally substituted by a halogen atom" include the above-mentioned "alkyl group" optionally substituted by 1 to 4, preferably 1 to 3, halogen atoms (e.g., fluorine atom, chlorine atom, bromine atom, iodine atom).

In the present specification, examples of the "C₁₋₆ alkyl group optionally substituted by a halogen atom" include the above-mentioned "C₁₋₆ alkyl group" optionally substituted by 1 to 4, preferably 1 to 3, halogen atoms (e.g., fluorine atom, chlorine atom, bromine atom, iodine atom).

In the present specification, examples of the "C₁₋₆ alkyl group substituted by a halogen atom" include the above-mentioned "C₁₋₆ alkyl group" substituted by 1 to 4, preferably 1 to 3, halogen atoms (e.g., fluorine atom, chlorine atom, bromine atom, iodine atom).

In the present specification, examples of the optionally substituted alkyl group" include the above-mentioned "alkyl group" optionally having substituents selected from the following, wherein the number of the substituents is 1 to 4, preferably 1 to 3.
(i) a halogen atom (e.g., fluorine atom, chlorine atom, bromine atom, iodine atom),
(ii) a cyano group,
(iii) a hydroxy group,
(iv) a nitro group,
(v) a formyl group,
(vi) an amino group,
(vii) a mono- or di-C₁₋₆ alkylamino group (e.g., methylamino, ethylamino, propylamino, dimethylamino, diethylamino, dipropylamino etc.),
(viii) a C₁₋₆ alkyl-carbonylamino group (e.g., acetylamino, ethylcarbonylamino etc.),
(ix) a C₁₋₆ alkoxy-carbonylamino group (e.g., methoxycarbonylamino, ethoxycarbonylamino, propoxycarbonylamino etc.),
(x) a C₃₋₈ cycloalkyl group (e.g., cyclopropyl, cyclobutyl, cyclopentyl, cyclohexyl etc.),
(xi) a C₆₋₁₂ aryl group (e.g., phenyl, 1-naphthyl, 2-naphthyl etc.) optionally substituted by substituent(s) selected from a halogen atom (e.g., fluorine atom, chlorine atom, bromine atom, iodine atom) and a C₁₋₆ alkoxy group (e.g., methoxy, ethoxy, propoxy etc.),
(xii) a C₁₋₆ alkoxy group (e.g., methoxy, ethoxy, propoxy, isopropoxy, butoxy, isobutoxy, sec-butoxy, tert-butoxy etc.) optionally substituted by a halogen atom (e.g., fluorine atom, chlorine atom, bromine atom, iodine atom),
(xiii) a C₇₋₁₂ aralkyloxy group (e.g., benzyloxy etc.),
(xiv) a C₆₋₁₂ aryloxy group (e.g., phenoxy etc.),
(xv) a carboxyl group,
(xvi) a C₁₋₆ alkoxy-carbonyl group (e.g., methoxycarbonyl, ethoxycarbonyl, propoxycarbonyl, isopropoxycarbonyl, tert-butoxycarbonyl etc.),
(xvii) a C₇₋₁₂ aralkyloxy-carbonyl group (e.g., benzyloxycarbonyl etc.),
(xviii) a C₆₋₁₂ aryloxy-carbonyl group (e.g., phenyloxycarbonyl etc.),
(xix) a C₁₋₆ alkyl-carbonyl group (e.g., acetyl, ethylcarbonyl, n-propylcarbonyl, isopropylcarbonyl, 2,2-dimethylpropylcarbonyl etc.),
(xx) a C₃₋₈ cycloalkyl-carbonyl group (e.g., cyclopropylcarbonyl, cyclobutylcarbonyl, cyclopentylcarbonyl, cyclohexylcarbonyl etc.),
(xxi) a C₇₋₁₂ aralkyl-carbonyl group (e.g., benzylcarbonyl etc.), (xxii) a carbamoyl group,
(xxiii) a thiocarbamoyl group,
(xxiv) a mono-or di-C₁₋₆ alkyl-carbamoyl group (e.g., methylcarbamoyl, ethylcarbamoyl, n-propylcarbamoyl, isopropylcarbamoyl, dimethylcarbamoyl, diethylcarbamoyl, dipropylcarbamoyl etc.),
(xxv) a mono-or di-C₇₋₁₂ aralkyl-carbamoyl group (e.g., benzylcarbamoyl, dibenzylcarbamoyl etc.),
(xxvi) a thiol group,
(xxvii) a C₁₋₆ alkylthio group (e.g., methylthio, ethylthio, propylthio etc.),
(xxviii) a C₇₋₁₂ aralkylthio group (e.g., benzylthio etc.), (xxix) a C₁₋₆ alkylsulfonyl group (e.g., methylsulfonyl, ethylsulfonyl, n-propylsulfonyl, isopropylsulfonyl etc.),
(xxx) a C₃₋₈ cycloalkylsulfonyl group (e.g., cyclopropylsulfonyl, cyclobutylsulfonyl, cyclopentylsulfonyl etc.),
(xxxi) a C₆₋₁₂ arylsulfonyl group (e.g., phenylsulfonyl, 1-naphthylsulfonyl, 2-naphthylsulfonyl etc.),
(xxxii) a C₇₋₁₂ aralkylsulfonyl group (e.g., benzylsulfonyl etc.),
(xxxiii) a 5- to 8-membered non-aromatic heterocyclic group containing, besides carbon atom, 1 to 4 hetero atoms selected from nitrogen atom, sulfur atom and oxygen atom (for example, pyrrolidinyl, tetrahydrofuryl, tetrahydrothienyl, piperidinyl, tetrahydropyranyl, morpholinyl, thiomorpholinyl, piperazinyl, azepanyl, oxazepanyl etc.),
(xxxiv) a 5- to 8-membered aromatic heterocyclic group containing, besides carbon atom, 1 to 4 hetero atoms selected from nitrogen atom, sulfur atom and oxygen atom (for example, furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.),
(xxxv) a 5- to 8-membered non-aromatic heterocyclyl containing, besides carbon atom, 1 to 4 hetero atoms selected from nitrogen atom, sulfur atom and oxygen atom-carbonyl group (e.g., pyrrolidinylcarbonyl, tetrahydrofurylcarbonyl, tetrahydrothienylcarbonyl, piperidinylcarbonyl, tetrahydropyranylcarbonyl, morpholinylcarbonyl, thiomorpholinylcarbonyl, piperazinylcarbonyl etc.),
(xxxvi) a 5- to 8-membered aromatic heterocyclyl containing, besides carbon atom, 1 to 4 hetero atoms selected from nitrogen atom, sulfur atom and oxygen atom-carbonyl group (e.g., furylcarbonyl, thienylcarbonyl, pyrrolylcarbonyl, oxazolylcarbonyl, isooxazolylcarbonyl, thiazolylcarbonyl, isothiazolylcarbonyl, imidazolylcarbonyl, pyrazolylcarbonyl, 1,2,3-oxadiazolylcarbonyl, 1,2,4-oxadiazolylcarbonyl, 1,3,4-oxadiazolylcarbonyl, furazanylcarbonyl, 1,2,3-thiadiazolylcarbonyl, 1,2,4-thiadiazolylcaxbonyl, 1,3,4-thiadiazolylcarbonyl, 1,2,3-triazolylcarbonyl, 1,2,4-triazolylcarbonyl, tetrazolylcarbonyl, pyridylcarbonyl, pyridazinylcarbonyl, pyrimidinylcarbonyl, pyrazinylcarbonyl, triazinylcarbonyl etc.),
(xxxvii) a ureido group,
(xxxviii) a C₁₋₆ alkylureido group (e.g., methylureido, ethylureido, propylureido etc.),
(xxxix) a C₆₋₁₂ arylureido group (e.g., phenylureido, 1-naphthylureido, 2-naphthylureido etc.) and
(xxxx) a C₁₋₄ alkylenedioxy group (e.g., methylenedioxy, ethylenedioxy, propylenedioxy etc.)

In the present specification, examples of the "cycloalkyl group" include a C₃₋₆ cycloalkyl group (e.g., cyclopropyl, cyclobutyl, cyclopentyl, cyclohexyl).
In the present specification, examples of the "C₃₋₆ cycloalkyl group" include a cycloalkyl group having a carbon number of 3 to 6 (e.g., cyclopropyl, cyclobutyl, cyclopentyl, cyclohexyl).

In the present specification, examples of the "C₁₋₆ alkyl group optionally substituted by a C₃₋₆ cycloalkyl group" include the above-mentioned "C₁₋₆ alkyl group" optionally substituted by 1 to 4, preferably 1 to 3, of the above-mentioned "C₃₋₆ cycloalkyl group".

In the present specification, examples of the "alkoxy group" include a C₁₋₆ alkoxy group (e.g., methoxy, ethoxy, n-propoxy, isopropoxy, n-butoxy, isobutoxy, sec-butoxy, tert-butoxy, pentoxy, hexoxy etc.).

In the present specification, examples of the "alkylthio group" include a C₁₋₆ alkylthio group (e.g., methylthio, ethylthio, n-propylthio, isopropylthio, n-butylthio, isobutylthio, sec-butylthio, tert-butylthio, pentylthio, hexylthio etc.).

In the present specification, examples of the "C₁₋₆ alkylthio group" include an alkylthio group wherein the alkyl moiety is the above-mentioned "C₁₋₆ alkyl" (e.g., methylthio, ethylthio, n-propylthio, isopropylthio, n-butylthio, isobutylthio, sec-butylthio, tert-butylthio, pentylthio, hexylthio etc.).

In the present specification, examples of the "aryl group" include a C₆₋₁₄ aryl group (e.g., phenyl, naphthyl etc.).

In the present specification, examples of the "aryl group optionally substituted by substituent(s) selected from halogen atom, an alkyl group optionally substituted by a halogen atom, an alkoxy group, an alkylthio group and an aryl group" for R¹ include the above-mentioned "aryl group" optionally substituted by 1 to 4, preferably 1 to 3, substituents selected from the above-mentioned "halogen atom", the above-mentioned "alkyl group optionally substituted by a halogen atom", the above-mentioned "alkoxy group", the above-mentioned "alkylthio group" and the above-mentioned "aryl group".

In the present specification, examples of the "heteroaryl group" include a 5- to 8-membered heteroaryl group containing, besides carbon atom, 1 to 4 hetero atoms selected from nitrogen atom, sulfur atom and oxygen atom (for example, furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl etc.).

In the present specification, examples of the "non-aromatic cyclic amino group" include a 5- to 8-membered non-aromatic cyclic amino group containing, besides carbon atom and nitrogen atom, 1 to 4 hetero atoms selected from sulfur atom and oxygen atom (for example, pyrrolidinyl, piperidino, morpholino, thiomorpholino, piperazinyl, azepanyl, 1,4-diazepanyl, oxazepanyl(e.g., 1,4-oxazepanyl) etc.).

In the present specification, examples of the "non-aromatic cyclic amino group optionally substituted by substituent(s) selected from halogen atom, an alkyl group, an alkoxy group and an aryl group" optionally formed by R³ and R⁴ together with the adjacent nitrogen atom include the above-mentioned "non-aromatic cyclic amino group" optionally substituted by 1 to 4, preferably 1 to 3, substituents selected from the above-mentioned "halogen atom", the above-mentioned "alkyl group", the above-mentioned "alkoxy group" and the above-mentioned "aryl group".

In the present specification, examples of the "non-aromatic cyclic amino group optionally substituted by substituent(s) selected from halogen atom, an alkyl group, an alkoxy group and an aryl group" optionally formed, together with the adjacent nitrogen atom, by R^{3'} and R^{4'} which are crosslinked to each other via a carbon-carbon bond include a non-aromatic cyclic amino group formed only by one nitrogen atom and two or more (for example, 4 to 7, preferably 4 or 5) carbon atoms, which is optionally substituted by 1 to 4, preferably 1 to 3, substituents selected from the above-mentioned "halogen atom", the above-mentioned "alkyl group", the above-mentioned "alkoxy group" and the above-mentioned "aryl group" (for example, pyrrolidinyl, piperidino, azepanyl etc.).

In the present specification, examples of the "alicyclic hydrocarbon group optionally substituted by substituent(s) selected from a halogen atom, an alkyl group and an alkoxy group" optionally formed by R^{2a} and R^{2b} together with the adjacent carbon atom include C₃₋₁₄ cycloalkyl group (preferably C₃₋₈ cycloalkyl group) such as cyclopropyl, cyclobutyl, cyclopentyl, cyclohexyl, perhydronaphthyl, perhydroanthranyl, bicyclo[2,2,1]heptyl and the like, C₃₋₁₄ cycloalkenyl group (preferably C₃₋₈ cycloalkenyl group) such as cyclopropenyl, cyclobuten-1-or 3-yl, cyclopenten-1-, 3- or 4-yl, cyclohexen-1-or 3-yl and the like, and the like, each of which is optionally substituted by 1 to 4, preferably 1 to 3 substituents selected from the above-mentioned "halogen atom", the above-mentioned "alkyl group" and the above-mentioned "alkoxy group".

In the present specification, examples of the "substituent" for R⁷, R⁸, R⁹ or R¹⁰ include the above-mentioned "optionally substituted alkyl group" and the substituents that the alkyl group of the above-mentioned "optionally substituted alkyl group" optionally has.

Compounds (Ix), (I), (Ia'), (Ia), (Ia'-1), (Ib"), (Ib"-1), (Ib), (Ib'), (Ib-1), (Ic) and (Ic') are explained in detail in the following.
1. Compound (Ix):
   X is a hydrogen atom or a halogen atom.
   X is preferably a hydrogen atom, a fluorine atom, a chlorine atom or a bromine atom.
      R¹ is
   (1) a group represented by the formula

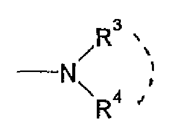

wherein R³ and R⁴ are the same or different and each is a hydrogen atom, a cycloalkyl group or an alkyl group optionally substituted by a cycloalkyl group, or R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ (R⁵ is a hydrogen atom, a cycloalkyl group, an alkyl group optionally substituted by a cycloalkyl group, an aryl group or a heteroaryl group),
(3) a group represented by -SR⁶ (R⁶ is an alkyl group, an aryl group or a heteroaryl group),
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group.

R¹ is preferably
(1) a group represented by the formula

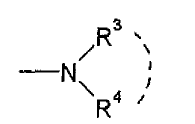

wherein R³ and R⁴ are the same or different and each is a hydrogen atom, a cycloalkyl group (particularly, a cyclopentyl group) or an alkyl group (particularly, methyl group, ethyl group, an n-propyl group, an isopropyl group, an isobutyl group, a sec-butyl group) optionally substituted by a cycloalkyl group (particularly, a cyclopropyl group), or R³ and R⁴ form, together with the adjacent nitrogen atom, a non-aromatic cyclic amino group (particularly, piperidino group, pyrrolidinyl group, morpholino group) optionally substituted by substituent(s) selected from a halogen atom, an alkyl group, an alkoxy group and an aryl group (particularly, an alkyl group (e.g., methyl group, ethyl group, an n-propyl group)), (2) a group represented by -OR⁵ (R⁵ is a hydrogen atom, a cycloalkyl group (particularly, a cyclobutyl group), an alkyl group (particularly, ethyl group, an isopropyl group, a sec-butyl group, tert-butyl group), an aryl group or a heteroaryl group) optionally substituted by a cycloalkyl group (particularly, a cyclopropyl group, a cyclopentyl group, a cyclohexyl group),
(4) an alkyl group (particularly, an n-propyl group, an isopropyl group, an isobutyl group, a sec-butyl group, a 1-ethylpropyl group),
(5) a cycloalkyl group (particularly, a cyclopropyl group, a cyclobutyl group, a cyclopentyl group),
(6) an aryl group (particularly, a phenyl group) optionally substituted by substituent(s) selected from a halogen atom (particularly, a fluorine atom), alkyl group (particularly, methyl group) optionally substituted by a halogen atom (particularly, a fluorine atom), an alkoxy group, an alkylthio group (particularly, a methylthio group) and an aryl group,
and
(7) a heteroaryl group (particularly, a furyl group), more preferably,
(1) a group represented by the formula

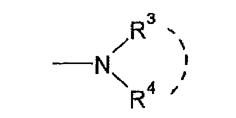

wherein R³ and R⁹ are the same or different and each is a hydrogen atom, a cycloalkyl group (particularly, a cyclopentyl group), or an alkyl group (particularly, a methyl group, an ethyl group, an n-propyl group, an isopropyl group, an isobutyl group, a sec-butyl group) optionally substituted by a cycloalkyl group (particularly, a cyclopropyl group), or R³ and R⁴ form, together with the adjacent nitrogen atom, a non-aromatic cyclic amino group (particularly, piperidino group, pyrrolidinyl group, morpholino group) optionally substituted by substituent(s) selected from a halogen atom, an alkyl group, an alkoxy group and an aryl group (particularly, an alkyl group (e.g., methyl group, ethyl group, an n-propyl group)).

Specific examples of R¹ include isopropylamino group, isobutylamino group, cyclopropylmethylamino group, dimethylamino group, diethylamino group, methyl(ethyl)amino group, methyl(n-prapyl)amino group, methyl(isopropyl)amino group, methyl(isobutyl)amino group, methyl(sec-butyl)amino group, methyl(cyclopropylmethyl)amino group, methyl(cyclopentyl)amino group, hydroxy group, isopropoxy group, sec-butoxy group, tert-butoxy group, 1-cyclopropylethoxy group, 1-cyclopentylethoxy group, 1-cyclohexylethoxy group, cyclobutyloxy group, an n-propyl group, an isopropyl group, an isobutyl group, a sec-butyl group, a 1-ethylpropyl group, pyrrolidinyl group, 2,5-dimethylpyrrolidinyl group, piperidino group, morpholino group, 2-methylmorpholino group, 2-ethylmorpholino group, 2-n-propylmorpholino group, 4-fluorophenyl group, 4-trifluoromethylphenyl group, 4-methylthiophenyl group, 3-furyl group, a cyclopropyl group, a cyclobutyl group, a cyclopentyl group and the like.

R^{2a} and R^{2b} are the same or different and each is a hydrogen atom or an optionally substituted alkyl group, or R^{2a} and R^{2b} form, together with the adjacent carbon atom, an alicyclic hydrocarbon group optionally substituted by substituent(s) selected from a halogen atom, an alkyl group and an alkoxy group.
R^{2a} and R^{2b} are the same or different and each is preferably a hydrogen atom or an alkyl group (particularly, methyl group, ethyl group).

n is 0 or 1. n is preferably 0.
As compound (Ix), the compounds of Examples 1 - 80 and the like are preferable.

2. Compound (I):
R¹ is
   (1) a group represented by the formula

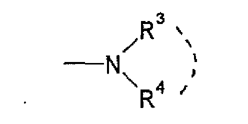

wherein R³ and R⁹ are the same or different and each is a hydrogen atom or an alkyl group optionally substituted by a cycloalkyl group, or R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ (R⁵ is an alkyl group optionally substituted by a cycloalkyl group, an aryl group or a heteroaryl group),
(3) a group represented by -SR⁶ (R⁶ is an alkyl group, an aryl group or a heteroaryl group),
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group or
(7) a heteroaryl group.

R¹ is preferably
(1) a group represented by the formula

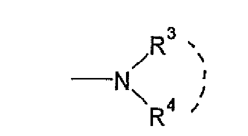

wherein R³ and R⁴ are the same or different and each is a hydrogen atom or an alkyl group (particularly, a methyl group, an ethyl group, an n-propyl group, an isopropyl group, an isobutyl group, a sec-butyl group) optionally substituted by a cycloalkyl group (particularly, a cyclopropyl group), or R³ and R⁴ form, together with the adjacent nitrogen atom, a non-aromatic cyclic amino group (particularly, piperidino group, pyrrolidinyl group, morpholino group) optionally substituted by substituent(s) selected from a halogen atom, an alkyl group, an alkoxy group and an aryl group (particularly, an alkyl group (e.g., methyl group, ethyl group, an n-propyl group)),

(2) a group represented by -OR⁵ (R⁵ is an alkyl group (particularly, ethyl group, an isopropyl group, a sec-butyl group, tert-butyl group) optionally substituted by a cycloalkyl group (particularly, a cyclopropyl group, a cyclopentyl group, a cyclohexyl group), an aryl group or a heteroaryl group),
(4) an alkyl group (particularly, an n-propyl group, an isopropyl group, an isobutyl group, a sec-butyl group, a 1-ethylpropyl group),
(5) a cycloalkyl group (particularly, a cyclopropyl group, a cyclobutyl group, a cyclopentyl group),
(6) an aryl group (particularly, a phenyl group) optionally substituted by substituent(s) selected from a halogen atom (particularly, a fluorine atom), an alkyl group (particularly, methyl group) optionally substituted by a halogen atom (particularly, a fluorine atom), an alkoxy group, an alkylthio group (particularly, a methylthio group) and an aryl group or
(7) a heteroaryl group (particularly, a furyl group), more preferably,
(1) a group represented by the formula

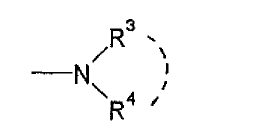

wherein R³ and R⁴ are the same or different and each is a hydrogen atom or an alkyl group (particularly, a methyl group, an ethyl group, an n-propyl group, an isopropyl group, an isobutyl group, a sec-butyl group) optionally substituted by a cycloalkyl group (particularly, a cyclopropyl group), or R³ and R⁴ form, together with the adjacent nitrogen atom, a non-aromatic cyclic amino group (particularly, piperidino group, pyrrolidinyl group, morpholino group) optionally substituted by substituent(s) selected from a halogen atom, an alkyl group, an alkoxy group and an aryl group (particularly, an alkyl group (e.g., methyl group, ethyl group, an n-propyl group)).

Specific examples of R¹ include isopropylamino group, isobutylamino group, cyclopropylmethylamino group, dimethylamino group, diethylamino group, methyl(ethyl)amino group, methyl(n-propyl)amino group, methyl(isopropyl)amino group, methyl(isobutyl)amino group, methyl(sec-butyl)amino group, methyl(cyclopropylmethyl)amino group, isopropoxy group, 1-cyclopropylethoxy group, 1-cyclopentylethoxy group, 1-cyclohexylethoxy group, sec-butoxy group, tert-butoxy group, an n-propyl group, an isopropyl group, an isobutyl group, a sec-butyl group, a 1-ethylpropyl group, a cyclopropyl group, a cyclobutyl group, pyrrolidinyl group, 2,5-dimethylpyrrolidinyl group, piperidino group, morpholino group, 2-methylmorpholino group, 2-ethylmorpholino group, 2-n-propylmorpholino group, 4-fluorophenyl group, 4-trifluoromethylphenyl group, 4-methylthiophenyl group, 3-furyl group and the like.

R^{2a} and R^{2b} are the same or different and each is a hydrogen atom or an optionally substituted alkyl group, or R^{2a} and R^{2b} form, together with the adjacent carbon atom, an alicyclic hydrocarbon group optionally substituted by substituent(s) selected from a halogen atom, an alkyl group and an alkoxy group.
R^{2a} and R^{2b} are the same or different and each is preferably a hydrogen atom or an alkyl group (particularly, methyl group, ethyl group).

n is 0 or 1. n is preferably 0.
As compound (1), the compounds of Examples 1 - 31, 38 - 51, 53 - 59, 61 and 63 - 80 and the like are preferable.

3. Compound (Ia'):
X is a hydrogen atom or a halogen atom.
X is preferably a hydrogen atom, a fluorine atom, a chlorine atom or a bromine atom.

R^{2a} and R^{2b} are the same or different and each is a hydrogen atom or an optionally substituted alkyl group, or R^{2a} and R^{2b} form, together with the adjacent carbon atom, an alicyclic hydrocarbon group optionally substituted by substituent(s) selected from a halogen atom, an alkyl group and an alkoxy group.
R^{2a} and R^{2b} are the same or different and each is preferably a hydrogen atom, a methyl group or an ethyl group.
R⁷, R⁸, R⁹ and R¹⁰ are the same or different and each is a hydrogen atom or a substituent.
R⁷, R⁸, R⁹ and R¹⁰ are the same or different and each is preferably a hydrogen atom or an optionally substituted alkyl group (particularly, methyl group, ethyl group and n-propyl group).

Note that (1) one or each of R^{2a} and R^{2b} is an optionally substituted alkyl group, or (2) R⁷, R⁸, R⁹ and R¹⁰ are the same or different and each is a hydrogen atom or a substituent, and R^{2a} and R^{2b} form, together with the adjacent carbon atom, an alicyclic hydrocarbon group optionally substituted by substituent(s) selected from a halogen atom, an alkyl group and an alkoxy group, or (3) at least one group of R⁷, R⁸, R⁹ and R¹⁰ is a substituent.
At least one group of R^{2a}, R^{2b}, R⁷, R⁸, R⁹ and R¹⁰ is preferably an optionally substituted alkyl group (particularly, a methyl group, an ethyl group or an n-propyl group).
As compound (Ia'), the compounds of Examples 3, 5, 7, 8, 9, 35 - 37, 48 - 50, 65, 66 and 73 - 78 and the like are preferable.

4. Compound (Ia):
R^{2a} and R^{2b} are the same or different and each is a hydrogen atom or an optionally substituted alkyl group, or R^{2a} and R^{2b} form, together with the adjacent carbon atom, an alicyclic hydrocarbon group optionally substituted by substituent(s) selected from a halogen atom, an alkyl group and an alkoxy group.
R^{2a} and R^{2b} are the same or different and each is preferably a hydrogen atom, a methyl group or an ethyl group.
R⁷ , R⁸, R⁹ and R¹⁰ are the same or different and each is a hydrogen atom or a substituent.
R⁷, R⁸, R⁹ and R¹⁰ are the same or different and each is preferably a hydrogen atom or an optionally substituted alkyl group (particularly, a methyl group, an ethyl group or an n-propyl group).

Note that (1) one or each of R^{2a} and R^{2b} is an optionally substituted alkyl group, or (2) R⁷, R⁸, R⁹ and R¹⁰ are the same or different and each is a hydrogen atom or a substituent, and R^{2a} and R^{2b} form, together with the adjacent carbon atom, an alicyclic hydrocarbon group optionally substituted by substituent(s) selected from a halogen atom, an alkyl group and an alkoxy group, or (3) at least one group of R⁷, R⁸, R⁹ and R¹⁰ is a substituent.
At least one group of R^{2a}, R^{2b}, R⁷, R⁸, R⁹ and R¹⁰ is preferably an optionally substituted alkyl group (particularly, a methyl group, an ethyl group or an n-propyl group).
As compound (Ia), the compounds of Examples 3, 5, 7, 8, 9, 48 - 50, 65, 66 and 73 - 78 and the like are preferable.

5. Compound (Ia'-1):
(1):
   X is a hydrogen atom.
   R^{2a} and R^{2b} are the same or different and each is a hydrogen atom or an alkyl group (provided that one or each of R^{2a} and R^{2b} is a C₁₋₆ alkyl group) .
   R^{2a} and R^{2b} are the same or different and each is preferably a hydrogen atom or a methyl group.

R⁷ and R⁸ are the same or different and each is a hydrogen atom or a C₁₋₆ alkyl group (provided that at least one group of R⁷ and R⁸ is a hydrogen atom).
R⁷ and R⁸ are the same or different and each is preferably a hydrogen atom, a methyl group, an ethyl group or an n-propyl group.
R⁹ and R¹⁰ are each a hydrogen atom.

(2):
X is a hydrogen atom or a halogen atom.
X is preferably a hydrogen atom, a fluorine atom, a chlorine atom or a bromine atom.
R^{2a} and R^{2b} are each a hydrogen atom.
R⁷, R⁹ and R¹⁰ are each a hydrogen atom.
R⁸ is a C₁₋₆ alkyl group.
R⁸ is preferably a methyl group.
   As compound (Ia'-1), the compounds of Examples 3, 5, 7 - 9, 35 - 37, 48 - 50, 65, 66 and 73 - 78 and the like are preferable.

6. Compound (Ib"):
X is a hydrogen atom or a halogen atom.
X is preferably a hydrogen atom or a chlorine atom.

R¹ is a group represented by the formula

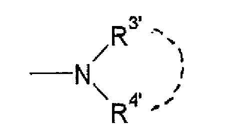

wherein R^{3'} is a hydrogen atom or an alkyl group optionally substituted by a cycloalkyl group, R^{4'} is a cycloalkyl group or an alkyl group optionally substituted by a cycloalkyl group, or R^{3'} and R^{4'} are crosslinked to each other via a carbon-carbon bond to form, together with the adjacent nitrogen atom, a non-aromatic cyclic amino group optionally substituted by substituent(s) selected from a halogen atom, an alkyl group, an alkoxy group and an aryl group, excluding a methylamino group.
R^{3'} is a hydrogen atom and a C₁₋₆ alkyl group (e.g., methyl group, ethyl group, an n-propyl group, an isopropyl group, an isobutyl group and sec-butyl group and the like) optionally substituted by cyclopropyl is preferable. R^{4'} is a cycloalkyl group (e.g., a cyclopentyl group and the like) and a C₁₋₆ alkyl group (e.g., a methyl group, an ethyl group, an n-propyl group, an isopropyl group, an isobutyl group and a sec-butyl group and the like) optionally substituted by cyclopropyl is preferable. In addition, R^{3'} and R^{4'} are preferably crosslinked to each other via a carbon-carbon bond to form, together with the adjacent nitrogen atom, a non-aromatic cyclic amino group (e.g., pyrrolidinyl group, piperidino group and the like) optionally substituted by an alkyl group (e.g., methyl group and the like).

Specific examples of R¹ include isopropylamino group, isobutylamino group, cyclopropylmethylamino group, methyl(cyclopropylmethyl)amino group, dimethylamino group, diethylamino group, methyl(ethyl)amino group, methyl(n-propyl)amino group, methyl(isopropyl)amino group, methyl(isobutyl)amino group, methyl(sec-butyl)amino group, methyl(cyclopentyl)amino group, pyrrolidinyl group, 2,5-dimethylpyrrolidinyl group, piperidino group and the like.

R^{2a} and R^{2b} are the same or different and each is a hydrogen atom or an optionally substituted alkyl group, or R^{2a} and R^{2b} form, together with the adjacent carbon atom, an alicyclic hydrocarbon group optionally substituted by substituent(s) selected from a halogen atom, an alkyl group and an alkoxy group.
R^{2a} and R^{2b} are the same or different and each is preferably a hydrogen atom or a methyl group.
As compound (Ib"), the compounds of Examples 10 - 23, 32 - 34, 38, 39, 51 - 53, 79 and 80 and the like are preferable.

7. Compound (Ib"-1):
[1]:
   X is a hydrogen atom or a halogen atom.
   X is preferably a hydrogen atom or a chlorine atom.

R¹ is (1) a group represented by the formula

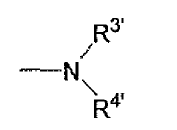

wherein R^{3'} is a hydrogen atom or a C₁₋₆ alkyl group, and R^{4'} is a cycloalkyl group, or a C₁₋₆ alkyl group optionally substituted by a C₃₋₆ cycloalkyl group, excluding a methylamino group, or (2) a group represented by the formula

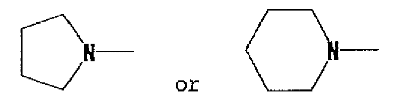

optionally substituted by 1 to 3 C₁₋₆ alkyl groups.
R^{3'} is preferably a hydrogen atom or a C₁₋₆ alkyl group (e.g., a methyl group, an ethyl group, an n-propyl group, an isopropyl group, an isobutyl group and a sec-butyl group and the like). R^{4'} is preferably a C₃₋₆ cycloalkyl group (e.g., a cyclopentyl group and the like) or a C₁₋₆ alkyl group (e.g., a methyl group, an ethyl group, an n-propyl group, an isopropyl group, an isobutyl group and a sec-butyl group and the like) optionally substituted by cyclopropyl.

Specific examples of R¹ include isopropylamino group, isobutylamino group, cyclopropylmethylamino group, methyl(cyclopropylmethyl)amino group, dimethylamino group, diethylamino group, methyl(ethyl)amino group, methyl(n-propyl)amino group, methyl(isopropyl)amino group, methyl(isobutyl)amino group, methyl(sec-butyl)amino group, methyl(cyclopentyl)amino group, pyrrolidinyl group, 2,5-dimethylpyrrolidinyl group, piperidino group and the like.

R^{2a} and R^{2b} are the same or different and each is a hydrogen atom or a C₁₋₆ alkyl group.
R^{2a} and R^{2b} are the same or different and each is preferably a hydrogen atom or a methyl group.

[2] :
X is a hydrogen atom or a halogen atom.
X is preferably a hydrogen atom or a chlorine atom.

R¹ is (1) a group represented by the formula

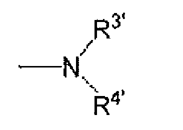

wherein R^{3'} is a hydrogen atom or a C₁₋₆ alkyl group, R^{4'} is a cycloalkyl group, or a C₁₋₆ alkyl group optionally substituted by a C₃₋₆ cycloalkyl group, excluding a methylamino group, or (2) a group represented by the formula

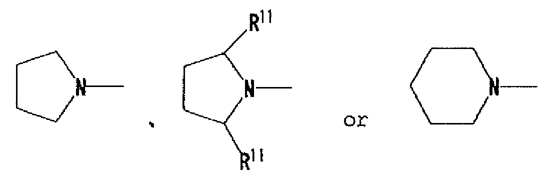

wherein R¹¹ is a hydrogen atom or a C₁₋₆ alkyl group.
R^{3'} is preferably a hydrogen atom or a C₁₋₆ alkyl group (e.g., a methyl group, an ethyl group, an n-propyl group, an isopropyl group, an isobutyl group and a sec-butyl group and the like). R^{4'} is preferably a C₃₋₆ cycloalkyl group (e.g., a cyclopentyl group and the like) or a C₁₋₆ alkyl group (e.g., a methyl group, an ethyl group, an n-propyl group, an isopropyl group, an isobutyl group and a sec-butyl group and the like) optionally substituted by cyclopropyl.
R¹¹ is preferably a methyl group.

Specific examples of R¹ include isopropylamino group, isobutylamino group, cyclopropylmethylamino group, methyl(cyclopropylmethyl)amino group, dimethylamino group, diethylamino group, methyl(ethyl)amino group, methyl(n-propyl)amino group, methyl(isopropyl)amino group, methyl(isobutyl)amino group, methyl(sec-butyl)amino group, methyl(cyclopentyl)amino group, pyrrolidinyl group, 2,5-dimethylpyrrolidinyl group, piperidino group and the like.

R^{2a} and R^{2b} are the same or different and each is a hydrogen atom or a C₁₋₆ alkyl group (provided that at least one group of R^{2a} and R^{2b} is a hydrogen atom).
R^{2a} and R^{2b} are the same or different and each is preferably a hydrogen atom or a methyl group.
As compound (Ib"-1), the compounds of Examples 10 - 23, 32 - 34, 38, 39, 51 - 53, 79 and 80 and the like are preferable.

8. Compound (Ib):
[1] :
   R¹ is a group represented by the formula

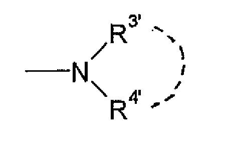

wherein R^{3'} is a hydrogen atom or an alkyl group optionally substituted by a cycloalkyl group, R^{4'} is an alkyl group optionally substituted by a cycloalkyl group, or R^{3'} and R^{4'} are crosslinked to each other via a carbon-carbon bond to form, together with the adjacent nitrogen atom, a non-aromatic cyclic amino group optionally substituted by substituent(s) selected from a halogen atom, an alkyl group, an alkoxy group and an aryl group, excluding a methylamino group.
R^{3'} is preferably a hydrogen atom or a C₁₋₆ alkyl group (e.g., a methyl group, an ethyl group, an n-propyl group, an isopropyl group, an isobutyl group and a sec-butyl group and the like) optionally substituted by cyclopropyl. R^{4'} is preferably a C₁₋₆ alkyl group (e.g., a methyl group, an ethyl group, an n-propyl group, an isopropyl group, an isobutyl group and a sec-butyl group and the like) optionally substituted by cyclopropyl. R^{3'} and R^{4'} are also preferably crosslinked to each other via a carbon-carbon bond to form, together with the adjacent nitrogen atom, a non-aromatic cyclic amino group (e.g., pyrrolidinyl group, piperidino group and the like) optionally substituted by an alkyl group (e.g., methyl group and the like).

Specific examples of R¹ include isopropylamino group, isobutylamino group, cyclopropylmethylamino group, methyl(cyclopropylmethyl)amino group, dimethylamino group, dimethylamino group, methyl(ethyl)amino group, methyl(n-propyl)amino group, methyl(isopropyl)amino group, methyl(isobutyl)amino group, methyl(sec-butyl)amino group, pyrrolidinyl group, 2,5-dimethylpyrrolidinyl group, piperidino group and the like.

R^{2a} and R^{2b} are the same or different and each is a hydrogen atom or an optionally substituted alkyl group, or R^{2a} and R^{2b} form, together with the adjacent carbon atom, an alicyclic hydrocarbon group optionally substituted by substituent(s) selected from a halogen atom, an alkyl group and an alkoxy group.
R^{2a} and R^{2b} are each preferably a hydrogen atom.
As compound (Ib), the compounds of Examples 10 - 23, 38, 39, 51, 53, 79 and 80 and the like are preferable.

[2] excluding a compound wherein R¹ is a methoxy group and R^{2a} and R^{2b} are each a hydrogen atom):
R¹ is
   (1) a group represented by -OR⁵ (R⁵ is a hydrogen atom, a cycloalkyl group, an alkyl group optionally substituted by a cycloalkyl group, an aryl group or a heteroaryl group),
   (2) a C₃₋₅ alkyl group (alkyl group having a carbon number of 3 to 5),
   (3) an aryl group optionally substituted by substituent(s) selected from a halogen atom, an alkyl group optionally substituted by a halogen atom, an alkoxy group, an alkylthio group and an aryl group,
   (4) a heteroaryl group, or
   (5) a cycloalkyl group.
R¹ is preferably
   (1) a group represented by -OR⁵ (R⁵ is a hydrogen atom, a cycloalkyl group (particularly, a cyclobutyl group), an alkyl group (particularly, an ethyl group, an isopropyl group, a sec-butyl group, a tert-butyl group) optionally substituted by a cycloalkyl group (particularly, a cyclopropyl group, a cyclopentyl group, a cyclohexyl group), an aryl group or a heteroaryl group),
   (2) a C₃₋₅ alkyl group (particularly, an n-propyl group, an isopropyl group, an isobutyl group, a sec-butyl group, a 1-ethylpropyl group),
   (3) an aryl group (particularly, a phenyl group) optionally substituted by substituent(s) selected from a halogen atom (particularly, a fluorine atom), an alkyl group (particularly, methyl group) optionally substituted by a halogen atom (particularly, a fluorine atom), an alkoxy group, an alkylthio group (particularly, a methylthio group) and an aryl group,
   (4) a heteroaryl group (particularly, a furyl group), or
   (5) a cycloalkyl group (particularly, a cyclopropyl group, a cyclobutyl group, a cyclopentyl group), more preferably (3) a phenyl group substituted by substituent(s) selected from a halogen atom (particularly, a fluorine atom), a C₁₋₆ alkyl group (particularly, methyl group) substituted by a halogen atom (particularly, a fluorine atom) and a C₁₋₆ alkylthio group (particularly, a methylthio group).

Specific examples of R¹ include hydroxy group, isopropoxy group, sec-butoxy group, tert-butoxy group, 1-cyclopropylethoxy group, 1-cyclopentylethoxy group, 1-cyclohexylethoxy group, cyclobutyloxy group, an n-propyl group, an isopropyl group, an isobutyl group, a sec-butyl group, a 1-ethylpropyl group, 4-fluorophenyl group, 4-trifluoromethylphenyl group, 4-methylthiophenyl group, 3-furyl group, a cyclopropyl group, a cyclobutyl group, a cyclopentyl group and the like.

R^{2a} and R^{2b} are the same or different and each is a hydrogen atom or an optionally substituted alkyl group, or R^{2a} and R^{2b} form, together with the adjacent carbon atom, an alicyclic hydrocarbon group optionally substituted by substituent(s) selected from a halogen atom, an alkyl group and an alkoxy group.
R^{2a} and R^{2b} are the same or different and each is preferably a hydrogen atom or an alkyl group (particularly, methyl group, ethyl group).
As compound (Ib), the compounds of Examples 4, 24 - 31, 40 - 47, 54 - 64 and 67 - 72 and the like are preferable.

9. Compound (Ib') [excluding a compound wherein R¹ is a methoxy group and R^{2a} and R^{2b} are each a hydrogen atom]:
R¹ is
   (1) a group represented by -OR⁵ (R⁵ is an alkyl group optionally substituted by a cycloalkyl group, an aryl group or a heteroaryl group),
   (2) a C₃ alkyl group (alkyl group having a carbon number of 3 (e.g., n-propyl, isopropyl)),
   (3) an aryl group optionally substituted by substituent(s) selected from a halogen atom, an alkyl group optionally substituted by a halogen atom, an alkoxy group, an alkylthio group and an aryl group, or
   (4) a heteroaryl group.
      R⁵ is preferably an alkyl group (particularly, a C₁₋₆ alkyl group such as an ethyl group, an isopropyl group and the like) optionally substituted by cyclopropyl.

R¹ is preferably
(1) a group represented by -OR⁵ (R⁵ is a C₁₋₆ alkyl group optionally substituted by cyclopropyl),
(2) a C₃ alkyl group (alkyl group having a carbon number of 3 (e.g., n-propyl, isopropyl)),
(3) a phenyl group optionally substituted by 1 - 3 substituents selected from a halogen atom, a C₁₋₆ alkyl group optionally substituted by a halogen atom and a C₁₋₆ alkylthio group, or
(4) a heteroaryl group.

R^{2a} and R^{2b} are the same or different and each is a hydrogen atom or an optionally substituted alkyl group, or R^{2a} and R^{2b} form, together with the adjacent carbon atom, an alicyclic hydrocarbon group optionally substituted by substituent(s) selected from a halogen atom, an alkyl group and an alkoxy group.
R^{2a} and R^{2b} are each preferably a hydrogen atom.
As compound (Ib'), the compounds of Examples 4, 24 - 31, 45 - 47, 54, 61, 63, 64, 67, 71 and 72 and the like are preferable.

10. Compound (Ib-1) [excluding a compound wherein R¹ is a methoxy group and R^{2a} and R^{2b} are each a hydrogen atom]:
R¹ is
   (1) a group represented by -OR⁵ (R⁵ is a hydrogen atom, a C₃₋₆ cycloalkyl group or a C₁₋₆ alkyl group optionally substituted by a C₃₋₆ cycloalkyl group),
   (2) a C₃₋₅ alkyl group,
   (3) a phenyl group optionally substituted by substituent(s) selected from a halogen atom, a C₁₋₆ alkyl group optionally substituted by a halogen atom, a C₁₋₆ alkylthio group and an aryl group,
   (4) a group represented by the formula 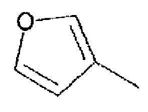
or

(5) a C₃₋₆ cycloalkyl group.
R¹ is preferably
(1) a group represented by -OR⁵ (R⁵ is a hydrogen atom, a C₃₋₆ cycloalkyl group (particularly, a cyclobutyl group), or a C₁₋₆ alkyl group (particularly, ethyl group, an isopropyl group, a sec-butyl group, tert-butyl group) optionally substituted by a C₃₋₆ cycloalkyl group (particularly, a cyclopropyl group, a cyclopentyl group, a cyclohexyl group)),
(2) a C₃₋₅ alkyl group (particularly, an n-propyl group, an isopropyl group, an isobutyl group, a sec-butyl group, a 1-ethylpropyl group),
(3) a phenyl group optionally substituted by substituent(s) selected from a halogen atom (particularly, a fluorine atom), a C₁₋₆ alkyl group (particularly, methyl group) optionally substituted by a halogen atom (particularly, a fluorine atom), and a C₁₋₆ alkylthio group (particularly, a methylthio group),
(4) a group represented by the formula

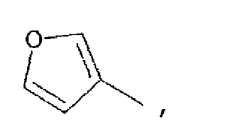

and
(5) a C₃₋₆ cycloalkyl group (particularly, a cyclopropyl group, a cyclobutyl group, a cyclopentyl group).

Specific examples of R¹ include hydroxy group, isopropoxy group, sec-butoxy group, tert-butoxy group, 1-cyclopropylethoxy group, 1-cyclopentylethoxy group, 1-cyclohexylethoxy group, cyclobutyloxy group, an n-propyl group, an isopropyl group, an isobutyl group, a sec-butyl group, a 1-ethylpropyl group, 4-fluorophenyl group, 4-trifluoromethylphenyl group, 4-methylthiophenyl group, 3-furyl group, a cyclopropyl group, a cyclobutyl group, a cyclopentyl group and the like.

R^{2a} and R^{2b} are the same or different and each is a hydrogen atom or an optionally substituted alkyl group.
R^{2a} and R^{2b} are the same or different and each is preferably a hydrogen atom or an alkyl group (particularly, methyl group, ethyl group).
As compound (Ib-1), the compounds of Examples 4, 24 - 31, 40 - 47, 54 - 64 and 67 - 72 and the like are preferable.

11. Compound (Ic):
R¹ is
   (1) a group represented by the formula

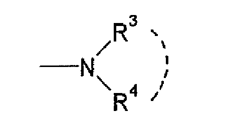

wherein R³ and R⁴ are the same or different and each is a hydrogen atom or an alkyl group, or R³ and R⁴ form, together with the adjacent nitrogen atom, a non-aromatic cyclic amino group optionally substituted by substituent(s) selected from a halogen atom, an alkyl group, an alkoxy group and an aryl group),
(2) a group represented by -OR⁵ (R⁵ is an alkyl group, an aryl group or a heteroaryl group),
(3) a group represented by -SR⁶ (R⁶ is an alkyl group, an aryl group or a heteroaryl group),
(4) an alkyl group,
(5) a cycloalkyl group,
(6) an aryl group optionally substituted by substituent(s) selected from a halogen atom, an alkyl group, an alkoxy group, an alkylthio group and an aryl group, or
(7) a heteroaryl group.

R¹ is preferably
(1) a group represented by the formula

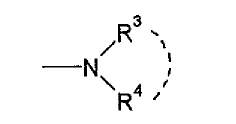

wherein R³ and R⁹ are the same or different and each is a hydrogen atom or an alkyl group, or R³ and R⁴ form, together with the adjacent nitrogen atom, a non-aromatic cyclic amino group optionally substituted by substituent(s) selected from a halogen atom, an alkyl group, an alkoxy group and an aryl group, more preferably,
R³ and R⁴ form, together with the adjacent nitrogen atom, a non-aromatic cyclic amino group (particularly, a morpholino group).

R^{2a} and R^{2b} are the same or different and each is a hydrogen atom or an optionally substituted alkyl group, or R^{2a} and R^{2b} form, together with the adjacent carbon atom, an alicyclic hydrocarbon group optionally substituted by substituent(s) selected from a halogen atom, an alkyl group and an alkoxy group.
R^{2a} and R^{2b} are the same or different and each is preferably a hydrogen atom or an alkyl group (particularly, methyl group).
As compound (Ic), the compounds of Examples 1 and 2 and the like are preferable.

12. Compound (Ic'):
One of R^{2a} and R^{2b} is a hydrogen atom and the other is a alkyl group.
Preferably, one of R^{2a} and R^{2b} is a hydrogen atom, and the other is a methyl group.

As the compounds of the present invention,
2-isopropyl-5,6,7,8-tetrahydro-1,6-naphthyridine or a salt thereof (Example 24),
2-isopropoxy-5,6,7,8-tetrahydro-1,6-naphthyridine or a salt thereof (Example 30),
2-cyclopropyl-5,6,7,8-tetrahydro-1,6-naphthyridine or a salt thereof (Example 43),
8-methyl-2-morpholin-4-yl-5,6,7,8-tetrahydro-1,6-naphthyridine or a salt thereof (Examples 5, 73, 74),
8-methyl-2-[(3R)-3-methylmorpholin-4-yl]-5,6,7,8-tetrahydro-1,6-naphthyridine or a salt thereof (Examples 48, 75, 76),
9-methyl-2-morpholin-4-yl-6,7,8,9-tetrahydro-5H-pyrido[3,2-c]azepine or a salt thereof (Example 2), and
N-isopropyl-N,8-dimethyl-5,6,7,8-tetrahydro-1,6-naphthyridin-2-amine or a salt thereof (Examples 53, 79, 80) are particularly preferable.

When compounds (Ix), (I), (Ia'), (Ia), (Ia'-1), (Ib"), (Ib"-1), (Ib), (Ib'), (Ib-1), (Ic) and (Ic') (hereinafter sometimes to be comprehensively abbreviated as compound (Ix)) are a salt, examples of the salt include salt with inorganic base, ammonium salt, salt with organic base, salt with inorganic acid, salt with organic acid, salt with basic or acidic amino acid and the like.
Preferable examples of the salt with inorganic base include alkali metal salts such as sodium salt, potassium salt and the like; alkaline earth metal salts such as calcium salt, magnesium salt, barium salt and the like; aluminum salt and the like.
Preferable examples of the salt with organic base include salts with trimethylamine, triethylamine, pyridine, picoline, ethanolamine, diethanolamine, triethanolamine, dicyclohexylamine, N,N'-dibenzylethylenediamine and the like.
Preferable examples of the salt with inorganic acid include salts with hydrochloric acid, hydrobromic acid, nitric acid, sulfuric acid, phosphoric acid and the like.
Preferable examples of the salt with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.
Preferable examples of the salt with basic amino acid include salts with arginine, lysine, ornithine and the like.
Preferable examples of the salt with acidic amino acid include salts with aspartic acid, glutamic acid and the like.
Of these salts, pharmaceutically acceptable salts are preferable.

Compound (Ix) encompasses a solvate, for example, hydrate. In addition, compound (Ix) may be labeled with an isotope (e.g., ³H, ¹⁴C, ³⁵S, ¹²⁵I and the like) and the like. Alternatively, compound (Ix) may be a non-solvate (including non-hydrate). In addition, compound (Ix) may be a deuterium exchanged compound.
When compound (Ix) of the present invention has an asymmetric center, isomers such as enantiomer, diastereomer and the like may be present. Such isomers and a mixture thereof are all encompassed in the scope of the present invention. When an isomer due to conformation is present, such isomer and a mixture thereof are also encompassed in compound (Ix) of the present invention.

The production methods of the compounds (Ix) of the present invention are explained in the following.
The compounds (Ix) of the present invention can be produced, for example, using the following method A, method B, method C, method D, method E, method F, method G, method H and method I. Unless otherwise specified, each symbol in the chemical formulas of each scheme is as defined above. The compounds (Iaa), (Ibb), (Icc) and (Id) - (Ii), which are the final products of each scheme, are all encompassed in compound (Ix). The compounds of each scheme may form salts and examples thereof include those similar to the salts of compound (Ix), and the like.

In each of the following reactions, when the starting material compound and intermediate has an amino group, a carboxyl group or a hydroxyl group as a substituent, these groups may be protected by a protecting group generally used in the peptide chemistry and the like. In this case, the object compound can be obtained by removing the protecting group as necessary after the reaction.
Examples of the amino-protecting group include formyl group, C₁₋₆ alkyl-carbonyl group, C₁₋₆ alkoxy-carbonyl group, benzoyl group, C₇₋₁₀ aralkyl-carbonyl group (e.g., benzylcarbonyl), C₇₋₁₄ aralkyloxy-carbonyl group (e.g., benzyloxycarbonyl, 9-fluorenylmethoxycarbonyl), trityl group, phthaloyl group, N,N-dimethylaminomethylene group, substituted silyl group (e.g., trimethylsilyl, triethylsilyl, dimethylphenylsilyl, tert-butyldimethylsilyl, tert-butyldiethylsilyl), C₂₋₅ alkenyl group (e.g., 1-allyl) and the like. These groups are optionally substituted by 1 to 3 substituents selected from a halogen atom, a C₁₋₆ alkoxy group and a nitro group.
Examples of the carboxyl-protecting group include C₁₋₆ alkyl group, C₇₋₁₁ aralkyl group (e.g., benzyl), a phenyl group, trityl group, substituted silyl group (e,g., trimethylsilyl, triethylsilyl, dimethylphenylsilyl, tert-butyldimethylsilyl, tert-butyldiethylsilyl), C₂₋₆ alkenyl group (e.g., 1-allyl) and the like.
Examples of the hydroxyl-protecting group include C₁₋₆ alkyl group, a phenyl group, trityl group, C₇₋₁₀ aralkyl group (e.g., benzyl), formyl group, C₁₋₆ alkyl-carbonyl group, benzoyl group, C₇₋₁₀ aralkyl-carbonyl group (e.g., benzylcarbonyl), 2-tetrahydropyranyl group, 2-tetrahydrofuranyl group, substituted silyl group (e.g., trimethylsilyl, triethylsilyl, dimethylphenylsilyl, tert-butyldimethylsilyl, tert-butyldiethylsilyl), C₂₋₆ alkenyl group (e.g., 1-allyl) and the like.
These groups are optionally substituted by 1 to 3 substituents selected from a halogen atom, a C₁₋₆ alkyl group, a C₁₋₆ alkoxy group and a nitro group.
The above-mentioned protecting groups can be removed by a method known per se, for example, the method described in Protective Groups in Organic Synthesis, John Wiley and Sons (1980) and the like. Specifically, a method using acid, base, ultraviolet rays, hydrazine, phenylhydrazine, sodium N-methyldithiocarbamate, tetrabutylammonium fluoride, palladium acetate, trialkylsilyl halide (for example, trimethylsilyl iodide, trimethylsilyl bromide and the like) and the like, reduction method and the like are employed.

While the compound obtained in each step can be used for the next reaction in the form of a reaction mixture or a crude product. Alternatively, it can also be isolated from a reaction mixture according to a conventional method, and easily purified by a separation means such as recrystallization, distillation, chromatography and the like.

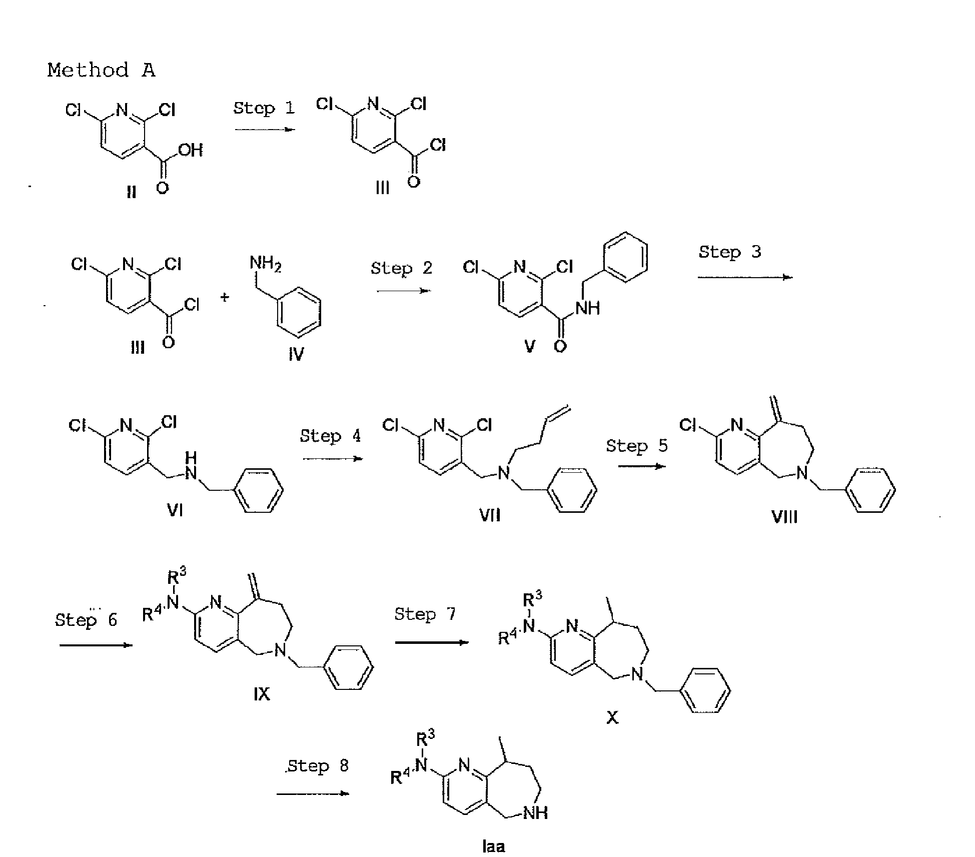

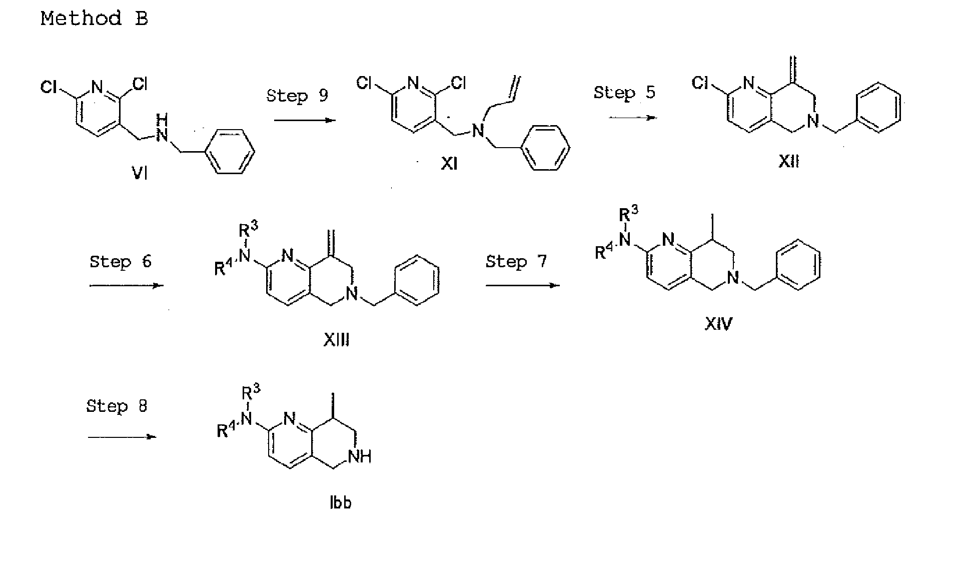

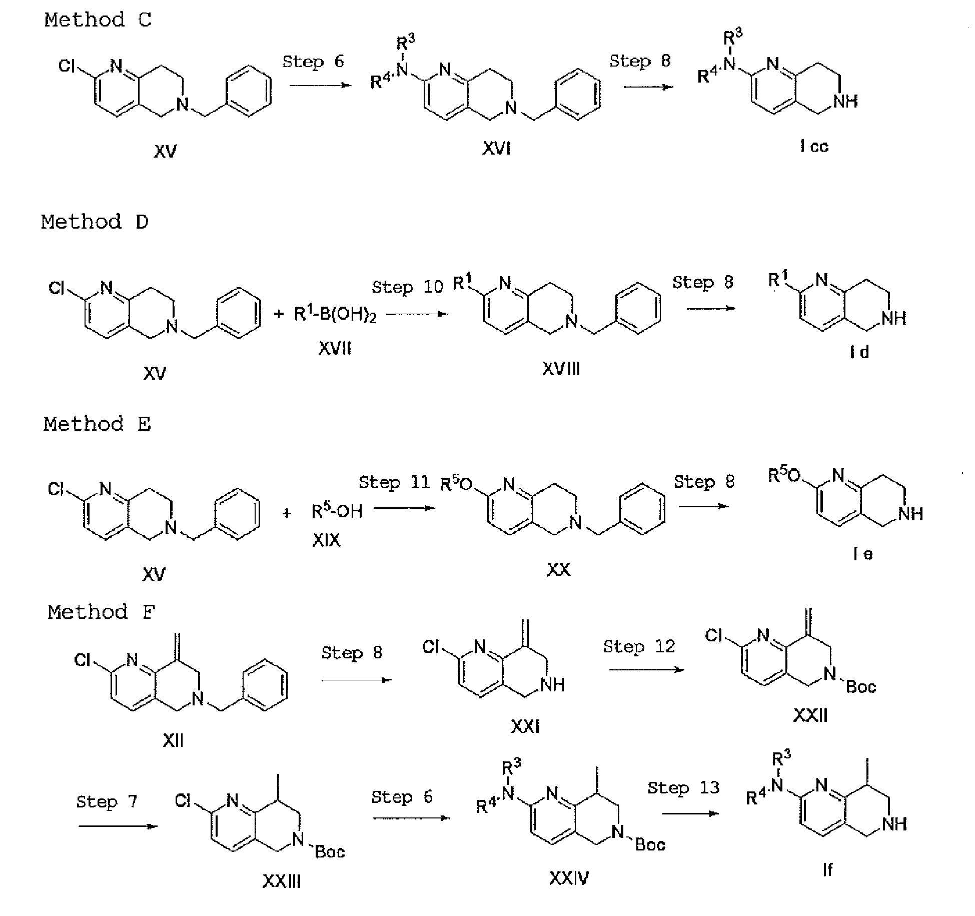

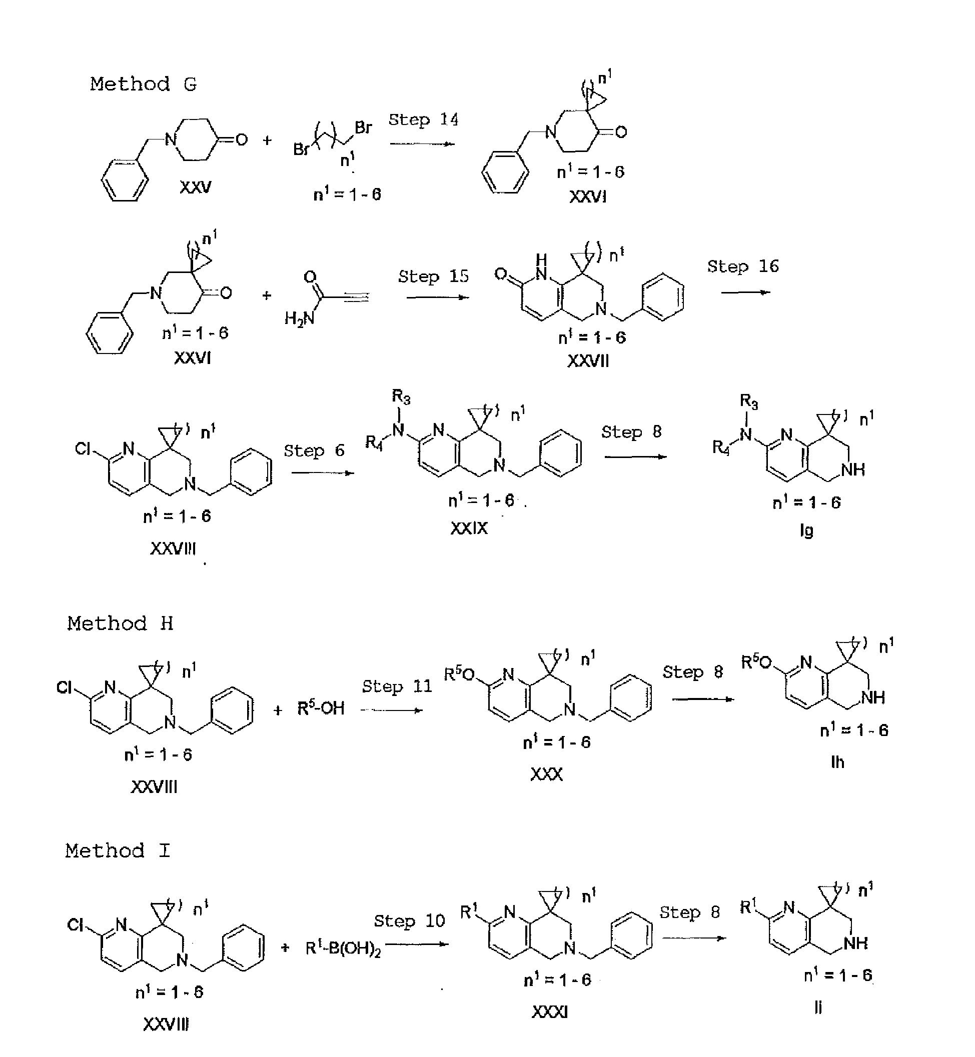

Each step described in the above-mentioned scheme (Method A, method B, method C, method D, method E, method F, method G, method H, method I) is explained in detail in the following.

### (Step 1)

This step is a step of converting compound (II) to a compound represented by the formula (III) or a salt thereof (hereinafter to be referred to as compound (III)) by halogenating compound (II) with a halogenating agent (in the scheme, halogen is shown by a chlorine atom for convenience). The halogenation can be carried out according to a method known per se [e.g., the method described in 4th ed. Jikken Kagaku Koza, vol. 22, page 115 and the like], for example, it can be carried out in the presence of a halogenating agent, in a solvent that does not adversely influence the reaction.
Compound (II) may be a commercially available product, or can be produced from the corresponding starting material compound according to a method known per se.
Examples of the halogenating agent include thionyl chloride, phosphorus pentachloride, phosphorus oxychloride, oxalyl chloride and the like. The amount thereof to be used is about 1 to about 10 molar equivalents, preferably about 1 to about 3 molar equivalents, per 1 mol of compound (II).
Examples of the solvent that does not adversely influence the reaction include hydrocarbons (e.g., benzene, toluene, xylene etc.), halogenated hydrocarbons (e.g., chloroform, 1,2-dichloroethane etc.), nitriles (e.g., acetonitrile etc.), ethers (e.g., dimethoxyethane, tetrahydrofuran etc.), aprotic polar solvents (e.g., N,N-dimethylformamide, dimethyl sulfoxide, hexamethylphosphoramide etc.) and a mixture thereof. The amount of the solvent to be used is generally 1-fold volume to 100-fold volume, relative to compound (II).
The reaction temperature is generally about -10 to about 200°C, preferably about 0 to about 150°C. The reaction time is generally about 0.5 to about 48 hr, preferably about 0.5 to about 16 hr.
The thus-obtained compound (III) can be isolated and purified by known separation and purification means, for example, concentration, concentration under reduced pressure, solvent extraction, crystallization, recrystallization, phase transfer, chromatography and the like. In addition, compound (III) may be used for the next reaction without isolation.

### (Step 2)

This step is a step of producing a compound represented by the formula (V) or a salt thereof (hereinafter to be referred to as compound (V)) by reacting compound (III) with a compound represented by the formula (IV) (hereinafter to be referred to as compound (IV)).
Compound (IV) (benzylamine) can be generally a commercially available product. The amount of compound (IV) to be used is generally about 1 molar equivalent to about 10 molar equivalents, preferably about 1 molar equivalent to about 2 molar equivalents, per 1 mol of compound (III).

The above-mentioned reaction is generally carried out in a solvent that does not adversely influence the reaction, and an appropriate base for promotion of the reaction may be added.
Examples of the solvent include hydrocarbons (e.g., benzene, toluene etc.), ethers (e.g., diethyl ether, dioxane, tetrahydrofuran etc.), esters (e.g., ethyl acetate etc.), halogenated hydrocarbons (e.g., chloroform, dichloromethane etc.), amides (e.g., N,N-dimethylformamide etc.), aromatic amines (e.g., pyridine etc.), water and the like. These solvents may be used in a mixture of two or more kinds at an appropriate ratio. The amount of the solvent to be used is generally 1-fold volume to 100-fold volume, relative to compound (III).
Examples of the base include alkali metal hydroxides (e.g., sodium hydroxide, potassium hydroxide etc.), hydrogen carbonates (e.g., sodium hydrogen carbonate, potassium hydrogen carbonate etc.), carbonates (e.g., sodium carbonate, potassium carbonate etc.), acetates (e.g., sodium acetate etc.), tertiary amines (e.g., trimethylamine, triethylamine, N-methylmorpholine etc.), aromatic amines (e.g., pyridine, picoline, N,N-dimethylaniline etc.) and the like. The amount of the base to be used is generally about 1 to about 100 molar equivalents, preferably about 1 to about 5 molar equivalents, per 1 mol of compound (III).
The reaction temperature is generally about -80°C to about 150°C, preferably about 0°C to about 100°C. The reaction time is generally about 0.1 hr to about 48 hr, preferably about 0.5 hr to about 16 hr.
The thus-obtained compound (V) can be isolated and purified by known separation and purification means, for example, concentration, concentration under reduced pressure, solvent extraction, crystallization, recrystallization, phase transfer, chromatography and the like. In addition, compound (V) may be used for the next reaction without isolation.

### (Step 3)

This step is a step of producing a compound represented by the formula (VI) or a salt thereof (hereinafter to be referred to as compound (VI)) by subjecting compound (V) to a reduction reaction. This reaction can be carried out according to a method known per se generally in the presence of a reducing agent, and where necessary, in a solvent that does not adversely influence the reaction.
Examples of the reducing agent include aluminum reagents (e.g., lithium aluminum hydrides (LiAlH₄), diisobutylaluminum hydrides (DIBAL-H), sodium bis(2-methoxyethoxy)aluminum hydrides (Red-Al), Alane (AlH₃) etc.), boron reagents (e.g., borane (BH₃), 9-borabicyclo[3.3.1]nonane (9-BBN), sodium borohydrides (NaBH₄), sodium cyanoborohydrides (NaBH₃CN), sodium triacetoxyborohydrides (NaBH(OAc)₃) etc.) and the like. Of these, lithium aluminum hydride and borane are preferable. While the amount of the reducing agent to be used varies depending on the kind of the solvent and the other reaction conditions, it is generally about 1 molar equivalent to about 10 molar equivalents, preferably about 1 molar equivalent to about 5 molar equivalents, per 1 mol of compound (V).

Examples of the solvent that does not adversely influence the reaction include alcohols (e.g., methanol, ethanol, propanol, 2-propanol, butanol, isobutanol, tert-butanol etc.), hydrocarbons (e.g., benzene, toluene, xylene, hexane, heptane etc.), halogenated hydrocarbons (e.g., dichloromethane, chloroform etc.), ethers (e.g., diethyl ether, diisopropyl ether, tert-butyl methyl ether, tetrahydrofuran, dioxane, dimethoxyethane etc.), carboxylic acids (e.g., acetic acid, trifluoroacetic acid etc.) and the like. These solvents may be used in a mixture of two or more solvents at an appropriate ratio. The amount of the solvent to be used is generally 1-fold volume to 100-fold volume, relative to compound (V).
The reaction temperature is generally within the range of about -80°C to about 200°C, preferably about -80°C to about 100°C. While the reaction time varies depending on the kind of the reducing agent, the reaction temperature and the like, it is generally, about 0.1 hr to about 100 hr, preferably about 0.5 hr to about 24 hr.
The thus-obtained compound (VI) can be isolated and purified by known separation and purification means, for example, concentration, concentration under reduced pressure, solvent extraction, crystallization, recrystallization, phase transfer, chromatography and the like. In addition, compound (VI) may be used for the next reaction without isolation.

### (Step 4)

This step is a step of producing a compound represented by the formula (VII) or a salt thereof (hereinafter to be referred to as compound (VII)) by subjecting compound (VI) to a homoallylation reaction with a homoallyl bromide. This reaction can be carried out according to a method known per se, generally, in the presence of a base, and where necessary, in a solvent that does not adversely influence the reaction.
The homoallyl bromide may be generally commercially available product. The amount of the homoallyl bromide to be used is generally about 1 molar equivalent to about 10 molar equivalents, preferably about 1 molar equivalent to about 3 molar equivalents, per 1 mol of compound (VI).
Examples of the solvent include hydrocarbons (e.g., benzene, toluene etc.), ethers (e.g., diethyl ether, dioxane, tetrahydrofuran etc.), esters (e.g., ethyl acetate etc.), halogenated hydrocarbons (e.g., chloroform, dichloromethane etc.), amides (e.g., N,N-dimethylformamide etc.), aromatic amines (e.g., pyridine etc.), water and the like. These solvents may be used in a mixture of two or more solvents at an appropriate ratio. The amount of the solvent to be used is generally 1-fold volume to 100-fold volume, relative to compound (VI).
Examples of the base include alkali metal hydroxides (e.g., sodium hydroxide, potassium hydroxide etc.), hydrogen carbonates (e.g., sodium hydrogen carbonate, potassium hydrogen carbonate etc.), carbonates (e.g., sodium carbonate, potassium carbonate etc.), acetates (e.g., sodium acetate etc.), tertiary amines (e.g., trimethylamine, triethylamine, N-methylmorpholine etc.), aromatic amines (e.g., pyridine, picoline, N,N-dimethylaniline etc.) and the like. The amount of the base to be used is generally about 1 molar equivalent to about 100 molar equivalents, preferably about 1 molar equivalent to about 5 molar equivalents, per 1 mol of compound (VI).
The reaction temperature is generally about -80°C to about 150°C, preferably about 0°C to about 100°C. The reaction time is generally about 0.1 hr to about 48 hr, preferably about 0.5 hr to about 16 hr.
The thus-obtained compound (VII) can be isolated and purified by known separation and purification means, for example, concentration, concentration under reduced pressure, solvent extraction, crystallization, recrystallization, phase transfer, chromatography and the like. In addition, compound (VII) may be used for the next reaction without isolation.

### (Step 5)

This step is a step of converting compound (VII) to a compound represented by the formula (VIII) or a salt thereof (hereinafter to be referred to as compound (VIII)) by subjecting compound (VII) to an intramolecular Heck reaction. This step can be carried out according to a method known per se [e.g., the method described in J. Org. Chem. 2006, vol.71, page 1732 and the like], for example, and in the presence of a transition metal catalyst and a base, in a solvent that does not adversely influence the reaction.
Examples of the transition metal catalyst to be used include palladium catalysts (e.g., palladium (II) acetate, tris(dibenzylideneacetone)dipalladium (0), palladium (II) chloride, tetrakis(triphenylphosphine)palladium (0) etc.) and the like. Ligands (e.g., 2,2'-bis(diphenylphosphino)-1,1'-binaphthyl, 2,2'-bis(di-p-tolylphosphino)-1,1'-binaphthyl, 2-dicyclohexylphosphino-2',4',6'-triisopropyl-1,1'-biphenyl, triphenylphosphine, tri-tert-butylphosphine, tris(2-methylphenyl)phosphine etc.) may be added as necessary. While the amount of the catalyst to be used varies depending on kind thereof, it is generally about 0.0001 to about 1 molar equivalent, preferably about 0.01 to about 0.5 molar equivalent, per 1 mol of compound (VII). The amount of the ligand to be used is generally about 0.0001 to about 4 molar equivalents, preferably about 0.01 to about 2 molar equivalents, per 1 mol of compound (VII).

Examples of the base to be used include organic amines (e.g., trimethylamine, triethylamine, diisopropylamine, N-methylmorpholine, 1,8-diazabicyclo[5.4.0]undec-7-ene, pyridine, N,N-dimethylaniline etc.), alkali metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide etc.), metal hydrides (potassium hydride, sodium hydride etc.), alkali metal alkoxides (sodium methoxide, sodium ethoxide, sodium-tert-butoxide, potassium-tert-butoxide etc.), alkali disilazides (e.g., lithium disilazide, sodium disilazide, potassium disilazide etc.) and the like. Of these, metal hydrides (potassium hydride, sodium hydride etc.); alkali metal alkoxides such as sodium-tert-butoxide, potassium-tert-butoxide and the like; and the like are preferable. The amount of the base to be used is generally about 0.1 to about 10 molar equivalents, preferably about 1 to about 5 molar equivalents, per 1 mol of compound (VII).
Examples of the solvent that does not adversely influence the reaction include hydrocarbons (e.g., benzene, toluene, xylene etc.), halogenated hydrocarbons (e.g., chloroform, 1,2-dichloroethane etc.), nitriles (e.g., acetonitrile etc.), ethers (e.g., dimethoxyethane, tetrahydrofuran etc.), aprotic polar solvents (e.g., N,N-dimethylforznamide, dimethyl sulfoxide, hexamethylphosphoramide etc.) and a mixture thereof and the like. The amount of the solvent to be used is generally 1-fold volume to 100-fold volume, relative to compound (VII).
The reaction temperature is generally about -10 to about 200°C, preferably about 0 to about 150°C. The reaction time is generally about 0.5 to about 48 hr, preferably about 0.5 to about 16 hr.
The thus-obtained compound (VIII) can be isolated and purified by known separation and purification means, for example, concentration, concentration under reduced pressure, solvent extraction, crystallization, recrystallization, phase transfer, chromatography and the like. In addition, compound (VIII) may be used for the next reaction without isolation.
The conversion of compound (XI) to compound (XII) in method B in the scheme can be also carried out by this step.

### (step 6)

This step is a step of producing a compound represented by the formula (IX) or a salt thereof (hereinafter to be referred to as compound (IX)) by reacting compound (VIII) with an amine represented by the formula: HNR³R⁴ wherein each symbol is as defined above, or a salt thereof. This step can be carried out according to a method known per se [e.g., the method described in J. Am. Chem. Soc. 2003, vol.125, page 6653, J. Org. Chem. 2000, vol.65, page 1174 and the like], for example, in the presence of a transition metal catalyst, a base and an amine represented by the formula: HNR³R⁴ or a salt thereof, in a solvent that does not adversely influence the reaction.
The amine represented by the formula: HNR³R⁴ or a salt thereof may be a commercially available product, or can be produced from the corresponding starting material compound according to a method known per se. The amount of the amine or a salt thereof to be used is generally about 1 molar equivalent to about 10 molar equivalents, preferably about 1 molar equivalent to about 3 molar equivalents, per 1 mol of compound (VIII).
Examples of the transition metal catalyst include palladium catalysts (e.g., palladium(II) acetate, tris(dibenzylideneacetone)dipalladium(0), palladium(II) chloride, tetrakis(triphenylphosphine)palladium(0) etc.), nickel catalysts (e.g., nickel chloride etc.) and the like. Ligands (e.g., 2-dicyclohexylphosphino-2' ,4' ,6'-triisopropyl-1,1'-biphenyl, triphenylphosphine, tri-tert-butylphosphine etc.) may be added as necessary. While the amount of the transition metal catalyst to be used varies depending on the kind, it is generally about 0.0001 molar equivalent to about 1 molar equivalent, preferably about 0.01 molar equivalent to about 0.5 molar equivalent, per 1 mol of compound (VIII). The amount of the ligand to be used is generally about 0.0001 molar equivalent to about 4 molar equivalents, preferably about 0.01 molar equivalent to about 2 molar equivalents, per 1 mol of compound (VIII).

Examples of the base include organic amines (e.g., trimethylamine, triethylamine, diisopropylamine, N-methylmorpholine, 1,8-diazabicyclo[5.4.0]undec-7-ene, pyridine, N,N-dimethylaniline etc.), alkali metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide etc.), metal hydrides (e.g., potassium hydride, sodium hydride etc.), alkali metal alkoxides (e.g., sodium methoxide, sodium ethoxide, sodium-tert-butoxide, potassium-tert-butoxide etc.), alkali metal disilazides (e.g., lithium disilazide, sodium disilazide, potassium disilazide etc.) and the like. Of these, alkali metal salts such as potassium carbonate, cesium carbonate, sodium phosphate, potassium phosphate and the like; alkali metal alkoxides such as sodium-tert-butoxide, potassium-tert-butoxide and the like; organic amines such as triethylamine, diisopropylamine and the like and the like are preferable. The amount of the base to be used is generally about 0.1 molar equivalent to about 10 molar equivalents, preferably about 1 molar equivalent to about 5 molar equivalents, per 1 mol of compound (VIII).
Examples of the solvent that does not adversely influence the reaction include hydrocarbons (e.g., benzene, toluene, xylene etc.), halogenated hydrocarbons (e.g., chloroform, 1,2-dichloroethane etc.), nitriles (e.g., acetonitrile etc.), ethers (e.g., dimethoxyethane, tetrahydrofuran etc.), aprotic polar solvents (e.g., N,N-dimethylformamide, dimethyl sulfoxide, hexamethylphosphoramide etc.) and a mixture thereof and the like. The amount of the solvent to be used is generally 1-fold volume to 100-fold volume, relative to compound (VIII).
The reaction temperature is generally about -10°C to about 200°C, preferably about 0°C to about 150°C. The reaction time is generally about 0.5 hr to about 48 hr, preferably about 0.5 hr to about 16 hr.
The thus-obtained compound (IX) can be isolated and purified by known separation and purification means, for example, concentration, concentration under reduced pressure, solvent extraction, crystallization, recrystallization, phase transfer, chromatography and the like. In addition, compound (IX) may be used for the next reaction without isolation.
The conversion of compound (XII) to compound (XIII) in method B in the scheme, the conversion of compound (XV) to compound (XVI) in method C in the scheme, the conversion of compound (XXIII) to compound (XXIV) in method F in the scheme, and the conversion of compound (XXVIII) to compound (XXIX) in method G in the scheme can be also carried out by this step.

### (Step 7)

This step is a step of producing compound (X) or a salt thereof (hereinafter to be referred to as compound (X)) by subjecting compound (IX) to a reduction reaction. The reduction reaction can be carried out according to a method known per se [e.g., the method described in Guide to Organic Chemical Experiments 3, synthesis reaction I, Kagaku Dojin, pages 38-40 and the like], for example, by reducing with hydrogen gas in the presence of various transition metal catalyst in a solvent that does not adversely influence the reaction.
Examples of the transition metal catalyst include platinum, palladium, rhodium, ruthenium, nickel, palladium oxide, palladium carbon, Wilkinson's complex and the like. While the amount of the transition metal catalyst to be used varies depending on the kind, it is generally about 0.1 molar equivalent to about 5 molar equivalents, preferably about 0.1 molar equivalent to about 2 molar equivalents, per 1 mol of compound (IX). The hydrogen gas may be used at a normal pressure or pressurized. In addition, formic acid, formic acid amine salt, phosphinate salt, hydrazine and the like can be used as a hydrogen source instead of hydrogen gas.
Examples of the solvent that does not adversely influence the reaction include hydrocarbons (e.g., benzene, toluene, xylene etc.), nitriles (e.g., acetonitrile etc.), ethers (e.g., dimethoxyethane, tetrahydrofuran), alcohols (e.g., methanol, ethanol etc.), aprotic polar solvents (e.g., dimethylformamide, dimethyl sulfoxide, hexamethylphosphoramide etc.), water and a mixture thereof and the like. The amount of the solvent to be used is generally 1-fold volume to 100-fold volume, relative to compound (IX).
The reaction temperature is generally about -100°C to about 100°C, preferably about -78°C to about 50°C. The reaction time is generally about 0.5 hr to about 48 hr, preferably about 0.5 hr to about 16 hr.
The thus-obtained compound (X) can be isolated and purified by known separation and purification means, for example, concentration, concentration under reduced pressure, solvent extraction, crystallization, recrystallization, phase transfer, chromatography and the like. In addition, compound (X) may be used for the next reaction without isolation.
The conversion of compound (XIII) to compound (XIV) in method B in the scheme, and the conversion of compound (XXII) to compound (XXIII) in method F in the scheme can be also carried out by this step.

### (Step 8)

This step is a step of converting compound (X) to a compound represented by the formula (Iaa) or a salt thereof (hereinafter to be referred to as compound (Iaa)) by removing the benzyl group of compound (X). The benzyl group can be removed according to a method known per se or the method described in Wiley-InterScience, 1999, "Protective Groups in Organic Synthesis, 3rd Ed." (Theodara W. Greene, Peter G.M. Wuts) and the like or a method analogous thereto, for example, by subjecting compound (X) to a catalytic hydrogenation or by treating compound (X) with an acid halide and the like.
The catalytic hydrogenation can be carried out in the presence of a catalyst under hydrogen atmosphere. Examples of the catalyst include palladiums (e.g., palladium carbon, palladium hydroxide carbon, palladium oxide etc.), nickels (e.g., activated nickel catalyst etc.), platinums (e.g., platinum oxide, platinum carbon etc.), rhodiums (e.g., rhodium carbon etc.) and the like. The amount thereof to be used is generally about 0.001 to about 1 molar equivalent, preferably about 0.01 to about 0.5 molar equivalent, per 1 mol of compound (X).
The catalytic hydrogenation is generally carried out in a solvent inert to the reaction. Examples of the solvent include alcohols (e.g., methanol, ethanol, propanol, butanol etc.), hydrocarbons (e.g., benzene, toluene, xylene etc.), halogenated hydrocarbons (e.g., dichloromethane, chloroform etc.), ethers (e.g., diethyl ether, dioxane, tetrahydrofuran etc.), esters (e.g., ethyl acetate etc.), amides (e.g., N,N-dimethylformamide etc.), carboxylic acids (e.g., acetic acid etc.), water and a mixture thereof. The amount of the solvent to be used is generally 1-fold volume to 100-fold volume, relative to compound (X).
The hydrogen pressure for catalytic hydrogenation is generally about 1 to about 50 atm, preferably about 1 to about 10 atm. Formic acid, formic acid amine salt, phosphinate salt, hydrazine and the like may be used as a hydrogen source instead of hydrogen gas. The reaction temperature is generally about 0°C to about 150°C, preferably about 20°C to about 100°C. The reaction time is generally about 5 min to about 72 hr, preferably about 0.5 hr to about 40 hr.

Examples of the treatment with an acid halide include method using 1-chloroethyl chloroformate, 2,2,2-trichloro-1,1-dimethylethyl chloroformate, β-trimethylsilylethyl chloroformate and the like. Of these, method using 1-chloroethyl chloroformate is preferable. The amount of the acid halide to be used is generally about 1 to about 10 molar equivalents, preferably about 1 to about 2 molar equivalents, per 1 mol of compound (X).
The reaction can be generally carried out in a solvent inert to the reaction. Examples of the solvent include hydrocarbons (e.g., benzene, toluene, xylene etc.), halogenated hydrocarbons (e.g., dichloromethane, chloroform etc.), ethers (e.g., diethyl ether, dioxane, tetrahydrofuran etc.), esters (e.g., ethyl acetate etc.), amides (e.g., N,N-dimethylformamide etc.), nitriles (e.g., acetonitrile etc.) and a mixture thereof. The amount of the solvent to be used is generally 1-fold volume to 100-fold volume, relative to compound (X).
The reaction temperature is generally about -80°C to about 150°C, preferably about 0°C to about 100°C. The reaction time is generally about 5 min to about 72 hr, preferably about 0.5 hr to about 20 hr.
When 1-chloroethyl chloroformate is used, compound (X) is reacted with 1-chloroethyl chloroformate, and the resulting compound is treated with an alcohol (e.g., methanol, ethanol etc.), an aqueous solution (e.g., aqueous sodium hydroxide solution etc.) or water to give compound (Iaa). The reaction temperature is generally about 0°C to about 150°C, preferably about 5°C to about 100°C. The reaction time is generally about 5 min to about 24 hr, preferably about 0.5 hr to about 5 hr.
The thus-obtained compound (Iaa) can be isolated and purified by known separation and purification means, for example, concentration, concentration under reduced pressure, solvent extraction, crystallization, recrystallization, phase transfer, chromatography and the like.
The conversion of compound (XIV) to compound (Ibb) in method B in the scheme, the conversion of compound (XVI) to compound (Icc) in method C in the scheme, the conversion of compound (XVIII) to compound (Id) in method D in the scheme, the conversion of compound (XX) to compound (Ie) in method E in the scheme, the conversion of compound (XII) to compound (XXI) in method F in the scheme, the conversion of compound (XXIX) to compound (Ig) in method G in the scheme, the conversion of compound (XXX) to compound (Ih) in method H in the scheme, and the conversion of compound (XXXI) to compound (Ii) in method I in the scheme can be also carried out by this step.

### (Step 9)

This step is a step of producing a compound represented by the formula (XI) or a salt thereof (hereinafter to be referred to as compound (XI)) by subjecting compound (VI) to an allylation reaction with allyl bromide. This reaction can be carried out according to a method known per se, generally, in the presence of a base, and where necessary, in a solvent that does not adversely influence the reaction.
The allyl bromide may be generally commercially available product. The amount of the allyl bromide to be used is generally about 1 molar equivalent to about 10 molar equivalents, preferably about 1 molar equivalent to about 3 molar equivalents, per 1 mol of compound (VI).
Examples of the solvent include hydrocarbons (e.g., benzene, toluene etc.), ethers (e.g., diethyl ether, dioxane, tetrahydrofuran etc.), esters (e.g., ethyl acetate etc.), halogenated hydrocarbons (e.g., chloroform, dichloromethane etc.), amides (e.g., N,N-dimethylformamide etc.), aromatic amines (e.g., pyridine etc.), water and the like. These solvents may be used in a mixture of two or more solvents at an appropriate ratio. The amount of the solvent to be used is generally 1-fold volume to 100-fold volume, relative to compound (VI).
Examples of the base include alkali metal hydroxides (e.g., sodium hydroxide, potassium hydroxide etc.), hydrogen carbonates (e.g., sodium hydrogen carbonate, potassium hydrogen carbonate etc.), carbonates (e.g., sodium carbonate, potassium carbonate etc.), acetates (e.g., sodium acetate etc.), tertiary amines (e.g., trimethylamine, triethylamine, N-methylmorpholine etc.), aromatic amines (e.g., pyridine, picoline, N,N-dimethylaniline etc.) and the like. The amount of the base to be used is generally about 1 molar equivalent to about 100 molar equivalents, preferably about 1 molar equivalent to about 5 molar equivalents, per 1 mol of compound (VI).
The reaction temperature is generally about -80°C to about 150°C, preferably about 0°C to about 100°C. The reaction time is generally about 0.1 hr to about 48 hr, preferably about 0.5 hr to about 16 hr.
The thus-obtained compound (XI) can be isolated and purified by known separation and purification means, for example, concentration, concentration under reduced pressure, solvent extraction, crystallization, recrystallization, phase transfer, chromatography and the like. In addition, compound (XI) may be used for the next reaction without isolation.

### (Step 10)

This step is a step of producing a compound represented by the formula (XVIII) or a salt thereof (hereinafter to be referred to as compound (XVIII)) by subjecting compound (XV) to a coupling reaction with a compound represented by the formula: R¹-B(OH)₂ wherein each symbol is as defined above, or a salt thereof. This step can be carried out according to a method known per se [e.g., the method described in Chemical Reviews, 1995, vol.95, page 2457 and the like] , for example, in the presence of a transition metal catalyst and a base, in a solvent that does not adversely influence the reaction.
Compound (XV) may be a commercially available product, or can be produced from the corresponding starting material compound according to a method known per se.
The compound represented by the formula: R¹-B(OH)₂ or a salt thereof may be a commercially available product, or can be produced from the corresponding starting material compound according to a method known per se. The amount of the compound represented by the formula: R¹-B(OH)₂ or a salt thereof to be used is generally about 1 molar equivalent to about 10 molar equivalents, preferably about 1 molar equivalent to about 3 molar equivalents, per 1 mol of compound (XV).

Examples of the transition metal catalyst include palladium catalysts (e.g., palladium acetate, palladium chloride, tetrakistriphenylphosphine palladium etc.), nickel catalysts (e.g., nickel chloride etc.) and the like. Where necessary, a ligand (e.g., triphenylphosphine, tri-tert-butylphosphine etc.) may be added, a metal oxide (e.g., copper oxide, silver oxide etc.) and the like may used as a cocatalyst. While the amount of the transition metal catalyst to be used varies depending on the kind, it is generally about 0.0001 molar equivalent to 1 molar equivalent, preferably about 0.01 molar equivalent to 0.5 molar equivalent, per 1 mol of compound (XV). The amount of the ligand to be used is generally about 0.0001 molar equivalent to 4 molar equivalents, preferably about 0.01 molar equivalent to 2 molar equivalents, per 1 mol of compound (XV). The amount of the cocatalyst to be used is generally about 0.0001 molar equivalent to 4 molar equivalents, preferably about 0.01 molar equivalent to 2 molar equivalents, per 1 mol of compound (XV).
Examples of the base include organic amines (e.g., trimethylamine, triethylamine, diisopropylamine, N-methylmorpholine, 1,8-diazabicyclo[5,4,0]undec-7-ene, pyridine, N,N-dimethylaniline etc.), alkali metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide etc.), metal hydrides (e.g., potassium hydride, sodium hydride etc.), alkali metal alkoxides (e.g., sodium methoxide, sodium ethoxide, sodium-tert-butoxide, potassium-tert-butoxide etc.), alkali metal disilazides (e.g., lithium disilazides, sodium disilazide, potassium disilazide etc.) and the like. Of these, alkali metal salts such as potassium carbonate, cesium carbonate, sodium phosphate, potassium phosphate and the like, alkali metal alkoxides such as sodium-tert-butoxide, potassium-tert-butoxide and the like, organic amines such as triethylamine, diisopropylamine and the like and the like are preferable. The amount of the base to be used is generally about 0.1 molar equivalent to 10 molar equivalents, preferably about 1 molar equivalent to 5 molar equivalents, per 1 mol of compound (XV).

Examples of the solvent that does not adversely influence the reaction include hydrocarbons (e.g., benzene, toluene, xylene etc.), halogenated hydrocarbons (e.g., chloroform, 1,2-dichloroethane etc.), nitriles (e.g., acetonitrile etc.), ethers (e.g., dimethoxyethane, tetrahydrofuran), alcohols (e.g., methanol, ethanol etc.), aprotic polar solvents (e.g., dimethylformamide, dimethyl sulfoxide, hexamethylphosphoramide etc.), water and a mixture thereof and the like. The amount of the solvent to be used is generally 1-fold volume to 100-fold volume, relative to compound (XV).
The reaction temperature is generally -10°C to 200°C, preferably about 0°C to 150°C. The reaction time is generally 0.5 hr to 48 hr, preferably 0.5 hr to 16 hr.
The thus-obtained compound (XVIII) can be isolated and purified by known separation and purification means, for example, concentration, concentration under reduced pressure, solvent extraction, crystallization, recrystallization, phase transfer, chromatography and the like. In addition, compound (XVIII) may be used for the next reaction without isolation.
The conversion of compound (XXVIII) to compound (XXXI) in method I in the scheme can be also carried out by this step.

### (Step 11)

This step is a step of converting compound (XV) to a compound represented by the formula (XX) or a salt thereof (hereinafter to be referred to as compound (X)) by subjecting compound (XV) to an alkoxylation with an alcohol represented by the formula: R⁵OH wherein each symbol is as defined above, or a salt thereof. This step can be carried out according to a method known per se [e.g., the method described in J. Am. Soc. Chem. 1997, vol.119, page 3395 and the like], for example, in the presence of a transition metal catalyst and a base in a solvent that does not adversely influence the reaction.
The alcohol represented by the formula: R⁵OH or a salt thereof may be a commercially available product, or can be produced from the corresponding starting material compound according to a method known per se. The amount of the alcohol or a salt thereof to be used is preferably about 1 molar equivalent to about 3 molar equivalents, per 1 mol of compound (XV).
Examples of the transition metal catalyst to be used include palladium catalysts (e.g., palladium (II) acetate, tris(dibenzylideneacetone)dipalladium (0), palladium (II) chloride, tetrakis(triphenylphosphine)palladium (0) etc.) and the like. Where necessary, a ligand (e.g., 2,2'-bis(diphenylphosphino)-1,1'-binaphthyl, 2,2'-bis(di-p-tolylphosphino)-1,1'-binaphthyl, 2-dicyclohexylphosphino-2',4',6'-triisopropyl-1,1'-biphenyl, triphenylphosphine, tri-tert-butylphosphine etc.) may be added. While the amount of the catalyst to be used varies depending on the kind, it is generally about 0.0001 to about 1 molar equivalent, preferably about 0.01 to about 0.5 molar equivalent, per 1 mol of compound (XV). The amount of the ligand to be used is generally about 0.0001 to about 4 molar equivalents, preferably about 0.01 to about 2 molar equivalents, per 1 mol of compound (XV).

Examples of the base to be used include organic amines (e.g., trimethylamine, triethylamine, diisopropylamine, N-methylmorpholine, 1,8-diazabicyclo[5.4.0]undec-7-ene, pyridine, N,N-dimethylaniline etc.), alkali metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide etc.), metal hydrides (potassium hydride, sodium hydride etc.), alkali metal alkoxides (sodium methoxide,
sodium ethoxide, sodium-tert-butoxide, potassium-tert-butoxide etc.), alkali disilazides (e.g., lithium disilazide, sodium disilazide, potassium disilazide etc.) and the like. Of these, metal hydrides (potassium hydride, sodium hydride etc.);
alkali metal alkoxides such as sodium-tert-butoxide, potassium-tert-butoxide and the like; and the like are preferable. The amount of the base to be used is generally about 0.1 to about 10 molar equivalents, preferably about 1 to about 5 molar equivalents, per 1 mol of compound (XV).
Examples of the solvent that does not adversely influence the reaction include hydrocarbons (e.g., benzene, toluene, xylene etc.), halogenated hydrocarbons (e.g., chloroform, 1,2-dichloroethane etc.), nitriles (e.g., acetonitrile etc.), ethers (e.g., dimethoxyethane, tetrahydrofuran etc.), aprotic polar solvents (e.g., N,N-dimethylformamide, dimethyl sulfoxide, hexamethylphosphoramide etc.) and a mixture thereof. The amount of the solvent to be used is generally 1-fold volume to 100-fold volume, relative to compound (XV).
The reaction temperature is generally about -10 to about 200°C, preferably about 0 to about 150°C. The reaction time is generally about 0.5 to about 48 hr, preferably about 0.5 to about 16 hr.
The thus-obtained compound (XX) can be isolated and purified by known separation and purification means, for example, concentration, concentration under reduced pressure, solvent extraction, crystallization, recrystallization, phase transfer, chromatography and the like. In addition, compound (XX) may be used for the next reaction without isolation.
The conversion of compound (XXVIII) to compound (XXX) in method H in the scheme can be also carried out by this step.

### (Step 12)

This step is a step of producing a compound represented by the formula (XXII) or a salt thereof (hereinafter to be referred to as compound (XXII)) by subjecting compound (XXI) to a tert-butoxycarbonylation reaction. The tert-butoxycarbonylation reaction can be carried out according to a conventional method, for example, using di-text-butyl dicarbonate, where necessary in the presence of a base in a solvent that does not adversely influence the reaction.
The di-tert-butyl dicarbonate may be generally, commercially available product. The amount of the di-tert-butyl dicarbonate to be used is generally about 1 molar equivalent to about 10 molar equivalents, preferably about 1 molar equivalent to about 3 molar equivalents, per 1 mol of compound (XXI).
Examples of the base include triethylamine, tributylamine, diisopropylethylamine, potassium carbonate, sodium carbonate, sodium hydride, potassium hydride and the like. The amount of the base to be used is generally about 1 molar equivalent to about 5 molar equivalents, relative to compound (XXI).
Examples of the solvent that does not adversely influence the reaction include ethers such as tetrahydrofuran and the like; halogenated hydrocarbons such as chloroform and the like; aromatic hydrocarbons such as toluene and the like; amides such as N,N-dimethylformamide and the like; sulfoxides such as dimethylsulfoxide and the like and the like. These solvents may be used in a mixture of two or more solvents at an appropriate ratio. The amount of the solvent to be used is generally 1-fold volume to 100-fold volume, relative to compound (XXI).
The reaction temperature is generally about -50°C to about 250°C, preferably 0°C to 120°C. The reaction time is generally about 0.5 to about 24 hr.
The thus-obtained compound (XXII) can be isolated and purified by known separation and purification means, for example, concentration, concentration under reduced pressure, solvent extraction, crystallization, recrystallization, phase transfer, chromatography and the like. In addition, compound (XXII) may be used for the next reaction without isolation.

### (Step 13)

This step is a step of producing a compound represented by the formula (If) or a salt thereof (hereinafter to be referred to as compound (If)) by removing the tert-butoxycarbonyl group (Boc group) of compound (XXIV). This reaction can be carried out according to a method known per se, generally by reacting compound (XXIV) with an acid in a solvent that does not adversely influence the reaction.
Examples of the acid include hydrochloric acid, hydrobromic acid, sulfuric acid, trifluoroacetic acid, trifluoromethanesulfonic acid, hydrogen chloride and the like. The amount of the acid to be used is preferably about 1 molar equivalent to about 100 molar equivalents, per 1 mol of compound (XXIV).
Examples of the solvent that does not adversely influence the reaction include alcohols (e.g., methanol etc.), ethers (e.g., tetrahydrofuran etc.), halogenated hydrocarbons (e.g., chloroform etc.), aromatic hydrocarbons (e.g., toluene etc.), amides (e.g., N,N-dimethylformamide etc.), sulfoxides (e.g., dimethyl sulfoxide etc.), esters (e.g., ethyl acetate etc.) and the like. These solvents may be used in a mixture of two or more solvents at an appropriate ratio. The amount of the solvent to be used is generally 1-folk volume to 100-fold volume, relative to compound (XXIV).
The reaction temperature is generally about -50°C to about 250°C, preferably 0°C to 120°C. The reaction time is generally about 0.5 hr to about 24 hr.
The thus-obtained compound (If) can be isolated and purified by known separation and purification means, for example, concentration, concentration under reduced pressure, solvent extraction, crystallization, recrystallization, phase transfer, chromatography and the like.

### (Step 14)

This step is a step of producing a compound represented by the formula (XXVI) or a salt thereof (hereinafter to be referred to as compound (XXVI)) by reacting compound (XXV) with a dihalogenoalkane in the presence of a base. This step can be carried out according to a method known per se [e.g., the method described in Biorg. Med. Chem. Lett. 1999, vol.9, page 2329 and the like], for example, in the presence of a dihalogenoalkane and a base in a solvent that does not adversely influence the reaction.
Compound (XXV) may be a commercially available product, or can be produced from the corresponding starting material compound according to a method known per se.
Examples of the dihalogenoalkane include 1,2-dibromoethane, 1,3-dibromopropane, 1,4-dibromobutane, 1,5-dibromopentane, 1,6-dibromohexane, 1,2-dichloroethane, 1,3-dichloropropane, 1,4-dichlorobutane, 1,5-dichloropentane, 1,6-dichlorohexane and the like. The dihalogenoalkane may be generally, commercially available product. While the amount of the dihalogenoalkane to be used varies depending on the kind, it is generally about 1 molar equivalent to about 3 molar equivalents, preferably about 1 molar equivalent to about 1.3 molar equivalents, per 1 mol of compound (XXV).

Preferable examples of the base include organic amines (e.g., trimethylamine, triethylamine, diisopropylamine, N-methylmorpholine, 1,8-diazabicyclo[5.4.0]undec-7-ene, pyridine, N,N-dimethylaniline etc.), alkali metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide etc.), metal hydrides (e.g., potassium hydride, sodium hydride etc.), alkali metal alkoxides (e.g., sodium methoxide, sodium ethoxide, sodium-tert-butoxide, potassium-tert-butoxide etc.), alkali metal disilazides (e.g., lithium disilazide, sodium disilazide, potassium disilazide etc.) and the like. Of these, alkali metal salt such as potassium carbonate, cesium carbonate, sodium phosphate, potassium phosphate and the like; alkali metal alkoxide such as sodium-tert-butoxide, potassium-tert-butoxide and the like; organic amines such as triethylamine, diisopropylamine etc. and the like are preferable. The amount of the base to be used is generally about 0.1 molar equivalent to about 10 molar equivalents, preferably about 1 molar equivalent to about 5 molar equivalents, per 1 mol of compound (XXV).
Examples of the solvent that does not adversely influence the reaction include hydrocarbons (e.g., benzene, toluene, xylene etc.), halogenated hydrocarbons (e.g., chloroform, 1,2-dichloroethane etc.), nitriles (e.g., acetonitrile etc.), ethers (e.g., dimethoxyethane, tetrahydrofuran etc.), aprotic polar solvents (e.g., N,N-dimethylformamide, dimethyl sulfoxide, hexamethyl phosphoramide etc.) and a mixture thereof and the like. The amount of the solvent to be used is generally 1-fold volume to 100-fold volume, relative to compound (XXV).
The reaction temperature is generally about -10°C to about 200°C, preferably about 0°C to about 150°C. The reaction time is generally about 0.5 hr to about 48 hr, preferably about 0.5 hr to about 16 hr.
The thus-obtained compound (XXVI) can be isolated and purified by known separation and purification means, for example, concentration, concentration under reduced pressure, solvent extraction, crystallization, recrystallization, phase transfer, chromatography and the like. In addition, compound (XXVI) may be used for the next reaction without isolation.

### (Step 15)

This step is a step of producing a compound represented by the formula (XXVII) or a salt thereof (hereinafter to be referred to as compound (XXVII)) by reacting compound (XXVI) with propiolic amide in the presence of a base. This step can be carried out according to a method known per se [e.g., the method described in patent document EP1595881 and the like], for example, in the presence of propiolic amide and a base in a solvent that does not adversely influence the reaction.
The propiolic amide may be generally commercially available product. While the amount of the propiolic amide to be used varies depending on the kind of compound (XXVI), it is generally about 1 molar equivalent to about 10 molar equivalents, preferably about 1 molar equivalent to about 3 molar equivalents, per 1 mol of compound (XXVI).
Examples of the base include organic amines (e.g., pyrrolidine, piperidine, morpholine, diethylamine, trimethylamine, triethylamine, diisopropylamine, N-methylmorpholine, 1,8-diazabicyclo[5.4.0]undec-7-ene, pyridine, N,N-dimethylaniline etc.), alkali metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide etc.), metal hydrides (e.g., potassium hydride, sodium hydride etc.), alkali metal alkoxides (e.g., sodium methoxide, sodium ethoxide, sodium-tert-butoxide, potassium-tert-butoxide etc.), alkali metal disilazides (e.g., lithium disilazide, sodium disilazide, potassium disilazide etc.) and the like. Of these, organic amines such as pyrrolidine, piperidine, morpholine, diethylamine and the like and the like are preferable. The amount of the base to be used is generally about 0.1 molar equivalent to about 10 molar equivalents, preferably about 1 molar equivalent to about 5 molar equivalents, per 1 mol of compound (XXVI).
Examples of the solvent that does not adversely influence the reaction include hydrocarbons (e.g., benzene, toluene, xylene etc.), halogenated hydrocarbons (e.g., chloroform, 1,2-dichloroethane etc.), nitriles (e.g., acetonitrile etc.), ethers (e.g., dimethoxyethane, tetrahydrofuran etc.), aprotic polar solvents (e.g., N,N-dimethylformamide, dimethyl sulfoxide, hexamethylphosphoramide etc.) and a mixture thereof and the like. The amount of the solvent to be used is generally 1-fold volume to 100-fold volume, relative to compound (XXVI).
The reaction temperature is generally about -10°C to about 200°C, preferably about 0°C to about 150°C. The reaction time is generally about 0.5 hr to about 48 hr, preferably about 0.5 hr to about 16 hr.
The thus-obtained compound (XXVII) can be isolated and purified by known separation and purification means, for example, concentration, concentration under reduced pressure, solvent extraction, crystallization, recrystallization, phase transfer, chromatography and the like. In addition, compound (XXVII) may be used for the next reaction without isolation.

### (Step 16)

This step is a step of converting compound (XXVII) to a compound represented by the formula (XXVIII) or a salt thereof (hereinafter to be referred to as compound (XXVIII)) by reacting compound (XXVII) with a halogenating agent (in the scheme, halogen is shown by a chlorine atom for convenience). The halogenation can be carried out according to a method known per se [e.g., the method described in patent document EP1595881 and the like], for example, in the presence of a halogenating agent in a solvent that does not adversely influence the reaction.
Examples of the halogenating agent include thionyl chloride, phosphorus pentachloride, phosphorus oxychloride, oxalyl chloride and the like. The amount thereof to be used is generally about 1 to about 10 molar equivalents, preferably about 1 to about 3 molar equivalents, per 1 mol of compound (XXVII).
Examples of the solvent that does not adversely influence the reaction include hydrocarbons (e.g., benzene, toluene, xylene etc.), halogenated hydrocarbons (e.g., chloroform, 1,2-dichloroethane etc.), nitriles (e.g., acetonitrile etc.), ethers (e.g., dimethoxyethane, tetrahydrofuran etc.), aprotic polar solvents (e.g., N,N-dimethylformamide, dimethyl sulfoxide, hexamethylphosphoramide etc.) and a mixture thereof. The amount of the solvent to be used is generally 1-fold volume to 100-fold volume, relative to compound (XXVII).
The reaction temperature is generally about -10 to about 200°C, preferably about 0 to about 150°C. The reaction time is generally about 0.5 to about 48 hr, preferably about 0.5 to about 16 hr.
The thus-obtained compound (XXVIII) can be isolated and purified by known separation and purification means, for example, concentration, concentration under reduced pressure, solvent extraction, crystallization, recrystallization, phase transfer, chromatography and the like. In addition, compound (XXVIII) may be used for the next reaction without isolation.

In addition, compound (Ix) can be also produced, for example, according to Method J, Method K, Method L, Method M, Method N, Method O, Method P, Method Q or Method R. Unless otherwise specified, each symbol in the chemical formulas of each scheme is as defined above. The compounds (Ij)-(Ir), which are the final products of each scheme, are all encompassed in compound (Ix). The compounds of each scheme may form salts and examples thereof include those similar to the salts of compound (Ix), and the like.
For example, when compound (Ix) is a compound represented by the formula (1j) or a salt thereof (hereinafter to be referred to as compound (1j)), the compound can be produced according to the following Method J.

### [Method J]

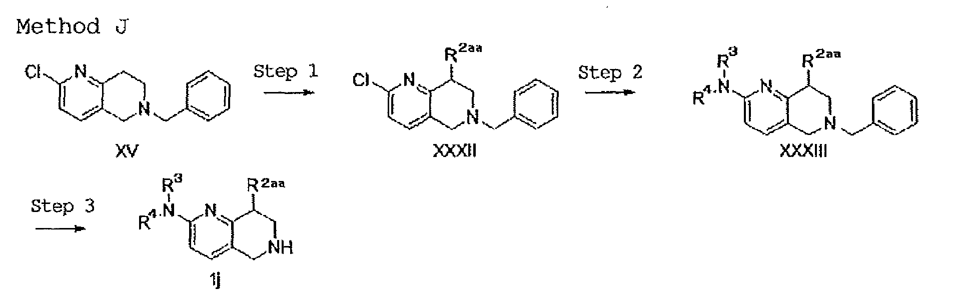

wherein R^{2aa} is an optionally substituted alkyl group, and the other symbols are each as defined above.
The "optionally substituted alkyl group" for R^{2aa} is as defined for the "optionally substituted alkyl group" for R^{2a}.

### (Step 1)

This step is a step of producing a compound represented by the formula (XXXII) or a salt thereof (hereinafter to be referred to as compound (XXXII)) by reacting compound (XV) with a compound represented by the formula: R^{2aa}-X wherein X is a leaving group, or a salt thereof.

Examples of the leaving group for X include halogen atoms (a chlorine atom, a bromine atom, an iodine atom etc.), substituted sulfonyloxy groups (C₁₋₆ alkylsulfonyloxy groups such as methanesulfonyloxy, ethanesulfonyloxy and the like; C₆₋₁₄ arylsulfonyloxy groups such as benzenesulfonyloxy, p-toluenesulfonyloxy and the like; C₇₋₁₆ aralkylsulfonyloxy groups such as benzylsulfonyloxy and the like, etc.), acyloxy groups (acetoxy group, benzoyloxy group etc.), an oxy group substituted by a hetero ring or aryl group (succinimidoxy, benzotriazoloxy, quinolyloxy, 4-nitrophenoxy etc.), a hetero ring (imidazole etc.) and the like, particularly a halogen atom is preferable.
The amount of the compound represented by the formula: R^{2aa}-X to be used is, for example, about 1 to 100 molar equivalents, preferably about 1 to 10 molar equivalents, per 1 mol of compound (XV).
This reaction can be generally carried out by reacting compound (XV) with a compound represented by the formula: R^{2aa}-X in a solvent in the presence of a base. Examples of the solvent include hydrocarbons (e.g., benzene, toluene, xylene etc.), halogenated hydrocarbons (e.g., chloroform, 1,2-dichloroethane etc.), nitriles (e.g., acetonitrile etc.), ethers (e.g., dimethoxyethane, tetrahydrofuran etc.), aprotic polar solvents (e.g., N,N-dimethylformamide, dimethyl sulfoxide, hexamethylphosphoramide etc.) and the like. These solvents may be used in a mixture at an appropriate ratio. The amount of the solvent to be used is generally 1-fold volume to 100-fold volume, relative to compound (XV).
Examples of the base include organic bases (trimethylamine, triethylamine, N-methylmorpholine, pyridine, picoline, N,N-dimethylaniline etc.), inorganic bases (potassium carbonate, sodium carbonate, potassium hydroxide, sodium hydroxide etc.), metal amides (lithium hexamethyl disilazide, sodium hexamethyl disilazide, potassium hexamethyl disilazide, lithium diisopropylamide etc.), metal hydrides (lithium hydride, sodium hydride, calcium hydride) and the like. The amount of the base to be used is, for example, about 1 to 100 molar equivalents, preferably about 1 to 10 molar equivalents, per 1 mol of compound (XV).
Where necessary, an additive may also be added to promote the reaction. Examples of the additive include iodides (sodium iodide, potassium iodide etc.) and the like. The amount thereof to be used is about 0.1 to 10 molar equivalents, preferably about 0.1 to 5 molar equivalents, per 1 mol of compound (XV).
The reaction temperature is generally -10 to 200°C, preferably about 0 to 110°C. The reaction time is generally 0.5 to 48 hr, preferably 0.5 to 16 hr.
The thus-obtained compound (XXXII) can be isolated and purified by known separation and purification means, for example, concentration, concentration under reduced pressure, solvent extraction, crystallization, recrystallization, phase transfer, chromatography and the like. In addition, compound (XXXII) may be used for the next reaction without isolation.

### (Step 2)

This step is a step of producing a compound represented by the formula (XXXIII) or a salt thereof (hereinafter to be referred to as compound (XXXIII)) by reacting compound (XXXII) with an amine represented by the formula: HNR³R⁴ wherein each symbol is as defined above, or a salt thereof. This step can be carried out in the same manner as in the method described in Method A, Step 6.

### (Step 3)

This step is a step of producing compound (1j) by removing the benzyl group of compound (XXXIII). This step can be carried out in the same manner as in the method described in Method A, Step 8.

For example, when compound (Ix) is a compound represented by the formula (1k) or a salt thereof (hereinafter to be referred to as compound (1k)), the compound can be produced according to the following Method K.

### [Method K]

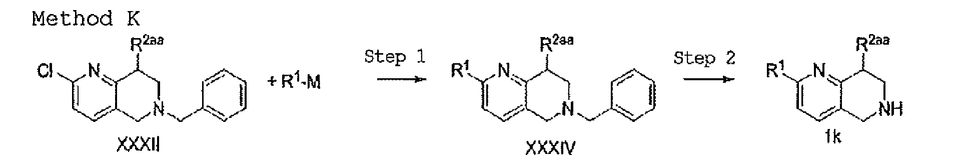

wherein each symbol is as defined above.

### (Step 1)

This step is a step of producing a compound represented by the formula (XXXIV) or a salt thereof (hereinafter to be referred to as compound (XXXIV)) by subjecting compound (XXXII) to a coupling reaction with a compound represented by the formula: R¹-M wherein R¹-M is R¹-B(OH)₂ or R¹-MgX (X is a halogen atom) and the like, and the other symbols are each as defined above, or a salt thereof.
The coupling reaction can be carried out according to a method known per se [e.g., the method described in Chemical Reviews, 1995, vol.95, page 2457 and the like], for example, in the presence of a transition metal catalyst in a solvent that does not adversely influence the reaction. Where necessary, a base may be added.
The compound represented by the formula: R¹-M may be a commercially available product, or can be produced from the corresponding starting material compound according to a method known per se. The amount of the compound represented by the formula: formula: R¹-M or a salt thereof to be used is generally about 1 molar equivalent to about 10 molar equivalents, preferably about 1 molar equivalent to about 5 molar equivalents, per 1 mol of compound (XXXII).
Examples of the transition metal catalyst include palladium catalysts (e.g., palladium acetate, palladium chloride, tetrakistriphenylphosphinepalladium etc.), nickel catalysts (e.g., nickel chloride etc.), iron catalysts (iron chloride, iron (III) acetylacetonate) and the like. Where necessary, a ligand (e.g., triphenylphosphine, tri-tert-butylphosphine etc.) may be added, and a metal oxide (e.g., copper oxide, silver oxide etc.) and the like may be used as a cocatalyst. While the amount of the transition metal catalyst to be used varies depending on the kind, it is generally about 0.0001 molar equivalent to 1 molar equivalent, preferably about 0.01 molar equivalent to 0.5 molar equivalent, per 1 mol of compound (XXXII). The amount of the ligand to be used is generally about 0.0001 molar equivalent to 4 molar equivalents, preferably about 0.01 molar equivalent to 2 molar equivalents, per 1 mol of compound (XXXII). The amount of the cocatalyst to be used is generally about 0.0001 molar equivalent to 4 molar equivalents, preferably about 0.01 molar equivalent to 2 molar equivalents, per 1 mol of compound (XXXII).
Examples of the base include organic amines (e.g., trimethylamine, triethylamine, diisopropylamine, N-methylmorpholine, 1,8-diazabicyclo[5,4,0]undec-7-ene, pyridine, N,N-dimethylaniline etc.), alkali metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide etc.), metal hydrides (e.g., potassium hydride, sodium hydride etc.), alkali metal alkoxides (e.g., sodium methoxide, sodium ethoxide, sodium-tert-butoxide, potassium-tert-butoxide etc.), alkali metal disilazides (e.g., lithium disilazide, sodium disilazide, potassium disilazide etc.) and the like. Of these, alkali metal salts such as potassium carbonate, cesium carbonate, sodium phosphate, potassium phosphate and the like, alkali metal alkoxides such as sodium-tert-butoxide, potassium-tert-butoxide and the like, organic amines such as triethylamine, diisopropylamine and the like and the like are preferable. The amount of the base to be used is generally about 0.1 molar equivalent to 10 molar equivalents, preferably about 1 molar equivalent to 5 molar equivalents, per 1 mol of compound (XXXII).
Examples of the solvent that does not adversely influence the reaction include hydrocarbons (e.g., benzene, toluene, xylene etc.), halogenated hydrocarbons (e.g., chloroform, 1,2-dichloroethane etc.), nitriles (e.g., acetonitrile etc.), ethers (e.g., dimethoxyethane, tetrahydrofuran etc.), aprotic polar solvents (e.g., N,N-dimethylformamide, dimethyl sulfoxide, hexamethylphosphoramide etc.) and a mixture thereof and the like. The amount of the solvent to be used is generally 1-fold volume to 100-fold volume, relative to compound (XXXII).
The thus-obtained compound (XXXIV) can be isolated and purified by known separation and purification means, for example, concentration, concentration under reduced pressure, solvent extraction, crystallization, recrystallization, phase transfer, chromatography and the like. In addition, compound (XXXIV) may be used for the next reaction without isolation.

### (Step 2)

This step is a step of producing compound (1k) by removing the benzyl group of compound (XXXIV). This step can be carried out in the same manner as in the method described in Method A, Step 8.

For example, when compound (Ix) is a compound represented by the formula (11) or a salt thereof (hereinafter to be referred to as compound (11)), the compound can be produced according to the following Method L.

### [Method L]

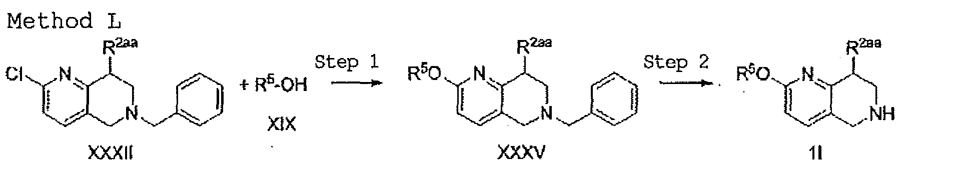

wherein each symbol is as defined above.

### (Step 1)

This step is a step of producing a compound represented by the formula (XXXV) or a salt thereof (hereinafter to be referred to as compound (XXXV)) by subjecting compound (XXXII) to an alkoxylation with a compound represented by the formula (XIX) or a salt thereof (hereinafter to be referred to as compound (XIX)). This step can be carried out in the same manner as in the method described in Method E, Step 11.

### (Step 2)

This step is a step of producing compound (11) by removing the benzyl group of compound (XXXV). This step can be carried out in the same manner as in the method described in Method A, Step 8.

For example, when compound (Ix) is a compound represented by the formula (1m) or a salt thereof (hereinafter to be referred to as compound (1m)), the compound can be produced according to the following Method M.

### [Method M]

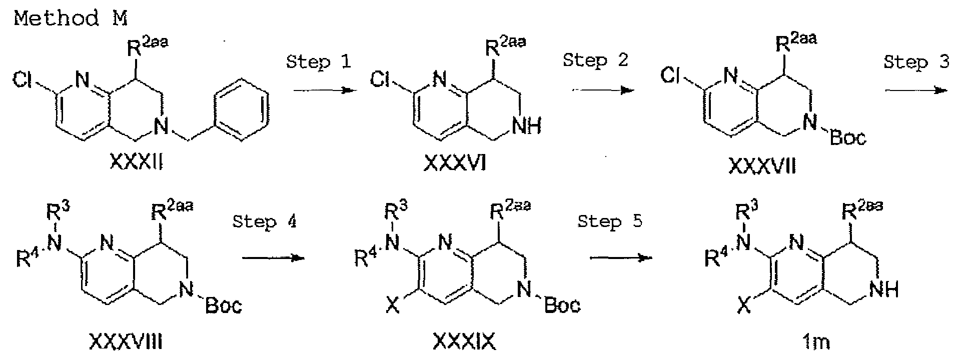

wherein X is a halogen atom, and the other symbols are each as defined above.

### (Step 1)

This step is a step of producing a compound represented by the formula (XXXVI) or a salt thereof (hereinafter to be referred to as compound (XXXVI)) by removing the benzyl group of compound (XXXII). This step can be carried out in the same manner as in the method described in Method A, Step 8.

### (Step 2)

This step is a step of producing a compound represented by the formula (XXXVII) or a salt thereof (hereinafter to be referred to as compound (XXXVII)) by subjecting compound (XXXVI) to a tert-butoxycarbonylation reaction. This step can be carried out in the same manner as in the method described in Method F, Step 12.

### (Step 3)

This step is a step of producing a compound represented by the formula (XXXVIII) or a salt thereof (hereinafter to be referred to as compound (XXXVIII)) by reacting compound (XXXVII) with an amine represented by the formula: HNR³R⁴ wherein each symbol is as defined above, or a salt thereof. This step can be carried out in the same manner as in the method described in Method A, Step 6.

### (Step 4)

This step is a step of converting compound (XXXVIII) to a compound represented by the formula (XXXIX) or a salt thereof (hereinafter to be referred to as compound (XXXIX)) by reacting compound (XXXVIII) with a halogenating agent. The halogenation can be carried out according to a method known per se, for example, in the presence of a halogenating agent in a solvent that does not adversely influence the reaction.
Examples of the halogenating agent include halogen elements, N-halogenated succinimide, N-halogenated pyridinium salt, N-fluoro-N'-chloromethyltriethylenediamine bis(tetrafluoroboric acid) and the like. The amount thereof to be used is generally about 1 to about 10 molar equivalents, preferably about 1 to about 3 molar equivalents, per 1 mol of compound (XXXVIII).
Examples of the solvent that does not adversely influence the reaction include hydrocarbons (e.g., benzene, toluene, xylene etc.), halogenated hydrocarbons (e.g., chloroform, 1,2-dichloroethane etc.), nitriles (e.g., acetonitrile etc.), ethers (e.g., dimethoxyethane, tetrahydrofuran etc.), aprotic polar solvents (e.g., N,N-dimethylformamide, dimethyl sulfoxide, hexamethylphosphoramide etc.) and a mixture thereof. The amount of the solvent to be used is generally 1-fold volume to 100-fold volume, relative to compound (XXXVIII).
The reaction temperature is generally about -10 to about 200°C, preferably about 0 to about 150°C. The reaction time is generally about 0.5 to about 48 hr, preferably about 0.5 to about 16 hr.
The thus-obtained compound (XXXIX) can be isolated and purified by known separation and purification means, for example, concentration, concentration under reduced pressure, solvent extraction, crystallization, recrystallization, phase transfer, chromatography and the like. In addition, compound (XXXIX) may be used for the next reaction without isolation.

### (Step 5)

This step is a step of producing compound (1m) by removing the tert-butoxycarbonyl group (Boc group) of compound (XXXIX). This step can be carried out in the same manner as in the method described in Method F, Step 13.

For example, when compound (Ix) is a compound represented by the formula (1n) or a salt thereof (hereinafter to be referred to as compound (1n)), the compound can be produced according to the following Method N.

### [Method N]

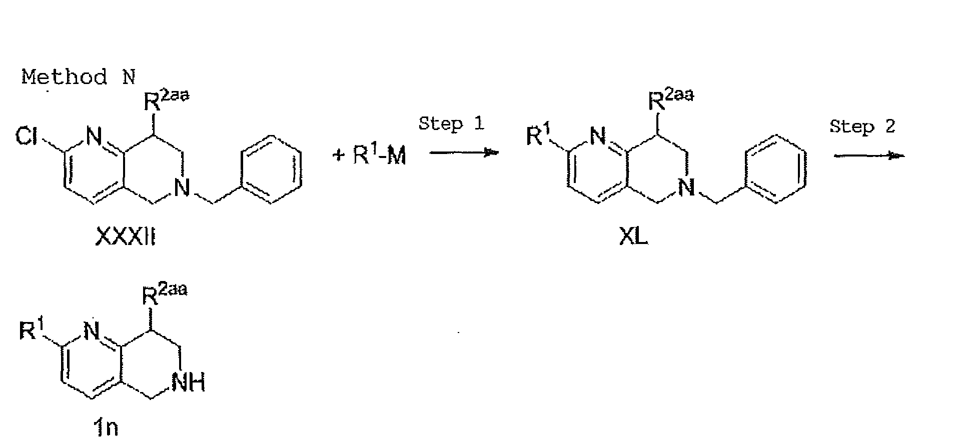

wherein each symbol is as defined above.

### (Step 1)

This step is a step of producing a compound represented by the formula (XL) or a salt thereof (hereinafter to be referred to as compound (XL)) by subjecting compound (XXXII) to a coupling reaction with a compound represented by the formula: R¹-M wherein each symbol is as defined above, or a salt thereof. This step can be carried out in the same manner as in the method described in Method K, Step 1.

### (Step 2)

This step is a step of producing compound (1n) by removing the benzyl group of compound (XL). This step can be carried out in the same manner as in the method described in Method A, Step 8.

For example, when compound (Ix) is a compound represented by the formula (1o) or a salt thereof (hereinafter to be referred to as compound (1o)), the compound can be produced according to the following Method O.

### [Method O]

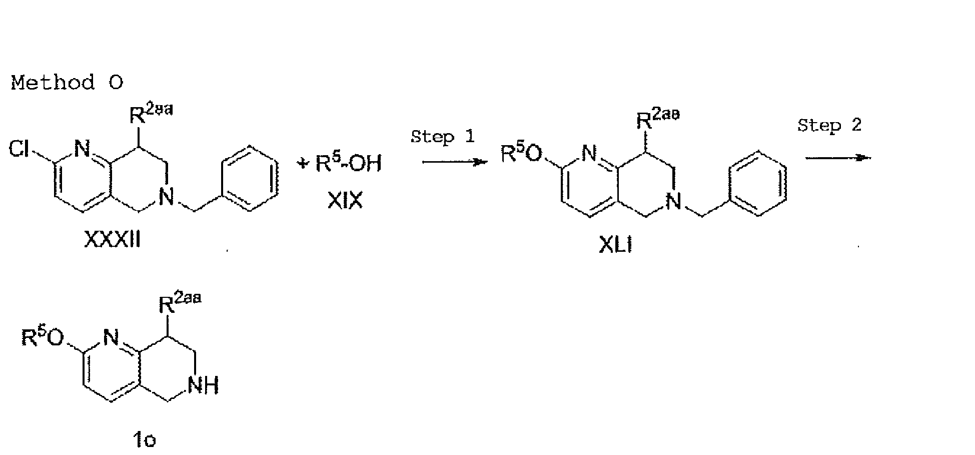

wherein each symbol is as defined above.

### (Step 1)

This step is a step of producing a compound represented by the formula (XLI) or a salt thereof (hereinafter to be referred to as compound (XLI)) by subjecting compound (XXXII) to an alkoxylation with compound (XIX). This step can be carried out in the same manner as in the method described in Method E, Step 11.

### (Step 2)

This step is a step of producing compound (1o) by removing the benzyl group of compound (XLI). This step can be carried out in the same manner as in the method described in Method A, Step 8.

For example, when compound (Ix) is a compound represented by the formula (1p) or a salt thereof (hereinafter to be referred to as compound (1p)), the compound can be produced according to the following Method P.

### [Method P]

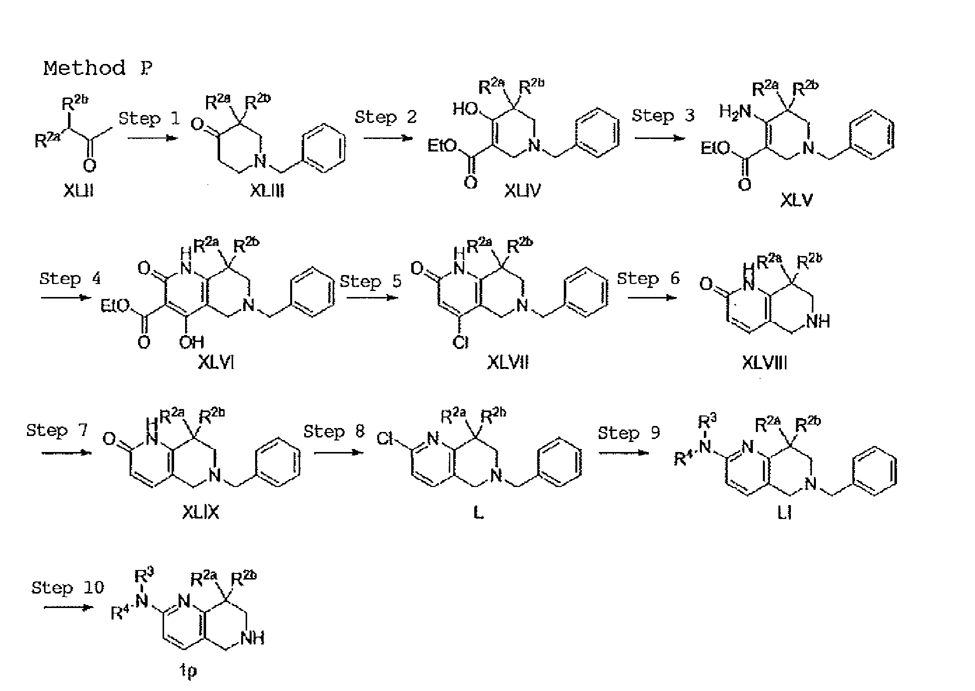

wherein each symbol is as defined above.

### (Step 1)

This step is a step of producing a compound represented by the formula (XLIII) or a salt thereof (hereinafter to be referred to as compound (XLIII)) by reacting a compound represented by the formula (XLII) or a salt thereof (hereinafter to be referred to as compound (XLII)) with benzylamine and formaldehyde in the presence of an acid and a base.
The reaction is generally carried out in a solvent that does not adversely influence the reaction. Examples of the solvent include aromatic hydrocarbons (toluene, xylene etc.), aliphatic hydrocarbons (heptane, hexane etc.), halogenated hydrocarbons (chloroform, dichloromethane etc.), ethers (diethyl ether, tetrahydrofuran, dioxane etc.), alcohols (methanol, ethanol, 2-propanol, butanol, benzyl alcohol etc.), nitriles (acetonitrile etc.), amides (N,N-dimethylformamide etc.), sulfoxides (dimethyl sulfoxide etc.) and the like. These solvents may be used in a mixture of two or more solvents at an appropriate ratio. The amount of the formaldehyde and benzylamine to be used is, for example, about 1 to 100 molar equivalents, preferably about 1 to 5 molar equivalents, per 1 mol of compound (XLII), respectively.
Examples of the acid to be used include mineral acids (hydrochloric acid, hydrobromic acid, sulfuric acid etc.), carboxylic acids (formic acid, acetic acid, propionic acid, trifluoroacetic acid etc.), sulfonic acids (methanesulfonic acid, p-toluenesulfonic acid etc.) and Lewis acids (aluminum chloride, zinc chloride, zinc bromide, boron trifluoride, titanium chloride etc.). Of these, hydrochloric acid, hydrobromic acid, sulfuric acid and the like are preferable. The amount of the acid to be used is, for example, about 0.1 to 10 molar equivalents, preferably about 1 to 5 molar equivalents, per 1 mol of compound (XLII).
Examples of the base to be used include organic amines (e.g., trimethylamine, triethylamine, diisopropylamine, N-methylmorpholine, 1,8-diazabicyclo[5.4.0]undec-7-ene, pyridine, N,N-dimethylaniline etc.), alkali metal salts (e.g., sodium hydrogen carbonate, potassium hydrogen carbonate, sodium carbonate, potassium carbonate, cesium carbonate, sodium phosphate, potassium phosphate, sodium hydroxide, potassium hydroxide etc.), metal hydrides (potassium hydride, sodium hydride etc.), alkali metal alkoxides (sodium methoxide, sodium ethoxide, sodium-tert-butoxide, potassium-tert-butoxide etc.), alkali disilazides (e.g., lithium disilazide, sodium disilazide, potassium disilazide etc.) and the like. The amount of the base to be used is generally about 0.1 to about 10 molar equivalents, preferably about 1 to about 5 molar equivalents, per 1 mol of compound (XLII).
The reaction temperature is generally about 0°C to 200°C, preferably about 20°C to 150°C. The reaction time is generally 0.5 to 48 hr, preferably 0.5 to 24 hr.
The thus-obtained compound (XLIII) can be isolated and purified by known separation and purification means, for example, concentration, concentration under reduced pressure, solvent extraction, crystallization, recrystallization, phase transfer, chromatography and the like. In addition, compound (XLIII) may be used for the next reaction without isolation.

### (Step 2)

This step is a step of producing a compound represented by the formula (XLIV) or a salt thereof (hereinafter to be referred to as compound (XLIV)) by reacting compound (XLIII) with an alkoxycarbonylating agent. This reaction can be carried out according to a method known per se, generally, in the presence of a base, and where necessary, in a solvent that does not adversely influence the reaction.

Examples of the alkoxycarbonylating agent include dimethyl carbonate, diethyl carbonate, methyl chloroformate, ethyl chloroformate and the like. The amount thereof to be used is about 1 to about 10 molar equivalents, preferably about 1 to about 3 molar equivalents, per 1 mol of compound (XLIII).

Examples of the solvent include hydrocarbons (e.g., benzene, toluene etc.), alcohols (e.g., methanol, ethanol etc.), ethers (e.g., diethyl ether, dioxane, tetrahydrofuran etc.), esters (e.g., ethyl acetate etc.), halogenated hydrocarbons (e.g., chloroform, dichloromethane etc.), amides (e.g., N,N-dimethylformamide etc.), aromatic amines (e.g., pyridine etc.) and the like. These solvents may be used in a mixture of two or more solvents at an appropriate ratio. The amount of the solvent to be used is generally 1-fold volume to 100-fold volume, relative to compound (XLIII).

Examples of the base include alkali metal hydroxides (e.g., sodium hydroxide, potassium hydroxide etc.), hydrogen carbonates (e.g., sodium hydrogen carbonate, potassium hydrogen carbonate etc.), carbonates (e.g., sodium carbonate, potassium carbonate etc.), acetates (e.g., sodium acetate etc.), tertiary amines (e.g., trimethylamine, triethylamine, N-methylmorpholine etc.), aromatic amines (e.g., pyridine, picoline, N,N-dimethylaniline etc.) and the like. Of these, sodium hydride, sodium ethoxide and the like are preferable. The amount of the base to be used is generally about 1 molar equivalent to about 100 molar equivalents, preferably about 1 molar equivalent to about 5 molar equivalents, per 1 mol of compound (XLIII).
The reaction temperature is generally about -80C to about 150°C, preferably about 0°C to about 100°C. The reaction time is generally about 0.1 hr to about 48 hr, preferably about 0.5 hr to about 16 hr.
The thus-obtained compound (XLIV) can be isolated and purified by known separation and purification means, for example, concentration, concentration under reduced pressure, solvent extraction, crystallization, recrystallization, phase transfer, chromatography and the like. In addition, compound (XLIV) may be used for the next reaction without isolation.

### (Step 3)

This step is a step of producing a compound represented by the formula (XLV) or a salt thereof (hereinafter to be referred to as compound (XLV)) by subjecting compound (XLIV) to an enamine-forming reaction. In the enamine-forming reaction, compound (XLIV) is condensed with ammonia or a salt thereof to give an enamine.
The enamine-forming reaction is generally carried out in a solvent that does not adversely influence the reaction. Examples of the solvent include aromatic hydrocarbons (toluene, xylene etc.), aliphatic hydrocarbons (heptane, hexane etc.), halogenated hydrocarbons (chloroform, dichloromethane etc.), ethers (diethyl ether, tetrahydrofuran, dioxane etc.), alcohols (methanol, ethanol, 2-propanol, butanol, benzyl alcohol etc.), nitriles (acetonitrile etc.), amides (N,N-dimethylformamide etc.), sulfoxides (dimethyl sulfoxide etc.) and the like. These solvents may be used in a mixture of two or more solvents at an appropriate ratio. The amount of the solvent to be used is generally 1-fold volume to 100-fold volume, relative to compound (XLIV).
Where necessary, a catalyst may also be added to advantageously perform the reaction. Examples of the catalyst include mineral acids (hydrochloric acid, hydrobromic acid, sulfuric acid etc.), carboxylic acids (formic acid, acetic acid, propionic acid, trifluoroacetic acid etc.), sulfonic acids (methanesulfonic acid, p-toluenesulfonic acid etc.), Lewis acids (aluminum chloride, zinc chloride, zinc bromide, boron trifluoride, titanium chloride etc.), acetates (sodium acetate, potassium acetate etc.) and molecular sieves (molecular sieves 3A, 4A, 5A etc.). The amount of the catalyst to be used is, for example, about 0.01 to 50 molar equivalents, preferably about 0.1 to 10 molar equivalents, per 1 mol of compound (XLIV).
The reaction temperature is generally about 0°C to 200°C, preferably about 20°C to 150°C. The reaction time is generally 0.5 to 48 hr, preferably 0.5 to 24 hr.
The thus-obtained compound (XLV) can be isolated and purified by known separation and purification means, for example, concentration, concentration under reduced pressure, solvent extraction, crystallization, recrystallization, phase transfer, chromatography and the like. In addition, compound (XLV) may be used for the next reaction without isolation.

### (Step 4)

This step is a step of converting a compound represented by the formula (XLVI) or a salt thereof (hereinafter to be referred to as compound (XLVI)) by subjecting compound (XLV) to an acylation reaction with an alkyl halogenated formylacetate, and then a cyclization reaction.
The acylation reaction can be carried out according to a method known per se, generally, in the presence of a base, and where necessary in a solvent that does not adversely influence the reaction.
Examples of the alkyl halogenated formylacetate include ethyl chloroformylacetate, ethyl bromoformylacetate, methyl chloroformylacetate, methyl bromoformylacetate and the like. The amount thereof to be used is about 1 to about 10 molar equivalents, preferably about 1 to about 3 molar equivalents, per 1 mol of compound (XLV).
Examples of the base include inorganic bases (alkali metal hydroxides such as lithium hydroxide, sodium hydroxide, potassium hydroxide and the like, alkali metal hydrogen carbonates such as sodium hydrogen carbonate, potassium hydrogen carbonate and the like, alkali metal carbonates such as sodium carbonate, potassium carbonate and the like), metal alkoxides (sodium methoxide, sodium ethoxide, potassium methoxide, potassium ethoxide etc.), organic bases (amines such as trimethylamine, triethylamine, diisopropylethylamine and the like, aromatic amines such as pyridine, 4-dimethylaminopyridine and the like, etc.) and the like. Of these, triethylamine, diisopropylethylamine and the like are preferable. While the amount of the base to be used varies depending on the kind of the solvent, the other reaction conditions, it is generally about 1 to 100 molar equivalents, preferably about 1 to 20 molar equivalents, per 1 mol of compound (XLV).
Examples of the solvent that does not adversely influence the reaction include aromatic hydrocarbons (toluene, xylene etc.), aliphatic hydrocarbons (heptane, hexane etc.), halogenated hydrocarbons (chloroform, dichloromethane etc.), ethers (diethyl ether, tetrahydrofuran, dioxane etc.), nitriles (acetonitrile etc.), amides (N,N-dimethylformamide etc.), sulfoxides (dimethyl sulfoxide etc.) and the like. These solvents may be used in a mixture of two or more solvents at an appropriate ratio. The amount of the solvent to be used is generally 1-fold volume to 100-fold volume, relative to compound (XLV).
The cyclization reaction can be carried out according to a method known per se, generally, in the presence of a base, and where necessary in a solvent that does not adversely influence the reaction.
Examples of the base include inorganic bases (alkali metal hydroxides such as lithium hydroxide, sodium hydroxide, potassium hydroxide and the like, alkali metal hydrogen carbonates such as sodium hydrogen carbonate, potassium hydrogen carbonate and the like, alkali metal carbonates such as sodium carbonate, potassium carbonate and the like), metal alkoxides (sodium methoxide, sodium ethoxide, potassium methoxide, potassium ethoxide etc.), organic bases (amines such as trimethylamine, triethylamine, diisopropylethylamine and the like, aromatic amines such as pyridine, 4-dimethylaminopyridine and the like, etc.) and the like. Of these, sodium methoxide, sodium ethoxide, potassium methoxide, potassium ethoxide and the like are preferable. While the amount of the base to be used varies depending on the kind of the solvent and the other reaction conditions, it is generally about 1 to 100 molar equivalents, preferably about 1 to 20 molar equivalents, per 1 mol of compound (XLV).
Examples of the solvent that does not adversely influence the reaction include aromatic hydrocarbons (toluene, xylene etc.), aliphatic hydrocarbons (heptane, hexane etc.), halogenated hydrocarbons (chloroform, dichloromethane etc.), ethers (diethyl ether, tetrahydrofuran, dioxane etc.), nitriles (acetonitrile etc.), amides (N,N-dimethylformamide etc.), sulfoxides (dimethyl sulfoxide etc.) and the like. These solvents may be used in a mixture of two or more solvents at an appropriate ratio. The amount of the solvent to be used is generally 1-fold volume to 100-fold volume, relative to compound (XLV).
The thus-obtained compound (XLVI) can be isolated and purified by known separation and purification means, for example, concentration, concentration under reduced pressure, solvent extraction, crystallization, recrystallization, phase transfer, chromatography and the like. In addition, compound (XLVI) may be used for the next reaction without isolation.

### (Step 5)

This step is a step of converting compound (XLVI) to a compound represented by the formula (XLVII) or a salt thereof (hereinafter to be referred to as compound (XLVII)) by subjecting compound (XLVI) to hydrolysis, and halogenating the resulting compound with a halogenating agent (in the scheme, halogen is shown by a chlorine atom for convenience).
The hydrolysis can be carried out according to a method known per se, generally, in the presence of a base, and where necessary in a solvent that does not adversely influence the reaction.
Examples of the base include inorganic bases (alkali metal hydroxides such as lithium hydroxide, sodium hydroxide, potassium hydroxide and the like, alkali metal hydrogen carbonates such as sodium hydrogen carbonate, potassium hydrogen carbonate and the like, alkali metal carbonates such as sodium carbonate, potassium carbonate and the like, alkoxides such as sodium methoxide, sodium ethoxide and the like, etc.), organic bases (amines such as trimethylamine, triethylamine, diisopropylethylamine and the like, aromatic amines such as pyridine, 4-dimethylaminopyridine and the like, etc.) and the like. Of these, lithium hydroxide, sodium hydroxide, potassium hydroxide and the like are preferable. While the amount of the base to be used varies depending on the kind of the solvent, and the other reaction conditions, it is generally about 1 to 100 molar equivalents, preferably about 1 to 20 molar equivalents, per 1 mol of compound (XLVI).
Examples of the solvent that does not adversely influence the reaction include hydrocarbons (benzene, toluene, xylene, hexane, heptane etc.), halogenated hydrocarbons (dichloromethane, chloroform etc.), ethers (diethyl ether, diisopropyl ether, t-butyl methyl ether, tetrahydrofuran, dioxane, dimethoxyethane etc.), nitriles (acetonitrile etc.), amides (N,N-dimethylformamide, N,N-dimethylacetamide etc.), sulfoxides (dimethyl sulfoxide etc.), water and the like. These solvents may be used in a mixture of two or more solvents at an appropriate ratio. The amount of the solvent to be used is generally 1-fold volume to 100-fold volume, relative to compound (XLVI).
The reaction temperature is, for example, within the range of about -50 to 200°C, preferably about 0 to 100°C. While the reaction time varies depending on the kind of compound (XLVI), the reaction temperature and the like, it is, for example, about 0.5 to 24 hr, preferably about 0.5 to 2 hr.
The halogenation can be carried out in the same manner as in the method described in Method G, Step 16.

### (Step 6)

This step is a step of producing a compound represented by the formula (XLVIII) or a salt thereof (hereinafter to be referred to as compound (XLVIII)) by subjecting compound (XLVII) to a reduction reaction. This step can be carried out in the same manner as in the method described in Method A, Step 7.

### (Step 7)

This step is a step of producing a compound represented by the formula (XLIX) or a salt thereof (hereinafter to be referred to as compound (XLIX)) by subjecting compound (XLVIII) to a benzylation reaction with benzylbromide. This reaction can be carried out according to a method known per se, generally, in the presence of a base, and where necessary, in a solvent that does not adversely influence the reaction.
The benzyl bromide may be generally, commercially available product. The amount of the benzyl bromide to be used is generally about 1 molar equivalent to about 10 molar equivalents, preferably about 1 molar equivalent to about 3 molar equivalents, per 1 mol of compound (XLVIII).
Examples of the solvent include hydrocarbons (e.g., benzene, toluene etc.), ethers (e.g., diethyl ether, dioxane, tetrahydrofuran etc.), esters (e.g., ethyl acetate etc.), halogenated hydrocarbons (e.g., chloroform, dichloromethane etc.), amides (e.g., N,N-dimethylformamide etc.), aromatic amines (e.g., pyridine etc.), water and the like. These solvents may be used in a mixture of two or more solvents at an appropriate ratio. The amount of the solvent to be used is generally 1-fold volume to 100-fold volume, relative to compound (XLVIII).
Examples of the base include alkali metal hydroxides (e.g., sodium hydroxide, potassium hydroxide etc.), hydrogen carbonates (e.g., sodium hydrogen carbonate, potassium hydrogen carbonate etc.), carbonates (e.g., sodium carbonate, potassium carbonate etc.), acetates (e.g., sodium acetate etc.), tertiary amines (e.g., trimethylamine, triethylamine, N-methylmorpholine etc.), aromatic amines (e.g., pyridine, picoline, N,N-dimethylaniline etc.) and the like. The amount of the base to be used is generally about 1 molar equivalent to about 100 molar equivalents, preferably about 1 molar equivalent to about 5 molar equivalents, per 1 mol of compound (XLVIII).
The reaction temperature is generally about -80°C to about 150°C, preferably about 0°C to about 100°C. The reaction time is generally about 0.1 hr to about 48 hr, preferably about 0.5 hr to about 16 hr.
The thus-obtained compound (XLIX) can be isolated and purified by known separation and purification means, for example, concentration, concentration under reduced pressure, solvent extraction, crystallization, recrystallization, phase transfer, chromatography and the like. In addition, compound (XLIX) may be used for the next reaction without isolation.

### (Step 8)

This step is a step of converting compound (XLIX) to a compound represented by the formula (L) or a salt thereof (hereinafter to be referred to as compound (L)) by halogenating compound (XLIX) with a halogenating agent (in the scheme, halogen is shown by a chlorine atom for convenience). This step can be carried out in the same manner as in the method described in Method G, Step 16.

### (Step 9)

This step is a step of producing a compound represented by the formula (LI) or a salt thereof (hereinafter to be referred to as compound (LI)) by reacting compound (L) with an amine represented by the formula: HNR³R⁴ wherein each symbol is as defined above, or a salt thereof. This step can be carried out in the same manner as in the method described in Method A, Step 6.

### (Step 10)

This step is a step of producing compound (1p) by removing the benzyl group of compound (LI). This step can be carried out in the same manner as in the method described in Method A, Step 8.

For example, when compound (Ix) is a compound represented by the formula (1q) or a salt thereof (hereinafter to be referred to as compound (2q)), the compound can be produced according to the following Method Q.

### [Method Q]

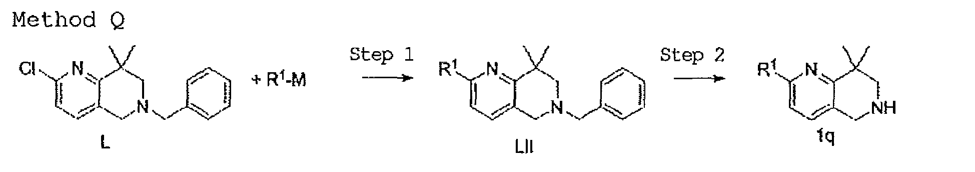

wherein each symbol is as defined above.

### (Step 1)

This step is a step of producing a compound represented by the formula (LII) or a salt thereof (hereinafter to be referred to as compound (LII)) by subjecting compound (L) to a coupling reaction with a compound represented by the formula: R¹-M wherein each symbol is as defined above, or a salt thereof. This step can be carried out in the same manner as in the method described in Method K, Step 1.

### (Step 2)

This step is a step of producing compound (1q) by removing the benzyl group of compound (LII). This step can be carried out in the same manner as in the method described in Method A, Step 8.

For example, when compound (Ix) is a compound represented by the formula (1r) or a salt thereof (hereinafter to be referred to as compound (1r)), the compound can be produced according to the following Method R.

### [Method R]

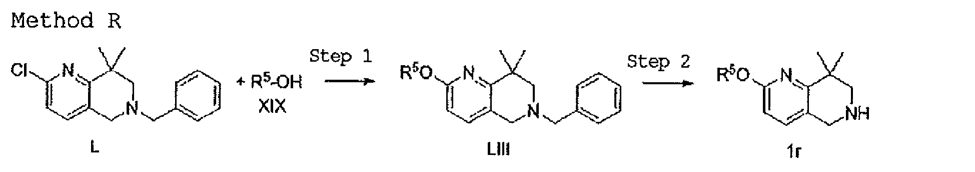

wherein each symbol is as defined above.

### (Step 1)

This step is a step of producing a compound represented by the formula (LIII) or a salt thereof (hereinafter to be referred to as compound (LIII)) by subjecting compound (L) to an alkoxylation with compound (XIX). This step can be carried out in the same manner as in the method described in Method E, Step 11.

### (Step 2)

This step is a step of producing compound (1r) by removing the benzyl group of compound (LIII). This step can be carried out in the same manner as in the method described in Method A, Step 8.

Compound (Ix) produced by such method, can be isolated and purified by a typical separation means such as recrystallization, distillation, chromatography, etc.
When compound (Ix) contains an optical isomer, a stereoisomer, a regioisomer or a rotamer, these are also encompassed in compound (Ix), and can be obtained as a single product according to synthesis and separation methods known *per se* (e.g., concentration, solvent extraction, column chromatography, recrystallization, etc.). For example, when compound (Ix) has an optical isomer, an optical isomer resolved from this compound is also encompassed in compound (Ix).
In addition, a deuterium exchange compound of compound (Ix) is also encompassed in compound (Ix).
The optical isomer can be produced according to a method known per se. To be specific, an optically active synthetic intermediate is used, or the final racemate product is subjected to optical resolution according to a conventional method to give an optical isomer.
The method of optical resolution may be a method known *per se,* such as a fractional recrystallization method, a chiral column method, a diastereomer method, etc.

### 1) Fractional recrystallization method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine, etc.) is formed, which is separated by a fractional recrystallization method, and if desired, a free optical isomer is obtained by a neutralization step.

### 2) Chiral column method

A method wherein a racemate or a salt thereof is applied to a column for separation of an optical isomer (a chiral column) to allow separation. In the case of a liquid chromatography, for example, a mixture of the optical isomers is applied to a chiral column such as ENANTIO-OVM (manufactured by Tosoh Corporation), CHIRAL series (manufactured by Daicel Chemical Industries, Ltd.) and the like, and developed with water, various buffers (e.g., phosphate buffer, etc.) and organic solvents (e.g., ethanol, methanol, isopropanol, acetonitrile, trifluoroacetic acid, diethylamine, etc.) solely or in admixture to separate the optical isomer. In the case of a gas chromatography, for example, a chiral column such as CP-Chirasil-DeX CB (manufactured by GL Sciences Inc.) and the like is used to allow separation.

### 3) Diastereomer method

A method wherein a racemic mixture is prepared into a diastereomeric mixture by chemical reaction with an optically active reagent, which is made into a single substance by a typical separation means (e.g., a fractional recrystallization method, a chromatography method, etc.) and the like, and is subjected to a chemical treatment such as hydrolysis and the like to separate an optically active reagent moiety, whereby an optical isomer is obtained. For example, when compound (Ix) contains a hydroxyl group, or primary or secondary amino in a molecule, the compound and an optically active organic acid (e.g., MTPA [α-methoxy-α-(trifluoromethyl)phenylacetic acid], (-)-menthoxyacetic acid, etc.) and the like are subjected to condensation reaction to give diastereomers in the ester form or in the amide form, respectively. When compound (Ix) has carboxyl, this compound and an optically active amine or optically active alcohol are subjected to condensation reaction to give diastereomers in the amide form or in the ester form, respectively. The separated diastereomer is converted to an optical isomer of the original compound by acid hydrolysis or base hydrolysis.

The compound (Ix) may be a crystal.
The crystal of the compound (Ix) can be produced by crystallization of compound (Ix) according to crystallization methods known *per se.*
Examples of the crystallization method include a method of crystallization from a solution, a method of crystallization from vapor, a method of crystallization from the melts and the like.

The "crystallization from a solution" is typically a method of shifting a non-saturated state to supersaturated state by varying factors involved in solubility of compounds (solvent composition, pH, temperature, ionic strength, redox state, etc.) or the amount of solvent. To be specific, for example, a concentration method, a slow cooling method, a reaction method (a diffusion method, an electrolysis method), a hydrothermal growth method, a flux method and the like can be mentioned. Examples of the solvent to be used include aromatic hydrocarbons (e.g., benzene, toluene, xylene, etc.), halogenated hydrocarbons (e.g., dichloromethane, chloroform, etc.), saturated hydrocarbons (e.g., hexane, heptane, cyclohexane, etc.), ethers (e.g., diethyl ether, diisopropyl ether, tetrahydrofuran, dioxane, etc.), nitriles (e.g., acetonitrile, etc.), ketones (e.g., acetone, etc.), sulfoxides (e.g., dimethyl sulfoxide, etc.), acid amides (e.g., N,N-dimethylformamide, etc.), esters (e.g., ethyl acetate, etc.), alcohols (e.g., methanol, ethanol, isopropyl alcohol, etc.), water and the like. These solvents are used alone or in a combination of two or more at a suitable ratio (e.g., 1:1 to 1:100 (a volume ratio)). Where necessary, a seed crystal can be used.
The "crystallization from vapor" is, for example, a vaporization method (a sealed tube method, a gas stream method), a gas phase reaction method, a chemical transportation method and the like.
The "crystallization from the melts" is, for example, a normal freezing method (a Czockralski method, a temperature gradient method and a Bridgman method, etc.), a zone melting method (a zone leveling method and a floating zone method, etc.), a special growth method (a VLS method and a liquid phase epitaxy method, etc.) and the like.

Preferable examples of the crystallization method include a method of dissolving compound (Ix) in a suitable solvent (e.g., alcohols such as methanol, ethanol, etc., and the like) at a temperature of 20 to 120°C, and cooling the resulting solution to a temperature not higher than the temperature of dissolution (e.g., 0 to 50°C, preferably 0 to 20°C) and the like.
The thus obtained crystals of compound (Ix) of the present invention can be isolated, for example, by filtration and the like.
As an analysis method of the obtained crystal is generally a method of crystal analysis by powder X-ray diffraction. As a method of determining crystal orientation, a mechanical method or an optical method and the like can also be used.
The crystal of compound (Ix) obtained by the above-mentioned production method (hereinafter to be abbreviated as "the crystal of the present invention") has high purity, high quality, and low hygroscopicity, is not denatured even after a long-term preservation under general conditions, and is extremely superior in the stability. In addition, it is also superior in the biological properties (e.g., pharmacokinetics (absorption, distribution, metabolism, excretion), efficacy expression etc.) and is extremely useful as a medicament.

In the present specification, the melting point means a melting point measured using, for example, a micro melting point apparatus (YANACO, MP-500D), a DSC (differential scanning calorimetry) apparatus (SEIKO, EXSTAR6000) and the like.

A prodrug of the compound (Ix) means a compound which is converted to the compound (Ix) with a reaction due to an enzyme, an gastric acid, etc. under the physiological condition in the living body, that is, a compound which is converted to the compound (Ix) with oxidation, reduction, hydrolysis, etc. according to an enzyme; a compound which is converted to the compound (Ix) by hydrolysis etc. due to gastric acid, etc. A prodrug for compound (Ix) may be a compound obtained by subjecting amino in compound (Ix) to an acylation, alkylation or phosphorylation [e.g., a compound obtained by subjecting amino in compound (Ix) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.]; a compound obtained by subjecting a hydroxy group in compound (Ix) to an acylation, alkylation, phosphorylation or boration (e.g., a compound obtained by subjecting a hydroxy group in compound (Ix) to an acetylation, palmitoylation, propanoylation, pivaloylation, succinylation, fumarylation, alanylation, dimethylaminomethylcarbonylation, etc.); a compound obtained by subjecting carboxyl in compound (Ix) to an esterification or amidation [e.g., a compound obtained by subjecting carboxyl in compound (Ix) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation, etc.] and the like. Any of these compounds can be produced from compound (Ix) by a method known per se.
A prodrug for compound (Ix) may also be one which is converted into compound (Ix) under a physiological condition, such as those described in IYAKUHIN no KAIHATSU (Development of Pharmaceuticals), Vol. 7, Design of Molecules, p.163-198, Published by HIROKAWA SHOTEN (1990).
Compound (Ix) of the present invention or a prodrug thereof (hereinafter to be abbreviated as the compound of the present invention) has a superior serotonin 5-HT_{2c} receptor activating action.
In addition, the compound of the present invention has low toxicity and is safe.

Accordingly, compound (I) of the present invention having a superior serotonin 5-HT_{2c} receptor activation action is useful as a prophylaxis or therapeutic drug for all serotonin 5-HT_{2c} associated diseases in mammals (e.g., mouse, rat, hamster, rabbit, cat, dog, bovine, sheep, monkey, human and the like), for example,
(1) lower urinary tract diseases (including all diseases having a lower urinary tract symptoms described below including, for example, overactive bladder, benign prostatic hyperplasia, interstitial cystitis, chronic prostatitis etc.): storage symptom (e.g., day time urinary frequency, nocturia, urinary urgency, urinary incontinence, stress urinary incontinence, urge urinary incontinence, mixed urinary incontinence, enuresis, nocturnal enuresis, overflow urinary incontinence, other urinary incontinence, enhanced, decreased or missing bladder sensation etc.), voiding symptom (e.g., weak urinary stream (or slow stream), split urinary stream, spraying stream, intermittent urinary stream, voiding postponement, straining at urination, terminal dribbling etc.), post-micturition symptom (e.g., sense of residual urine, post-micturition dribble etc.), symptom due to sexual intercourse (e.g., coital pain, vaginal dryness, urinary incontinence etc.), symptom due to pelvic organ prolapse (e.g., foreign body sensation, lumbago etc.), genital organ pain or lower urinary tract pain (e.g., cystalgia, urethral pain, pudendalgia, vaginodynia, scrotal pain, perineal pain, pelvic pain etc.), genital organ or urinary tract pain syndrome (e.g., cystalgia syndrome, urethral pain syndrome, pudendalgia syndrome, vaginal syndrome, scrotal pain syndrome, perineal pain syndrome, pelvic pain syndrome etc.), symptom syndrome suggesting lower urinary tract dysfunction (e.g., overactive bladder syndrome, a lower urinary tract symptom suggesting bladder outlet obstruction etc.), polyuria, urolithiasis (e.g., ureteral calculus, urethral calculus) and the like,
(2) metabolic diseases [for example, diabetes (insulin dependent diabetes, diabetic complications, diabetic retinopathy, diabetic microangiopathy, diabetic neuropathy etc.), impaired glucose tolerance, obesity [e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity], benign prostatic hyperplasia, sexual dysfunction and the like]
(3) central nervous system diseases [for example, neurodegenerative diseases (e.g., Alzheimer's disease, Down's disease, Parkinson's disease, Creutzfeldt-Jakob disease, amyotrophic lateral sclerosis (ALS), Huntington chorea, diabetic neuropathy, multiple sclerosis etc.), mental diseases (e.g., schizophrenia, depression, mania, anxiety neurosis, obsessive-compulsive neurosis, panic disorder, epilepsy, alcohol dependence, drug dependence, anxiety, anxious mental state, emotional abnormality, cyclothymia, nervous erethism, autism, faint, addiction, low sex drive etc.), disorders such as central nervous system and peripheral nerve disorders (e.g., head trauma, spinal trauma, brain edema, disorders of sensory function, abnormality of sensory function, disorders of autonomic nervous function, abnormality of autonomic nervous function, whiplash injury etc.), memory disorders (e.g., senile dementia, amnesia, cerebrovascular dementia etc.), cerebrovascular disorder (e.g., cerebral hemorrhage, cerebral infarction and the like and sequelae or complication thereof, asymptomatic cerebrovascular accident, transient cerebral ischemic attack, hypertensive encephalopathia, blood-brain barrier disorder, etc.), recurrence and sequelae of cerebrovascular disorders (e.g., neural symptoms, mental symptoms, subjective symptoms, disorders of daily living activities etc.), central nervous system hypofunction after brain blood vessel occlusion, disorder or abnormality of autoregulation ability of brain circulation or renal circulation, sleep disorder etc.]
(4) sexual dysfunction diseases [for example, male erectile dysfunction, dysspermia, female sexual dysfunction etc.]
(5) digestive organ diseases [for example, an irritable bowel syndrome, inflammatory bowel disease, ulcerative colitis, Crohn's disease, diseases caused by a spiral urease-positive gram-negative bacterium (e.g., Helicobacter pylori, etc.) (e.g., gastritis, gastric ulcer, etc.), gastric cancer, postgastrostomy disorder, indigestion, esophageal ulcer, pancreatitis, polyp of the colon, cholelithiasis, hemorrhoids, peptic ulcer, situational ileitis, gluttony, constipation, diarrhea, borborygmus, etc.]
(6) inflammatory or allergic diseases [for example, allergic rhinitis, conjunctivitis, gastrointestinal allergy, pollinosis, anaphylaxis, dermatitis, herpes, psoriasis, bronchitis, expectoration, retinopathy, postoperative and posttraumatic inflammation, regression of puffiness, pharyngitis, cystitis, meningitidis, inflammatory ophthalmic diseases, etc.] (7) osteoarthropathy diseases [for example, rheumatoid arthritis (chronic rheumatoid arthritis), arthritis deformans, rheumatoid myelitis, osteoporosis, abnormal growth of cells, bone fracture, bone refracture, osteomalacia, osteopenia, Paget's disease of bone, rigid myelitis, articular tissue destruction by gonarthrosis deformans and similar diseases thereto, etc.]
(8) respiratory diseases [for example, cold syndrome, pneumonia, asthma, pulmonary hypertension, pulmonary thrombi/pulmonary obliteration, pulmonary sarcoidosis, pulmonary tuberculosis, interstitial pneumonia, silicosis, adult respiratory distress syndrome, chronic obstructive pulmonary disease, cough, etc.]
(9) infectious diseases [HIV infectious diseases, virus infectious diseases due to cytomegalo virus, influenza virus, herpes virus and the like, rickettsia infectious diseases, bacterial infectious diseases, sexually-transmitted diseases, carinii pneumonia, Helicobacter pylori infectious disease, systemic fungal infectious diseases, tuberculosis, invasive staphylococcal infectious diseases, acute viral encephalitis, acute bacterial meningitidis, AIDS encephalitis, septicemia, sepsis, sepsis gravis, septic shock, endotoxin shock, toxic shock syndromes, etc.]
(10) cancers [for example, primary, metastatic or recurrent breast cancer, prostatic cancer, pancreatic cancer, gastric cancer, lung cancer, colorectal cancer (colon cancer, rectal cancer, anal cancer), esophagus cancer, duodenal cancer, head and neck cancer (tongue cancer, pharynx cancer, larynx cancer), brain tumor, neurinoma, non-small cell lung cancer, small cell lung cancer, hepatic cancer, renal cancer, colic cancer, uterine cancer (cancer of the uterine body, uterine cervical cancer), ovarian cancer, bladder cancer, skin cancer, hemangioma, malignant lymphoma, malignant melanoma, thyroid cancer, bone tumor, hemangioma, angiofibroma, retinosarcoma, penis cancer, pediatric solid cancer, Kaposi's sarcoma, Kaposi's sarcoma caused by AIDS, tumor of the maxillary sinus, fibrous histiocytoma, smooth muscle sarcoma, rhabdomyosarcoma, liposarcoma, fibroid tumors of the uterus, osteoblastoma, osteosarcoma, chondrosarcoma, carcinomatous mesothelial tumor, tumors such as leukemia, Hodgkin's disease, etc.] (11) circulatory diseases [for example, acute coronary artery syndromes (e.g., acute cardiac infarction, unstable angina,
   etc.), peripheral arterial occlusion, Raynaud's disease, Buerger's disease, restenosis after coronary-artery intervention (percutaneous transluminal coronary angioplasty (PTCA), directional coronary atherectomy (DCA), stenting, etc.), restenosis after coronary-artery bypass operation, restenosis after intervention (angioplasty, atherectomy, stenting, etc.) or bypass operation in other peripheral artery, ischemic cardiac diseases (e.g., cardiac infarction, angina, etc.), myocarditis, intermittent claudication, lacunar infarction, arteriosclerosis (e.g., atherosclerosis, etc.), cardiac failure (acute cardiac failure, chronic cardiac failure including congestive cardiac failure), arrhythmia, progress of atherosclerotic plaque, thrombosis, hypertension, hypertensive tinnitus, hypotension, etc.]
(12) pains [e.g., headache, migraine, neuralgia, pelvic visceral pain (including cystalgia), etc.]
(13) autoimmune diseases [for example, collagen disease, systemic lupus erythematosus, scleroderma, polyarteritis, myasthenia gravis, multiple sclerosis, Sjogren's syndrome, Behcet's disease, etc.]
(14) hepatic diseases [e.g., hepatitis (including chronic hepatitis), cirrhosis, interstitial hepatic diseases, etc.]
(15) pancreatic diseases [e.g., pancreatitis (including chronic pancreatitis), etc.]
(16) renal diseases [e.g., nephritis, glomerulonephritis, glomerulosclerosis, renal failure, thrombotic microangiopathy, dialysis complications, organ disorders including nephropathia by radiation, diabetic nephropathy, etc.]
(17) endocrine diseases [e.g., Addison's disease, Cushing's syndrome, melanocytoma, primary aldosteronism, etc.]
(18) other diseases [for example,
   (a) transplant rejection [e.g., posttransplantational rejection, posttransplantational polycythemia, hypertension, organ disorder and/or vascular hypertrophy, graft-versus-host disease, etc.]
   (b) abnormality in characteristic of blood and/or blood components [e.g., enhancement in platelet aggregation, abnormality of erythrocyte deformability, enhancement in leukocyte adhesiveness, increase in blood viscosity, polycythemia, vascular peliosis, autoimmune hemolytic anemia, disseminated intravascular coagulation syndrome (DIC), multiple myelopathy, etc.]
   (c) gynecologic diseases [e.g., climacteric disorder, gestational toxicosis, endometriosis, hysteromyoma, ovarian disease, mammary disease, premenstrual syndrome, pelvic organ prolapse, rectal prolapse, cystoceles, enteroceles etc.]
   (d) dermatic diseases [e.g., keloid, angioma, psoriasis, pruritus, etc.]
   (e) ophthalmic diseases [e.g., glaucoma, ocular hypertension disease, etc.]
   (f) otolaryngological diseases [e.g., Menuel syndrome, tinnitus, gustation disorder, dizziness, disequilibrium, dysphagia, etc.]
   (g) diseases due to environmental and/or occupational factors (e.g., radiation disorder, disorders by ultraviolet ray/infrared ray/laser ray, altitude sickness, etc.)
   (h) ataxia, stiffness, tremor, motion impairment, akinesia and the like
   (i) chronic fatigue syndrome
   (j) sudden infant death syndrome
   (k) hiccup
   (l) diseases causing palpitation, vertigo, heartburn and the like.
In addition, the serotonin 5-HT_{2c} receptor activators including the compound of the present invention can also be used for the prophylaxis or treatment of diseases caused by prolapses of an organ from its normal position due to weakened pelvic floor muscles such as cystocele, enterocele and the like (e.g., proctocele and the like).
Pelvic organ prolapse, rectal prolapse, cystocele and enterocele are diseases characterized by the protrusion of bladder, uterus, small intestine, rectal or the like beyond the vaginal orifice or the rectal orifice due to the insufficient contractile force of the pelvic floor muscles.
In the above-mentioned diseases, the compound of the present invention is particularly useful as a serotonin 5-HT_{2c} receptor activator, as an ameliorator for a lower urinary tract symptoms such as overactive bladder, stress urinary incontinence, and as a prophylactic or therapeutic drug for the lower urinary tract symptoms, a prophylactic or therapeutic agent for obesity, or a prophylactic or therapeutic agent for pelvic organ prolapse.

As a useful compound that can be used as a prophylactic or therapeutic drug for cystoceles or enteroceles, various serotonin 5-HT_{2c} receptor activators can be used besides the aforementioned compounds of the present invention and, for example, one having an inhibitory activity as evidenced by 50% inhibitory concentration (IC₅₀) of not more than about 1000 nM, preferably not more than about 100 nM, by a binding assay is more preferable. Specifically, as the serotonin 5-HT_{2c} receptor agonist, compounds described in
EP0572863, EP0863136, EP1213017, USP3253989, USP3676558, USP3652588, USP4082844, USP4971969, USP5494928, USP5646173, USP6310208, WO97/42183 WO98/30546 WO98/30548 WO98/33504, WO99/02159, WO99/43647(USP6281243), WO00/12475(USP6380238), WO00/12502(USP6365598), WO00/12510(USP6433175), WO00/12475, WO00/12481(USP6552062), WO00/12482, WO00/12502, WO00/16761, WO00/17170, WO00/28993, WO00/35922(USP6372745), WO00/44737, WO00/44753, WO00/64899, WO00/77001, WO00/77002, WO00/77010, WO00/76984(USP6465467), WO01/09111, WO01/09122, WO01/09123(USP6638936), WO01/09126, WO01/12602, WO01/12603(USP6706750), WO01/40183, WO01/66548(USP6583134), WO01/70207, WO01/70223, WO01/72752(USP6734301), WO01/83487, WO02/04456, WO02/04457, WO02/08178, WO02/10169, WO02/24700, WO02/24701 WO02/36596 WO02/40456, WO02/40457 WO02/42304 WO02/44152(USP6479534), WO02/48124, WO02/51844(USP6610685), WO02/59124, WO02/59127, WO02/59129, WO02/72584, WO02/74746, WO02/83863, WO02/98350, WO02/98400, WO02/98860, WO03/00663, WO03/00666, WO03/04501, WO03/06466, WO03/11281, WO03/14118, WO03/14125, WO03/24976, WO03/28733, WO03/33497, WO03/57161, WO03/57213, WO03/57673, WO03/57674, WO03/62205, WO03/64423, WO03/86306, WO03/87086, WO03/89409, WO03/91250, WO03/91251, WO03/91257, WO03/97636, WO04/00829, WO04/00830(USP6667303), WO04/56324 WO04/78718 WO04/81010 WO04/087156 WO04/87662 WO04/87692 WO04/89897 WO04/096196 WO04/96201 WO04/112769 US2004192754, WO05/00849 WO05/03096 EP1500391, WO05/16902 WO05/19180 US2005080074, WO05/40146 WO05/41856, WO05/42490, WO05/42491, WO05/44812, WO05/082859, WO05/000309, WO05/019179, WO05/121113, WO05/049623, WO05/105082, WO05/109987, WO05/121113, WO05/113535, US06/003990, US06/014777, US06/014778, WO06/000902, WO06/028961, WO06/020817, WO06/020049, WO06/019940, WO06/004931, US06/025601, WO06/044762, WO06/047032, WO06/050007, WO06/052887, WO06/077025, WO06/065600, WO06/103511,WO06/116165, WO06/047228, WO06/117304, US06/241172, US06/241176, WO06/116136, WO06/116148, WO06/116151, WO06/116171, WO06/116170, WO06/116218, WO06/116169, WO06/077025, US07/032481, WO07/025144, WO07/028082, WO07/028132, WO07/028131, WO07/028083, WO07/030150, US2007/0049613, WO07/140213, WO07/084622, WO08/052075, WO08/052078, WO08/052086, WO08/052087, WO08/052088, WO08/010073, US08/0146583, WO08/117169, WO08/009125, US08/0255092,

US08/0269196 and the like are used. Among these, particularly, (1) WAY-161503

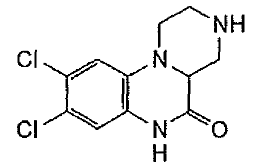

(2) m-CPP

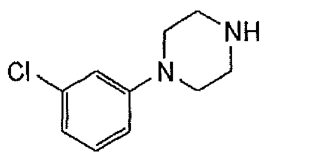

(3) PNU-22394A

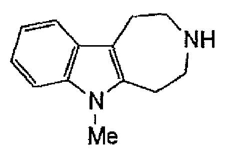

(4) Ro60-0175

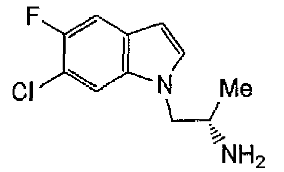

(5) ORG-12962

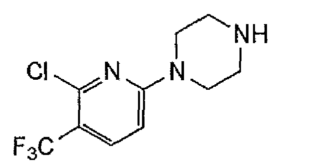

(6) Nordexfenfluramine

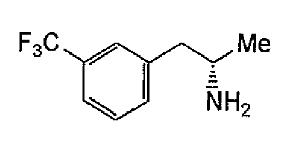

(7) MK-212

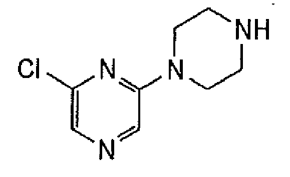

(8) Oxaflozane

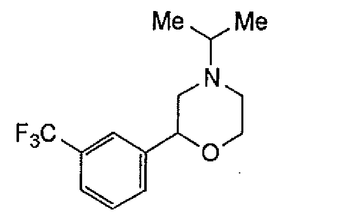

(9) a compound represented by the following structural formula described in WO00/12510 (USP6433175)

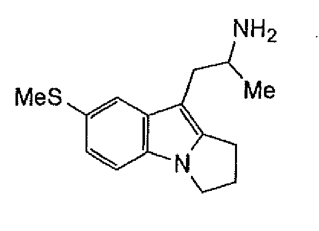

(10) a compound represented by the following structural formula described in WO02/51844 (USP6610685)

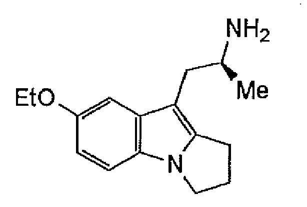

(11) a compound represented by the following structural formula described in WO01/66 (USP6583134)

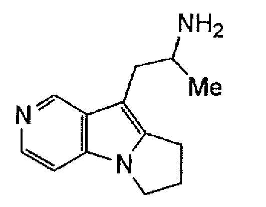

(12) a compound represented by the following structural formula described in WO00/12482

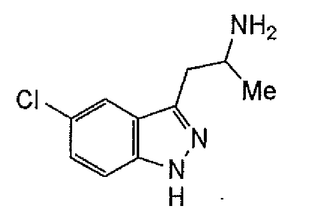

(13) a compound represented by the following structural formula described in WO03/2497

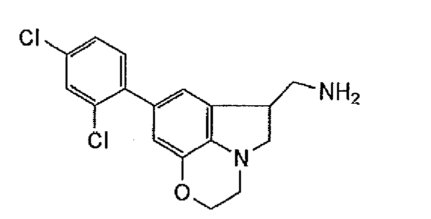

(14) ALX-2218

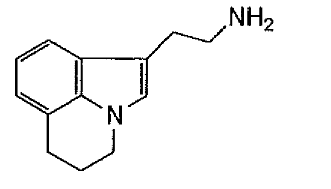

(15) ALX-2226

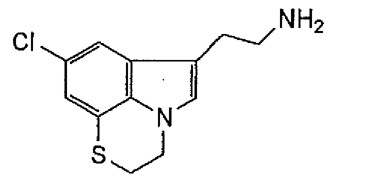

(16) Ro60-0332

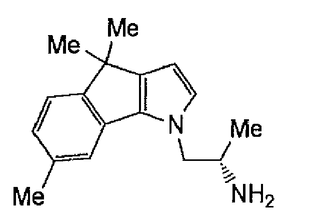

(17) VER-2692

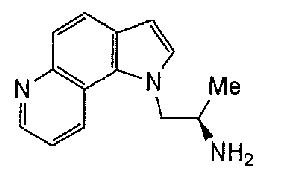

(18) VER-6925

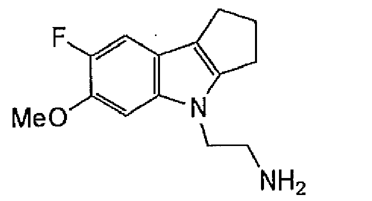

(19) VER-7397

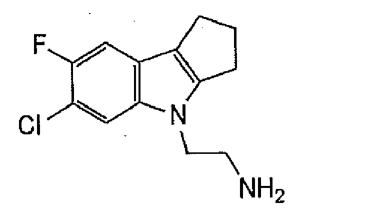

(20) VER-7499

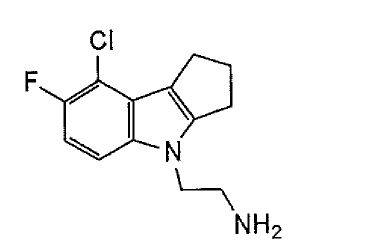

(21) VER-7443

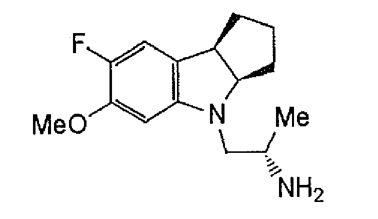

(22) VER-3993

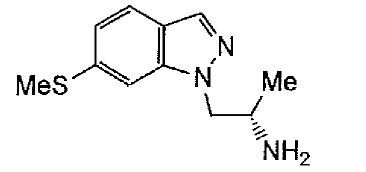

(23) YM-348

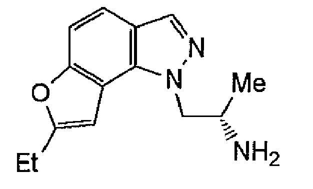

(24) a compound represented by the following structural formula described in WO03/57213

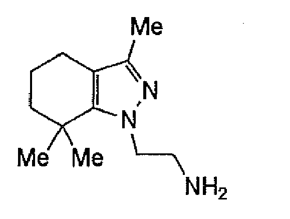

(25) a compound represented by the following structural formula described in WO03/57674

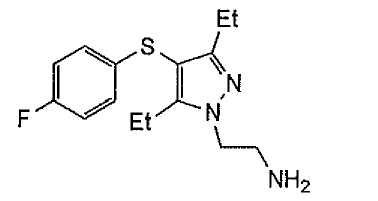

(26) a compound represented by the following structural formula described in WO02/98860

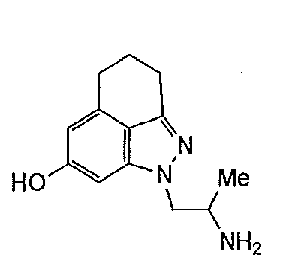

(27) VER-5593

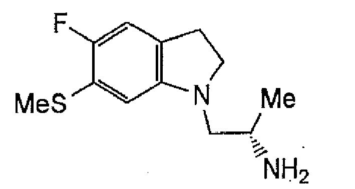

(28) VER-5384

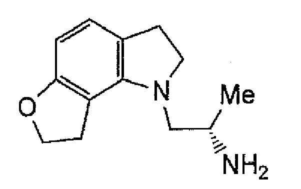

(29) VER-3323

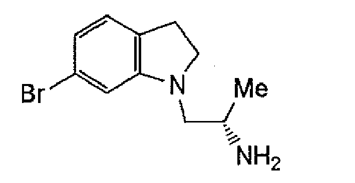

(30) a compound represented by the following structural formula described in WO02/44152 (USP6479534)

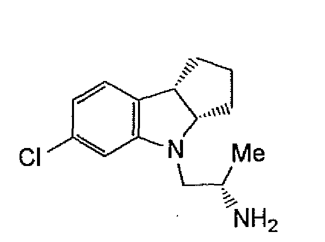

(31) a compound represented by the following structural formula described in WO0/44737

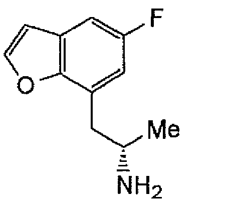

(32) APD-356

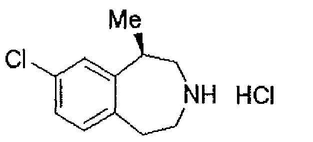

(33) AR-10A
(34) a compound represented by the following structural formula described in WO02/40456

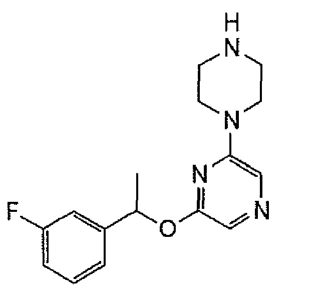

(35) BVT-933
(36) a compound represented by the following structural formula described in WO02/0817

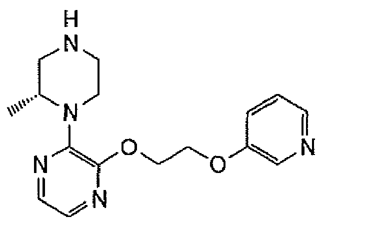

(37) PNU-243922

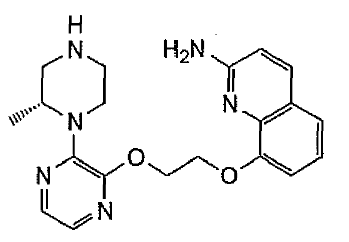

(38) a compound represented by the following structural formula described in WO03/00666

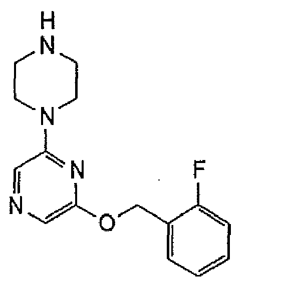

(39) a compound represented by the following structural formula described in WO01/09123 (USP6638936)

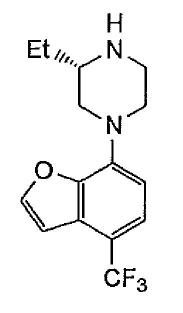

(40) a compound represented by the following structural formula described in WO01/09122

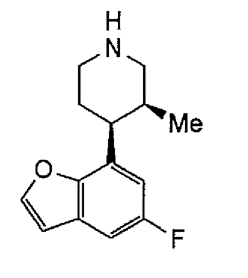

(41) a compound represented by the following structural formula described in WO01/09126

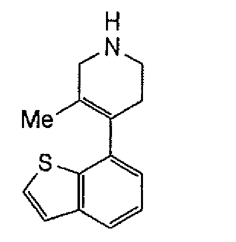

(42) Org-37684

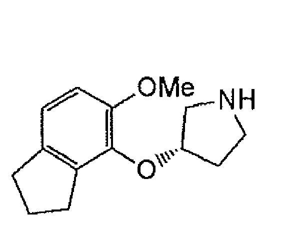

(43) Org-36262 (Ro60-0527)

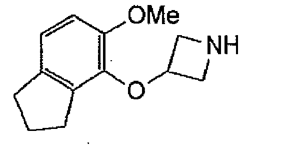

(44) a compound represented by the following structural formula described in EP0572863

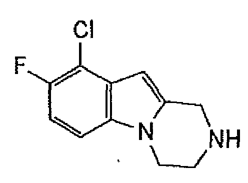

(45) Ro60-0017 (Org-35013)

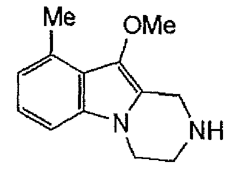

(46) VER-3881

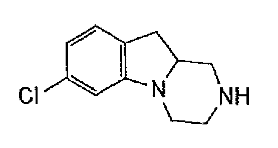

(47) a compound represented by the following structural formula described in WO02/72584

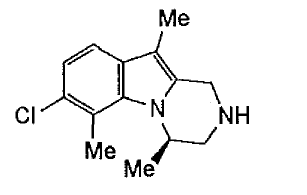

(48) Ro0721256

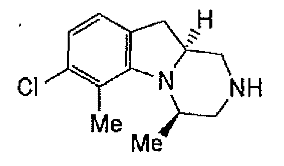

(49) PNU-181731A

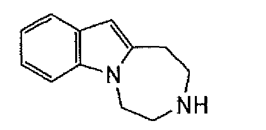

(50) a compound represented by the following structural formula described in WO02/59127

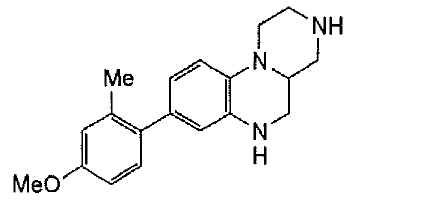

(51) a compound represented by the following structural formula described in WO03/14118

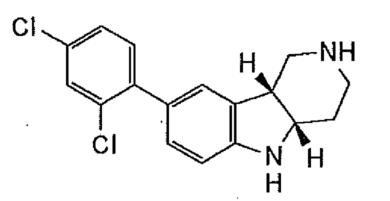

(52) a compound represented by the following structural formula described in WO03/33497

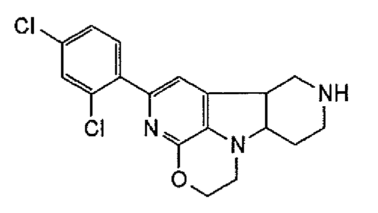

(53) IL-639

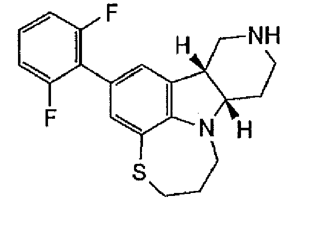

(54) IK-264

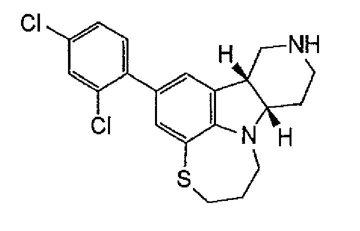

(55) VR-1065
(56) Ro60-0759

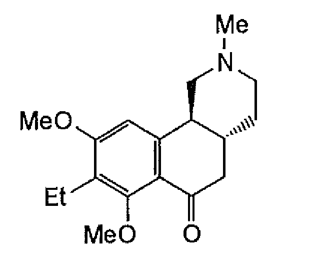

(57) Ro60-0869

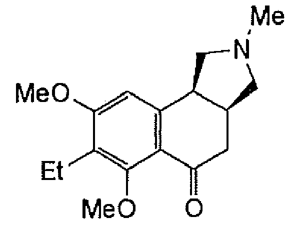

(58) a compound represented by the following structural formula described in WO00/64899

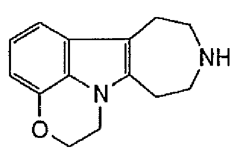

(59) PNU-57378E

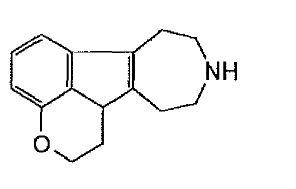

(60) a compound represented by the following structural formula described in WO03/06466

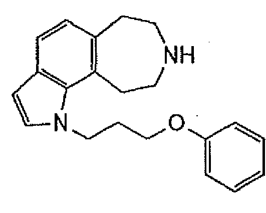

(61) a compound represented by the following structural formula described in WO02/74746

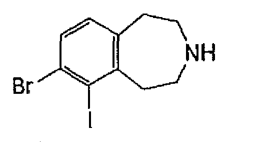

(62) a compound represented by the following structural formula described in WO02/42304

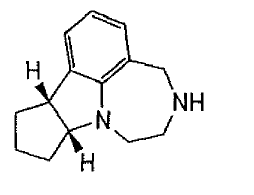

(63) WAY-470

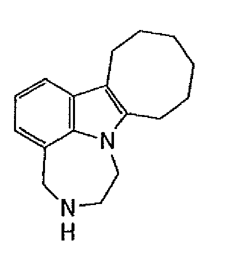

(64) WAY-629

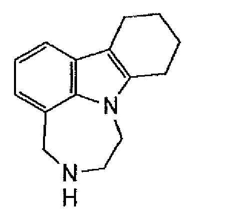

(65) a compound represented by the following structural formula described in WO97/42183

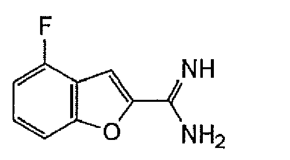

(66) a compound represented by the following structural formula described in WO02/48124

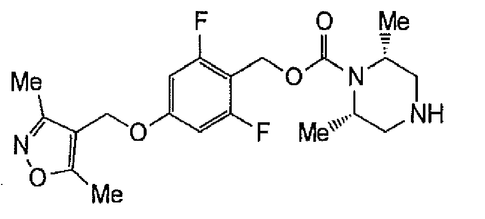

(67) WAY-162545

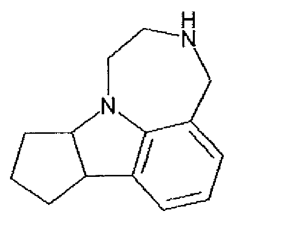

(68) WAY-163909

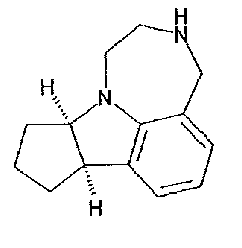

(69) IX-065

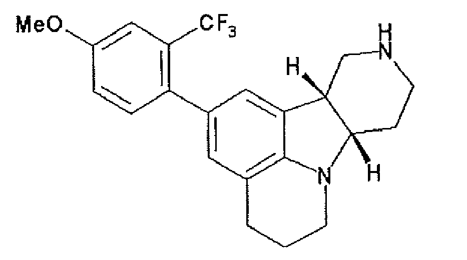

(70)A-37215

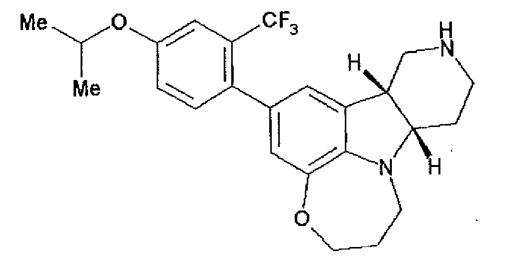

(71) a compound represented by the following structural formula described in WO05/42491

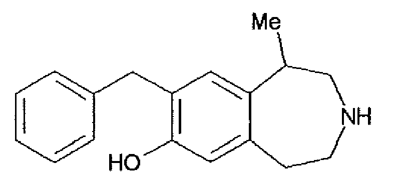

(72) a compound represented by the following structural formula described in WO05/16902

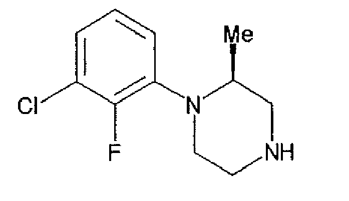

(73) a compound represented by the following structural formula described in WO04/99150

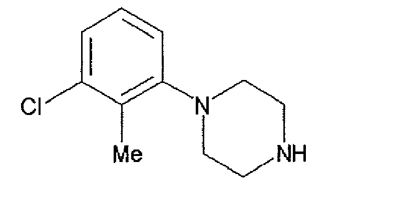

(74) PAL-287
(75) SCA-136

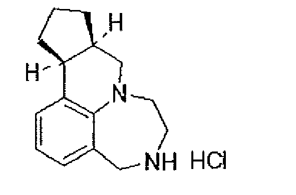

(76) a compound represented by the following structural formula described in WO08/010073

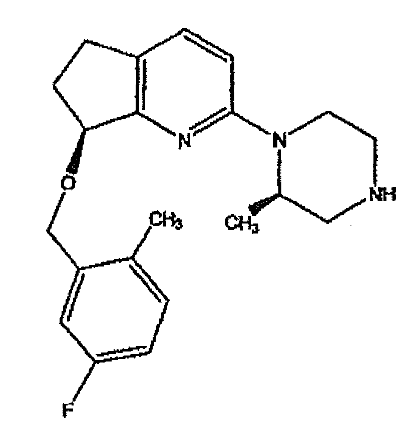

(77) ATHX-105
and the like are preferably used.
Preparations comprising the compound of the present invention may be in any solid forms of powders, granules, tablets, capsules, orally disintegrable films, etc., and in any liquid forms of syrups, emulsions, injections, etc.
The preparations of the present invention for prophylaxis or treatment can be produced by any conventional methods, for example, blending, kneading, granulation, tableting, coating, sterilization, emulsification, etc., in accordance with the forms of the preparations to be produced. For the production of such pharmaceutical preparations, for example, each of the items in General Rules for Preparations in the Japanese Pharmacopoeia, can be made reference to. In addition, the preparations of the present invention may be formulated into a sustained release preparation containing active ingredients and biodegradable polymer compounds. The sustained release preparation can be produced according to the method described in JP-A-9-263545.

In the preparations of the present invention, the content of the compound in the present invention varies depending on the forms of the preparations, but is generally in the order of 0.01 to 100 % by weight, preferably 0.1 to 50 % by weight, more preferably 0.5 to 20 % by weight, relative to the total weight of each preparation.
When the compound of the present invention is used in the above-mentioned pharmaceutical products, it may be used alone, or in admixture with a suitable, pharmacologically acceptable carrier, for example, excipients (e.g., starch, lactose, sucrose, calcium carbonate, calcium phosphate, etc.), binders (e.g., starch, arabic gum, carboxymethyl cellulose, hydroxypropyl cellulose, crystalline cellulose, alginic acid, gelatin, polyvinylpyrrolidone, etc.), lubricants (e.g., stearic acid, magnesium stearate, calcium stearate, talc, etc.), disintegrants (e.g., calcium carboxymethylcellulose, talc, etc.), diluents (e.g., water for injection, physiological saline, etc.) and if desired, with the additives (e.g., a stabilizer, a preservative, a colorant, a fragrance, a dissolution aid, an emulsifier, a buffer, an isotonic agent, etc.) and the like, by ordinary methods. It can be formulated into the solid preparations such as powders, fine granules, granules, tablets, capsules, etc., or into the liquid preparations such as injections, etc., and can be administered orally or parenterally. When the compound of the present invention is formed as a preparation for topical administration and administered, it can also be directly administered to the affected part of an articular disease. In this case, an injection is preferable. It can also be administered as a parenteral agent for topical administration (e.g., intramuscular injection, subcutaneous injection, organ injection, injection to the vicinity of a joint and the like, solid preparation such as implant, granule, powder and the like, liquid such as suspension and the like, ointment etc.) and the like.

For formulation into an injection, for example, the compound of the present invention is formulated into an aqueous suspension with a dispersing agent (e.g., surfactant such as Tween 80, HCO-60 and the like, polysaccharides such as carboxymethylcellulose, sodium alginate, hyaluronic acid and the like, polysorbate etc.), preservative (e.g., methylparaben, propylparaben etc.), isotonic agent (e.g., sodium chloride, mannitol, sorbitol, glucose etc.), buffer (e.g., calcium carbonate etc.), pH adjuster (e.g., sodium phosphate, potassium phosphate etc.) and the like to give a preparation for practical injection. In addition, an oily suspension can be obtained by dispersing the compound of the present invention together with vegetable oil such as sesame oil, corn oil and the like or a mixture thereof with a phospholipid such as lecithin and the like, or medium-chain fatty acid triglyceride (e.g., miglyol 812 etc.) to give an injection to be actually used.

An agent for the prophylaxis or treatment of the present invention can be used along with other medicament.
As the drug that can be mixed with or concomitantly used with the compound of the present invention (hereinafter to be abbreviated as concomitant drug), for example, the following drugs can be used.

### (1) Other drugs for treating stress urinary incontinence

Adrenaline α1 receptor agonists (e.g., ephedrine hydrochloride, midodrine hydrochloride), adrenaline β2 receptor agonists (e.g., Clenbuterol), noradrenaline uptake inhibitors, noradrenaline and serotonin uptake inhibitors (e.g., duloxetine), tricyclic antidepressants (e.g., imipramine hydrochloride), anticholinergic agents or smooth muscle stimulants (e.g., oxybutynin hydrochloride, propiverine hydrochloride, celimeverine hydrochloride), female hormone drugs (e.g., conjugated estrogen (premarin), estriol) and the like.

### (2) Agent for treating diabetes

Insulin preparations [e.g., animal insulin preparations extracted from the bovine or swine pancreas; human insulin preparations synthesized by a genetic engineering technique using Escherichia coli or a yeast; insulin zinc; protamine zinc insulin; a fragment or a derivative of insulin (e.g., INS-1, etc.)], insulin sensitizers (e.g., pioglitazone hydrochloride, troglitazone, rosiglitazone or its maleate, JTT-501, MCC-555, YM-440, GI-262570, KRP-297, FK-614, CS-011, etc.), α-glucosidase inhibitors (e.g., voglibose, acarbose, miglitol, emiglitate, etc.), biguanides (e.g., phenformin, metformin, buformin, etc.), sulfonylureas (e.g., tolbutamide, glibenclamide, gliclazide, chlorpropamide, tolazamide, acetohexamide, glyclopyramide, glimepiride, etc.) and other insulin secretagogues (e.g., repaglinide, senaglinide, mitiglinide or its calcium salt hydrate, GLP-1, nateglinide, etc.), dipeptidylpeptidase IV inhibitors (e.g., vildagliptin, sitagliptin, saxagliptin, alogliptin, NVP-DPP-728, PT-100, P32/98, etc.), β3 agonists (e.g., CL-316243, SR-58611-A, UL-TG-307, AJ-9677, AZ40140, etc.), amylin agonists (e.g., pramlintide, etc.), phosphotyrosine phosphatase inhibitors (e.g., vanadic acid, etc.), gluconeogenesis inhibitors (e.g., glycogen phosphorylase inhibitors, glucose-6-phosphatase inhibitors, glucagon antagonists, etc.), SGLT (sodium-glucose cotransporter) inhibitors (e.g., T-1095, etc.) and the like.

### (3) Agent for treating diabetic complications

Aldose reductase inhibitors (e.g., tolrestat, epalrestat, zenarestat, zopolrestat, fidarestat (SNK-860), minalrestat (ARI-509), CT-112, etc.), neurotrophic factors (e.g., NGF, NT-3, etc.), AGE inhibitors (e.g., ALT-945, pimagedine, pyratoxathine, N-phenacylthiazolium bromide (ALT-766), EXO-226, etc.), active oxygen scavengers (e.g., thioctic acid, etc.), cerebral vasodilators (e.g., tiapride, etc.) and the like.

### (4) Antihyperlipidemic agent

Statin compounds inhibiting cholesterol synthesis (e.g., pravastatin, simvastatin, lovastatin, atorvastatin, fluvastatin, cerivastatin or their salt (e.g., sodium salt, etc.), etc.), squalene synthase inhibitors, fibrate compounds having triglyceride lowering action (e.g., bezafibrate, clofibrate, simfibrate, clinofibrate, etc.) and the like.

### (5) Hypotensive agent

Angiotensin converting enzyme inhibitors (e.g., captopril, enalapril, delapril, etc.), angiotensin II antagonists (e.g., losartan, candesartan cilexetil, etc.), calcium antagonists (e.g., manidipine, nifedipine, amlodipine, efonidipine, nicardipine, etc.), clonidine, and the like.

### (6) Antiobesity agent

Antiobesity drugs acting on the central nervous system (e.g. dexfenfluramine, fenfluramine, phentermine, sibutramine, anfepramone, dexamphetamine, mazindol, phenylpropanolamine, clobenzorex, etc.), pancreatic lipase inhibitors (e.g. orlistat, etc.), β3 agonists (e.g. CL-316243, SR-58611-A, UL-TG-307, AJ-9677, AZ40140, etc.), anorectic peptides (e.g. leptin, CNTF (Ciliary Neurotrophic Factor), etc.), cholecystokinin agonists (e.g. lintitript, FPL-15849, etc.).

### (7) Diuretic agent

Xanthine derivatives (e.g., theobromine sodium salicylate, theobromine calcium salicylate, etc.), thiazide preparations (e.g., ethiazide, cyclopenthiazide, trichlormethiazide, hydrochlorothiazide, hydroflumethiazide, benzylhydrochlorothiazide, penflutizide, polythiazide, methyclothiazide, etc.), antialdosterone preparations (e.g., spironolactone, triamterene, etc.), carbonic anhydrase inhibitors (e.g., acetazolamide, etc.), chlorobenzenesulfonamide preparations (e.g., chlorthalidone, mefruside, indapamide, etc.), azosemide, isosorbide, ethacrynic acid, piretanide, bumetanide, furosemide, etc.

### (8) Chemotherapeutic agent

Alkylating agents (e.g., cyclophosphamide, ifosamide, etc.), metabolic antagonists (e.g., methotrexate, 5-fluorouracil, etc.), antitumor antibiotics (e.g., mitomycin, adriamycin, etc.), plant-derived antitumor agents (e.g., vincristine, vindesine, taxol, etc.), cisplatin, carboplatin, etoposide, etc. Among these, 5-fluorouracil derivatives such as Furtulon and Neo-Furtulon are preferred.

### (9) Immunotherapeutic agent

Microorganism- or bacterium-derived components (e.g., muramyl dipeptide derivatives, Picibanil, etc.), immunopotentiator polysaccharides (e.g., lentinan, schizophyllan, krestin, etc.), genetically engineered cytokines (e.g., interferons, interleukins (IL), etc.), colony stimulating factors (e.g., granulocyte colony stimulating factor, erythropoietin, etc.) and the like. Among these, IL-1, IL-2, IL-12, etc. are preferred.

### (10) Therapeutic agent recognized to ameliorate cachexia in animal models or clinical practice

Progesterone derivatives (e.g., megestrol acetate) [Journal of Clinical Oncology, vol. 12, pp. 213-225, 1994], metoclopramide pharmaceuticals, tetrahydrocannabinol pharmaceuticals (the above references are applied to both), fat metabolism ameliorating agents (e.g., eicosapentaenoic acid) [British Journal of Cancer, vol. 68, pp. 314-318, 1993], growth hormones, IGF-1, and antibodies to the cachexia-inducing factors such as TNF-α, LIF, IL-6 and oncostatin M.

### (11) Antiinflammatory agent

Steroids (e.g., dexamethasone, etc.), sodium hyaluronate, cyclooxygenase inhibitors (e.g., indomethacin, ketoprofen, loxoprofen, meloxicam, ampiroxicam, celecoxib, rofecoxib, etc.) and the like.

### (12) Miscellaneous

Glycosylation inhibitors (e.g., ALT-711, etc.), nerve regeneration promoting drugs (e.g., Y-128, VX853, prosaptide, etc.), drugs acting on the central nervous system (e.g., antidepressants such as desipramine, amitriptyline, imipramine, fluoxetine, paroxetine, doxepin, etc.), anticonvulsants (e.g., lamotrigine, carbamazepine), antiarrhythmic drugs (e.g., mexiletine), acetylcholine receptor ligands (e.g., ABT-594), endothelin receptor antagonists (e.g., ABT-627), monoamine uptake inhibitors (e.g., tramadol), indoleamine uptake inhibitors (e.g., fluoxetine, paroxetine), narcotic analgesics (e.g., morphine), GABA receptor agonists (e.g., gabapentin), GABA uptake inhibitors (e.g., tiagabine), α₂ receptor agonists (e.g., clonidine), local analgesics (e.g., capsaicin), protein kinase C inhibitors (e.g., LY-333531), antianxiety drugs (e.g., benzodiazepines), phosphodiesterase inhibitors (e.g., sildenafil), dopamine receptor agonists (e.g., apomorphine), dopamine receptor antagonists (e.g., haloperidol), serotonin receptor agonists (e.g., tandospirone citrate, sumatryptan), serotonin receptor antagonists (e.g., cyproheptadine hydrochloride, ondansetron), serotonin uptake inhibitors (e.g., fluvoxamine maleate, fluoxetine, paroxetine), hypnotics (e.g., triazolam, zolpidem), anticholinergic agents, α₁ receptor blocking agents (e.g., tamsulosin, silodosin, naftopidil), muscle relaxants (e.g., baclofen), potassium channel openers (e.g., nicorandil), calcium channel blocking agents (e.g., nifedipine), agents for preventing and/or treating Alzheimer's disease (e.g., donepezil, rivastigmine, galanthamine), agents for treating Parkinson's disease (e.g., L-dopa), agents for preventing and/or treating multiple sclerosis (e.g., interferon β-1a), histamine H₁ receptor inhibitors (e.g., promethazine hydrochloride), proton pump inhibitors (e.g., lansoprazole, omeprazole), antithrombotic agents (e.g., aspirin, cilostazol), NK-2 receptor antagonists, agents of treating HIV infection (saquinavir, zidovudine, lamivudine, nevirapine), agents of treating chronic obstructive pulmonary diseases (salmeterol, thiotropium bromide, cilomilast), etc.

Anticholinergic agents include, for example, atropine, scopolamine, homatropine, tropicamide, cyclopentolate, butylscopolamine bromide, propantheline bromide, methylbenactyzium bromide, mepenzolate bromide, flavoxate, pirenzepine, ipratropium bromide, trihexyphenidyl, oxybutynin, propiverine, darifenacin, tolterodine, temiverine, trospium chloride or a salt thereof (e.g., atropine sulfate, scopolamine hydrogen bromide, homatropine hydrogen bromide, cyclopentolate hydrochloride, flavoxate hydrochloride, pirenzepine hydrochloride, trihexyphenidyl hydrochloride, oxybutynin hydrochloride, tolterodine tartrate, etc.), preferably, oxybutynin, propiverine, darifenacin, tolterodine, temiverine, trospium chloride or a salt thereof (e.g., oxybutynin hydrochloride, tolterodine tartrate, etc.). In addition, acetylcholinesterase inhibitors (e.g., distigmine, etc.) and the like can be used.
NK-2 receptor antagonists include, for example, a piperidine derivative such as GR159897, GR149861, SR48968 (saredutant), SR144190, YM35375, YM38336, ZD7944, L-743986, MDL105212A, ZD6021, MDL105172A, SCH205528, SCH62373, R-113281, etc., a perhydroisoindole derivative such as RPR-106145, etc., a quinoline derivative such as SB-414240, etc., a pyrrolopyrimidine derivative such as ZM-253270, etc., a pseudopeptide derivative such as MEN11420 (nepadutant), SCH217048, L-659877, PD-147714 (CAM-2291), MEN10376, S16474, etc., and others such as GR100679, DNK333, GR94800, UK-224671, MEN10376, MEN10627, or a salt thereof, and the like.

In combination of the compound of the present invention and the concomitant drug, the administration time of the compound of the present invention and the concomitant drug is not restricted, and the compound of the present invention or a pharmaceutical composition thereof and the concomitant drug or a pharmaceutical composition thereof can be administered to the administration subject simultaneously, or may be administered at different times. The dosage of the concomitant drug may be determined according to the administration amount clinically used, and can be appropriately selected depending on the administration subject, administration route, disease, combination and the like.
The concomitant administration mode is not particularly restricted, and it is sufficient that the compound of the present invention and the concomitant drug are combined in administration. Examples of such administration mode include the following methods:
(1) The compound of the present invention or a pharmaceutical composition thereof and the concomitant drug are simultaneously produced to give a single preparation which is administered. (2) The compound of the present invention or a pharmaceutical composition thereof and the concomitant drug or a pharmaceutical composition thereof are separately produced to give two kinds of preparations which are administered simultaneously by the same administration route. (3) The compound of the present invention or a pharmaceutical composition thereof and the concomitant drug or a pharmaceutical composition thereof are separately produced to give two kinds of preparations which are administered by the same administration route only at the different times. (4) The compound of the present invention or a pharmaceutical composition thereof and the concomitant drug or a pharmaceutical composition thereof are separately produced to give two kinds of preparations which are administered simultaneously by different administration routes. (5) The compound of the present invention or a pharmaceutical composition thereof and the concomitant drug or a pharmaceutical composition thereof are separately produced to give two kinds of preparations which are administered by different administration routes at different times (for example, the compound of the present invention or a pharmaceutical composition thereof; the concomitant drug or a pharmaceutical composition thereof are administered in this order, or in the reverse order).
The mixing ratio of compound of the present invention and a concomitant drug in the combination drug of the present invention can be appropriately determined according to the subject of administration, administration route, disease and the like.
For example, while the content of compound of the present invention in the combination drug of the present invention varies depending on the form of the preparation, it is generally about 0.01 to about 100 wt%, preferably about 0.1 to about 50 wt%, more preferably about 0.5 to about 20 wt%, relative to the whole preparation.

While the content of the concomitant drug in the combination drug of the present invention varies depending on the form of the preparation, it is generally about 0.01 to about 100 wt%, preferably about 0.1 to about 50 wt%, more preferably about 0.5 to about 20 wt%, relative to the whole preparation.
While the content of the additive such as a carrier and the like in the combination drug of the present invention varies depending on the form of the preparation, it is generally about 1 to about 99.99 wt%, preferably about 10 to about 90 wt%, relative to the whole preparation.
Similar contents can be employed when the compound of the present invention and the concomitant drug are independently formulated.
While the dose varies depending on the kind of the compound of the present invention or a pharmaceutically acceptable salt thereof, administration route, symptom, age of patients and the like, for example, for oral administration to an adult patient with stress urinary incontinence, obesity and/or pelvic organ prolapse, it is about 0.005 - 50 mg, preferably about 0.05 - 10 mg, more preferably about 0.2 - 4 mg/kg body weight/day as the compound of the present invention, which can be administered in 1 to about 3 portions.
When the pharmaceutical composition of the present invention is a sustained-release preparation, the dose varies depending on the kind and content of the compound in the present invention, dosage form, period of sustained drug release, subject animal of administration (e.g., mammals such as human, rat, mouse, cat, dog, rabbit, bovine, swine and the like) and administration object. For parenteral administration, for example, about 0.1 - about 100 mg of the compound in the present invention only needs to be released in one week from the administered preparation.
The dose of the concomitant drug may be set within the range such that it causes no problems of side effects. The daily dose as the concomitant drug varies depending on severity of symptoms, age, sex, weight and sensitivity of the subject to be administered, time and interval of administration, property, formulation and kinds of pharmaceutical preparation, kinds of active ingredients, etc., and is not particularly limited. In the case of oral administration, a daily dosage in terms of drugs is usually in the order of about 0.001 to 2000 mg, preferably about 0.01 to 500 mg, and more preferably about 0.1 to 100 mg, per 1 kg body weight of mammals, which may be administered once a day or in two to four divided portions a day.

In administering the combination drug of the present invention, it may be administered at the same time or, the concomitant drug may be administered before administering the compound of the present invention, and vice versa. In case of staggered administration, the time interval varies depending on the active ingredients to be administered, a formulation and an administration route. For example, if the concomitant drug is administered first, the compound of the present invention may be administered 1 minute to 3 days, preferably 10 minutes to 1 day, more preferably 15 minutes to 1 hour after administering the concomitant drug. If the compound of the present invention is administered first, the concomitant drug may be administered 1 minute to 1 day, preferably 10 minutes to 6 hours, more preferably 15 minutes to 1 hour after administering the compound of the present invention.
The pharmaceutical composition of the present invention shows low toxicity and can be used safely. Particularly, since the Example compounds shown below are superior in the absorption by oral administration, they can be advantageously used for oral preparations.

The screening method of the present invention can be performed by examining an influence of a test substance on the total urethral resistance of an animal (e.g., non-human mammal) between administration and without administration of the substance based on the measurement of the leak point pressure. The "leak point pressure" refers to an intravesicular pressure when leakage of urine has occurred without contraction of detrusor muscles, and shows the maximum urethral resistance that can resist an increase in the intravesicular pressure. In the screening method of the present invention, the leak point pressure is an intravesicular pressure when incontinence is induced by injecting saline rapidly and transiently with, preferably, an instrument capable of controlling the rate such as an infusion pump and the like into the bladder of an animal showing no micturition reflex due to an operation such as transection of the spinal cord and the like but showing functional bladder-spinal cord-urethral reflex. The injection rate of saline into the bladder is set so that leakage of the intravesical fluid is observed preferably in 1 sec - 1 hr, more preferably 5 sec - 30 sec. A specific measurement method of the leak point pressure is described in detail in the below-mentioned Examples.

The gender of the "animal" to be used in the present invention is preferably female, and the species is, for example, a non-human animal such as monkey, dog, cat, rabbit, guinea pig, hamster, rat, mouse, Mongolian gerbil and the like can be mentioned. Among these, rats (Wistar, SD and the like) are most preferable.
While the weeks of age, body weight, experience of birth and the like of the "animals" to be used in the present invention are not particularly limited as long as they are applicable to the desired screening, the conditions may be changed as appropriate. The "animals" to be used in the present invention may be normal animals (those free of pathology and the like), but those showing lower leak point pressure upon a rise in the intravesical pressure are preferably used. Here, the lower leak point pressure is preferably caused by transection or injury of the nerve relating to urethra closing reflex or decreased weight of the pelvic floor muscles and the external urethral sphincter. To lower the reflex contraction force of the pelvic floor muscles and the external urethral sphincter, physical, chemical or biological transection or injury of the nerve relating to urethra closing reflex (for example, pelvic nerve, pudendal nerve, nerve heading for iliococcygeal muscle/pubococcygeal muscle and the like), childbirth, ovariectomy, mechanical vaginal expansion, induction of diabetes, drug administration or a combination of these is only needed.
As the drug, for example, α-bungarotoxin, d-tubocurarine, pancuronium, decamethonium, suxamethonium, which are myoneural junction blockers, botulinum toxin A which is a neurotoxin, and the like are used.
These animal models can be produced according to a known method, for example, the methods described in Urology, 1998, vol. 52, p. 143-151, The Journal of Urology, 2001, vol. 166, p. 311-317, and The Journal of Urology, 2006, vol. 176, p. 819-823.

According to the screening method of the present invention, moreover, a leak point pressure upon a rapid and transient rise in the intravesicular pressure of an animal can be measured efficiently and stably. As a method for rapidly and transiently rising the intravesicular pressure by a stable procedure, for example, saline is rapidly injected (preferably not more than 3600 ml/hr, more preferably 360 ml/hr) into the bladder with an infusion pump and the like, infusion is stopped simultaneously with leakage of saline from the meatus urethra, and the intravesicular pressure is released and the like.
As a test substance, known or novel synthetic compounds, physiologically active substances derived from naturally occurring substances, peptides, proteins and the like, as well as, for example, tissue extracts, cell culture supernatants and the like of warm-blooded animals (for example, mouse, rat, swine, bovine, sheep, monkey, human and the like) are used.
Using this screening method, a substance that increases the leak point pressure upon a rise in the intravesical pressure can be screened for using, as an index, an increase in the leak point pressure by the administration of a test substance as compared to that without administration thereof.
For example, when this screening method is applied to rats and the leak point pressure has increased by about 5 cmH₂O, preferably not less than about 10 cmH₂O, more preferably not less than about 15 cmH₂O, by the administration of a test substance as compared to that without administration thereof, the test substance is determined to have a stress urinary incontinence-improving effect.

In the screening method of the present invention, a leak point pressure upon a rise in the intravesicular pressure of an animal is measured, and can be beneficially and efficiently applied to screening for a substance used for the prophylaxis or treatment of stress urinary incontinence (for example, adrenaline α1 receptor agonist, adrenaline β2 receptor agonist, serotonin receptor agonist, serotonin uptake inhibitory substance, noradrenaline uptake inhibitory substance, serotonin and noradrenaline uptake inhibitory substance and the like).
For example, a prophylactic or therapeutic drug for stress urinary incontinence can be evaluated by administering about 0.0001 - about 1000 mg/kg (preferably, about 0.001 about 100 mg/kg) of a test substance to a non-human mammal in the screening method of the present invention, and examining the treatment effect of the test substance with the leak point pressure as an index. Here, the concept of the prophylaxis of stress urinary incontinence includes suppress of decline of urethral resistance, and the concept of the treatment of stress urinary incontinence includes improvement of stress urinary incontinence, suppression of progression, and prophylaxis of aggravation. The timing of administration of a test substance to an animal in the evaluation method of the present invention is, for example, before or after a leak point pressure lowering treatment, during a leak point pressure measurement and the like. Drugs aiming at the prophylaxis or treatment of stress urinary incontinence can be evaluated according to the respective administration periods.
The animals to be used in the present invention may be normal animals (those free of pathology and the like), but those showing pathology such as stress urinary incontinence, overactive bladder, benign prostatic hyperplasia, detrusor underactivity, diabetes, diabetes neurosis, hypertension, obesity, hyperlipidemia, arteriosclerosis, gastric ulcer, asthma, chronic closure respiratory diseases, uterus cancer, cerebrovascular disorder, brain damage, spinal trauma and the like, for example, obese rats (Wistar fatty rat) and the like may be used for the aforementioned leak point pressure measurement. When an animal showing such pathology is subjected to the aforementioned leak point pressure measurement, a new stress urinary incontinence animal model can be searched for, and the animal model can be effectively applied to screening for a medical substance used for then prophylaxis or treatment of such complications, can be applied to screening for a medical substance effective for, for example, the aforementioned pathology (e.g., digestive organ diseases such as gastric ulcer and the like, and the like) alone and noninfluential on stress urinary incontinence, and can be applied to screening to exclude a test substance that induces stress urinary incontinence from those to be selected.

Furthermore, the screening method of the present invention can also be applied effectively to screening for a medical substance for the prophylaxis or treatment of stress urinary incontinence by applying a test substance and determining a leak point pressure increasing effect, as well as screening for various medical substances by applying a test substance and determining an influence (including aggravation, no influence, improvement) on urethral resistance upon a rise in the intravesical pressure. That is, it can be applied effectively to screening to exclude a test substance that aggravates stress urinary incontinence from a medical substance to be selected; select a test substance noninfluential on stress urinary incontinence as a medical substance for the prophylaxis or treatment of a disease other than stress urinary incontinence; select a test substance showing a stress urinary incontinence-improving effect as a medical substance for the prophylaxis or treatment of stress urinary incontinence or complications of stress urinary incontinence and a certain kind of disease (e.g., urological diseases such as overactive bladder and the like, and the like); and the like. The screening method of the present invention, unlike conventional methods, can be applied as a useful evaluation system to screening for various medical substances for any object mentioned above, since evaluation is possible by rapidly increasing the intravesicular pressure conveniently by a stable procedure in an animal showing no micturition reflex but showing functional bladder-spinal cord-urethra reflex.
A substance obtained by the screening method of the present invention can be formulated into a preparation and used as a prophylactic or therapeutic agent for stress urinary incontinence, like the aforementioned substance of the present invention.

The screening methods of a prophylactic or therapeutic drug for cystoceles or enteroceles are described in the following.
The screening methods of the present invention can be performed by administering a test substance to a test animal and without administration thereof, and examining the influence of the test substance on the contractile responses of the pelvic floor muscles in the animal (e.g., non-human mammal) in terms of reflex closure responses in the urethra or urethral resistance (leak point pressure) upon a rise in intravesical pressure. The animals used in the screening methods of the present invention have the hypogastric nerve and the pudendal nerve cut bilaterally. Muscular tissues that influence urethral closure pressure include the internal urethral sphincter, which is a smooth muscle, the external urethral sphincter and the pelvic floor muscles, which are striated muscles, and the like; the pelvic floor muscles include the iliococcygeus muscle and the pubococcygeus muscle. The internal urethral sphincter is under the control of the hypogastric nerve, the external urethral sphincter is under the control of the pudendal nerve, and the iliococcygeus muscle and the pubococcygeus muscle are under the control of the nerves that lead to the iliococcygeus muscle and the pubococcygeus muscle (no unified names across species available). When a water reservoir connected to the urinary bladder is raised to a given height, a reflex urethral contractile response is observed in the middle urethra (see "American Journal of Physiology Renal Physiology", 2004, vol. 287, p. F434-441); by increasing the intravesical pressure in an animal having the hypogastric nerve and the pudendal nerve cut bilaterally, and monitoring the resulting urethral contractile responses or leak point pressure, urethral closure responses or urethral resistance, which are attributed mainly to the pelvic floor muscles (the iliococcygeus muscle and the pubococcygeus muscle), can be measured, and the contractile responses of the pelvic floor muscles can be evaluated. "Leak point pressure upon a rise in intravesical pressure" refers to an intravesical pressure upon occurrence of urine leakage without contraction of the detrusor, showing the maximum urethral resistance against rises in intravesical pressure. Applying "the reflex contractile responses of the pelvic floor muscles upon a rise in intravesical pressure", the reflex contractile responses of the pelvic floor muscles can be evaluated also by measuring reflex closure responses in the vagina. When a drug that increases such values can be found by examining the reflex closure response of the urethra or vagina and the urethral resistance (leak point pressure) upon a rise in the intravesical pressure, a prophylactic or therapeutic drug for cystoceles or enteroceles can be obtained.

As the gender of the "animal" to be used in the present invention, female is preferable, and the species is a non-human animal such as monkey, dog, cat, rabbit, guinea pig, hamster, rat, mouse, Mongolian gerbil and the like can be mentioned, and rat (Wistar, SD and the like) is most preferable.
While the age in weeks, body weight, delivery or non-delivery and the like of the "animal" to be used in the present invention are not particularly limited as long as they are applicable to the objective screening, these conditions may be appropriately changed. As the "animal" to be used in the present invention, normal animals (animals free of pathosis and the like) may be used. In addition, animals with functional decline of pelvic floor muscles, such as those having partially sectioned or injured nerve involved in the contraction of the pelvic floor muscles or having decreased weight of the pelvic floor muscles, can also be used. To decrease the reflex contractile force of the pelvic floor muscles or the weight of the pelvic floor muscles, a part of the nerve involved in the reflex contraction reflex of the pelvic floor muscles (e.g., pelvic nerve, nerve to
iliococcygeus muscle/pubococcygeus muscle and the like) is physically, chemically or biologically sectioned or injured,
the animal is made to deliver, the ovary is removed, vagina is mechanically expanded, diabetes is induced, a drug is administered, or these may be combined.
As the drug, for example, α-bungarotoxin, d-tubocurarine, pancuronium, decamethonium, suxamethonium and the like, which are neuromuscular junction blockers, botulinum toxin A, which is a neurotoxin, and the like are used.
These model animals can be produced according to a known method, for example, the method described in Urology, 1998, vol. 52, p. 143-151, Journal of Urology, 2001, vol. 166, p. 311-317, The Journal of Urology, 2006, vol. 176, p. 819-823.
As the test substance, a known or novel synthetic compound, a physiological active substance derived from a naturally occurring substance, peptide, protein and the like and, for example, tissue extract, cell culture supernatant and the like of warm-blooded mammals (e.g., mouse, rat, swine, bovine, sheep, monkey, human and the like) are used.

Using this screening method, a substance that increases the contractile force of the pelvic floor muscles can be screened for using, as an index, the fact that the reflex closure response or the leak point pressure increases when a test substance is administered than without administration.
For example, by performing the present screening method using rats, a test substance can be evaluated to have an improving effect on cystoceles and enteroceles when the contractile response of the pelvic floor muscles increased by not less than about 100%, preferably not less than about 20%, more preferably not less than about 50%, with administration of the test substance as compared to the absence of administration of the test substance.

The screening method of the present invention measures the contractile response of the pelvic floor muscles during an abrupt increase in abdominal pressure in an animal, and is useful for and efficiently applicable to the screening for a substance usable for the prophylaxis or treatment of cystoceles or enteroceles.
For example, in the screening method of the present invention, about 0.0001 - about 1000 mg/kg (preferably about 0.001 - about 100 mg/kg) of a test substance is administered to a non-human mammal, and the treatment effect of the test substance is examined with the contractile response of the pelvic floor muscles as an index, whereby a drug for the prophylaxis or treatment of cystoceles or enteroceles can be evaluated. Here, the concept of the treatment of cystoceles or enteroceles includes improvement, suppression of progression and prophylaxis of aggravation of cystoceles and enteroceles. In the evaluation method of the present invention, a test substance is administered to an animal before or after the treatment for decreasing the reflex contractile force of pelvic floor muscles (or leak point pressure), or during the measurement of contractile force (or leak point pressure) and the like. A drug can be evaluated for the purpose of preventing or treating cystoceles and enteroceles according to each administration period.
While the animals to be used in the present invention may be a normal animal (animal free of pathosis), for example, an animal (e.g., overweight rat (Wistar fatty rat) and the like) showing the pathosis of urinary incontinence, overactive bladder, benign prostatic hyperplasia, detrusor underactivity, diabetes, diabetes neuropathy, hypertension, obesity, hyperlipidemia, arteriosclerosis, gastric ulcer, asthma, chronic obstructive respiratory disease, uterus cancer, cerebrovascular disorder, brain damage, spinal cord injury and the like may be used for the measurement of the aforementioned reflex contraction of the pelvic floor muscles. When animals showing such pathosis are subjected to the aforementioned measurement of the contractile force of the pelvic floor muscles (or leak point pressure), a new model animal of cystoceles and enteroceles can be searched for, and the measurement can be effectively applied to the screening for a pharmaceutical substance for the prophylaxis or treatment of the complications. For example, the measurement is applicable to the screening for a pharmaceutical substance effective only for the aforementioned pathosis (e.g., digestive disease such as gastric ulcer etc., and the like) and free of an influence on the cystoceles and enteroceles, and also applicable to the screening for the purpose of removing a test substance inducing cystoceles and enteroceles from a pharmaceutical substance to be selected.

Furthermore, the screening method of the present invention can be effectively applied to the screening for a pharmaceutical substance for the prophylaxis or treatment of cystoceles or enteroceles by applying a test substance and testing increasing effect on the contractile force of pelvic floor muscles (or leak point pressure), as well as effectively applied to the screening for the purpose of applying a test substance and selecting a test substance showing an improving effect on cystoceles and enteroceles, as a pharmaceutical substance for the prophylaxis or treatment of cystoceles or enteroceles or the complications with a certain kind of disease; and the like. The substance obtained by the screening method of the present invention can be formed as a preparation in the same manner as in the aforementioned substance of the present invention and can be used as an agent for the prophylaxis or treatment of cystoceles or enteroceles. Examples

The present invention is further described in detail with reference to Examples, Formulation Examples and Experimental Examples which are not intended to restrict the invention and may be modified without departing from the scope of the invention.
In Examples, column chromatography was performed using Purif-8 or Purif-α2 manufactured by MORITEX and under observation by a UV detector. The silica gel used for column chromatography was Purif-Pack manufactured by MORITEX. The room temperature referred herein means temperature generally from about 10°C to 30°C.
The abbreviations in Examples mean the following.
LC: liquid chromatography
MS: mass spectrometry spectrum
ESI: electrospray ionization method
M: molecular weight
NMR: nuclear magnetic resonance spectrum
Hz: hertz
J: coupling constant
m: multiplet
q: quartet
t: triplet
d: doublet
dd: double doublet
s: singlet
dt: double triplet
brs: broad singlet
Boc: tert-butyloxycarbonyl group
Ph: phenyl group
N: normal concentration
DMF: N,N-dimethylformamide
THF: tetrahydrofuran
DMSO: dimethyl sulfoxide
DME: dimethoxyethane
MeOH: methanol
NMP: 1-methyl-pyrrolidin-2-one
(Boc)₂O: di-tert-butyl bicarbonate
XPhos: dicyclohexyl(2',4',6'-triisopropylbiphenyl-2-yl)phosphine
BINAP: 2,2'-bis(diphenylphosphino)-1,1'-binaphthyl
5-HT: serotonin (or 5-hydroxytryptamine)

LC-MS in Examples was measured under the following conditions.

### Analysis by LC-MS

Measurement device: Waters LC-MS system
HPLC: Agilent HP1100
MS: Micromass ZQ

### HPLC conditions

Column: CAPCELL PAK C18UG120, S-3 µm, 1.5×35 mm

### (Shiseido)

Solvent: Solution A; water containing 0.05% trifluoroacetic acid, Solution B; acetonitrile containing 0.05% trifluoroacetic acid
Gradient cycle: 0.00 min (Solution A/Solution B=90/10), 2.00 min (Solution A/Solution B=5/95), 2.75 min (solution A/Solution B=5/95), 2.76 min (Solution A/Solution B=90/10), 3.60 min (Solution A/Solution B=90/10)
Injection volume: 2 µL, flow rate: 0.5 mL/min, detection method: UV 220 nm

### MS conditions

Ionization method: ESI

Purification by preparative HPLC in Examples was carried out under the following conditions.
Instrument: Gilson high-throughput purification system
Column: CombiPrep ODS-AS-5 µm, 50x20 mm (YMC)
Solvent: Solution A; water containing 0.1% trifluoroacetic acid, Solution B; acetonitrile containing 0.1% trifluoroacetic acid
Gradient cycle: 0.00 min (Solution A/Solution B=95/5), 1.00 min (Solution A/Solution B=95/5), 5.20 min (Solution A/Solution B=5/95), 6.40 min (Solution A/Solution B=5/95), 6.50 min (Solution A/Solution B=95/5), 6.60 min (Solution A/Solution B=95/5)
Flow rate: 25 mL/min, detection method: UV 220 nm

### Example 1

### 9-Methyl-2-morpholin-4-yl-6,7,8,9-tetrahydro-5H-pyrido[3,2-c]azepine

### (step 1)

To a solution of 2,6-dichloronicotinic acid (50.0 g) and DMF (10 mL) in toluene (1 L) was added dropwise oxalyl dichloride (22.3 mL) under ice-cooling, and the mixture was stirred at room temperature for 1.5 hr. The reaction mixture was concentrated, and THF (200 mL) was added to the residue. To this solution was added a solution of benzylamine (30.7 g) in THF (300 mL), and 1N aqueous sodium hydroxide solution (500 mL) was added. After stirring at room temperature for 1 hr, the reaction solution was poured into water, and the mixture was extracted with ethyl acetate. The organic layer was washed with saturated brine, dried over anhydrous magnesium sulfate, and concentrated. The obtained residue was recrystallized from ethyl acetate to give N-benzyl-2,6-dichloronicotinamide (56.2 g, 77%) as a solid.
¹H-NMR(CDCl₃):δ4.67(2H,d,J=5.6Hz), 6.79(1H,brs), 7.28-7.49(6H,m), 8.15(1H,d,J=8.1Hz)
MS(ESI+):281(M+H)

### (step 2)

To a solution of the compound (30.0 g) obtained in step 1 in THF (200 mL) was added a 1 mol/L solution (373 mL) of BH₃ THF complex in THF under ice-cooling, and the mixture was stirred at 70°C for 2 hr. The reaction solution was cooled to room temperature, methanol was added under ice-cooling, and the mixture was concentrated. To the residue was added 6N hydrochloric acid (200 mL), and the mixture was refluxed for 2 hr. To the reaction solution was added 2N aqueous sodium hydroxide solution (200 mL) at room temperature, and the mixture was extracted with ethyl acetate. The extract was washed with water, dried over anhydrous magnesium sulfate, and concentrated. The obtained residue was purified by silica gel column chromatography (solvent gradient: 0→30% ethyl acetate/hexane) to give N-benzyl-1-(2,6-dichloropyridin-3-yl)methanamine (15.8 g, 55%) as an oil.
¹H-NMR(CDCl₃):δ3.82(2H,s), 3.87(2H,s), 7.23-7.37(6H,m), 7.81(1H,d,J=7.9Hz)
MS(ESI+):267(M+H)

### (step 3)

To a solution of the compound (8.88 g) obtained in step 2 in acetonitrile (100 mL) were added potassium carbonate (9.18 g), sodium iodide (0.498 g) and 4-bromo-1-butene (4.94 g), and the mixture was stirred at 85°C for 30 hr. The reaction solution was poured into water, and the mixture was extracted with ethyl acetate. The extract was washed with water, dried over anhydrous magnesium sulfate, and concentrated. The residue was purified by silica gel column chromatography (solvent gradient: 5→10% ethyl acetate/hexane) to give N-benzyl-N-[(2,5-dichloropyridin-3-yl)methyl]but-3-en-1-amine (6.17 g, 58%) as an oil.
¹H-NMR(CDCl₃):δ2.25-2.32(2H,m), 2.56(2H,t,J=7.3Hz), 3.64(4H,s), 4.95-5.07(2H,m), 5.68-5.82(1H,m), 7.23-7.34(6H,m), 7.92(1H,d,J=7.9Hz)
MS(ESI+):322 (M+H)

### (step 4)

A mixed solution of the compound (7.68 g) obtained in step 3, potassium carbonate (6.61 g), tetrabutylammonium bromide (15.4 g), palladium acetate (0.537 g) and tri(2-methylphenyl)phosphine (0.727 g) in DMF (50 mL) was stirred under a nitrogen atmosphere at 130°C for 20 hr. The reaction solution was cooled, filtered through celite, and the filtrate was concentrated. The obtained residue was purified by silica gel column chromatography (solvent gradient: 7→25% ethyl acetate/hexane) to give 6-benzyl-2-chloro-9-methylene-6,7,8,9-tetrahydro-5H-pyrido[3,2-c]azepine (1.90 g, 28%) as an oil. ¹H-NMR(CDCl₃):δ2.52-2.58(2H,m), 2.94-3.02(2H,m), 3.58(2H,s), 3.67(2H,s), 5.25-5.28(1H,m), 5.60(1H,d,J=1.5Hz), 7.02-7.05(1H,m), 7.09-7.12(1H,m), 7.16-7.30(5H,m)
MS(ESI+):285(M+H)

### (step 5)

A mixture of the compound (0.500 g) obtained in step 4, sodium tert-butoxide (0.253 g), morpholine (0.305 g), tris(dibenzylideneacetone)dipalladium (0) (0.0643 g), XPhos (0.100 g) and toluene (5 mL) was stirred under a nitrogen atmosphere at 100°C for 20 hr. The reaction solution was cooled to room temperature, filtered through celite, and concentrated. The obtained residue was purified by silica gel column chromatography (solvent gradient: 10→30% ethyl acetate/hexane) to give 6-benzyl-9-methylene-2-morpholin-4-yl-6,7,8,9-tetrahydro-5H-pyrido[3,2-c]azepine (0.317 g, 54%) as an oil.
¹H-NMR(CDCl₃):δ2.61-2.69(2H,m), 2.97-3.06(2H,m), 3.46-3.55(4H,s), 3.65(2H,s), 3.71(2H,s), 3.79-3.88(4H,m), 5.18-5.27(1H,m), 5.60-5.69(1H,m), 6.46(1H,d,J=8.5Hz), 7.11(1H,d,J=8.5Hz), 7.28-7.39(5H,m)
MS(ESI+):336(M+H)

### (step 6)

A suspension of the compound (0.317 g) obtained in step 5 and 10% palladium/carbon catalyst (0.200 g) in methanol (3 mL) was stirred under a hydrogen atmosphere at room temperature for 2 hr. The insoluble material was filtered off through celite, and the filtrate was concentrated. The obtained residue was purified by basic silica gel column chromatography (solvent gradient: 70→100% ethyl acetate/hexane) to give 9-methyl-2-morpholin-4-yl-6,7,8,9-tetrahydro-5H-pyrido-[3,2-c]azepine (0.160 g, 69%) as an oil.
¹H-NMR(CDCl₃):δ1.37(3H,d,J=6.8Hz), 1.43-1.59(1H,m), 1.59-1.82(1H,m), 3.05-3.17(1H,m), 3.17-3.28(1H,m), 3.29-3.40(1H,m) 3.42-3.55(4H,m), 3.79-3.88(6H,m), 6.34(1H,d,J=8.3Hz), 7.22(1H,d,J=8.3Hz)
MS(ESI+):248(M+H)

### Example 2

### 9-Methyl-2-morpholin-4-yl-6,7,8,9-tetrahydro-5H-pyrido[3,2-c]azepine hydrochloride

A mixture of the compound (0.160 g) obtained in Example 1 and 4N hydrogen chloride-ethyl acetate solution (5 mL) was stirred at room temperature for 16 hr, and concentrated. The obtained residue was recrystallized from ethanol/diisopropyl ether to give 9-methyl-2-morpholin-4-yl-6,7,8,9-tetrahydro-5H-pyrido[3,2-c]azepine hydrochloride (0.134 g, 73%) as a solid. ¹H-NMR(DMSO-d₆):δ1.30(3H,d,J=7.0Hz), 1.52-1.70(1H,m), 1.82-1.94(1H,m), 3.22-3.38(3H,m), 3.43-3.50(4H,m), 3.64-3.73(4H,m), 4.10-4.28(2H,m), 6.63(1H,d,J=8.5Hz), 7.53(1H,d,J=8.5Hz), 8.90(2H,brs)
MS(ESI+):248(M-HCl+H)

### Example 3

### 8-Ethyl-2-morpholin-4-yl-5,6,7,8-tetrahydro-1,6-naphthyridine

### (step 1)

A mixed solution of the compound (2.00 g) obtained in Example 1, step 3, potassium carbonate (1.72 g), palladium acetate (0.139 g), and tri(2-methylphenyl)phosphine (0.417 g) in DMF (60 mL) was stirred under a nitrogen atmosphere at 100°C for 16 hr. The reaction solution was filtered through celite, poured into water, and the mixture was extracted with ethyl acetate, dried over anhydrous magnesium sulfate, and concentrated. The obtained residue was purified by silica gel column chromatography (solvent gradient: 5→30% ethyl acetate/hexane) to give a 1:3 mixture of exo form and endo form as an oil. A mixture of the obtained mixture (0.627 g), sodium tert-butoxide (0.211 g), morpholine (0.383 g), tris(dibenzylideneacetone)dipalladium (0) (0.0807 g), XPhos (0.126 g) and toluene (5 mL) was stirred under a nitrogen atmosphere at 100°C for 15 hr. The reaction solution was filtered through celite, and concentrated. The obtained residue was purified by silica gel column chromatography (solvent gradient: 10→40% ethyl acetate/hexane) to give a 1:3 mixture of exo form and endo form as an oil. The mixture was separated by HPLC to give (8E)-6-benzyl-8-ethylidene-2-morpholin-4-yl-5,6,7,8-tetrahydro-1,6-naphthyridine (0.517 g, 29%) as an oil.
¹H-NMR(CDCl₃):δ1.73(3H,d,J=7.4Hz), 3.44-3.53(6H,m), 3.57(2H,s), 3.69(2H,s), 3.75-3.87(4H,m), 6.47(1H,d,J=8.5Hz), 6.86-6.98(1H,m), 7.13(1H,d,J=8.5Hz), 7.22-7.40(5H,m) MS(ESI+):336(M+H)

### (step 2)

A suspension of the compound (0.052 g) obtained in step 1, 10% palladium/carbon catalyst (0.10 g), and methanol (5 mL) was stirred under a hydrogen atmosphere at room temperature for 2 hr. The insoluble material was filtered off through celite, and the filtrate was concentrated. The obtained residue was purified by basic silica gel column chromatography (solvent gradient; 10%→100% ethyl acetate/hexane) to give 8-ethyl-2-morpholin-4-yl-5,6,7,8-tetrahydro-1,6-naphthyridine (0.0078 g, 20%) as an oil.
¹H-NMR(CDCl₃):δ0.93(3H,t,J=7.4Hz), 1.37-1.58(1H,m), 1.91-2.09(1H,m), 2.44-2.58(1H,m), 2.88(1H,dd,J=12.8Hz,6.2Hz), 3.17(1H,dd,J=12.8Hz,5.1Hz), 3.28-3.50(4H,m), 3.68-3.85(6H,m), 6.37(1H,d,J=8.5Hz), 7.07(1H,d,J=8.5Hz)
MS(ESI+):248 (M+H)

### Example 4

### 2-(4-Fluorophenyl)-8-methyl-5,6,7,8-tetrahydro-1,6-naphthyridine hydrochloride

### (step 1)

To a suspension of the compound (23.0 g) obtained in Example 1, step 2 and potassium carbonate (11.9 g) in DMF (400 mL) was added dropwise allyl bromide (10.4 g), and the reaction mixture was stirred at room temperature for 10 hr. The reaction mixture was poured into water, and the mixture was extracted 3 times with ethyl acetate. The organic layer was washed with saturated brine, dried over anhydrous magnesium sulfate, and concentrated. The obtained residue was purified by silica gel column chromatography (solvent gradient: 0→50% ethyl acetate/hexane) to give N-benzyl-N-[(2,6-dichloropyridin-3-yl)methyl]prop-2-en-1-amine (17.6 g, 67%) as an oil.
¹H-NMR(CDCl₃):δ3.10(2H,d,J=6.4Hz), 3.64(4H,d,J=3.0Hz), 5.11-5.29(2H,m), 5.79-5.97(1H,m), 7.20-7.37(6H,m), 7.94(1H,d,J=8.1Hz)
MS(ESI+):307(M+H)

### (step 2)

A suspension of the compound (10.0 g) obtained in step 1, palladium acetate (0.731 g), tri(2-methylphenyl)phosphine (1.98 g) and potassium carbonate (6.75 g) in DMF (400 mL) was stirred under a nitrogen atmosphere at 115°C for 4 hr. The reaction mixture was cooled to room temperature, and filtered through celite. The filtrate was concentrated, and the obtained residue was purified by silica gel column chromatography (solvent gradient: 0→50% ethyl acetate/hexane) to give 6-benzyl-2-chloro-8-methylene-5,6,7,8-tetrahydro-1,6-naphthyridine (3.32 g, 38%) as an oil.
¹H-NMR(CDCl₃):δ3.51(2H,s), 3.69(4H,s), 5.16(1H,s), 6.34(1H,s), 7.12(1H,d,J=8.0Hz), 7.22-7.40(6H,m)

### (step 3)

To a solution of the compound (2.00 g) obtained in step 2 in dichloroethane (20 mL) was added 1-chloroethyl chloroformate (1.27 g) and the mixture was stirred at 90°C for 1 hr. The reaction mixture was cooled to room temperature, and concentrated. To the obtained residue was added methanol (20 mL), and the mixture was stirred under reflux for 2 hr. The reaction mixture was cooled to room temperature, and concentrated. The obtained solid was washed with ethyl acetate/diisopropyl ether to give 2-chloro-8-methylene-5,6,7,8-tetrahydro-1,6-naphthyridine dihydrochloride (1.78 g, 95%) as a brown powder.
¹H-NMR(CDCl₃):δ4.18(2H,brs), 4.41(2H,brs), 5.55(1H,s), 6.34(1H,s), 7.52(1H,d,J=8.3Hz), 7.84(1H,d,J=8.3Hz), 9.78(2H,brs)

### (step 4)

A mixed solution of the compound (1.78 g) obtained in step 3 and (Boc)₂O (2.30 g) in THF/1N aqueous sodium hydroxide solution (20 mL/20 mL) was stirred at room temperature for 16 hr. The organic layer was separated, and the aqueous layer was extracted 3 times with ethyl acetate. The organic layers were combined, washed with saturated brine, dried over anhydrous magnesium sulfate, and concentrated. The obtained residue was purified by silica gel column chromatography (solvent gradient: 0→50% ethyl acetate/hexane) to give tert-butyl 2-chloro-8-methylene-7,8-dihydro-1,6-naphthyridine-6(5H)-carboxylate (1.42 g, 72%) as an oil.
¹H-NMR(CDCl₃):δ1.47(9H,s), 4.33(2H,s), 4.62(2H,s), 5.29(1H,brs), 6.31(1H,s), 7.18(1H,d,J=8.1Hz), 7.40(1H,d,J=8.1Hz)

### (step 5)

A suspension of the compound (1.42 g) obtained in step 4 and palladium-fibroin catalyst (1.00 g) in methanol (30 mL) was stirred under a hydrogen atmosphere at room temperature for 1 hr. The reaction mixture was filtered through celite, and the catalyst was washed with methanol. The filtrate was concentrated, and the obtained residue was purified by silica gel column chromatography (solvent gradient: 0→50% ethyl acetate/hexane) to give tert-butyl 2-chloro-8-methyl-7,8-dihydro-1,6-naphthyridine-6(5H)-carboxylate (0.280 g, 20%) as an oil.
¹H-NMR(CDCl₃):δ1,33(3H,d,J=7.2Hz), 1.50(9H,s), 3.04(1H,brs), 3.60(2H,brs), 4.45(1H,brs), 4.74(1H,brs), 7.15(1H,d,J=8.1Hz), 7.36(1H,d,J=8.1Hz)

### (step 6)

To a mixed solution of the compound (0.200 g) obtained in step 5, 4-fluorophenylboronic acid (0.148 g) and potassium carbonate (0.0978 g) in DME/water (10 mL/1 mL) was added under an argon atmosphere tetrakis(triphenylphosphine)palladium (0) (Pd(PPh₃)₄) (0.0817 g), and the reaction vessel was irradiated in a microwave reaction apparatus at 160°C for 20 min. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 0→50% ethyl acetate/hexane) to give tert-butyl 2-(4-fluorophenyl)-8-methyl-7,8-dihydro-1,6-naphthyridine-6(5H)-carboxylate (0.204 g, 84%) as a white powder.
¹H-NMR(CDCl₃):δ1.41(3H,d,J=7.0Hz), 1.51(9H,s), 3.07-3.21(1H,m), 3.44-3.89(2H,m), 4.44-4.91(2H,m), 7.08-7.18(2H,m), 7.40-7.53(2H,m), 7.94-8.03(2H,m)
MS(ESI+):343 (M+H)

### (step 7)

A solution of the compound (0.204 g) obtained in step 6 in 4N hydrogen chloride-ethyl acetate solution (6 mL) was stirred at room temperature for 16 hr. The reaction solution was concentrated, and the residue was recrystallized from ethanol/diisopropyl ether to give 2-(4-fluorophenyl)-8-methyl-5,6,7,8-tetrahydro-1,6-naphthyridine hydrochloride (0.0797 g, 48%) as a yellow powder.
¹H-NMR(DMSO-d₆):δ1.45(3H,d,J=7.1Hz), 3.18(1H,brs), 3.26-3.37(1H,m), 3.61-3.69(1H,m), 4.36(2H,brs), 7.29-7.38(2H,m), 7.75(1H,d,J=8.3Hz), 7.89(1H,d,J=8.3Hz), 8.14-8.20(2H,m), 9.49(2H,brs)

### MS(ESI+):243(M-HCl+H)

### Example 5

### 8-Methyl-2-morpholin-4-yl-5,6,7,8-tetrahydro-1,6-naphthyridine dihydrochloride

### (step 1)

A mixture of the compound (0.130 g) obtained in Example 4, step 5, morpholine (0.0801 g), tris(dibenzylideneacetone)dipalladium (0) (0.0168 g), XPhos (0.0801 g), sodium tert-butoxide (0.0663 g) and toluene (5 mL) was stirred under a nitrogen atmosphere with heating at 100°C for 3 hr. To the reaction solution was added water, and the insoluble material was filtered off through celite. The aqueous layer was extracted with ethyl acetate, and the organic layer was washed with saturated brine, dried over anhydrous magnesium sulfate, and concentrated. The obtained residue was purified by silica gel column chromatography (solvent gradient: 0→50% ethyl acetate/hexane) to give tert-butyl 8-methyl-2-morpholin-4-yl-7,8-dihydro-1,6-naphthyridine-6(5H)-carboxylate (0.0559 g, 36%) as an oil.
¹H-NMR(CDCl₃):δ1.28(3H,d,J=7.2Hz), 1.49(9H,s), 2.82-2.98(1H,m), 3.40-3.56(5H,m),3.57-3.77(1H,m), 3.82(4H,t,J=4.8Hz), 4.40-4.60(2H,m), 6.48(1H,d,J=8.5Hz), 7.22(1H,d,J=8.5Hz)

### (step 2)

A mixed solution of the compound (0.0559 g) obtained in step 1 and 4N hydrogen chloride-ethyl acetate solution (2 mL) was stirred at room temperature for 16 hr. The reaction solution was concentrated, and the residue was recrystallized from ethanol to give 8-methyl-2-morpholin-4-yl-5,6,7,8-tetrahydro-1,6-naphthyridine dihydrochloride (0.0127 g, 32%) as an orange powder.
¹H-NMR(DMSO-d₆):δ1.31(3H,d,J=6.4Hz), 2.97-3.21(2H,m), 3.39-3.49(4H,m), 3.49-3.59(1H,m), 3.69(4H,t,J=4.7Hz), 4.14(2H,brs), 6.79(1H,d,J=8.7Hz), 7.43(1H,d,J=8.7Hz), 9.43(2H,brs) MS(ESI+):234(M-2HCl+H)

### Example 6

### 2-Morpholin-4-yl-5,6,7,8-tetrahydro-1,6-naphthyridine

### (step 1)

A solution of 1-benzyl-4-piperidone (12.8 g) and pyrrolidine (11.2 mL) in toluene (42 mL) was heated for 18 hr under azeotropic conditions while removing generated water. The reaction solution was cooled to room temperature, and concentrated. To the obtained residue were added acrylamide (9.60 g) and p-toluenesulfonic acid monohydrate (0.600 g), and the mixture was heated at 90°C for 1 hr, then at 120°C for 2 hr. The reaction mixture was cooled to room temperature, the resulting solid was filtered, and the solid was washed with acetone and ether. The filtrates were combined, concentrated, diluted with ethyl acetate, and washed with water. The organic layer was dried over anhydrous magnesium sulfate, and concentrated. The obtained residue and the solid were combined, p-toluenesulfonic acid monohydrate (0.600 g) and dioxane (42 mL) were added, and the mixture was refluxed for 18 hr. The reaction mixture was cooled to room temperature, and the obtained solid was washed with ethyl acetate to give 6-benzyl-3,4,5,6,7,8-hexahydro-1,6-naphthyridin-2(1H)-one (10.4 g, 64%) as a yellow powder.
¹H-NMR(CDCl₃):δ2.08-2.25(4H,m), 2.50(2H,t,J=8.1Hz), 2.64(2H,t,J=5.7Hz), 2.94(2H,s), 3.61(2H,s), 6.74(1H,brs), 7.21-7.38(5H,m)
MS(EST+):243(M+H)

### (step 2)

A solution of the compound (10.0 g) obtained in step 1, chloranil (10.7 g), and phosphoryl chloride (31.6 g) in toluene (130 mL) was refluxed under a nitrogen atmosphere for 18 hr. The reaction mixture was cooled to room temperature, and concentrated. The residue was basified with 4N aqueous sodium hydroxide solution, and the mixture was extracted 3 times with ethyl acetate. The organic layer was washed with saturated brine, dried over anhydrous magnesium sulfate, and concentrated. The obtained residue was purified by silica gel column chromatography (solvent gradient: 0→50% ethyl acetate/hexane) to give 6-benzyl-2-chloro-5,6,7,8-tetrahydro-1,6-naphthyridine (2.98 g, 28%) as an oil.
¹H-NMR(CDCl₃):δ2.83(2H,t,J=6.1Hz), 3.02(2H,t,J=6.1Hz), 3.58(2H,s), 3.71(2H,s), 7.07(1H,d,J=8.0Hz), 7.20-7.40(6H,m) MS(ESI+):259(M+H)

### (step 3)

A mixture of the compound (0.500 g) obtained in step 2, morpholine (0.337 g), tris(dibenzylideneacetone)dipalladium (0) (0.0708 g), XPhos (0.111 g), sodium tert-butoxide (0.279 g) and toluene (5 mL) was stirred by heating at 100°C for 16 hr under a nitrogen atmosphere. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 10→100% ethyl acetate/hexane) to give 6-benzyl-2-morpholin-4-yl-5,6,7,8-tetrahydro-1,6-naphthyridine (0.481 g, 81%) as a yellow powder. ¹H-NMR(CDCl₃):δ2.76-2.83(2H,m), 2.83-2.91(2H,m), 3.44(4H,t,J=4.9Hz), 3.51(2H,s), 3.68(2H,s), 3.81(4H,t,J=4.9Hz), 6.42(1H,d,J=8.7Hz), 7.12(1H,d,J=8.7Hz), 7.23-7.41(5H,m) MS(ESI+):310 (M+H)

### (step 4)

A suspension of the compound (0.350 g) obtained in step 3 and 10% palladium/carbon catalyst (0.100 g) in methanol (10 mL) was stirred under a hydrogen atmosphere at 50°C for 2 hr. The catalyst was filtered off through celite, and the filtrate was concentrated. The obtained solid was collected by filtration, and washed with diisopropyl ether to give 2-morpholin-4-yl-5,6,7,8-tetrahydro-1,6-naphthyridine (0.162 g, 65%) as a white powder.
¹H-NMR(CDCl₃):δ2.77(2H,t,J=6.1Hz), 3.18(2H,t,J=6.1Hz), 3.42-3.49(4H,m), 3.79-3.85(4H,m), 3.90(2H,s), 6.45(1H,d,J=8.3Hz), 7.16(1H,d,J=8.3Hz)
MS(ESI+):220(M+H)

### Example 7

### 2-[(3R)-3-Methylmorpholin-4-yl]-5,6,7,8-tetrahydro-1,6-naphthyridine hydrochloride

### (step 1)

A mixture of the compound (0.500 g) obtained in Example 6, step 2, (3R)-methylmorpholine p-toluenesulfonate (0.580 g), tris(dibenzylideneacetone)dipalladium (0) (0.0708 g), XPhos (0.111 g), sodium tert-butoxide (0.464 g) and toluene (5 mL) was stirred by heating at 100°C for 16 hr under a nitrogen atmosphere. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 0→50% ethyl acetate/hexane) to give 6-benzyl-2-[(3R)-3-methylmorpholin-4-yl]-5,6,7,8-tetrahydro-1,6-naphthyridine (0.234 g, 38%) as a yellow powder.
¹H-NMR(CDCl₃):δ1.17(3H,d,J=6.8Hz), 2.86(4H,brs), 3.15(1H,dt,J=3.7Hz,12.5Hz), 3.52(2H,brs), 3.56-3.66(1H,m), 3.67-3.74(2H,m), 3.74-3.83(3H,m), 3.94-4.02(1H,m), 4.20-4.31(1H,m), 6.37(1H,d,J=8.7Hz), 7.10(1H,d,J=8.7Hz), 7.24-7.48(5H,m)
MS(ESI+):324(M+H)

### (step 2)

A suspension of the compound (0.150 g) obtained in step 1 and 10% palladium/carbon catalyst (0.100 g) in methanol (10 mL) was stirred under a hydrogen atmosphere at room temperature for 16 hr. The catalyst was filtered off through celite, and the filtrate was concentrated. The obtained residue was dissolved in ethyl acetate, and 4N hydrogen chloride-ethyl acetate solution (0.128 mL) was added. The reaction solution was stirred at room temperature for 10 min, and concentrated. The obtained solid was collected by filtration and washed with diisopropyl ether to give 2-[(3R)-3-methylmorpholin-4-yl]-5,6,7,8-tetrahydro-1,6-naphthyridine hydrochloride (0.0908 g, 73%) as a white powder. ¹H-NMR(DMSO-d₆):δ1.08(3H,d,J=6.4Hz), 2.89(2H,t,J=6.3Hz), 3.00(1H,dt,J=3.8Hz,12.7Hz), 3.39(2H,t,J=6.4Hz), 3.42-3.50(1H,m), 3.56-3.63(1H,m), 3.67-3.75(1H,m), 3.80-3.97(2H,m), 4.11(2H,s), 4.22-4.33(1H,m), 6.69(1H,d,J=8.7Hz), 7.39(1H,d,J=8.7Hz), 9.36(2H,brs)
MS(ESI+):234(M-HCl+H)

### Example 8

### 2-[(3S)-3-Methylmorpholin-4-yl]-5,6,7,8-tetrahydro-1,6-naphthyridine hydrochloride

### (step 1)

A mixture of the compound (0.500 g) obtained in Example 6, step 2, (3S)-methylmorpholine p-toluenesulfonate (0.580 g), tris(dibenzylideneacetone)dipalladium (0) (0.0708 g), XPhos (0.111 g), sodium tert-butoxide (0.464 g) and toluene (5 mL) was stirred by heating at 100°C for 16 hr under a nitrogen atmosphere. To the reaction solution was added water, and the insoluble material was filtered off through celite. The object product was extracted with ethyl acetate, washed with saturated brine, dried over anhydrous magnesium sulfate, and concentrated. The obtained residue was purified by silica gel column chromatography (solvent gradient: 0→50% ethyl acetate/hexane) to give 6-benzyl-2-[(3S)-3-methylmorpholin-4-yl]-5,6,7,8-tetrahydro-1,6-naphthyridine (0.276 g, 44%) as a yellow powder.
¹-H-NMR(CDCl₃):δ1.17(3H,d,J=6.6Hz), 2.77-2.92(4H,m), 3.15(1H,dt,J=3.8Hz,12.4Hz), 3.52(2H,brs), 3.56-3.67(1H,m), 3.71(2H,brs), 3.75-3.84(3H,m), 3.94-4.03(1H,m), 4.20-4.31(1H,m), 6.37(1H,d,J=8.5Hz), 7.10(1H,d,J=8.5Hz), 7.24-7.47(5H,m)
MS(ESI+):324(M+H)

### (step 2)

A suspension of the compound (0.200 g) obtained in step 1 and 10% palladium/carbon catalyst (0.100 g) in methanol (10 mL) was stirred under a hydrogen atmosphere at room temperature for 16 hr. The catalyst was filtered off through celite, and the filtrate was concentrated. The obtained residue was dissolved in ethyl acetate, and 4N hydrogen chloride-ethyl acetate solution (0.167 mL) was added. The reaction solution was stirred at room temperature for 10 min, and concentrated. The obtained solid was collected by filtration and washed with diisopropyl ether to give 2-[(3S)-3-methylmorpholin-4-yl]-5,6,7,8-tetrahydro-1,6-naphthyridine hydrochloride (0.137 g, 80%) as a white powder. ¹H-NMR(DMSO-d₆):δ1.09(3H,d,J=6.4Hz), 2.85-2.93(2H,m), 3.00(1H,dt,J=3.6Hz,12.6Hz), 3.34-3.50(3H,m), 3.55-3.64(1H,m), 3.67-3.75(1H,m), 3.80-3.96(2H,m), 4.11(2H,brs), 4.22-4.33(1H,m), 6:70(1H,d,J=8.7Hz), 7.40(1H,d,J=8.7Hz), 9.32(2H,brs)

### MS(ESI+):234(M-HCl+H)

### Example 9

### 2-[(3R)-3-Ethylmorpholin-4-yl]-5,6,7,8-tetrahydro-1,6-naphthyridine hydrochloride

Using the compound obtained in Example 6, step 2 and (3R)-3-ethylmorpholine p-toluenesulfonate and in the same manner as in the method described in Example 7, 2-[(3R)-3-ethylmorpholin-4-yl]-5,6,7,8-tetrahydro-1,6-naphthyridine hydrochloride (0.177 g, 33%) was obtained as a yellow powder. ¹H-NMR(DMSO-d₆):δ0.84(3H,t,J=7.4Hz), 1.38-1.55(1H,m), 1.64-1.83(1H,m), 2.88(2H,t,J=6.1Hz), 3.01(1H,dt,J=3.6Hz,12.7Hz), 3.29-3.54(4H,m), 3.78-4.07(4H,m), 4.10(2H,s), 6.69(1H,d,J=8.7Hz), 7.37(1H,d,J=8.7Hz), 9.39(2H,brs) MS(ESI+):248(M-HCl+H)

### Example 10

### 2-Pyrrolidin-1-yl-5,6,7,8-tetrahydro-1,6-naphthyridine hydrochloride

### (step 1)

A mixture of the compound (0.500 g) obtained in Example 6, step 2, pyrrolidine (0.275 g), tris(dibenzylideneacetone)dipalladium (0) (0.0708 g), XPhos (0.111 g), sodium tert-butoxide (0.279 g) and toluene (5 mL) was stirred by heating at 100°C for 16 hr under a nitrogen atmosphere. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 0→60% ethyl acetate/hexane) to give 6-benzyl-2-pyrrolidin-1-yl-5,6,7,8-tetrahydro-1,6-naphthyridine (0.272 g, 48%) as a yellow powder.
¹H-NMR(CDCl₃):δ1.92-2.01(4H,m), 2.75-2.83(2H,m), 2.84-2.91(2H,m), 3.37-3.46(4H,m), 3.49(2H,s), 3.68(2H,s), 6.16(1H,d,J=8.3Hz), 7.04(1H,d,J=8.3Hz), 7.23-7.42(5H,m) MS(ESI+):294(M+H)

### (step 2)

A suspension of the compound (0.200 g) obtained in step 1 and 20% palladium hydroxide/carbon catalyst (0.200 g) in methanol (10 mL) was stirred at 50°C for 2 hr under a hydrogen atmosphere. The catalyst was filtered off through celite, and the filtrate was concentrated. The obtained residue was dissolved in a mixed solution of ethyl acetate (10 mL) and methanol (5 mL), 4N hydrogen chloride-ethyl acetate solution (0.229 mL) was added, and the mixture was stirred at room temperature for 30 min. The reaction solution was concentrated, and the obtained solid was recrystallized from ethanol/diisopropyl ether to give 2-pyrrolidin-1-yl-5,6,7,8-tetrahydro-1,6-naphthyridine hydrochloride (0.100 g, 61%) as a white powder.
¹H-NMR(CDCl₃):δ1.86-1.98(4H,m), 2.87(2H,t,J=6.3Hz), 3.28-3.42(6H,m), 4.09(2H,s), 6.36(1H,d,J=8.7Hz), 7.31(1H,d,J=8.7Hz), 9.29(2H,brs)
MS(ESI+):204 (M+H)

### Example 11

### 2-Piperidin-1-yl-5,6,7,8-tetrahydro-1,6-naphthyridine hydrochloride

Using the compound obtained in Example 6, step 2 and piperidine and in the same manner as in the method described in Example 10, 2-piperidin-1-yl-5,6,7,8-tetrahydro-1,6-naphthyridine hydrochloride (0.0785 g, 23%) was obtained as a white powder.
¹H-NMR(DMSO-d₆):δ1.44-1.66(6H,m), 2.87(2H,t,J=6.3Hz), 3.39(2H,t,J=6.3Hz), 3.44-3.54(4H,m), 4.09(2H,s), 6.73(1H,d,J=8.7Hz), 7.34(1H,d,J=8.7Hz), 9.30(2H,brs) MS(ESI+):218(M-HCl+H)

### Example 12

### N,N-Dimethyl-5,6,7,8-tetrahydro-1,6-naphthyridin-2-amine hydrochloride

### (step 1)

A mixture of the compound (0.500 g) obtained in Example 6, step 2, dimethylamine hydrochloride (0.315 g), tris(dibenzylideneacetone)dipalladium (0) (0.0708 g), XPhos (0.111 g), sodium tert-butoxide (0.650 g) and toluene (10 mL) was stirred by heating at 100°C for 16 hr under a nitrogen atmosphere. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 0→100% ethyl acetate/hexane) to give 6-benzyl-N,N-dimethyl-5,6,7,8-tetrahydro-1,6-naphthyridin-2-amine (0.495 g, 96%) as an oil.
¹H-NMR(CDCl₃):δ2.76-2.83(2H,m), 2.83-2.90(2H,m), 3.03(6H,s), 3.49(2H,s), 3.68(2H,s), 6.32(1H,d,J=8.7Hz), 7.06(1H,d,J=8.7Hz), 7.23-7.42(5H,m)
MS(ESI+):268(M+H)

### (step 2)

A suspension of the compound (0.475 g) obtained in step 1 and 20% palladium hydroxide/carbon catalyst (0.200 g) in methanol (10 mL) was stirred under a hydrogen atmosphere at room temperature for 16 hr. The catalyst was filtered off through celite, and the filtrate was concentrated. The obtained residue was dissolved in a mixed solution of ethyl acetate (10 mL) and methanol (5 mL), 4N hydrogen chloride-ethyl acetate solution (0.444 mL) was added, and the mixture was stirred at room temperature for 30 min. The reaction solution was concentrated, and the obtained solid was recrystallized from ethanol/diisopropyl ether to give N,N-dimethyl-5,6,7,8-tetrahydro-1,6-naphthyridin-2-amine hydrochloride (0.204 g, 54%) as a yellow powder. ¹H-NMR(DMSO-d₆):δ2.89-3.15(8H,m),3.40(2H,brs), 4.11(2H,brs), 6.71(1H,brs), 7.46(1H,brs), 9.56(2H,brs)
MS(ESI+):178(M-HCl+H)

### Example 13

### N,N-Diethyl-5,6,7,8-tetrahydro-1,6-naphthyridin-2-amine hydrochloride

Using the compound obtained in Example 6, step 2 and diethylamine and in the same manner as in the method described in Example 10, N,N-diethyl-5,6,7,8-tetrahydro-1,6-naphthyridin-2-amine hydrochloride (0.101 g, 23%) was obtained as a white powder.
¹H-NMR(DMSO-d₆):δ1.07(6H,t,J=7.0Hz), 2.86(2H,t,J=6.2Hz), 3.38(2H,t,J=6.2Hz), 3.46(4H,q,J=7.0Hz), 4.07(2H,s), 6.50(1H,d,J=8.9Hz), 7.29(1H,d,J=8.9Hz), 9.33(2H,brs) MS(ESI+):206(M-HCl+H)

### Example 14

### N-Methyl-N-propyl-5,6,7,8-tetrahydro-1,6-naphthyridin-2-amine hydrochloride

Using the compound obtained in Example 6, step 2 and N-methylpropylamine and in the same manner as in the method described in Example 10, N-methyl-N-propyl-5,6,7,8-tetrahydro-1,6-naphthyridin-2-amine hydrochloride (0.203 g, 46%) was obtained as a yellow powder.
¹H-NMR(DMSO-d₆):δ0.84(3H,t,J=7.4Hz), 1.44-1.59(2H,m), 2.87(2H,t,J=6.2Hz), 2.97(3H,s), 3.28-3.49(4H,m), 4.07(2H,s), 6.53(1H,d,J=8.7Hz), 7.31(1H,d,J=8.7Hz), 9.43(2H,brs) MS(ESI+):206(M-HCl+H)

### Example 15

### N-Ethyl-N-methyl-5,6,7,8-tetrahydro-1,6-naphthyridin-2-amine hydrochloride

Using the compound obtained in Example 6, step 2 and N-methylethylamine and in the same manner as in the method described in Example 10, N-ethyl-N-methyl-5,6,7,8-tetrahydro-1,6-naphthyridin-2-amine hydrochloride (0.286 g, 34%) was obtained as a yellow powder.
¹H-NMR(DMSO-d₆):δ1.05(3H,t,J=7.0Hz), 2.91(2H,brs), 2.98(3H,brs), 3.37(2H,brs), 3.55(2H,q,J=7.0Hz), 4.09(2H,brs), 6.57(1H,brs), 7.36(1H,brs), 9.39(2H,brs)
MS(ESI+):192(M-HCl+H)

### Example 16

### N-Isopropyl-5,6,7,8-tetrahydro-1,6-naphthyridin-2-amine hydrochloride

### (step 1)

To a mixture of the compound (1.00 g) obtained in Example 6, step 2, isopropylamine (0.457 g), XPhos (0.221 g), sodium tert-butoxide (0.561 g) and toluene (20 mL) was added under an argon atmosphere tris(dibenzylideneacetone)dipalladium (0) (0.142 g), and the reaction solution was stirred at 100°C for 2 hr. The reaction solution was poured into water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 5→100% ethyl acetate/hexane) to give 6-benzyl-N-isopropyl-5,6,7,8-tetrahydro-1,6-naphthyridin-2-amine (0.540 g, 50%) as an oil.
¹H-NMR(CDCl₃):δ1.20(6H,d,J=6.4Hz), 2.74-2.87(4H,m), 3.48(2H,s) 3.65-3.81(3H,s), 6.18(1H,d,J=8.5Hz), 7.06(1H,d,J=8.5Hz), 7.23-7.43(5H,m)
MS(ESI+):282(M+H)

### (step 2)

A suspension of the compound (0.100 g) obtained in step 1 and 10% palladium/carbon catalyst (0.100 g) in methanol (5 mL) was stirred under a hydrogen atmosphere at room temperature for 16 hr. The catalyst was filtered off through celite, and the filtrate was concentrated. The obtained residue was dissolved in a mixed solution of ethyl acetate (10 mL) and methanol (5 mL), 2N hydrogen chloride-methanol solution (0.148 mL) was added, and the mixture was stirred at room temperature for 1 hr. The reaction solution was concentrated, and the obtained solid was recrystallized from ethanol/ethyl acetate to give N-isopropyl-5,6,7,8-tetrahydro-1,6-naphthyridin-2-amine hydrochloride (0.0170 g, 21%) as a white powder.
¹H-NMR(DMSO-d₆):δ1.11(6H,d,J=6.4Hz), 2.78-2.86(2H,m), 3.36(2H,t,J=6.4Hz), 3.87-4.00(1H,m), 4.03(2H,s), 6.34(2H,m), 7.17(1H,d,J=8.5Hz), 9.04(2H,brs)
MS(ESI+):192(M-HCl+H)

### Example 17

### N-Isopropyl-N-methyl-5,6,7,8-tetrahydro-1,6-naphthyridin-2-amine hydrochloride

### (step 1)

A solution (5 mL) of the compound (0.440 g) obtained in Example 16, step 1, formalin (2.66 mL), sodium triacetoxyborohydride (2.32 g), and acetic acid (0.200 mL) in dichloromethane was stirred at room temperature for 16 hr. To the reaction solution were added ethyl acetate and water, the organic layer was separated, and the aqueous layer was extracted 3 times with ethyl acetate. The organic layers were combined, washed with saturated brine, dried over anhydrous magnesium sulfate, and concentrated. The obtained residue was purified by silica gel column chromatography (solvent gradient: 5→50% ethyl acetate/hexane) to give 6-benzyl-N-isopropyl-N-methyl-5,6,7,8-tetrahydro-1,6-naphthyridin-2-amine (0.206 g, 48%) as an oil.
¹H-NMR(CDCl₃) :δ1.13(6H,d,J=6.4Hz), 2.75-2.89(4H,m), 2.80(3H,s), 3.48(2H,s), 3.68(2H,s), 4.73-4.90(1H,m), 6.30(1H,d,J=8.7Hz), 7.04(1H,d,J=8.7Hz), 7.23-7.42(5H,m)
MS(ESI+):296(M-HCl+H)

### (step 2)

A suspension of the compound (0.204 g) obtained in step 1 and 10% palladium/carbon catalyst (0.100 g) in methanol (5 mL) was stirred under a hydrogen atmosphere at room temperature for 16 hr. The catalyst was filtered off through celite, and the filtrate was concentrated. The obtained residue was dissolved in ethyl acetate (10 mL), 4N hydrogen chloride-ethyl acetate solution (0.173 mL) was added, and the mixture was stirred at room temperature for 1 hr. The reaction solution was concentrated, and the obtained solid was recrystallized from ethanol/ethyl acetate to give N-isopropyl-N-methyl-5,6,7,8-tetrahydro-1,6-naphthyridin-2-amine hydrochloride (0.0970 g, 58%) as a white powder.
¹H-NMR(DMSO-d₆):δ1.09(6H,t,J=6.8Hz), 2.77(3H,s), 2.87(2H,t,J=6.3Hz), 3.39(2H,t,J=6.3Hz), 4.09(2H,s), 4.74-4.89(1H,m), 6.54(1H,d,J=8.7Hz), 7.33(1H,d,J=8.7Hz), 9.23(2H,brs)
MS(ESI+):206(M-HCl+H)

### Example 18

### N-Isobutyl-5,6,7,8-tetrahydro-1,6-naphthyridin-2-amine

### (step 1)

To a mixture of the compound (0.500 g) obtained in Example 6, step 2, isobutylamine (0.580 mL), (R)-1-[(S)-2-(dicyclohexylphosphino)ferrocenyl]ethyl di-tert-butylphosphine (SL-J009-1) (0.0321 g), sodium tert-butoxide (0.279 g) and toluene (10 mL) was added under an argon atmosphere palladium acetate (0.0130 g), and the mixture was stirred under an argon atmosphere at 100°C for 2 hr. The reaction solution was poured into water, and the resulting product was extracted with ethyl acetate. The organic layer was washed with water and saturated brine, dried over sodium sulfate, and concentrated. The obtained residue was purified by silica gel column chromatography (solvent gradient; 0→40% ethyl acetate/hexane) to give 6-benzyl-N-isobutyl-5,6,7,8-tetrahydro-1,6-naphthyridin-2-amine (0.483 g, 80%) as an oil. ¹H-NMR(CDCl₃):δ0.97(6H,t,J=6.8Hz), 1.75-1.90(1H,m), 2.75-2.85(4H,m), 3.00(2H,t,J=6.4Hz), 3.48(2H,s), 3.67(2H,s), 4.55(1H,brs), 6.18(1H,d,J=8.4Hz), 7.06(1H,d,J=8.4Hz), 7.25-7.40(5H,m)

### (step 2)

A suspension of the compound (0.0600 g) obtained in step 1 and 20% palladium hydroxide/carbon catalyst (0.0100 g) in methanol (2 mL) was stirred under a hydrogen atmosphere at room temperature for 16 hr. The catalyst was filtered off, and the filtrate was concentrated. The obtained residue was purified by basic silica gel column chromatography (solvent gradient; 50→100% ethyl acetate/hexane) to give N-isobutyl-5,6,7,8-tetrahydro-1,5-naphthyridin-2-amine (0.0240 g, 58%) as a white powder.
¹H-NMR(CDCl₃):δ0.98(6H,t,J=6.6Hz), 1.82-1.91(2H,m), 2.77(2H,t,J=5.8Hz), 3.01(2H,dd,J=6.2Hz,6.2Hz), 3.17(2H,t,J-5.8Hz), 3.86(2H,s), 4.53(1H,brs), 6.22(1H,d,J=8.4Hz), 7.10(1H,d,J=8.4Hz)
MS(ESI+):206(M+H)

### Example 19

### N-Isobutyl-N-methyl-5,6,7,8-tetrahydro-1,6-naphthyridin-2-amine

### (step 1)

A solution (8 mL) of the compound (0.423 g) obtained in Example 18, step 1, paraformaldehyde (0.129 g), sodium triacetoxyborohydride (0.455 g) and acetic acid (1 mL) in dichloromethane was stirred at room temperature four 2 hr. Paraformaldehyde (0.129 g) and sodium triacetoxyborohydride (0.455 g) were added again, and the mixture was stirred at room temperature for 16 hr. To the reaction solution were added ethyl acetate and 1N aqueous sodium hydroxide solution and the organic layer was separated. The organic layer was washed with saturated brine, dried over anhydrous sodium sulfate, and concentrated. The obtained residue was purified by silica gel column chromatography (solvent gradient: 0→40% ethyl acetate/hexane) to give 6-benzyl-N-isobutyl-N-methyl-5,6,7,8-tetrahydro-1,6-naphthyridin-2-amine (0.304 g, 69%) as a white powder.
¹H-NMR(CDCl₃) :δ0.88(6H,t,J=6.3Hz), 1.90-2.10(1H,m), 2.77-2.84(4H,m), 3.03(3H,s), 3.27(2H,d,J=7.3Hz), 3.48(2H,s), 3.68(2H,s), 6.27(1H,d,J=8.6Hz), 7.03(1H,d,J=8.6Hz), 7.25-7.41(5H,m)

### (step 2)

A suspension of the compound (0.275 g) obtained in step 1 and 20% palladium hydroxide/carbon catalyst (0.0300 g) in methanol (10 mL) was stirred under a hydrogen atmosphere at 40°C for 16 hr. The catalyst was filtered off, and the filtrate was concentrated. The obtained solid was recrystallized from hexane to give N-isobutyl-N-methyl-5,6,7,8-tetrahydro-1,6-naphthyridin-2-amine (0.163 g, 84%) as a white powder.
¹H-NMR(CDCl₃):50.90(6H,t,J=6.7Hz), 1.61(1H,brs), 2.00-2.10(1H,m), 2.79(2H,t,J=6.0Hz), 3.04(3H,s), 3.16(2H,t,J=6.0Hz), 3.28(2H,t,J=7.5Hz), 3.86(2H,s), 6.30(1H,d,J=8.6Hz), 7.06(1H,d,J=8.6Hz)
MS(ESI+):220(M+H)

### Example 20

### N-(Cyclopropylmethyl)-5,6,7,8-tetrahydro-1,6-naphthyridin-2-amine hydrochloride

### (step 1)

Using the compound obtained in Example 6, step 2 and 1-cyclopropylmethanamine and in the same manner as in the method described in Example 18, step 1, 6-benzyl-N-(cyclopropylmethyl)-5,6,7,8-tetrahydro-1,6-naphthyridin-2-amine (0.464 g, 82%) was obtained as an oil.
¹H-NMR(CDCl₃):δ0.20-0.25(2H,m), 0.48-0.54(2H,m), 1.00-1.20(1H,m), 2.75-2.84(4H,m), 3.05(2H,t,J=6.4Hz), 3.48(2H,s), 3.67(2H,s), 4.61(1H,s), 6.28(1H,d,J=8.3Hz), 7.06(1H,d,J=8.3Hz), 7.26-7.39(5H,m)

### (step 2)

A suspension of the compound (0.060 g) obtained in step 1 and 20% palladium hydroxide/carbon catalyst (0.010 g) in methanol (2 mL) was stirred under a hydrogen atmosphere at room temperature for 16 hr. The catalyst was filtered off, and the filtrate was concentrated. The obtained residue was purified by basic silica gel column chromatography (solvent gradient; 50→100% ethyl acetate/hexane), 4N hydrogen chloride-ethyl acetate solution (0.056 mL) was added, and the mixture was concentrated. The obtained residue was recrystallized from ethanol/diisopropyl ether to give N-(cyclopropylmethyl)-5,6,7,8-tetrahydro-1,6-naphthyridin-2-amine hydrochloride (0.020 g, 41%) as a white powder.
¹H-NMR(DMSO-d₆):δ0.27-0.32(2H,m), 0.50-0.56(2H,m), 1.00-1.20(1H,m), 3.11(2H,brs), 3.32(2H,brs), 3.38(2H,brs), 3.90(1H,brs), 4.10(2H,s), 7.0-7.10(1H,m), 7.65-7.80(1H,m), 9.78-9.88(2H,m)
MS(ESI+):204(M-HCl+H)

### Example 21

### N-(Cyclopropylmethyl)-N-methyl-5,6,7,8-tetrahydro-1,6-naphthyridin-2-amine p-toluenesulfonate

### (step 1)

Using the compound obtained in Example 20, step 1 and in the same manner as in the method described in Example 19, step 1, 6-benzyl-N-(cyclopropylmethyl)-N-methyl-5,6,7,8-tetrahydro-1,6-naphthyridin-2-amine (0.349 g, 82%) was obtained as a white powder.
¹H-NMR(CDCl₃):δ0.20-0.25(2H,m), 0.43-0.49(2H,m), 0.95-1.10(1H,m), 2.77-2.85(4H,m), 3.05(3H,s), 3.41(2H,d,J=6.6Hz), 3.49(2H,s), 3.68(2H,s), 6.34(1H,d,J=8.6Hz), 7.05(1H,d,J=8.6Hz), 7.25-7.41(5H,m)

### (step 2)

A suspension of the compound (0.327 g) obtained in step 1 and 20% palladium hydroxide/carbon catalyst (0.030 g) in methanol (10 mL) was stirred under a hydrogen atmosphere at 40°C for 16 hr. The catalyst was filtered off, and the filtrate was concentrated. The obtained residue was purified by basic silica gel column chromatography (solvent gradient: 0 →10% methanol/ethyl acetate), and the obtained oil and p-toluenesulfonic acid monohydrate (0.164 g) were dissolved in methanol, and the solution was concentrated. The obtained residue was recrystallized from ethanol/diisopropyl ether to give N-(cyclopropylmethyl)-N-methyl-5,6,7,8-tetrahydro-1,6-naphthyridin-2-amine p-toluenesulfonate (0.164 g, 40%) as a white powder.
¹H-NMR(DMSO-d₆):δ0.22-0.27(2H,m), 0.39-0.45(2H,m), 0.95-1.05(1H,m), 2.29(3H,s), 2.88(2H,brs), 3.03(3H,s), 3.40-3.45(4H,m), 4.12(2H,brs), 6.62(1H,brs), 7.11(2H,d,J=8.0Hz), 7.34(1H,brs), 7.47(2H,d,J=8.0Hz), 8.90(2H,brs)
MS(ESI+):218(M-C₇H₈O₃S+H)

### Example 22

### N-Methyl-N-[(1R)-1-methylpropyl]-5,6,7,8-tetrahydro-1,6-naphthyridin-2-amine p-toluenesulfonate

### (step 1)

Using the compound obtained in Example 6, step 2 and (2R)-butan-2-amine and in the same manner as in the method described in Example 18, step 1 and Example 19, step 1, 6-benzyl-N-methyl-N-[(1R)-1-methylpropyl]-5,6,7,8-tetrahydro-1,6-naphthyridin-2-amine (0.174 g, 53%) was obtained as an oil. ¹H-NMR(CDCl₃):δ0.82(3H,t,J=7.4Hz), 1.09(3H,t,J=6.8Hz), 1.40-1.60(2H,m), 2.77(3H,s), 2.77-2.90(4H,m), 3.48(2H,s), 3.67(2H,s), 4.50-4.65(1H,m), 6.30(1H,d,J=8.7Hz), 7.03(1H,d,J=8.7Hz), 7.25-7.40(5H,m)

### (step 2)

A suspension of the compound (0.174 g) obtained in step 1 and 20% palladium hydroxide/carbon catalyst (0.0200 g) in methanol (5 mL) was stirred under a hydrogen atmosphere at 40°C for 16 hr. The catalyst was filtered off, and the filtrate was concentrated. The obtained oil and p-toluenesulfonic acid monohydrate (0.124 g) were dissolved in methanol, and the solution was concentrated. The obtained residue was recrystallized from ethyl acetate to give N-methyl-N-[(1R)-1-methylpropyl]-5,6,7,8-tetrahydro-1,6-naphthyridin-2-amine p-toluenesulfonate (0.180 g, 82%) as a white powder.
¹H-NMR(DMSO-d₆):δ0.75(3H,t,J=7.3Hz), 1.07(3H,t,J=6.6Hz), 1.40-1.60(2H,m), 2.29(3H,s), 2.76(3H,brs), 2.76-2.95(2H,m), 3.40-3.50(2H,m), 4.12(2H,brs), 4.57(1H,brs), 6.55(1H,brs), 7.11(2H,d,J-7.9Hz), 7.33(1H,brs), 7.47(2H,d,J=7.9Hz), 8.88(2H,brs)
MS(ESI+):220 (M-C₇H_{B}O₃S+H)
[α]_{D}²⁵ -11.8(c 0.1, MeOH)

### Example 23

### N-Methyl-N-[(1S)-1-methylpropyl]-5,6,7,8-tetrahydro-1,6-naphthyridin-2-amine p-toluenesulfonate

Using the compound obtained in Example 6, step 2 and (2S)-butan-2-amine and in the same manner as in the method described in Example 22, N-methyl-N-[(1S)-1-methylpropyl]-5,6,7,8-tetrahydro-1,6-naphthyridin-2-amine p-toluenesulfonate (0.157 g, 80%) was obtained as a white powder. ¹H-NMR(DMSO-d₆):δ0.75(3H,t,J=7.4Hz), 1.07(3H,t,J=6.4Hz), 1.40-1.60(2H,m), 2.29(3H,s), 2.77(3H,brs), 2.77-2.95(2H,m), 3.40-3.50(2H,m), 4.12(2H,brs), 4.59(1H,brs), 6.55(1H,brs), 7.11(2H,d,J=7.9Hz), 7.34(1H,brs), 7.47(2H,d,J=7.9Hz), 8.88(2H,brs)
MS (ESI+) : 220 (M-C₇H₈O₃S+H)
[α]_{D}²⁵ +7.8(c 0.1, MeOH)

### Example 24

### 2-Isopropyl-5,6,7,8-tetrahydro-1,6-naphthyridine hydrochloride

### (step 1)

To a mixed solution of the compound (0.500 g) obtained in Example 6, step 2 and iron (III) acetylacetonate (0.0682 g) in THF/NMP (10 mL/1 mL) was added a 1 mol/L solution (9.70 mL) of isopropylmagnesium chloride in ether under ice-cooling, and the mixture was stirred at room temperature for 16 hr. To the reaction solution was added 8N aqueous sodium hydroxide solution, and the resulting product was extracted with ethyl acetate. The organic layer was washed with saturated brine, dried over anhydrous magnesium sulfate, and concentrated. The obtained residue was purified by silica gel column chromatography (solvent gradient: 0→60% ethyl acetate/hexane) to give 6-benzyl-2-isopropyl-5,6,7,8-tetrahydro-1,6-naphthyridine (0.313 g, 61%) as an oil.
¹H-NMR(CDCl₃):δ1.26(6H,d,J=6.8Hz), 2.85(2H,t,J=5.9Hz), 2.96-3.06(3H,m), 3.58(2H,s), 3.70(2H,s), 6.93(1H,d,J=8.0Hz), 7.21(1H,d,J=8.0Hz), 7.24-7.42(5H,m)
MS(ESI+) :267 (M-HCl+H)

### (step 2)

A suspension of the compound (0.299 g) obtained in step 1 and 20% palladium hydroxide/carbon catalyst (0.200 g) in methanol (10 mL) was stirred under a hydrogen atmosphere at room temperature for 16 hr. The catalyst was filtered off through celite, and the filtrate was concentrated. The obtained residue was dissolved in a mixed solution of ethyl acetate (10 mL) and methanol (5 mL), a 4N hydrogen chloride-ethyl acetate solution (0.280 mL) was added, and the mixture was stirred at room temperature for 30 min. The reaction solution was concentrated, and the obtained solid was recrystallized from ethanol/diisopropyl ether to give 2-isopropyl-5,6,7,8-tetrahydro-1,6-naphthyridine hydrochloride (0.180 g, 76%) as a white powder.
¹H-NMR(DMSO-d₆):δ1.21(6H,d,J=6.8Hz), 2.90-3.01(1H,m), 3.05(2H,t,J=6.4Hz), 3.45(2H,t,J=6.4Hz), 4.24(2H,s), 7.18(1H,d,J=8.0Hz), 7.56(1H,d,J=8.0Hz), 9.52(2H,brs) MS(ESI+):177(M-HCl+H)

### Example 25

### 2-Propyl-5,6,7,8-tetrahydro-1,6-naphthyridine hydrochloride

Using the compound obtained in Example 6, step 2 and a 1 mol/L solution of cyclopropylmagnesium bromide in THF and in the same manner as in the method described in Example 24, 2-propyl-5,6,7,8-tetrahydro-1,6-naphthyridine hydrochloride (0.0749 g, 21%) was obtained as a brown powder. ¹H-NMR(DMSO-d₆):δ0.90(3H,t,J=7.4Hz), 1.60-1.75(2H,m), 2.73(2H,t,J=7.4Hz), 3.13(2H,brs), 3.45(2H,brs), 4.27(2H,brs), 7.22-7.37(1H,m), 7.70(1H,brs), 9.79(2H,brs)
MS(ESI+):177(M-HCl+H)

### Example 26

### 2-(3-Furyl)-5,6,7,8-tetrahydro-1,6-naphthyridine dihydrochloride (step 1)

To a solution of the compound (2.00 g) obtained in Example 6, step 2 in dichloroethane (30 mL) was added 1-chloroethyl chloroformate (0.917 mL), and the mixture was stirred at 90°C for 2 hr. The reaction mixture was cooled to room temperature, and concentrated. To the obtained residue was added methanol (20 mL), and the mixture was stirred under reflux for 1 hr. To the reaction mixture was added diisopropyl ether (30 mL), and the precipitate was collected by filtration. A mixed solution of the obtained solid (1.55 g) and (Boc)₂O (1.69 g) in THF/1N aqueous sodium hydroxide solution (23 mL/23 mL) was stirred at room temperature for 16 hr. The organic layer was separated, and the aqueous layer was extracted with ethyl acetate. The organic layers were combined, washed with saturated brine, dried over anhydrous sodium sulfate, and concentrated to give tert-butyl 2-chloro-7,8-dihydro-1,6-naphthyridine-6(5H)-carboxylate (1.56 g, 75%) as a white powder.
¹H-NMR(CDCl₃):δ1.49(9H,s), 2.97(2H,t,J=6.0Hz), 3.73(2H,d,J=6.0Hz), 4.56(2H,s), 7.17(1H,d,J=8.2Hz), 7.38(1H,d,J=8.2Hz)

### (step 2)

To a mixed solution of the compound (0.300 g) obtained in step 1, 3-furylboronic acid (0.187 g) and potassium carbonate (0.154 g) in DME/water (6 mL)/0.6 mL) was added under an argon atmosphere tetrakis(triphenylphosphine)palladium (0) (Pd(PPh₃)₄) (0.129 g), and the reaction vessel was irradiated in a microwave reaction apparatus at 150°C for 20 min. To the reaction solution was added water, and the resulting product was extracted with ethyl acetate. The organic layer was washed with saturated brine, dried over anhydrous sodium sulfate, and concentrated. The obtained residue was purified by silica gel column chromatography (solvent gradient: 0→30% ethyl acetate/hexane) to give tert-butyl 2-(3-furyl)-7,8-dihydro-1,6-naphthyridine-6(5H)-carboxylate (0.310 g, 92%) as an oil. ¹H-NMR(CDCl₃):δ1.50(9H,s), 2.95-3.05(2H,m), 3.73-3.77(2H,m), 4.58(2H,s), 6.87(1H,dd,J=1.7Hz,0.7Hz), 7.29(1H,d,J=8.2Hz), 7.39(1H,d,J=8.2Hz), 7.47(1H,dd,J=1.7Hz,1.7Hz), 7.99(1H,s)

### (step 3)

A mixture of the compound (0.310 g) obtained in step 2 and 4N hydrogen chloride-ethyl acetate solution (3 mL) was stirred at room temperature for 2 hr. The precipitate was collected by filtration, and the obtained solid was recrystallized from methanol/diisopropyl ether to give 2-(3-furyl)-5,6,7,8-tetrahydro-1,6-naphthyridine dihydrochloride (0.224 g, 80%) as a white powder.
¹H-NMR(DMSO-d₆):δ3.20-3.24(2H,m), 3.47(2H,brs), 4.30-4.33(2H,m), 7.16(1H,s), 7.77(1H,d,J-8.3Hz), 7.82(1H,s), 7.85(1H,d,J=8.3Hz), 8.51(1H,s), 8.70(1H,brs), 9.82(2H,brs)

### MS(ESI+):201(M-2HCl+H)

### Example 27

### 2-(4-Fluorophenyl)-5,6,7,8-tetrahydro-1,6-naphthyridine dihydrochloride

Using the compound obtained in Example 26, step 1 and 4-fluorophenylboronic acid and in the same manner as in the method described in Example 26, 2-(4-fluorophenyl)-5,6,7,8-tetrahydro-1,6-naphthyridine dihydrochloride (0.265 g, 85%) was obtained as a white powder.
¹H-NMR(DMSO-d₆):δ3.23(2H,brs), 3.49(2H,brs), 4.34(2H,brs), 7.35(2H,d,J=8.6Hz), 7.86(1H,d,J=8.2Hz), 7.93(1H,d,J=8.2Hz), 8.12-8.16(2H,m), 8.75(1H,brs), 9.97(2H,brs)
MS(ESI+):229(M-2HCl+H)

### Example 28

### 2-[4-(Methylthio)phenyl]-5,6,7,8-tetrahydro-1,6-naphthyridine dihydrochloride

Using the compound obtained in Example 26, step 1 and 4-(methylthio)phenylboronic acid and in the same manner as in the method described in Example 26, 2-[4-(methylthio)phenyl]-5,6,7,8-tetrahydro-1,6-naphthyridine dihydrochloride (0.317 g, 99%) was obtained as a white powder.
¹H-NMR(DMSO-d₆):δ3.17(3H,s), 3.20(2H,t,J=6.0Hz), 3.49(2H,brs), 4.32-4.35(2H,m), 5.24(1H,brs), 7.38(2H,d,J=8.7Hz), 7.81(1H,d,J=8.2Hz), 7.90(1H,d,J=8.2Hz), 8.04(2H,d,J=8.7Hz), 9.76(2H,brs)

### MS(ESI+):257(M-2HCl+H)

### Example 29

### 2-[4-(Trifluoromethyl)phenyl]-5,6,7,8-tetrahydro-1,6-naphthyridine dihydrochloride

Using the compound obtained in Example 26, step 1 and 4-(trifluoromethyl)phenylboronic acid and in the same manner as in the method described in Example 26, 2-[4-(trifluoromethyl)phenyl]-5,6,7,8-tetrahydro-1,6-naphthyridine dihydrochloride (0.284 g, 87%) was obtained as a white powder. ¹H-NMR(DMSO-d₆):δ3.29-3.23(2H,m), 3.51(2H,brs), 4.36(2H,brs), 6.79(1H,brs), 7.83-7.88(3H,m), 8.01(1H,d,J=8.1Hz), 8.31(2H,d,J=8.1Hz), 9.82(2H,brs)
MS(ESI+):257(M-2HCl+H)

### Example 30

### 2-Isopropoxy-5,6,7,8-tetrahydro-1,6-naphthyridine hydrochloride

### (step 1)

To a solution (5 mL) of isopropyl alcohol (0.089 mL) in toluene was added sodium hydride (0.093 g), and the mixture was stirred under a nitrogen atmosphere at 70°C for 30 min. The compound (0.30 g) obtained in Example 6, step 2, BINAP (0.022 g) and tris(dibenzylideneacetone)dipalladium (0) (0.016 g) were added, and the mixture was stirred under an argon atmosphere at 100°C for 2 hr. The reaction solution was poured into water, and the resulting product was extracted with ethyl acetate. The organic layer was washed with water and saturated brine, dried over anhydrous sodium sulfate, and concentrated. The residue was purified by silica gel column chromatography (solvent gradient; 0→3% ethyl acetate/hexane) to give 6-benzyl-2-isopropoxy-5,6,7,8-tetrahydro-3,6-naphthyridine(0.18 g, 55%) as an oil.
¹H-NMR(CDCl₃):δ1.31(6H,d,J=6.2Hz), 2.77-2.81(2H,m), 2.87-2.95(2H,m), 3.51(2H,s), 3.69(2H,s), 5.20-5.30(1H,m), 6.44(1H,d,J=8.4Hz), 7.14(1H,d,J=8.4Hz), 7.25-7.39(5H,m)

### (step 2)

A suspension of the compound (0.18 g) obtained in step 1 and 20% palladium hydroxide/carbon catalyst (0.020 g) in methanol (3 mL) was stirred under a hydrogen atmosphere at 40°C for 16 hr. The catalyst was filtered off, and the filtrate was concentrated. The obtained residue was purified by basic silica gel column chromatography (solvent gradient; 0→40% ethyl acetate/hexane) to give an oil. To the obtained oil was added 4N hydrogen chloride-ethyl acetate solution and the mixture was concentrated. The obtained residue was recrystallized from ethanol/diisopropyl ether to give 2-isopropoxy-5,6,7,8-tetrahydro-1,6-naphthyridine hydrochloride (0.037 g, 26%) as a white powder.
¹H-NMR(DMSO-d₆):δ1.27(6H,d,J=6.2Hz), 2.96(2H,t,J=6.2Hz), 3.42(2H,brs), 4.17(2H,s), 5.18-5.26(1H,m), 6.65(1H,d,J=8.5Hz), 7.54(1H,d,J=8.5Hz), 9.46(2H,brs)
MS(ESI):193(M-HCl+H)

### Example 31

### 2-(1-Cyclopropylethoxy)-5,6,7,8-tetrahydro-1,6-naphthyridine hydrochloride

### (step 1)

To a solution (5 mL) of 1-cyclopropylethanol (0.11 mL) in toluene was added sodium hydride (0.093 g) and the mixture was stirred under a nitrogen atmosphere at 70°C for 30 min. The compound (0.30 g) obtained in Example 6, step 2, BINAP (0.022 g) and tris(dibenzylideneacetone)dipalladium (0) (0.016 g) were added, and the mixture was stirred under an argon atmosphere at 100°C for 2 hr. The reaction solution was poured into water, and the resulting product was extracted with ethyl acetate. The organic layer was washed with water and saturated brine, dried over anhydrous sodium sulfate, and concentrated. The residue was purified by silica gel column chromatography (solvent gradient; 0→30% ethyl acetate/hexane) to give 6-benzyl-2-(1-cyclopropylethoxy)-5,6,7,8-tetrahydro-1,6-naphthyridine (0.27 g, 77%) as an oil.
¹H-NMR(CDCl₃):δ0.20-0.35(1H,m), 0.35-0.60(3H,m), 1.03-1.12(1H,m), 1.35(3H,d,J=6.2Hz), 2.76-2.80(2H,m), 2.84-2.88(2H,m), 3.50(2H,s), 3.68(2H,s), 4.58-4.67(1H,m), 6.47(1H,d,J=8.3Hz), 7.13(1H,d,J=8.3Hz), 7.24-7.39(5H,m)

### (step 2)

A suspension of the compound (0.27 g) obtained in step 1 and 20% palladium hydroxide/carbon catalyst (0.020 g) in methanol (3 mL) was stirred under a hydrogen atmosphere at 40°C for 16 hr. The catalyst was filtered off, and the filtrate was concentrated. The obtained residue was purified by basic silica gel column chromatography (solvent gradient; 0→40% ethyl acetate/hexane) to give an oil. To the obtained oil was added a 4N hydrogen chloride-ethyl acetate solution, and the mixture was concentrated. The obtained residue was recrystallized from ethanol/diisopropyl ether to give 2-(1-cyclopropylethoxy)-5,6,7,8-tetrahydro-1,6-naphthyridine hydrochloride (0.058 g, 25%) as a white powder. ¹H-NMR(DMSO-d₆):δ0.20-0.40(2H,m), 0.40-0.60(2H,m), 1.00-1.20(1H,m), 1.30(3H,d,J=6.2Hz), 2.91-2.95(2H,m), 3.39-3.43(2H,m), 4.17(2H,s), 4.50-4.70(1H,m), 6.67(1H,d,J=8.4Hz), 7.53(1H,d,J=8.4Hz), 9.38(2H,brs)
MS(ESI+):219(M-HCl+H)

### Example 32

### 3-Chloro-N-isobutyl-N-methyl-5,6,7,8-tetrahydro-1,6-naphthyridin-2-amine hydrochloride

### (step 1)

Using the compound obtained in Example 26, step 1 and N,2-dimethylpropan-1-amine and in the same manner as in the method described in Example 6, step 3, tert-butyl 2-[methyl(2-methylpropyl)amino]-7,8-dihydro-1,6-naphthyridine-6(5H)-carboxylate (1.02 g, 86%) was obtained as an oil. ¹H-NNR(CDCl₃):δ0.90(6H,d,J=6.8Hz), 1.48(9H,s), 1.95-2.15(1H,m), 2.75-2.81(2H,m), 3.04(3H,s), 3.28(2H,d,J=7.3Hz), 3.65-3.70(2H,m), 4.42(2H,s), 6.34(1H,d,J=8.6Hz), 7.13(1H,d,J=8.6Hz) MS(ESI+): 320 (M+H)

### (step 2)

A solution (3 mL) of the compound (0.200 g) obtained in step 1 and N-chlorosuccinimide (0.100 g) in dichloromethane was stirred at room temperature for two days. To the reaction solution was added water, and the resulting product was extracted with ethyl acetate. The organic layer was washed with saturated brine, dried over anhydrous sodium sulfate, and concentrated. The obtained residue was purified by silica gel column chromatography (solvent gradient: 0→40% ethyl acetate/hexane) to give tert-butyl 3-chloro-2-[methyl(2-methylpropyl)amino]-7,8-dihydro-1,6-naphthyridine-6(5H)-carboxylate (0.115 g, 52%) as an oil.
¹H-NMR(CDCl₃):δ0.88(6H,d,J=6.6Hz), 1.49(9H,s), 1.90-2.05(1H,m), 2.75-2.82(2H,m), 2.95(3H,s), 3.19(2H,d,J=7.4Hz), 3.67-3.70(2H,m), 9.45(2H,s), 7.27(1H,s)
MS(ESI+): 354 (M+H)

### (step 3)

A mixture of the compound (0.115 g) obtained in step 2 and 4N hydrogen chloride-ethyl acetate solution (3 mL) was stirred at room temperature for 2 hr. To the reaction solution was added 1N aqueous sodium hydroxide solution, and the resulting product was extracted with ethyl acetate. The organic layer was washed with saturated brine, dried over anhydrous sodium sulfate, and concentrated. To the obtained residue was added a 4N hydrogen chloride-ethyl acetate solution, and the mixture was concentrated. The obtained residue was recrystallized from ethanol/diisopropyl ether to give 3-chloro-N-isobutyl-N-methyl-5,6,7,8-tetrahydro-1,6-naphthyridin-2-amine hydrochloride (0.0260 g, 28%) as a white powder.
¹H-NMR(DMSO-d₆):δ0.82(6H,d,J=6.6Hz), 1.90-2.00(1H,m), 2.92(3H,s), 3.21(2H,d,J=7.5Hz), 3.35-3.45(2H,m), 3.57(2H,m), 4.16(2H,brs), 7.64(1H,s), 9.32(2H,brs)
MS(ES1+):254(M-HCl+H)

### Example 33

### 3-Chloro-N-isopropyl-N-methyl-5,6,7,8-tetrahydxo-1,6-naphthyridin-2-amine dihydrochloride

### (step 1)

Using the compound obtained in Example 26, step 1 and isopropylamine and in the same manner as in the method described in Example 18, step 1 and Example 19, step 1, tert-butyl 2-[methyl(1-methylethyl)amino]-7,8-dihydro-1,6-naphthyridine-6(5H)-carboxylate (0.591 g, 65%) was obtained as an oil.
¹H-NMR(CDCl₃):δ1.15(6H,d,J=6.6Hz), 1.48(9H,s), 2.78-2.82(2H,m), 2.82(3H,s), 3.66-3.70(2H,m), 4.42(2H,s), 4.75-4.90(1H,m), 6.37(1H,d,J=8.7Hz), 7.16(1H,d,J=8.7Hz)

### (step 2)

Using the compound obtained in step 1 and in the same manner as in the method described in Example 32, steps 2 and 3, 3-chloro-N-isopropyl-N-methyl-5,6,7,8-tetrahydro-1,6-naphthyridin-2-amine dihydrochloride (0.082 g, 84%) was obtained as a white powder.
¹H-NMR(DMSO-d₆):δ1.13(6H,d,J=5.6Hz), 2.71(3H,s), 2.91-2.95(2H,m), 3.40-3.45(2H,m), 4.00-4.15(1H,m), 4.16(2H,s), 4.51(1H,brs), 7.66(1H,s), 9.42(2H,brs)
MS(ESI+):240(M-2HCl+H)

### Example 34

### 3-Chloro-N-ethyl-N-methyl-5,6,7,8-tetrahydro-1,6-naphthyridin-2-amine hydrochloride

Using the compound obtained in Example 26, step 1 and N-methylethylamine and in the same manner as in the method described in Example 32, 3-chloro-N-ethyl-N-methyl-5,6,7,8-tetrahydro-1,6-naphthyridin-2-amine hydrochloride (0.039 g, 47%) was obtained as a white powder.
¹H-NMR(DMSO-d₆):δ1.14(3H,t,J=7.0Hz), 2.86(3H,s), 2.87-3.00(2H,m), 3.32(2H,q,J=7.0Hz), 3.45(2H,m), 4.16(2H,s), 7.66(1H,s), 9.21(1H,brs), 9.33(1H,brs)
MS(ESI+):226(M-HCl+H)

### Example 35

### 3-Chloro-2-[(3R)-3-methylmorpholin-4-yl]-5,6,7,8-tetrahydro-1,6-naphthyridine dihydrochloride

### (step 1)

Using the compound obtained in Example 26, step 1 and (3R)-3-methylmorpholine p-toluenesulfonate and in the same manner as in the method described in Example 7, step 1, tert-butyl 2-[(3R)-3-methylmorpholin-4-yl]-7,8-dihydro-1,6-naphthyridine-6(5H)-carboxylate (0.441 g, 36%) was obtained as an oil.
¹H-NMR(CDCl₃):δ1.20(3H,d,J=6.6Hz), 1.49(9H,s), 2.80(2H,t,J=5.9Hz), 3.17(1H,dt,J=3.8Hz,12.4Hz), 3.61(1H,dt,J=3.2Hz,12.1Hz), 3.69(2H,t,J=5.9Hz), 3.77-3.78(2H,m), 3.85(1H,dd,J=2.7Hz,13.0Hz), 4.00(1H,dd,J=2.8Hz,11.3Hz), 4.10-4.15(1H,m),4.44(2H,s), 6.44(1H,d,J=8.6Hz), 7.21(1H,d,J=8.6Hz)
MS(ESI+):368(M+H)

### (step 2)

Using the compound obtained in step 1 and in the same manner as in the method described in Example 32, step 2 and Example 26, step 3, 3-chloro-2-[(3R)-3-methylmorpholin-4-yl]-5,6,7,8-tetrahydro-1,6-naphthyridine dihydrochloride (0.057 g, 62%) was obtained as a white powder. ¹H-NMR(DMSO-d₆):δ1.02(3H,d,J=6.6Hz), 2.95-3.05(3H,m), 3.30-3.51(4H,m), 3.62-3.81(3H,m), 3.85-3.89(1H,m), 4.20(2H,s), 5.25(1H,brs), 7.76(1H,s), 9.60(2H,brs)
MS(ESI+):268(M-2HCl+H)

### Example 36

### 3-Fluoro-2-[(3R)-3-methylmorpholin-4-yl]-5,6,7,8-tetrahydro-1,6-naphthyridine dihydrochloride

### (step 1)

A solution of the compound (0.0930 g) obtained in Example 35, step 1 and N-fluoro-N'-chloromethyltriethylenediamine bis(tetrafluoroborate) (0.110 g) in acetonitrile was stirred under a nitrogen atmosphere at room temperature for two days. To the reaction solution was added water, and the resulting product was extracted with ethyl acetate. The organic layer was washed with saturated brine, dried over anhydrous sodium sulfate, and concentrated. The obtained residue was separated by HPLC to give tert-butyl 3-fluoro-2-[(3R)-3-methylmorpholin-4-yl]-7,8-dihydro-1,6-naphthyridine-6(5H)-carboxylate (0.00580 g, 6%) as an oil
¹H-NMR(CDCl₃):δ1.21(3H,d,J=6.8Hz), 1.49(9H,s), 2.75-2.80(2H,m), 3.33-3.43(1H,m), 3.53-3.58(1H,m), 3.61-3.74(4H,m), 3.82-3.95(2H,m), 4.13-4.16(1H,m), 4.45(2H,s), 6.97(1H,d,J=13.2Hz) MS(ESI+) : 352 (M+H)

### (step 2)

Using the compound obtained in step 1 and in the same manner as in the method described in Example 26, step 3, 3-fluoro-2-[(3R)-3-methylmorpholin-4-yl]-5,6,7,8-tetrahydro-1,6-naphthyridine dihydrochloride (0.00119 g, 22%) was obtained as a white powder.
¹H-NMR(DMSO-d₆):δ1.13(3H,d,J=6.6Hz), 2.85-2.95(2H,m), 3.20-3.30(2H,m), 3.50-3.75(6H,m), 3.87(1H,d,J=11.4Hz), 4.05-4.15(1H,m), 4.17(2H,s), 7.46(1H,d,J=14.1Hz), 9.13(2H,brs) MS(ESI+):252(M-2HCl+H)

### Example 37

### 3-Bromo-2-[(3R)-3-methylmoxpholin-4-yl]-5,6,7,8-tetrahydro-1,6-naphthyridine dihydrochloride

Using the compound obtained in Example 35, step 1 and N-bromosuccinimide and in the same manner as in the method described in Example 32, step 2 and Example 26, step 3, 3-bromo-2-[(3R)-3-methylmorpholin-4-yl]-5,6,7,8-tetrahydro-1,6-naphthyridine dihydrochloride (0.352 g, 73%) was obtained as a white powder.
¹H-NMR(DMSO-d₆):δ0.97(3H,d,J=6.0Hz), 2.90-3.05(3H,m), 3.28-3.36(1H,m), 3.41-3.47(3H,m), 3.65-3.83(4H,m), 4.20(2H,s), 7.39(1H,s), 9.67(2H,brs)

MS(ESI+):312(M-2HCl+H)

### Example 38

### N-Methyl-N-[(1R)-1-methylpropyl]-5,6,7,8-tetrahydro-1,6-naphthyridin-2-amine hydrobromide

A suspension of the compound (1.650 g) obtained in Example 22, step 1 and 20% palladium hydroxide/carbon catalyst (0.165 g) in methanol (30 mL) was stirred under a hydrogen atmosphere at 40°C for 16 hr. The catalyst was filtered off, and the filtrate was concentrated. The obtained oil was purified by basic silica gel column chromatography (solvent gradient: 30→70% ethyl acetate/hexane) and hydrobromic acid-ethanol solution and ethyl acetate were added. The solution was concentrated, and the obtained residue was recrystallized from ethanol/diisopropyl ether to give N-methyl-N-[(1R)-1-methylpropyl]-5,6,7,8-tetrahydro-1,6-naphthyridin-2-amine hydrobromide(0.879 g, 55%) as a white powder. ¹H-NMR(DMSO-d₆):δ0.74(3H,t,J=7.4Hz); 1.05(3H,t,J=6.6Hz), 1.45-1.60(2H,m), 2.74(3H,brs), 2.84-2.88(2H,m), 3.40-3.44(2H,m), 4.11(2H,s), 4.50-4.70(1H,m), 6.56(1H,d,J=8.8Hz), 7.32(1H,d,J-8.8Hz), 8.87(2H,brs)
MS(ESI+):220(M-HBr+H)
[α]_{D}²⁵ -13.9° (c 1.0, MeOH)

### Example 39

### N-Methyl-N-[(1S)-1-methylpropyl]-5,6,7,8-tetrahydro-1,6-naphthyridin-2-amine hydrobromide

Using the compound obtained in Example 6, step 2 and (2S)-butan-2-amine and in the same manner as in the method described in Example 18, step 1, Example 19, step 1 and Example 38, N-methyl-N-[(1S)-1-methylpropyl]-5,6,7,8-tetrahydro-1,6-naphthyridin-2-amine hydrobromide(0.739 g, 58%) was obtained as a white powder.
¹H-NMR(DMSO-d₆):δ0.74(3H,t,J=7.4Hz), 1.05(3H,t,J=6.1Hz), 1.45-1.60(2H,m), 2.74(3H,brs), 2.84-2.88(2H,m), 3.40-3.44(2H,m), 4.11(2H,s), 4.50-4.70(1H,m), 6.56(1H,d,J=8.7Hz), 7.32(1H,d,J=8.9Hz), 8.88(2H,brs)
MS(ESI+):220(M-HBr+H)
[α]_{D}²⁵ +14.3°(c 1.0, MeOH)

### Example 40

### 2-Isobutyl-5,6,7,8-tetrahydro-1,6-naphthyridine hydrochloride

Using the compound obtained in Example 6, step 2 and a 1 mol/L solution of isobutylmagnesium bromide in THF and in the same manner as in the method described in Example 24, 2-isobutyl-5,6,7,8-tetrahydro-1,6-naphthyridine hydrochloride (0.138 g, 31%) was obtained as a white powder.
¹H-NMR(DMSO-d₆):δ0.87(6H,d,J=6.4Hz), 1.93-2.09(1H,m), 2.57(2H,d,J=7.2Hz), 3.06(2H,t,J=6.3Hz), 3.45(2H,brs), 4.26(2H,brs), 7.13(1H,d,J=8.0Hz), 7.57(1H,d,J=8.0Hz), 9.50(2H,brs)
MS(ESI+):191(M-HCl+H)

### Example 41

### 2-(1-Methylpropyl)-5,6,7,8-tetrahydro-1,6-naphthyridine hydrochloride

Using the compound obtained in Example 6, step 2 and a 1 mol/L solution of sec-butylmagnesium bromide in THF and in the same manner as in the method described in Example 24, 2-(1-methylpropyl)-5,6,7,8-tetrahydro-1,6-naphthyridine hydrochloride (0.979 g, 22%) was obtained as a white powder. ¹H-NMR(DMSO-d₆):δ0.77(3H,t,J=7.4Hz), 1.19(3H,d,J=6.8Hz), 1.47-1.76(2H,m), 2.67-2.81(1H,m), 3.07(2H,t,J=6.1Hz), 3.41-3.48(2H,m), 4.25(2H,brs), 7.16(1H,d,J=8.0Hz), 7.59(1H,d,J=8.0Hz), 9.76(2H,brs)
MS(ESI+):191(M-HCl+H)

### Example 42

### 2-(1-Ethylpropyl)-5,6,7,8-tetrahydro-1,6-naphthyridine hydrobromide

### (step 1)

To a mixed solution of the compound (0.500 g) obtained in Example 6, step 2 and iron (III) acetylacetonate (0.0682 g) in THF/NMP (10 mL)/1 mL) was added a 1 mol/L solution (9.70 mL) of 1-ethylpropylmagnesium bromide in THF under ice-cooling, and the mixture was stirred at room temperature for 16 hr. To the reaction solution was added 8N aqueous sodium hydroxide solution, and the resulting product was extracted with ethyl acetate. The organic layer was washed with saturated brine, dried over anhydrous magnesium sulfate, and concentrated. The obtained residue was purified by silica gel column chromatography (solvent gradient: 0→60% ethyl acetate/hexane) to give 6-benzyl-2-(1-ethylpropyl)-5,6,7,8-tetrahydro-1,6-naphthyridine (0.160 g, 28%) as an oil.
¹H-NMR(CDCl₃) :δ0.77(3H,t,J=7.6Hz), 1.59-1.72(4H,m), 2.45-2.57(1H,m), 2.84(2H,t,J=6.1Hz), 3.03(2H,t,J=6.1Hz), 3.59(2H,s), 3.70(2H,s), 6.85(1H,d,J=8.0Hz), 7.19(1H,d,J=8.0Hz), 7.25-7.42(5H,m)
MS(ESI+):295(M+H)

### (step 2)

A suspension of the compound (0.299 g) obtained in step 1 and 20% palladium hydroxide/carbon catalyst (0.200 g) in methanol (10 mL) was stirred under a hydrogen atmosphere at room temperature for 16 hr. The catalyst was filtered off through celite, and the filtrate was concentrated. The obtained residue was dissolved in a mixed solution of ethyl acetate (10 mL) and methanol (5 mL), 4N hydrogen chloride-ethyl acetate solution (0.280 mL) was added, and the mixture was stirred at room temperature for 30 min and concentrated. To the residue was added 1N aqueous sodium hydroxide solution, and the resulting product was extracted with ethyl acetate. The organic layer was washed with saturated brine, dried over anhydrous sodium sulfate, and concentrated. The obtained residue was dissolved in methanol, a 5% hydrogen bromide-methanol solution (0.901 mL) was added, and the mixture was stirred at room temperature for 1 hr and concentrated. The obtained residue was recrystallized from ethanol/diisopropyl ether to give 2-(1-ethylpropyl)-5,6,7,8-tetrahydro-1,6-naphthyridine hydrobromide(0.0704 g, 45%) as a white powder. ¹H-NMR(DMSO-d₆):δ0.71(6H,t,J=7.4Hz), 1.55-1.71(4H,m), 2.45-2.59(1H,m), 3.07(2H,t,J=6.2Hz), 3.49(2H,brs), 4.30(2H,brs), 7.14(1H,d,J=7.7Hz), 7.60(1H,d,J=7.7Hz), 9.05(2H,brs) MS(ESI+):205(M-HBr+H)

### Example 43

### 2-Cyclopropyl-5,6,7,8-tetrahydro-1,6-naphthyridine hydrochloride

### (step 1)

To a mixed solution of the compound (0.500 g) obtained in Example 6, step 2, cyclopropylboronic acid (0.199 g), and potassium phosphate (1.23 g) in toluene/water (8 mL/0.4 mL) were added under an argon atmosphere palladium acetate (0.0217 g) and tricyclohexylphosphine (0.0542 g), the reaction solution was stirred at 100°C for 24 hr. To the reaction solution was added water, and the insoluble material was filtered off through celite. The resulting product was extracted with ethyl acetate, and the organic layer was washed with saturated brine, dried over anhydrous sodium sulfate, and concentrated. The obtained residue was purified by silica gel column chromatography (solvent gradient: 10→80% ethyl acetate/hexane) to give 6-benzyl-2-cyclopropyl-5,6,7,8-tetrahydro-1,6-naphthyridine (0.401 g, 78%) as a yellow powder. ¹H-NMR(CDCl₃):δ.87-0.97(4H,m), 1.94-2.06(1H,m), 2.78-2.85(2H,m), 2.92-2.99(2H,m), 3.56(2H,s), 3.69(2H,s), 6.78(1H,d,J=8.0Hz), 7.12(1H,d,J=8.0Hz), 7.23-7.41(5H,m) MS(ESI+):265(M+H)

### (step 2)

To a solution of the compound (0.401 g) obtained in step 1 in toluene (5 mL) was added 1-chloroethyl chloroformate (0.198 mL), and the mixture was stirred at 80°C for 4 hr. The reaction mixture was cooled to room temperature, and concentrated. To the obtained residue was added methanol (5 mL), and the mixture was stirred under reflux for 2 hr. The reaction mixture was concentrated, and the obtained solid was recrystallized from ethanol/diisopropyl ether to give 2-cyclopropyl-5,6,7,8-tetrahydro-1,6-naphthyridine hydrochloride (0.187 g, 59%) as a pale-yellow powder.
¹H-NMR(DMSO-d₆):δ0.83-0.98(4H,m), 2.98(2H,t,J=6.3Hz), 3.42(2H,t,J=6.3Hz), 4.21(2H,s), 7.16(1H,d,J=8.0Hz), 7.49(1H,d,J=8.0Hz), 9.56(2H,brs)
MS(ESI+):175(M-HCl+H)

### Example 44

### 2-Cyclobutyl-5,6,7,8-tetrahydro-1,6-naphthyridine hydrochloride

Using the compound obtained in Example 6, step 2 and a 1 mol/L solution of cyclobutylmagnesium bromide in THF and in the same manner as in the method described in Example 24, 2-cyclobutyl-5,6,7,8-tetrahydro-1,6-naphthyridine hydrochloride (0.325 g, 74%) was obtained as a yellow powder. ¹H-NMR(DMSO-d₆):δ1.75-1.89(1H,m), 1.89-2.08(1H,m), 2.21-2.29(4H,m), 3.04-3.15(2H,m), 3.45(2H,brs), 3.56-3.72(1H,m), 4.26(2H,brs), 7.24(1H,brs), 7.62(1H,brs), 9.53(2H,brs) MS(ESI+):189(M-HCl+H)

### Example 45

### 2-tert-Butoxy-5,6,7,8-tetrahydro-1,6-naphthyridine hydrochloride

### (step 1)

A mixture of the compound (5.00 g) obtained in Example 6, step 2, BINAP (0.361 g), tris(dibenzylideneacetone)dipalladium (0) (0.265 g), sodium tert-butoxide (3.71 g) and toluene (100 mL) was stirred under an argon atmosphere at 100°C for 3 hr. To the reaction solution was added water, and the insoluble material was filtered off through celite. The resulting product was extracted with ethyl acetate, and the organic layer was washed with saturated brine, dried over anhydrous magnesium sulfate, and concentrated. The residue was purified by silica gel column chromatography (solvent gradient; 0→50% ethyl acetate/hexane) to give 6-benzyl-2-tert-butoxy-5,6,7,8-tetrahydro-1,6-naphthyridine (5.11 g, 89%) as a yellow powder. ¹H-NMR(CDCl₃):δ1.54(9H,s), 2.76-2.83(2H,m), 2.85-2.93(2H,m), 3.51(2H,s), 3.69(2H,s), 6.42(1H,d,J=8.3Hz), 7.11(1H,d,J=8.3Hz), 7.24-7.42(5H,m)
MS(ESI+) : 297 (M+H)

### (step 2)

A suspension of the compound (0.500 g) obtained in step 1 and 10% palladium/carbon catalyst (0.200 g) in methanol (20 mL) was stirred under a hydrogen atmosphere at room temperature for 16 hr. The catalyst was filtered off through celite, and the filtrate was concentrated. The obtained residue was dissolved in ethyl acetate/methanol (5 mL/5 mL), 4N hydrogen chloride-ethyl acetate solution (0.422 mL) was added, and the mixture was stirred at room temperature for 1 hr. The reaction solution was concentrated, and the obtained solid was recrystallized from ethanol/ethyl acetate to give 2-tert-butoxy-5,6,7,8-tetrahydro-1,6-naphthyridine hydrochloride (0.303 g, 74%) as a white powder.
¹H-NMR(DMSO-d₆):δ1.54(9H,s), 2.96(2H,t,J=6.2Hz), 3.42(2H,t,J=6.2Hz), 4.16(2H,s), 6.58(1H,d,J=8.5Hz), 7.49(1H,d,J=8.5Hz), 9.42(2H,brs)
MS(ESI+):207(M-HCl+H)

### Example 46

### 2-(1-Cyclopentylethoxy)-5,6,7,8-tetrahydro-1,6-naphthyridine p-toluenesulfonate

Using the compound obtained in Example 6, step 2 and 1-cyclopentylethanol and in the same manner as in the method described in Example 45, step 1 and Example 21, step 2, 2-(1-cyclopentylethoxy)-5,6,7,8-tetrahydro-1,6-naphthyridine p-toluenesulfonate (0.182 g, 75%) was obtained as a white powder. ¹H-NMR(DMSO-d₆):δ1.22(3H,d,J=6.3Hz), 1.21-1.40(2H,m), 1.45-1.63(4H,m), 1.63-1.80(2H,m), 2.04-2.15(1H,m), 2.29(3H,s), 2.94(2H,t,J=6.4Hz), 3.45(2H,t,J=6.4Hz), 4.20(2H,s), 4.95-5.10(1H,m), 6.66(1H,d,J=8.5Hz), 7.11(2H,d,J=7.9Hz), 7.47(2H,d,J=7.9Hz), 7.53(1H,d,J=8.5Hz), 8.91(2H,brs)
MS(ESI+):247(M-C₇H_{B}O₃S+H)

### Example 47

### 2-(1-Cyclohexylethoxy)-5,6,7,8-tetrahydro-1,6-naphthyridine p-toluenesulfonate

Using the compound obtained in Example 6, step 2 and 1-cyclohexylethanol and in the same manner as in the method described in Example 45, step 1 and Example 21, step 2, 2-(1-cyclohexylethoxy)-5,6,7,8-tetrahydro-1,6-naphthyridine p-toluenesulfonate (0.248 g, 80%) was obtained as a white powder. ¹H-NMR(DMSO-d₆):δ0.90-1.30(5H,m), 1.19(3H,d,J=6.2Hz), 1.45-1.90(6H,m), 2.29(3H,s), 2.94(2H,t,J=6.4Hz), 3.45(2H,t,J=6.4Hz), 4.20(2H,s), 4.90-5.05(1H,m) 6.65(1H,d,J=8.5Hz), 7.11(2H,d,J=7.9Hz), 7.47(2H,d,J=7.9Hz), 7.53(1H,d,J=8.5Hz), 8.93(2H,brs)

### MS(ESI+) : 261 (M-C₇H₈O₃S+H)

### Example 48

### 8-Methyl-2-[(3R)-3-methylmorpholin-4-yl]-5,6,7,8-tetrahydro-1,6-naphthyridine hydrochloride

### (step 1)

Under an argon atmosphere, to a solution of diisopropylamine (16.5 mL) in THF (200 mL) was added dropwise 1.6 mol/L n-butyllithium hexane solution (58.0 mL) at -78°C, and the mixture was stirred for 1 hr. To this reaction solution was added dropwise a solution of the compound (20.0 g) obtained in Example 6, step 2 in THF (50 mL) under an argon atmosphere at -78C, and the mixture was stirred for 1 hr. To the obtained reaction solution was added dropwise methyl iodide (5.05 mL) under an argon atmosphere at -78°C, and the mixture was stirred for 1 hr. To the reaction solution was added a saturated aqueous ammonium chloride solution, and the mixture was warmed to room temperature and ether and water were added. The organic layer was separated, and the aqueous layer was extracted twice with ethyl acetate. The organic layers were combined, washed with saturated brine, dried over anhydrous magnesium sulfate, and concentrated. The residue was purified by silica gel column chromatography (solvent gradient; 0→50% ethyl acetate/hexane) to give 6-benzyl-2-chloro-8-methyl-5,6,7,8-tetrahydro-1,6-naphthyridine (17.8 g, 84%) as a pale-yellow powder.
¹H-NMR(CDCl₃):δ1.37(3H,d,J=6.8Hz), 2.52(1H,dd,J=11.4,6.1Hz), 2.87(1H,dd,J=11.4,4.9Hz), 3.01-3.15(1H,m), 3.50-3.63(2H,m), 3.62-3.76(2H,m), 7.05(1H,d,J=8.3Hz), 7.22(1H,d,J=8.3Hz), 7.25-7.41(5H,m)
MS(ESI+):273(M+H)

### (step 2)

Using the compound obtained in step 1 and (3R)-methylmorpholine p-toluenesulfonate and in the same manner as in the method described in Example 7, 8-methyl-2-[(3R)-3-methylmorpholin-4-yl]-5,6,7,8-tetrahydro-1,6-naphthyridine hydrochloride (0.0803 g, 15%) was obtained as a pale-yellow powder.
¹H-NMR(DMSO-d₆):δ1.06-1.15(3H,m), 1.32(3H,d,J=6.4Hz), 2.97-3.18(3H,m), 3.40-3.57(2H,m), 3.57-3.77(2H,m), 3.79-3.99(2H,m), 4.12(2H,brs), 4.24-4.39(1H,m), 6.73(1H,d,J=8.5Hz) 7.42(1H,d,J=8.5Hz), 9.53(2H,brs)
MS(ESI+):248(M-HCl+H)

### Example 49

### 2-[(3R)-3-Ethylmorpholin-4-yl]-8-methyl-5,6,7,8-tetrahydro-1,6-naphthyridine hydrochloride

Using the compound obtained in Example 48, step 1 and (3R)-ethylmorpholine p-toluenesulfonate and in the same manner as in the method described in Example 7, 2-[(3R)-3-ethylmorpholin-4-yl]-8-methyl-5,6,7,8-tetrahydro-1,6-naphthyridine hydrochloride (0.177 g, 33%) was obtained as a yellow powder.
¹H-NMR(DMSO-d₆):δ0.80-0.90(3H,m), 1.26-1.35(3H,m), 1.38-1.56(1H,m), 1.65-1.84(1H,m), 2.95-3.15(3H,m), 3.37-3.57(3H,m), 3.79-4.19(6H,m), 6.67(1H,d,J=8.7Hz), 7.36(1H,d,J=8.7Hz), 9.59(2H,brs)
MS(ESI+):262(M-HCl+H)

### Example 50

### 8-Methyl-2-(3-propylmorpholin-4-yl)-5,6,7,8-tetrahydro-1,6-naphthyridine hydrochloride

Using the compound obtained in Example 48, step 1 and 3-propylmorpholine p-toluenesulfonate and in the same manner as in the method described in Example 7, 8-methyl-2-(3-propylmorpholin-4-yl)-5,6,7,8-tetrahydro-1,6-naphthyridine hydrochloride (0.0777 g, 23%) was obtained as a yellow powder. ¹H-NMR(DMSO-d₆):δ0.81-0.93(3H,m), 1.12-1.47(6H,m), 1.64-1.82(1H,m), 2.94-3.15(3H,m), 3.36-3.66(3H,m), 3.77-3.99(3H,m), 4.11(2H,brs), 4.15-4.29(1H,m), 6.67(1H,d,J=8.7Hz), 7.36(1H,d,J=8.7Hz), 9.52(2H,brs)
MS(ESI+):276(M-HCl+H)

### Example 51

### 2-(2,5-Dimethylpyrrolidin-1-yl)-8-methyl-5,6,7,8-tetrahydro-1,6-naphthyridine hydrochloride

Using the compound obtained in Example 48, step 1 and 2,5-dimethylpyrrolidine and in the same manner as in the method described in Example 10, 2-(2,5-dimethylpyrrolidin-1-yl)-8-methyl-5,6,7,8-tetrahydro-1,6-naphthyridine hydrochloride (0.140 g, 27%) was obtained as a yellow powder. ¹H-NMR(DMSO-d₆):δ1.23(3H,d,J=6.4Hz), 1.26(3H,d,J=6.4Hz), 1.32(3H,d,J=6.4Hz), 1.63-1.75(2H,m), 1.94-2.09(2H,m), 2.94-3.11(2H,,m), 3.46-3.56(1H,m), 3.89-4.04(2H,m), 4.09(2H,brs), 6.3B(1H,d,J=8.7Hz), 7.29(1H,d,J=8.7Hz), 9.45(2H,brs) MS(ESI+):246(M-HCl+H)

### Example 52

### N-Cyclopentyl-N,8-dimethyl-5,6,7,8-tetrahydro-1,6-naphthyridin-2-amine hydrochloride

To a mixture of the compound (0.500 g) obtained in Example 48, step 1, cyclopentylamine (0.312 g), XPhos (0.105 g), sodium tert-butoxide (0.266 g) and toluene (10 mL) was added under an argon atmosphere tris(dibenzylideneacetone)dipalladium (0) (0.0671 g), and the reaction solution was stirred at 100°C for 16 hr. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 5→100% ethyl acetate/hexane). A solution (10 mL) of the obtained compound, formalin (2.27 mL), sodium triacetoxyborohydride (2.31 g) and acetic acid (0.4 mL) in DMF was stirred at room temperature for 16 hr. To the reaction solution were added water and 8N aqueous sodium hydroxide solution, and the resulting product was extracted with ethyl acetate. The organic layer was washed with saturated brine, dried over anhydrous magnesium sulfate, and concentrated. The obtained residue was purified by silica gel column chromatography (solvent gradient: 0→50% ethyl acetate/hexane) to give 6-benzyl-N-cyclopentyl-N,8-dimethyl-5,6,7,8-tetrahydro-1,6-naphthyridin-2-amine (0.114 g, 18%) as a white powder.
¹H-NMR(CDCl₃):δ1.32(3H,d,J=6.8Hz), 1.46-1.78(6H,m), 1.78-1.93(2H,m), 2.36-2.46(1H,m), 2.83-3.00(2H,m), 2.86(3H,s), 3.41-3.53(2H,m), 3.59-3.72(2H,m), 4.84-4.97(1H,m), 6.31(1H,d,J=8.7Hz), 7.03(1H,d,J=8.7Hz), 7.21-7.43(5H,m) MS(ESI+):336(M+H)

### (step 2)

A suspension of the compound (0.114 g) obtained in step 1 and 20% palladium hydroxide/carbon catalyst (0.200 g) in methanol (10 mL) was stirred under a hydrogen atmosphere at room temperature for 16 hr. The catalyst was filtered off through celite, and the filtrate was concentrated. The obtained residue was dissolved in methanol, and 4N hydrogen chloride-ethyl acetate solution (0.0850 mL) was added. The reaction solution was stirred at room temperature for 30 min, and concentrated. The obtained residue was recrystallized from ethanol/diisopropyl ether to give N-cyclopentyl-N,8-dimethyl-5,6,7,8-tetrahydro-1,6-naphthyridin-2-amine hydrochloride (0.0551 g, 58%) as a yellow powder.
¹H-NMR(DMSO-d₆):δ1.31(3H,d,J=6.4Hz), 1.44-1.64(4H,m), 1.64-1.85(4H,m), 2.83(3H,s), 2.96-3.12(2H,m), 3.46-3.59(1H,m), 4.09(2H,s), 4.81-4.95(1H,m), 6.57(1H,d,J=8.7Hz), 7.31(1H,d,J=8.7Hz), 9.37(2H,brs)
MS(ESI+):246(M-HCl+H)

### Example 53

### N-Isopropyl-N,8-dimethyl-5,6,7,8-tetrahydro-1,6-naphthyridin-2-amine hydrochloride

Using the compound obtained in Example 48, step 1 and isopropylamine and in the same manner as in the method described in Example 52, N-isopropyl-N,8-dimethyl-5,6,7,8-tetrahydro-1,6-naphthyridin-2-amine hydrochloride (0.108 g, 23%) was obtained as a pale-yellow powder.
¹H-NMR(DMSO-d₆):δ.10(3H,d,J=6.4Hz), 1.12(3H,d,J=6.4Hz), 1.32(3H,d,J=6.4Hz), 2.81(3H,s), 3.05(2H,brs), 3.45-3.57(1H,m), 4.07-4.13(2H,m), 4.68-4.85(1H,m), 6.58(1H,brs), 7.27-7.42(1H,m), 9.37(2H,brs)
MS(ESI+):220(M-HCl+H)

### Example 54

### 2-Isopropyl-8-methyl-5,6,7,8-tetrahydro-1,6-naphthyridine hydrochloride

Using the compound obtained in Example 48, step 1 and a 1 mol/L solution of isopropylmagnesium chloride in THF and in the same manner as in the method described in Example 24, 2-isopropyl-8-methyl-5,6,7,8-tetrahydro-1,6-naphthyridine hydrochloride (0.0893 g, 27%) was obtained as a white powder. H-NMR(DMSO-d₆):δ.22(6H,d,J=6.8Hz), 1.36(3H,d,J=6.8Hz), 2.92-3.05(1H,m), 3.05-3.28(2H,m), 3.58(1H,dd,J=11.9,5.1Hz), 4.26(2H,s), 7.17(1H,d,J=8.0Hz), 7.55(1H,d,J=8.0Hz), 9.38(2H,brs)
MS(ESI+):191(M-HCl+H)

### Example 55

### 2-Isobutyl-8-methyl-5,6,7,8-tetrahydro-1,6-naphthyridine hydrochloride

Using the compound obtained in Example 48, step 1 and a 1 mol/L solution of isobutylmagnesium bromide in THF and in the same manner as in the method described in Example 24, 2-isobutyl-8-methyl-5,6,7,8-tetrahydro-1,6-naphthyridine hydrochloride (0.161 g, 43%) was obtained as a white powder. ¹H-NMR(DMSO-d₆):δ0.88(6H,d,J=6.4Hz), 1.35(3H,d,J=6.8Hz), 1.94-2.14(1H,m), 2.58(2H,d,J=7.2Hz), 3.05-3.29(2H,m), 3.58(1H,dd,J=11.7,5.3Hz), 4.26(2H,s), 7.11(1H,d,J=8.0Hz), 7.54(1H,d,J=8.0Hz), 9.51(2H,brs)
MS(ESI+):205(M-HCl+H)

### Example 56

### 8-Methyl-2-(1-methylpropyl)-5,6,7,8-tetrahydro-1,6-naphthyridine hydrochloride

Using the compound obtained in Example 48, step 1 and a 1 mol/L solution of sec-butylmagnesium bromide in THF and in the same manner as in the method described in Example 24, 8-methyl-2-(1-methylpropyl)-5,6,7,8-tetrahydro-1,6-naphthyridine hydrochloride (0.0671 g, 19%) was obtained as a white powder. ¹H-NMR(DMSO-d₆):δ0.77(3H,t,J=7.4Hz), 1.15-1.22(3H,m), 1.35(3H,d,J=6.8Hz), 1.47-1.79(2H,m), 2.68-2.82(1H,m), 3.06-3.27(2H,m), 3.54-3.64(1H,m) 4.27(2H,s), 7.14(1H,d,J=8.0Hz), 7.55(1H,d,f=8.0Hz), 9.44(2H,brs)
MS(ESI+):205(M-HCl+H)

### Example 57

### 2-Cycloprapyl-8-methyl-5,6,7,8-tetrahydxo-1,6-naphthyridine hydrochloride

Using the compound obtained in Example 48, step 1 and cyclopropylboronic acid and in the same manner as in the method described in Example 43, 2-cyclopropyl-8-methyl-5,6,7,8-tetrahydro-1,6-naphthyridine hydrochloride (0.260 g, 63%) was obtained as a white powder.
¹H-NMR(DMSO-d₆):δ0.85-1.00(4H,m), 1.31(3H,d,J=6.8Hz), 2.02-2.14(1H,m), 3.01-3.23(2H,m), 3.55(1H,m), 4.23(2H,brs), 7.17(1H,d,J=8.1Hz), 7.99(1H,d,J=8.1Hz), 9.53(2H,brs) MS(ESI+):189(M-HCl+H)

### Example 58

### 2-Cyclobutyl-8-methyl-5,6,7,8-tetrahydro-1,6-naphthyridine hydrobromide

### (step 1)

To a mixture of chlorocyclobutane (1.00 g), turnings magnesium (3.36 g) and THF (50 mL) was added iodine (1 piece), and the mixture was refluxed for 10 min. A solution of chlorocyclobutane (9.00 g) in THF (50 mL) was added dropwise to the reaction solution, and the mixture was further refluxed for 16 hr and cooled to room temperature.

### (step 2)

To a mixed solution of the obtained compound (0.486 g) in Example 4, step 5 and iron (III) acetylacetonate (0.0608 g) in THF/NMP (5 mL/1 mL) was added the solution (8.60 mL) obtained in step 1 under ice-cooling, and the mixture was stirred at room temperature for 6 hr. To the reaction solution was added 1N hydrochloric acid, and the resulting product was extracted with ethyl acetate. The organic layer was washed with saturated brine, dried over anhydrous magnesium sulfate, and concentrated. The obtained residue was purified by silica gel column chromatography (solvent gradient: 0→50% ethyl acetate/hexane) to give tert-butyl 2-cyclobutyl-8-methyl-7,8-dihydro-l,6-naphthyridine-6(5H)-carboxylate (0.0872 g, 17%) as an oil.
¹H-NMR(CDCl₃):δ1.33(3H,d,J=7.2Hz), 1.49(9H,s), 1.81-1.95(1H,m), 1.95-2.13(1H,m), 2.21-2.41(4H,m), 2.99-3.13(1H,m), 3.48-3.81(3H,m), 4.28-4.85(2H,m), 7.02(1H,d,J=8.0Hz), 7.31(1H,d,J=8.0Hz)
MS(ESI+:303(M+H)

### (step 3)

A mixture of the compound (0.132 g) obtained in step 2 and 4N hydrogen chloride-ethyl acetate solution (6 mL) was stirred at room temperature for 2 hr. To the reaction solution was added 1N aqueous sodium hydroxide solution, and the resulting product was extracted with ethyl acetate. The organic layer was washed with saturated brine, dried over anhydrous magnesium sulfate, and concentrated. The obtained residue was purified by basic silica gel column chromatography (solvent gradient: 5→100% ethyl acetate/hexane), and the resulting product was dissolved in methanol. A 5% hydrogen bromide methanol solution (0.389 mL) was added, and the mixture was stirred at room temperature for 30 min, and concentrated. The obtained residue was recrystallized from ethanol/diisopropyl ether to give 2-cyclobutyl-8-methyl-5,6,7,8-tetrahydro-1,6-naphthyridine hydrobromide(0.0404 g, 33%) as a white powder.
¹H-NMR(DMSO-d₆):δ1.38(3H,d,J=6.8Hz), 1.77-1.91(1H,m), 1.91-2.08(1H,m), 2.18-2.31(4H,m), 3.09-3.28(2H,m), 3.54-3.70(2H,m), 4.29(2H,s), 7.16(1H,d,J=8.0Hz), 7.55(1H,d,J=8.0Hz), 9.03(2H,brs)
MS(ESI+):203(M-HBr+H)

### Example 59

### 2-Cyclopentyl-8-methyl-5,6,7,8-tetrahydro-1,5-naphthyridine hydrochloride

Using the compound obtained in Example 48, step 1 and a 1 mol/L solution of cyclopentylmagnesium bromide in THF and in the same manner as in the method described in Example 24, 2-cyclopentyl-8-methyl-5,6,7,8-tetrahydro-1,6-naphthyridine hydrochloride (0.0954 g, 21%) was obtained as a white powder. ¹H-NMR(DMSO-d₆):δ1.51(3H,d,J=7.0Hz), 1.55-1.88(6H,m), 1.96-2.10(2H,m), 3.08-3.21(2H,m), 3.35-3.50(1H,m), 3.66(1H,dd,J=12.6,5.3Hz), 4.36(2H,s), 7.04(1H,d,J=7.9Hz), 7.30(1H,d,J=7.9Hz), 10.20(2H,brs)
MS(ESI+) :217 (M-HCl+H)

### Example 60

### 8-Methyl-5,6,7,8-tetrahydro-1,6-naphthyridin-2(1H)-one hydrochloride

### (step 1)

A mixture of the compound (0.500 g) obtained in Example 48, step 1, BINAP (0.0342 g), tris(dibenzylideneacetone)dipalladium (0) (0.0252 g), sodium tert-butoxide (0.352 g) and toluene (5 mL) was stirred under an argon atmosphere at 100°C for 16 hr. To the reaction solution was added water, and the insoluble material was filtered off through celite. The resulting product was extracted with ethyl acetate, and the organic layer was washed with saturated brine, dried over anhydrous magnesium sulfate, and concentrated. The residue was purified by silica gel column chromatography (solvent gradient; 0→50% ethyl acetate/hexane) to give 6-benzyl-2-tert-butoxy-8-methyl-5,6,7,8-tetrahydro-1,6-naphthyridine (0.422 g, 74%) as a yellow powder.
¹H-NMR(CDCl₃):δ1.31(3H,d,J=6.8Hz), 1.56(9H,s), 2.38(1H,dd,J=10.9,7.2Hz), 2.89-3.06(2H,m), 3.41-3.59(2H,m), 3.61-3.72(2H,m), 6.40(1H,d,J=8.7Hz), 7.10(1H,d,J=8.7Hz), 7.23-7.42(5H,m)
MS(ESI+):311(M+H)

### (step 2)

A suspension of the compound (0.422 g) obtained in step 1 and 20% palladium hydroxide/carbon catalyst (0.200 g) in methanol (5 mL) was stirred under a hydrogen atmosphere at room temperature for 16 hr. The catalyst was filtered off through celite, and the filtrate was concentrated. The obtained residue was dissolved in ethyl acetate/methanol (5 mL/1 mL), a 4N hydrogen chloride-ethyl acetate solution (0.340 mL) was added, and the mixture was stirred at room temperature for 1 hr. The reaction solution was concentrated, and the obtained solid was recrystallized from ethanol/diisopropyl ether to give 8-methyl-5,6,7,8-tetrahydro-1,6-naphthyridin-2(1H)-one hydrochloride (0.260 g, 95%) as a white powder.
¹H-NMR(DMSO-d₆):δ1.32(3H,d,J=7.0Hz), 2.91-3.14(2H,m), 3.33-3.48(1H,m), 3.95(2H,brs), 6.29(1H,d,J=9.2Hz), 7.30(1H,d,J=9.2Hz), 9.35(1H,brs), 9.67(1H,brs)
MS(ESI+):165(M-HCl+H)

### Example 61

### 2-Isopropoxy-8-methyl-5,6,7,8-tetrahydro-1,6-naphthyridine hydrochloride

Using the compound obtained in Example 48, step 1 and 2-propanol and in the same manner as in the method described in Example 30, 2-isopropoxy-8-methyl-5,6,7,8-tetrahydro-1,6-naphthyridine hydrochloride (0.170 g, 38%) was obtained as a white powder.
¹H-NMR(DMSO-d₆):δ1.29(6H,d,J=6.1Hz), 1.33(3H,d,J=6.8Hz), 3.00-3.23(2H,m), 3.55(1H,dd,J=11.7,5.3Hz), 4.18(2H,s), 5.15-5.29(1H,m), 6.64(1H,d,J=8.3Hz), 7.52(1H,d,J=8.3Hz), 9.58(2H,brs)
MS(ESI+):207(M-HCl+H)

### Example 62

### 2-Cyclobutoxy-8-methyl-5,6,7,8-tetrahydro-1,6-naphthyridine hydrochloride

Using the compound obtained in Example 48, step 1 and cyclobutanol and in the same manner as in the method described in Example 30, 2-cyclobutoxy-8-methyl-5,6,7,8-tetrahydro-1,6-naphthyridine hydrochloride (0.124 g, 27%) was obtained as a white powder.
¹H-NMR(DMSO-d₆):δ1.32(3H,d,J=6.8Hz), 1.56-1.85(2H,m), 1.93-2.14(2H,m), 2.31-2.47(2H,m), 2.99-3.21(2H,m), 3.55(1H,dd,J=11.2,4.6Hz), 4.18(2H,s), 5.02-5.15(1H,m), 6.68(1H,d,J=8.5Hz), 7.54(1H,d,J=8.5Hz), 9.53(2H,brs) MS(ESI+):219(M-HCl+H)

### Example 63

### 8-Methyl-2-[(1S)-1-methylpropoxy]-5,6,7,8-tetrahydro-1,6-naphthyridine hydrochloride

Using the compound obtained in Example 48, step 1 and (2S)-butan-2-ol and in the same manner as in the method described in Example 30, 8-methyl-2-[(1S)-1-methylpropoxyl-5,6,7,8-tetrahydro-1,6-naphthyridine hydrochloride (0.136 g, 29%) was obtained as a white powder.
¹H-NMR(DMSO-d₆):δ0.85-0.94(3H,m), 1.25(3H,d,J=6.4Hz), 1.30-1.37(3H,m), 1.52-1.75(2H,m), 2.98-3.24(2H,m), 3.50-3.59(1H,m), 4.17(2H,s), 4.97-5.17(1H,m), 6.65(1H,d,J=8.5Hz), 7.52(1H,d,J=8.5Hz), 9.69(2H,brs)
MS(ESI+):221(M-HCl+H)

### Example 64

### 8-Methyl-2-[(1R)-1-methylpropoxy]-5,6,7,8-tetrahydro-1,6-naphthyridine hydrochloride

Using the compound obtained in Example 48, step 1 and (2R)-butan-2-ol and in the same manner as in the method described in Example 30, 8-methyl-2-[(1R)-1-methylpropoxy]-5,6,7,8-tetrahydro-1,6-naphthyridine hydrochloride (0.147 g, 31%) was obtained as a white powder.
¹H-NMR(DMSO-d₆):δ0.84-0.95(3H,m), 1.25(3H,d,J=5.3Hz), 1.29-1.37(3H,m), 1.52-1.75(2H,m), 3.02-3.21(2H,m), 3.52-3.61(1H,m), 4.19(2H,s), 4.98-5.17(1H,m), 6.66(1H,d,J=8.3Hz), 7.52(1H,d,J=8.3Hz), 9.41(2H,brs)
MS(ESI+):221(M-HCl+H)

### Example 65

### 8,8-Dimethyl-2-[(3R)-3-methylmorpholin-4-yl]-5,6,7,8-tetrahydro-1,6-naphthyridine hydrochloride

### (step 1)

To a solution of formalin (61.1 mL) in ethanol (150 mL) was added dropwise benzylamine (36.4 mL) under ice-cooling, and the obtained reaction mixture was added dropwise to a solution of 3-methylbutan-2-one (30.6 g), and concentrated hydrochloric acid (31.0 mL) in ethanol (150 mL) under refluxing. The reaction solution was stirred for 16 hr under reflux, and cooled to room temperature. N-Ethyldiisopropylamine (50.2 mL) and formalin (8.15 mL) were added to the reaction solution, and the mixture was stirred under reflux for 16 hr, and cooled to room temperature. To the reaction solution was added a solution of potassium hydroxide (20.7 g) in water (70 mL), and ethanol was evaporated under reduced pressure. The resulting product was extracted with ethyl acetate, and the organic layer was washed with saturated brine, dried over anhydrous magnesium sulfate, and concentrated. The obtained residue was purified by silica gel column chromatography (solvent gradient: 5→15% ethyl acetate/hexane) to give 1-benzyl-3,3-dimethylpiperidin-4-one (51.8 g, 71%) as an oil.
¹H-NMR(CDCl₃):δ1.13(6H,s), 2.40(2H,s), 2.52(2H,t,J=6.2Hz), 2.72(2H,t,J=6.2Hz), 3.56(2H,s), 7.23-7.41(5H,m)
MS (ESI+) :218 (M+H)

### (step 2)

A solution of the compound (25.0 g) obtained in step 1 in THF (100 mL) was added dropwise under reflux to a suspension of diethyl carbonate (34.0 g) and sodium hydride (13.8 g) in THF (100 mL), and the mixture was stirred for 16 hr. The reaction mixture was cooled to room temperature, saturated aqueous ammonium chloride solution was added under ice-cooling, and ether and water were added. The organic layer was separated, and the aqueous layer was extracted with ethyl acetate. The organic layer was washed with saturated brine, dried over anhydrous magnesium sulfate, and concentrated. The obtained residue was purified by silica gel column chromatography (solvent gradient: 0→50% ethyl acetate/hexane) to give ethyl 1-benzyl-5,5-dimethyl-4-oxopiperidine-3-carboxylate (27.8 g, 84%) as an oil.
¹H-NMR(CDCl₃):δ1.16(6H,s), 1.27(3H,t,J=7.2Hz), 2.27(2H,s), 3.19(2H,s), 3.59(2H,s), 4.19(2H,q,J=7.2Hz), 7.24-7.39(5H,m), 12.21(1H,s)
MS(ESI+):290(M+H)

### (step 3)

A solution of the compound (33.1 g) obtained in step 2 and ammonium acetate (88.1 g) in methanol (300 mL) was stirred for 96 hr, and concentrated. To the residue was added ethyl acetate, and the organic layer was washed with saturated aqueous sodium hydrogen carbonate solution. The aqueous layer was extracted with ethyl acetate, and the organic layer was washed with saturated brine, dried over anhydrous magnesium sulfate, and concentrated to give ethyl 4-amino-1-benzyl-5,5-dimethyl-1,2,5,6-tetrahydropyridine-3-carboxylate (33.0 g, quantitative yield) as an oil.
¹H-NMR(CDCl₃):δ1.15(6H,s), 1.25(3H,t,J=7.1Hz), 2.21(2H,s), 3.25(2H,s), 4.12(2H,q,J=7.1Hz), 7.21-7.40(5H,m) MS(ESI+):289(M+H)

### (step 4)

To a solution of the compound (33.0 g) obtained in step 3 and triethylamine (31.9 mL) in THF (400 mL) was added dropwise ethyl chloroformylacetate (21.0 g) under ice-cooling, and the mixture was stirred at room temperature for 16 hr. The reaction mixture was concentrated, and ethyl acetate was added to the residue. The organic layer was washed with saturated aqueous sodium hydrogen carbonate solution. The aqueous layer was extracted with ethyl acetate, and the organic layer was washed with saturated brine, dried over anhydrous magnesium sulfate, and concentrated. The obtained residue was dissolved in ethanol (500 mL), sodium ethoxide (23.3 g) was gradually added under ice-cooling, and the mixture was stirred at room temperature for 16 hr. The reaction mixture was concentrated, and the residue was washed with 20% ethyl acetate/hexane solution, and dissolved in water. The aqueous layer was neutralized with 1N hydrochloric acid, and the resulting solid was collected by filtration and washed with ethyl acetate. The aqueous layer was extracted with ethyl acetate, and the organic layer was washed with saturated brine, dried over anhydrous magnesium sulfate, and concentrated. The obtained solid was collected, and washed with ethanol and ethyl acetate to give ethyl 6-benzyl-4-hydroxy-8,8-dimethyl-2-oxo-1,2,5,6,7,8-hexahydro-1,5-naphthyridine-3-carboxylate (15.0 g,37%) as a white powder.
¹H-NMR(CDCl₃):δ1.35(6H,s), 1.38(3H,t,J=7.2Hz), 2.38(2H,s), 3.42(2H,s), 3.68(2H,s), 4.37(2H,q,J=7.2Hz), 7.24-7.41(5H,m), 11.39(1H,brs), 13.62(1H,s)
MS(ESI+):356(M+H)

### (step 5)

A mixture of the compound (14.0 g) obtained in step 4 and 2N aqueous sodium hydroxide solution (200 mL) was stirred under reflux for 24 hr, and cooled to room temperature. The reaction solution was neutralized with 1N hydrochloric acid, and the resulting solid was collected by filtration, washed with an ethanol/ethyl acetate mixed solution, and dried. To a solution of the obtained solid (10.0 g) in DMF (100 mL) was added dropwise phosphorus oxychloride(10.0 g) under ice-cooling, and the mixture was stirred at 90°C for 2 hr. The reaction solution was cooled to room temperature, poured into a saturated aqueous sodium hydrogen carbonate solution, and the resulting solid was collected by filtration. The filtrate was extracted with ethyl acetate, and the organic layer was washed with saturated brine, dried over anhydrous magnesium sulfate, and concentrated. The obtained residue and solid were combined, washed with an ethanol/ethyl acetate mixed solution and dried to give 6-benzyl-4-chloro-8,8-dimethyl-5,6,7,8-tetrahydro-1,6-naphthyridin-2(1H)-one (6.32 g, 64%) as a pale-yellow powder.
¹H-NMR(CDCl₃):δ1.32(6H,s), 2.39(2H,s), 3.44(2H,s), 3.68(2H,s), 6.49(1H,s), 7.24-7.40(5H,m), 11.24(1H,brs
MS(ESI+):303(M+H)

### (step 6)

A suspension of the compound (6.32 g) obtained in step 5 and 20% palladium hydroxide-carbon catalyst (2.00 g) in methanol (100 mL) was stirred under a hydrogen atmosphere at room temperature for 16 hr. The insoluble material was filtered off through celite, and the filtrate was concentrated to give 8,8-dimethyl-5,6,7,8-tetrahydro-1,6-naphthyridin-2(1H)-one (3.30 g, 89%) as a white powder.
¹H-NMR(DMSO-d₆):δ1.36(6H,s), 3.16(2H,s), 3.95(2H,s), 6.29(1H,d,J=9.5Hz), 7.31(1H,d,J=9.5Hz), 9.59(1H,brs), 11.42(1H,brs)

### (step 7)

A mixture of the compound (3.00 g) obtained in step 6, potassium carbonate (4.65 g), benzyl bromide (2.88 g) and DMF (50 mL) was stirred at room temperature for 24 hr, and the reaction mixture was poured into water. The resulting product was extracted with ethyl acetate, and the organic layer was washed with saturated brine, dried over anhydrous magnesium sulfate, and concentrated. The obtained solid was washed with a mixed solution of ethanol-diisopropyl ether to give 6-benzyl-8,8-dimethyl-5,6,7,8-tetrahydro-1,6-naphthyridin-2(1H)-one (3.23 g, 71%) as a white powder.
¹H-NMR(DMSO-d₆):δ1.21(6H,s), 2.32(2H,s), 3.26(2H,s), 3.60(2H,s), 6.11(1H,d,J=9.2Hz), 7.13(H,d,J=9.2Hz), 7.21-7.39(5H,m), 11.24(1H,brs)
MS(ESI+):269(M+H)

### (step 8)

A solution of the compound (3.50 g) obtained in step 7, phosphorus oxychloride (10.0 g), and DMF (5 mL) in toluene (30 mL) was stirred at 110°C for 2 hr, and cooled to room temperature. The reaction mixture was concentrated, poured into ice water, and basified with an 8N aqueous sodium hydroxide solution. The resulting product was extracted with ethyl acetate, and the organic layer was washed with saturated brine, dried over anhydrous magnesium sulfate, and concentrated. The obtained residue was purified by silica gel column chromatography (solvent gradient: 0-50% ethyl acetate/hexane) to give 6-benzyl-2-chloro-8,8-dimethyl-5,6,7,8-tetrahydro-1,6-naphthyridine (3.46 g, 93%) as an oil. ¹H-NMR(CDCl₃):51.32(6H,s), 2.52(2H,s), 3.56(2H,s), 3.67(2H,s), 7.02(H,d,J=8.0Hz), 7.20(1H,d,J=8.0Hz), 7.24-7.42(5H,m) MS(ESI+):287(M+H)

### (step 9)

Using the compound obtained in step 8 and in the same manner as in the method described in Example 7, 8,8-dimethyl-2-[(3R)-3-methylmorpholin-4-yl]-5,6,7,8-tetrahydro-1,6-naphthyridine hydrochloride (0.0642 g, 16%) was obtained as a orange powder.
¹H-NMR(DMSO-d₆):δ1.09(3H,d,J=6.8Hz), 1.32(6H,s), 3.01(1H,td,J=12.5,3.8Hz), 3.26(2H,brs), 3.41-3.53(1H,m), 3.58-3.65(1H,m), 3.69-3.76(1H,m), 3.83(1H,dd,J=13.3,1.9Hz), 3.93(1H,dd,J=11.2,3.6Hz), 4.12(2H,brs), 4.27-4.37(1H,m), 6.67(1H,d,J=8.7Hz), 7.38(1H,d,J=8.7Hz), 9.42(2H,brs)
MS(ESI+):262(M-HCl+H)

### Example 66

### 8,8-Dimethyl-2-morpholin-4-yl-5,6,7,8-tetrahydro-1,6-naphthyridine hydrochloride

Using the compound obtained in Example 65, step 8 and in the same manner as in the method described in Example 6, step 3 and step 4, 8,8-dimethyl-2-morpholin-4-yl-5,6,7,8-tetrahydro-1,6-naphthyridine hydrochloride (0.241 g, 61%) was obtained as a white powder.
¹H-NMR(DMSO-d₆):δ1.32(6H,s), 3.25(2H,s), 3.40-3.47(4H,m), 3.66-3.72(4H,m), 4.12(2H,s), 6.74(1H,d,J=8.7Hz), 7.40(1H,d,J=8.7Hz), 9.44(2H,brs)
MS(ESI+):248(M-HCl+H)

### Example 67

### 2-Isopropyl-8,8-dimethyl-5,6,7,8-tetrahydro-1,6-naphthyridine hydrochloride

Using the compound obtained in Example 65, step 8 and a 1 mol/L solution of isopropylmagnesium chloride in THF and in the same manner as in the method described in Example 24, 2-isopropyl-8,8-dimethyl-5,6,7,8-tetrahydro-1,6-naphthyridine hydrochloride (0.0535 g, 16%) was obtained as a white powder. ¹H-NMR(DMSO-d₆):δ1.23(6H,d,J=6.8Hz), 1.40(6H,s), 2.99-3.15(1H,m), 3.26-3.35(2H,m), 4.26(2H,brs), 7.22(1H,brs), 7.62(1H,brs), 9.82(2H,brs)
MS(ESI+):205(M-HCl+H)

### Example 68

### 8,8-Dimethyl-2-(1-methylpropyl)-5,6,7,8-tetrahydro-1,6-naphthyridine hydrochloride

Using the compound obtained in Example 65, step 8 and a 1 mol/L solution of sec-butylmagnesium bromide in THF and in the same manner as in the method described in Example 24, 8,8-dimethyl-2-(1-methylpropyl)-5,6,7,8-tetrahydro-1,6-naphthyridine hydrochloride (0.0701 g, 20%) was obtained as a white powder.
¹H-NMR(DMSO-d₆):δ0.90(3H,t,J=7.4Hz), 1.26(3H,d,J=6.1Hz), 1.35(6H,s), 1.52-1.77(2H,m), 3.27(2H,s), 4.16(2H,s), 4.99-5.12(1H,m), 6.63(1H,d,J=8.7Hz), 7.52(1H,d,J=8.7Hz), 9.73(2H,brs)
MS(ESI+):219(M-HCl+H)

### Example 69

### 2-Cyclopropyl-8,8-dimethyl-5,6,7,8-tetrahydro-1,6-naphthyridine hydrochloride

Using the compound obtained in Example 65, step 8 and cyclopropylboronic acid and in the same manner as in the method described in Example 43, 2-cyclopropyl-8,8-dimethyl-5,6,7,8-tetrahydro-1,6-naphthyridine hydrochloride (0.105 g, 32%) was obtained as a white powder.
¹H-NMR(DMSO-d₆):50.83-1.01(4H,m), 1.33(6H,s), 2.04-2.23(1H,m), 3.21-3.35(2H,m), 4.18-4.27(2H,m), 7.16(1H,d,J=8.0Hz), 7.51(1H,d,J=8.0Hz), 9.67(2H,brs)
MS(ESI+):203(M-HCl+H)

### Example 70

### 2-Cyclobutyl-8,8-dimethyl-5,6,7,8-tetrahydro-1,6-naphthyridine hydrochloride

Using the compound obtained in Example 65, step 8 and a 1 mol/L solution of cyclobutylmagnesium bromide in THF and in the same manner as in the method described in Example 24, 2-cyclobutyl-8,8-dimethyl-5,6,7,8-tetrahydro-1,6-naphthyridine hydrochloride (0.194 g, 55%) was obtained as a white powder. ¹H-NMR(DMSO-d₆):δ1.38(6H,s), 1.78-1.91(1H,m), 1.91-2.07(1H,m), 2.19-2.31(4H,m), 3.33(2H,s), 3.54-3.68(1H,m), 4.26(2H,s), 7.12(1H,d,J=8.0Hz), 7.53(1H,d,J=8.0Hz), 9.41(2H,brs) MS(ESI+):217(M-HCl+H)

### Example 71

### 2-Isopropoxy-8,8-dimethyl-5,6,7,8-tetrahydro-1,6-naphthyridine hydrochloride

Using the compound obtained in Example 65, step 8 and 2-propanol and in the same manner as in the method described in Example 30, 2-isopropoxy-8,8-dimethyl-5,6,7,8-tetrahydro-1,6-naphthyridine hydrochloride (0.0667 g, 19%) was obtained as a white powder.
¹H-NMR(DMSO-d₆):δ1.30(6H,d,J=6.1Hz), 1.35(6H,s), 3.28(2H,s), 4.17(2H,s), 5.14-5.28(1H,m), 6.62(1H,d,J=8.3Hz), 7.52(1H,d,J=8.3Hz), 9.59(2H,brs)
MS(ESI+):221(M-HCl+H)

### Example 72

### 8,8-Dimethyl-2-[(1S)-1-methylpropoxy]-5,6,7,8-tetrahydro-1,6-naphthyridine hydrochloride

Using the compound obtained in Example 65, step 8 and (2S)-butan-2-ol and in the same manner as in the method described in Example 30, 8,8-dimethyl-2-[(1S)-1-methylpropoxy]-5,6,7,8-tetrahydro-1,6-naphthyridine hydrochloride (0.115 g, 31%) was obtained as a white powder. ¹H-NMR(DMSO-d₆):δ0.90(3H,t,J=7.4Hz), 1.26(3H,d,J=6.1Hz), 1.35(6H,s), 1.52-1.77(2H,m), 3.27(2H,s), 4.16(2H,s), 4.99-5.12(1H,m), 6.63(1H,d,J=8.7Hz), 7.52(1H,d,J=8.7Hz), 9.73(2H,brs)
MS(ESI+):235(M-HCl+H)

### Example 73

### (8R^{*})-8-Methyl-2-morpholin-4-yl-5,6,7,8-tetrahydro-1,6-naphthyridine hydrochloride

### (step 1)

A solution of the compound (13.7 g) obtained in Example 48, step 1 and 1-chloroethyl chloroformate (8.64 g) in toluene was stirred at 80°C for 2 hr, and the mixture was stirred at room temperature for 16 hr. The reaction solution was concentrated, and methanol (50 mL) was added to the residue. The mixture was refluxed for 1 hr, and cooled to room temperature. To the reaction solution was added diisopropyl ether (100 mL), and the resulting solid was collected by filtration and washed with diisopropyl ether. A mixed solution of the obtained solid and (Boc)₂O (14.2 g) in THF/1N aqueous sodium hydroxide solution (100 mL)/100 mL) was stirred at room temperature for 16 hr. The organic layer was separated, and the aqueous layer was extracted 3 times with ethyl acetate. The organic layer was washed with saturated brine, dried over anhydrous magnesium sulfate, and concentrated. The obtained residue was purified by silica gel column chromatography (solvent gradient: 0→50% ethyl acetate/hexane) to give tert-butyl 2-chloro-8-methyl-7,8-dihydro-1,6-naphthyridine-6(5H)-carboxylate (12.1 g, 98%) as a white solid.
¹H-NMR(CDCl₃):δ1.33(3H,d,J=7.0Hz), 1.49(9H,s), 2.98-3.12(1H,m), 3.50-3.77(2H,m), 4.35-4.51(1H,m), 4.74(1H,brs), 7.14(1H,d,J=8.1Hz), 7.36(1H,d,J=8.1Hz)
MS(ESI+):283(M+H)

### (step 2)

The compound (12.1 g) obtained in step 1 was subjected to optical resolution using CHIRALPAK AD (50×500 mm) manufactured by Daicel Chemical Industries, Ltd. and 2% 2-propanol/hexane solvent (flow rate 80 mL/min) under detection at UV 220 nm, whereby compound C (5.99 g, 99.9%ee) with early elution time and compound B (5.92 g, 99.9%ee) with late elution time were obtained.

### (step 3)

A mixture of the compound C (0.500 g) obtained in step 2, morpholine (0.308 g), tris(dibenzylideneacetone)dipalladium (0) (0.0648 g), XPhos (0.101 g), sodium tert-butoxide (0.255 g) and toluene (10 mL) was stirred under a nitrogen atmosphere at 100°C for 3 hr. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 2→40% ethyl acetate/hexane) to give tert-butyl (8R^{*})-8-methyl-2-morpholin-4-yl-7,8-dihydro-1,6-naphthyridine-6(5H)-carboxylate (0.555 g, 94%) as an oil. ¹H-NMR(CDCl₃):δ1.28(3H,d,J=7.2Hz), 1.49(9H,s), 2.81-2.98(1H,m), 3.36-3.57(5H,m), 3.60-3.79(1H,m), 3.80-3.84(4H,m), 4.34-4.64(2H,m), 6.48(1H,d,J=8.3Hz), 7.22(1H,d,J=8.3Hz) MS(ESI+):334(M+H)

### (step 4)

A mixture of the compound (0.555 g) obtained in step 3 and 4N hydrogen chloride-ethyl acetate solution (5 mL) was stirred at room temperature for 3 hr, and concentrated. To the obtained residue were added 8N aqueous sodium hydroxide solution and saturated brine, and the resulting product was extracted with ethyl acetate. The organic layer was washed with saturated brine, dried over anhydrous sodium sulfate, and concentrated. The obtained residue was dissolved in methanol, and 4N hydrogen chloride-ethyl acetate solution (0.416 mL) was added. The reaction solution was stirred at room temperature for 10 min, and concentrated. The obtained residue was recrystallized from ethanol/diisopropyl ether to give (8R^{*})-8-methyl-2-morpholin-4-yl-5,6,7,8-tetrahydro-1,6-naphthyridine hydrochloride (0.354 g, 78%) as a white powder. ¹H-NMR(DMSO-d₆):δ1.31(3H,d,J=6.9Hz), 2.97-3.16(2H,m), 3.40-3.47(4H,m), 3.49-3.58(1H,m), 3.65-3.72(4H,m), 4.13(2H,s), 6.75(1H,d,J=8.9Hz), 7.41(1H,d,J=8.9Hz), 9.47(2H,brs) MS(ESI+):234(M-HCl+H)

### Example 74

### (8S^{*})-8-Methyl-2-morpholin-4-yl-5,6,7,8-tetrahydro-1,6-naphthyridine hydrochloride

### (step 1)

A mixture of the compound B (0.500 g) obtained in Example 73, step 2, morpholine (0.308 g), tris(dibenzylideneacetone)dipalladium (0) (0.0648 g), XPhos (0.101 g), sodium tert-butoxide (0.255 g) and toluene (10 mL) was stirred under a nitrogen atmosphere at 100°C for 16 hr. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 2→40% ethyl acetate/hexane) to give tert-butyl (8S^{*})-8-methyl-2-morpholin-4-yl-7,8-dihydro-1,6-naphthyridine-6(5H)-carboxylate (0.267 g, 45%) as an oil.
¹H-NMR(CDCl₃):δ1.28(3H,d,J=6.8Hz), 1.49(9H,s), 2.88(1H,brs), 3.33-3.57(5H,m), 3.59-3.78(1H,m), 3.78-3.86(4H,m), 4.33-4.66(2H,m), 6.48(1H,d,J=8.7Hz) 7.22(1H,d,J=8.7Hz) MS(ESI+):334(M+H)

### (step 2)

A mixture of the compound (0.267 g) obtained in step 1 and 4N hydrogen chloride-ethyl acetate solution (5 mL) was stirred at room temperature for 3 hr, and concentrated. To the obtained residue were added 8N aqueous sodium hydroxide solution and saturated brine, and the resulting product was extracted with ethyl acetate. The organic layer was washed with saturated brine, dried over anhydrous sodium sulfate, and concentrated. The obtained residue was dissolved in methanol, and 4N hydrogen chloride-ethyl acetate solution (0.200 mL) was added. The reaction solution was stirred at room temperature for 10 min, and concentrated. The obtained residue was recrystallized from ethanol/diisopropyl ether to give (8S^{*})-8-methyl-2-morpholin-4-yl-5,6,7,8-tetrahydro-1,6-naphthyridine hydrochloride (0.192 g, 89%) as a white powder.
¹H-NMR(DMSO-d₆):δ1.31(3H,d,J=6.4Hz), 2.98-3.15(2H,m), 3.39-3.48(4H,m), 3.48-3.61(1H,m), 3.64-3.74(4H,m), 4.14(2H,s), 6.75(1H,d,J=8.7Hz), 7.40(1H,d,J=8.7Hz), 9.25-9.52(2H,m) MS(ESI+):234(M-HCl+H)

### Example 75

### (8R^{*})-8-Methyl-2-[(3R)-3-methylmorpholin-4-yl]-5,6,7,8-tetrahydro-1,6-naphthyridine hydrochloride

### (step 1)

A mixture of the compound C (0.500 g) obtained in Example 73, step 2, (3R)-3-methylmorpholine p-toluenesulfonate (0.483 g), tris(dibenzylideneacetone)dipalladium (0) (0.0648 g), XPhos (0.101 g), sodium tert-butoxide (0.425 g) and toluene (10 mL) was stirred under a nitrogen atmosphere at 100°C for 16 hr. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 2→40% ethyl acetate/hexane) to give tert-butyl (BR^{*})-8-methyl-2-[(3R)-3-methylmorpholin-4-yl]-7,8-dihydro-1,6-naphthyridine-6(5H)-carboxylate (0.295 g, 48%) as an oil.
¹H-NMR(CDCl₃):δ1.19(3H,d,J=6.6Hz), 1.28(3H,d,J=7.0Hz), 1.49(9H,s), 2.89(1H,brs), 3.16(1H,td,J=12.4,3.8Hz), 3.31-3.56(1H,m), 3.57-3.85(5H,m), 4.00(1H,dd,J=11.3,2.8Hz), 4.28-4.39(1H,m), 4.40-4.65(2H,m), 6.42(1H,d,J=8.5Hz), 7.20(1H,d,J=8.5Hz)
MS(ESI+):348(M+H)

### (step 2)

A mixture of the compound (0.295 g) obtained in step 1 and 4N hydrogen chloride-ethyl acetate solution (5 mL) was stirred at room temperature for 2 hr, and concentrated. To the obtained residue were added 8N aqueous sodium hydroxide solution and saturated brine, and the resulting product was extracted with ethyl acetate. The organic layer was washed with saturated brine, dried over anhydrous sodium sulfate, and concentrated. The obtained residue was dissolved in methanol, 4N hydrogen chloride-ethyl acetate solution (0.212 mL) was added. The reaction solution was stirred at room temperature for 10 min, and concentrated. The obtained residue was recrystallized from ethanol/diisopropyl ether to give (8R^{*})-8-methyl-2-[(3R)-3-methylmorpholin-4-yl]-5,6,7,8-tetrahydro-1,6-naphthyridine hydrochloride (0.165 g, 68%) as a pale-yellow powder.
¹H-NMR(DMSO-d₆):δ1.09(3H,d,J=6.4Hz), 1.30(3H,d,J=6.8Hz), 2.93-3.16(3H,m), 3.40-3.65(3H,m), 3.68-3.76(1H,m),
3.83(1H,dd,J=13.3,1.9Hz), 3.93(1H,dd,J=11.4,3.4Hz), 4.12(2H,s), 4.26-4.36(1H,m), 6.69(1H,d,J=8.7Hz), 7.39(1H,d,J=8.7Hz), 9.49(2H,brs)
MS(ESI+):248(M-HCl+H)

### Example 76

### (8S^{*})-8-Methyl-2-[(3R)-3-methylmorpholin-4-yl]-5,6,7,8-tetrahydro-1,6-naphthyridine hydrochloride

### (step 1

A mixture of the compound B (0.500 g) obtained in Example 73, step 2, (3R)-3-methylmorpholine p-toluenesulfonate (0.483 g), tris(dibenzylideneacetone)dipalladium (0) (0.0648 g), XPhos (0.101 g), sodium tert-butoxide (0.425 g) and toluene (10 mL) was stirred under a nitrogen atmosphere at 100°C for 3 hr. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 2→40% ethyl acetate/hexane) to give tert-butyl (8S^{*})-8-methyl-2-[(3R)-3-methylmorpholin-4-yl]-7,8-dihydro-1,6-naphthyridine-6(5H)-carboxylate (0.363 g, 59%) as an oil.
¹H-NMR(CDCl₃):51.21(3H,d,J=6.8Hz), 1.28(3H,d,J=6.8Hz), 1.49(9H,s), 2.80-2.96(1H,m), 3.16(1H,td,J=12.7,3.8Hz), 3.29-3.54(1H,m), 3.56-3.84(4H,m), 3.86-3.94(1H,m), 3.96-4.04(1H,m), 4.21-4.32(1H,m), 4.34-4.64(2H,m), 6.41(1H,d,J=8.3Hz), 7.19(1H,d,J=8.3Hz)
MS(ESI+):348(M+H)

### (step 2)

A mixture of the compound (0.363 g) obtained in step 1 and 4N hydrogen chloride-ethyl acetate solution (5 mL) was stirred at room temperature for 2 hr, and concentrated. To the obtained residue were added 8N aqueous sodium hydroxide solution and saturated brine, and the resulting product was extracted with ethyl acetate. The organic layer was washed with saturated brine, dried over anhydrous sodium sulfate, and concentrated. The obtained residue was dissolved in methanol, and 4N hydrogen chloride-ethyl acetate solution (0.273 mL) was added. The reaction solution was stirred at room temperature for 10 min, and concentrated. The obtained residue was recrystallized from ethanol/diisopropyl ether to give (8S^{*})-8-methyl-2-[(3R)-3-methylmorpholin-4-yl]-5,6,7,8-tetrahydro-1,6-naphthyridine hydrochloride (0.211 g, 82%) as a yellow powder.
¹H-NMR(DMSO-d₆):δ1.10(3H,d,J=6.8Hz), 1.31(3H,d,J=6.8Hz), 2.95-3.15(3H,m), 3.31-3.66(3H,m), 3.67-3.76(1H,m), 3.86-3.97(2H,m), 4.13(2H,brs), 4.24-4.35(1H,m), 6.68(1H,d,J=8.9Hz), 7.39(1H,d,J=8.9Hz), 9.40(2H,brs)
MS(ESI+):248(M-HCl+H)

### Example 77

### (8R^{*})-2-[(3R)-3-Ethylmorpholin-4-yl]-8-methyl-5,6,7,8-tetrahydro-1,6-naphthyridine hydrochloride

### (step 1)

A mixture of the compound C (0.500 g) obtained in Example 73, step 2, (3R)-3-ethylmorpholine p-toluenesulfonate (0.508 g), tris(dibenzylideneacetone)dipalladium (0) (0.0648 g), XPhos (0.101 g), sodium tert-butoxide (0.425 g) and toluene (10 mL) was stirred under a nitrogen atmosphere at 100°C for 16 hr. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 2→40% ethyl acetate/hexane) to give tert-butyl (8R^{*})-2-[(3R)-3-ethylmorpholin-4-yl-8-methyl-7,8-dihydro-1,6-naphthyridine-6(5H)-carboxylate (0.135 g, 21%) as an oil.
¹H-NMR(CDCl₃):δ0.90(3H,t,J=7.6Hz), 1.27(3H,d,J=6.8Hz), 1.49(9H,s), 1.54-1.67(1H,m), 1.76-1.94(1H,m), 2.80-2.97(1H,m), 3.17(1H,td,J=12.5,3.8Hz), 3.30-3.55(1H,m), 3.55-3.86(4H,m), 3.90-4.01(2H,m), 4.04-4.15(1H,m), 4.34-4.63(2H,m), 6.39(1H,d,J=8.7Hz), 7.18(1H,d,J=8.7Hz)

### MS(ESI+):362(M+H)

### (step 2)

A solution of the compound (0.135 g) obtained in step 1 and 4N hydrogen chloride-ethyl acetate solution (5 mL) in methanol (1 mL) was stirred at room temperature for 3 hr, and concentrated. To the obtained residue was added 8N aqueous sodium hydroxide solution, and the resulting product was extracted with ethyl acetate. The organic layer was washed with saturated brine, dried over anhydrous sodium sulfate, and concentrated. The obtained residue was dissolved in methanol, and 4N hydrogen chloride-ethyl acetate solution (0.0774 mL) was added. The reaction solution was stirred at room temperature for 10 min, and concentrated. The obtained residue was recrystallized from ethanol/diisopropyl ether to give (8R^{*})-2-[(3R)-3-ethylmorpholin-4-yl]-8-methyl-5,6,7,8-tetrahydro-1,6-naphthyridine hydrochloride (0.0809 g, 73%) as a pale-yellow powder.
¹H-NMR(DMSO-d₆):δ0.84(3H,t,J=7.6Hz), 1.30(3H,d,J=6.8Hz), 1.37-1.54(1H,m), 1.64-1.82(1H,m), 2.95-3.14(3H,m), 3.37-3.61(3H,m), 3.80-3.94(3H,m), 4.06-4.19(3H,m), 6.68(1H,d,J=8.7Hz), 7.36(1H,d,J=8.7Hz), 9.23-9.49(2H,m)
MS (ESI+) :262 (M-HCl+H)

### Example 78

### (8S^{*})-2-[(3R)-3-Ethylmorpholin-4-yl]-8-methyl-5,6,7,8-tetrahydro-1,6-naphthyridine hydrochloride

A mixture of the compound B (0.500 g) obtained in Example 73, step 2, (3R)-3-ethylmorpholine p-toluenesulfonate (0.508 g), tris(dibenzylideneacetone)dipalladium (0) (0.0648 g), XPhos (0.101 g), sodium tert-butoxide (0.425 g) and toluene (10 mL) was stirred under a nitrogen atmosphere at 100°C for 16 hr. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 2→40% ethyl acetate/hexane). A solution of the obtained compound (0.304 g) and 4N hydrogen chloride-ethyl acetate solution (5 mL) in methanol (1 mL) was stirred at room temperature for 2 hr, and concentrated. To the obtained residue were added 8N aqueous sodium hydroxide solution and saturated brine, and the resulting product was extracted with ethyl acetate. The organic layer was washed with saturated brine, dried over anhydrous sodium sulfate, and concentrated. The obtained residue was dissolved in methanol, and 4N hydrogen chloride-ethyl acetate solution (0.210 mL) was added. The reaction solution was stirred at room temperature for 10 min, and concentrated. The obtained residue was recrystallized from ethanol/diisopropyl ether to give (8S*)-2-[(3R)-3-ethylmorpholin-4-yl]-8-methyl-5,6,7,8-tetrahydro-1,6-naphthyridine hydrochloride (0.228 g, 91%) as a pale-yellow powder.
¹H-NMR(DMSO-d₆):δ0.85(3H,t,J=7.4Hz), 1.30(3H,d,J=6.4Hz), 1. 38-1.54(1H,m), 1.66-1.83(1H,m), 2.94-3.13(3H,m), 3.36-3.59(3H,m), 3.79-4.08(4H,m), 4.11(2H,s), 6.67(1H,d,J=8.7Hz), 7.36(1H,d,J=8.7Hz), 9.29-9.52(2H,m)
MS(ESI+):262(M-HCl+H)

### Example 79

### (8R^{*})-N-Isopropyl-N,8-dimethyl-5,6,7,8-tetrahydro-1,6-naphthyridin-2-amine hydrochloride

### (step 1)

A mixture of the compound C (0.500 g) obtained in Example 73, step 2, isopropylamine (0.209 g), tris(dibenzylideneacetone)dipalladium (0) (0.0648 g), XPhos (0.101 g), sodium tert-butoxide (0.255 g) and toluene (10 mL) was stirred under a nitrogen atmosphere at 100°C for 16 hr. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 2→40% ethyl acetate/hexane). To a solution of the obtained compound (0.340 g), formalin (0.287 g), and acetic acid (0.200 g) in acetonitrile (10 mL) was added sodium triacetoxyborohydride (0.353 g), and the mixture was stirred at room temperature for 48 hr. To the reaction solution was added saturated brine, and the resulting product was extracted with ethyl acetate. The organic layer was washed with saturated brine, dried over anhydrous magnesium sulfate, and concentrated. The obtained residue was purified by silica gel column chromatography (solvent gradient: 0→30% ethyl acetate/hexane) to give tert-butyl (8R^{*})-2-(isopropylmethylamino)-8-methyl-7,8-dihydro-1,6-naphthyridine-6(5H)-carboxylate (0.170 g, 30%) as an oil.
¹H-NMR(CDCl₃):δ1.14(3H,d,J=7.0Hz), 1.16(3H,d,J=7.0Hz), 1.29(3H,d,J=6.8Hz), 1.44-1.51(9H,m), 2.77-2.95(4H,m), 3.28-3.56(1H,m), 3.56-3.87(1H,m), 4.27-4.63(2H,m), 4.76-4.93(1H,m), 6.35(1H,d,J=8.7Hz), 7.14(1H,d,J=8.7Hz)
MS(ESI+):320(M+H)

### (step 2)

A solution of the compound (0.170 g) obtained in step 1 and 4N hydrogen chloride-ethyl acetate solution (5 mL) in methanol (1 mL) was stirred at room temperature for 16 hr, and concentrated. To the obtained residue were added 8N aqueous sodium hydroxide solution and saturated brine, and the resulting product was extracted with ethyl acetate. The organic layer was washed with saturated brine, dried over anhydrous sodium sulfate, and concentrated. The obtained residue was dissolved in methanol, and 4N hydrogen chloride-ethyl acetate solution (0.133 mL) was added. The reaction solution was stirred at room temperature for 10 min, and concentrated. The obtained residue was recrystallized from ethanol/diisopropyl ether to give (8R^{*})-N-isopropyl-N,8-dimethyl-5,6,7,8-tetrahydro-1,6-naphthyridin-2-amine hydrochloride (0.101 g, 74%) as a yellow powder. ¹H-NMR(DMSO-d6):δ1.09(3H,d,J=6.4Hz), 1.11(3H,d,J=6.4Hz), 1.31(3H,d,J=6.4Hz), 2.79(3H,s), 2.95-3.13(1H,m), 3.38(1H,brs), 3.46-3.59(1H,m), 4.10(2H,brs), 4.70-4.87(1H,m), 6.54(1H,d,J=8.7Hz), 7.32(1H,d,J=8.7Hz), 9.37(2H,brs) MS(ESI+):220(M-HCl+H)

### Example 80

### (8S^{*})-N-Isopropyl-N,8-dimethyl-5,6,7,8-tetrahydro-1,6-naphthyridin-2-amine hydrochloride

### (step 1)

A mixture of the compound B (0.500 g) obtained in Example 73, step 2, isopropylamine (0.209 g), tris(dibenzylideneacetone)dipalladium (0) (0.0648 g), XPhos (0.101 g), sodium tert-butoxide (0.255 g) and toluene (10 mL) was stirred under a nitrogen atmosphere at 100°C for 16 hr. To the reaction solution was added water, the insoluble material was filtered off through celite, and the resulting product was extracted with ethyl acetate. The organic layer was washed with saturated brine, dried over anhydrous magnesium sulfate, and concentrated. The obtained residue was purified by silica gel column chromatography (solvent gradient: 2→40% ethyl acetate/hexane). To a solution of the obtained compound (0.373 g), formalin (0.315 g), and acetic acid (0.220 g) in acetonitrile (10 mL) was added sodium triacetoxyborohydride (0.388 g), and the mixture was stirred at room temperature for 24 hr. To the reaction solution was added saturated brine, and the resulting product was extracted with ethyl acetate. The organic layer was washed with saturated brine, dried over anhydrous magnesium sulfate, and concentrated. The obtained residue was purified by silica gel column chromatography (solvent gradient: 0→30% ethyl acetate/hexane) to give tert-butyl (8S^{*})-2-(isopropylmethylamino)-8-methyl-7,8-dihydro-1,6-naphthyridine-6(5H)-carboxylate (0.207 g, 37%) as an oil.
¹H-NMR(CDCl₃):δ1.11-1.18(6H,m), 1.23-1.31(3H,m), 1.49(9H,s), 2.79-2.95(4H,m), 3.30-3.56(1H,m), 3.56-3.87(1H,m), 4.30-4.63(2H,m), 4.77-4.92(1H,m) 6.35(1H,d,J=8.7Hz), 7.14(1H,d,J=8.7Hz)
MS(ESI+):320(M+H)

### (step 2)

A solution of the compound (0.207 g) obtained in step 1 and 4N hydrogen chloride-ethyl acetate solution (5 mL) in methanol (1 mL) was stirred at room temperature for 2 hr, and concentrated. To the obtained residue were added 8N aqueous sodium hydroxide solution and saturated brine, and the resulting product was extracted with ethyl acetate. The organic layer was washed with saturated brine, dried over anhydrous sodium sulfate, and concentrated. The obtained residue was dissolved in methanol, and 4N hydrogen chloride-ethyl acetate solution (0.162 mL) was added. The reaction solution was stirred at room temperature for 10 min, and concentrated. The obtained residue was recrystallized from ethanol/diisopropyl ether to give (8S^{*})-N-isopropyl-N,8-dimethyl-5,6,7,8-tetrahydro-1,6-naphthyridin-2-amine hydrochloride (0.118 g, 71%) as a yellow powder.
¹H-NMR(DMSO-d₆):δ1.10(3H,d,J=6.8Hz), 1.11(3H,d,J=6.8Hz), 1.31(3H,d,J=6.8Hz), 2.79(3H,s), 3.04(1H,brs), 3.38-3.59(2H,m), 4.10(2H,brs), 4.71-4.87(1H,m), 6.54(1H,d,J=8.7Hz), 7.32(1H,d,J=8.7Hz), 9.35(2H,brs)
MS(ESI+):220(M-HCl+H)

The chemical structural formulas of the compounds obtained in the Examples are shown in the following Tables 1 to 5.

**Table 1**

| Ex. No | structural formula | salt |
|---|---|---|
| 1 | 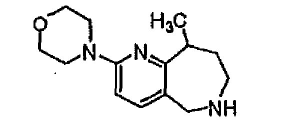 | |
| 2 | 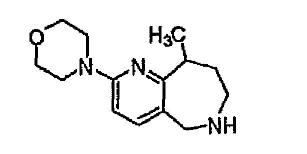 | HCl |
| 3 | 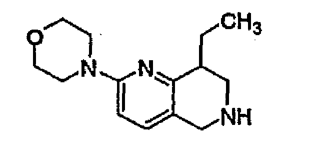 | |
| 4 | 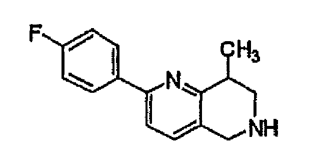 | HCl |
| 5 | 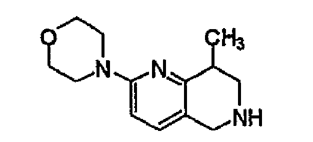 | 2HCl |

**Table 2**

| | | | | | | |
|---|---|---|---|---|---|---|
| | 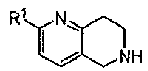 | | | | 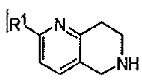 | |

| Ex. No. | R¹ | salt | | Ex. No. | R¹ | salt |
|---|---|---|---|---|---|---|
| 6 | 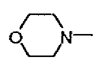 | | | 19 | 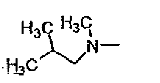 | |
| 7 | 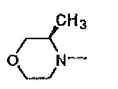 | HCl | | 20 | 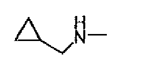 | HCl |
| 8 | 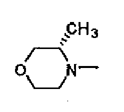 | HCl | | 21 | 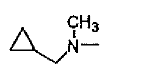 | TsOH |
| 9 | 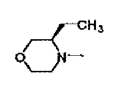 | HCl | | 22 | 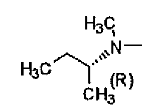 | TsOH |
| 10 | 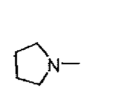 | HCl | | 23 | 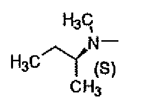 | TsOH |
| 11 | 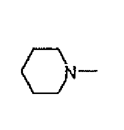 | HCl | | 24 | 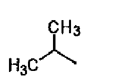 | HCl |
| 12 | 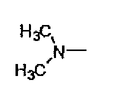 | HCl | | 25 | CH₃CH₂CH₂ | HCl |
| 13 | 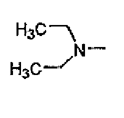 | HCl | | 26 | 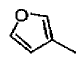 | 2HCl |
| 14 | 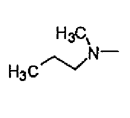 | HCl | | 27 | 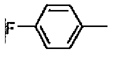 | 2HCl |
| 15 | 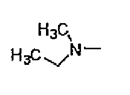 | HCl | | 28 | 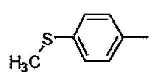 | 2HCl |
| 16 | 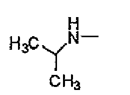 | HCl | | 29 | 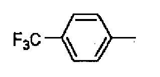 | 2HCl |
| 17 | 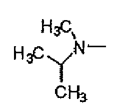 | HCl | | 30 | 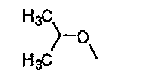 | HCl |
| 18 | 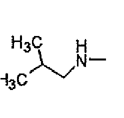 | | | 31 | 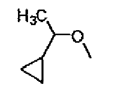 | HCl |

**Table 3**

| Ex. No. | structural formula | salt | | Ex. No. | structural formula | salt |
|---|---|---|---|---|---|---|
| 32 | 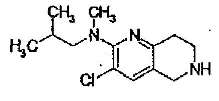 | HCl | | 42 | 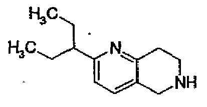 | HBr |
| 33 | 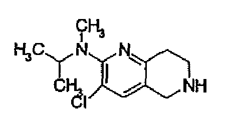 | 2HCl | | 43 | 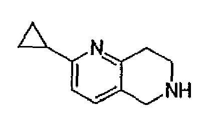 | HCl |
| 34 | 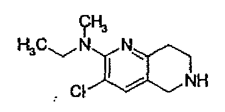 | HCl | | 44 | 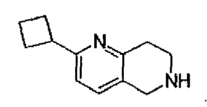 | HCl |
| 35 | 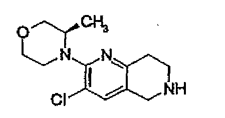 | 2HCl | | 45 | 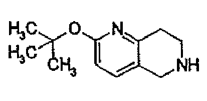 | HCl |
| 36 | 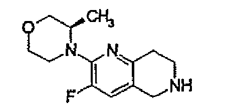 | 2HCl | | 46 | 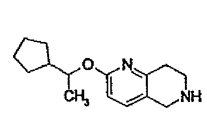 | TsOH |
| 37 | 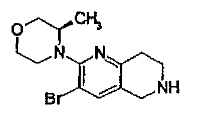 | 2HCl | | 47 | 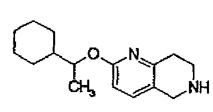 | TsOH |
| 38 | 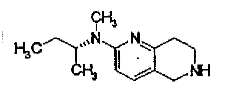 | HBr | | 48 | 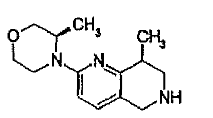 | HCl |
| 39 | 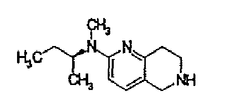 | HBr | | 49 | 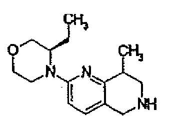 | HCl |
| 40 | 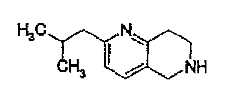 | HCl | | 50 | 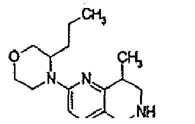 | HCl |
| 41 | 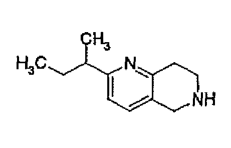 | HCl | | 51 | 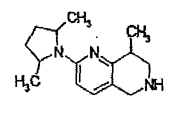 | HCl |

**[Table 4]**

| Ex. No. | structural formula | salt | | Ex. No. | structural formula | salt |
|---|---|---|---|---|---|---|
| 52 | 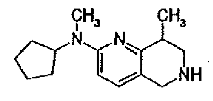 | HCl | | 62 | 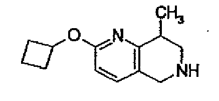 | HCl |
| 53 | 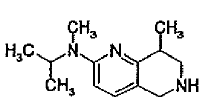 | HCl | | 63 | 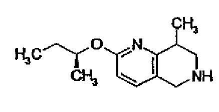 | HCl |
| 54 | 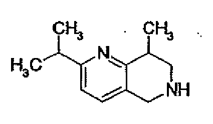 | HCl | | 64 | 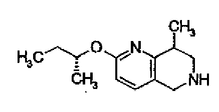 | HCl |
| 55 | 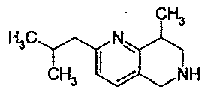 | HCl | | 65 | 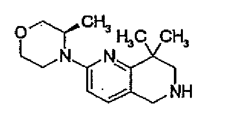 | HCl |
| 56 | 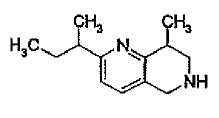 | HCl | | 66 | 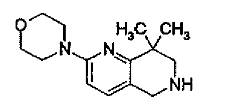 | HCl |
| 57 | 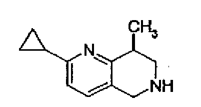 | HCl | | 67 | 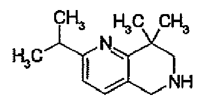 | HCl |
| 58 | 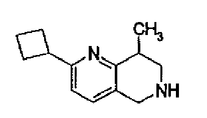 | HBr | | 68 | 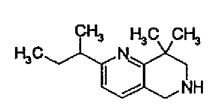 | HCl |
| 59 | 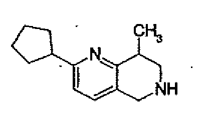 | HCl | | 69 | 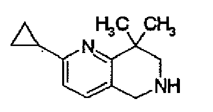 | HCl |
| 60 | 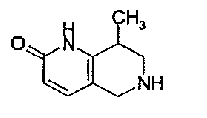 | HCl | | 70 | 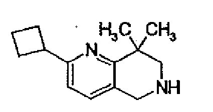 | HCl |
| 61 | 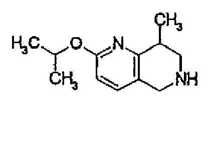 | HCl | | 71 | 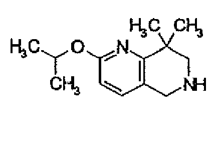 | HCl |

**[Table 5]**

| Ex. No. | structural formula | salt | | Ex. No. | structural formula | salt |
|---|---|---|---|---|---|---|
| 72 | 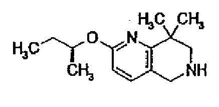 | HCl | | 77 | 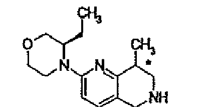 | HCl |
| 73 | 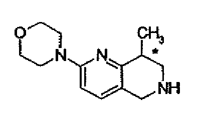 | HCl | | 78 | 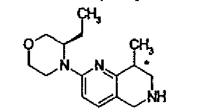 | HCl |
| 74 | 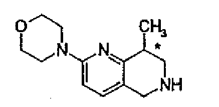 | HCl | | 79 | 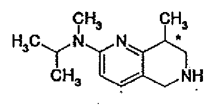 | HCl |
| 75 | 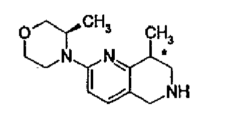 | HCl | | 80 | 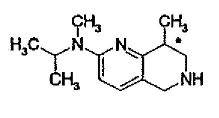 | HCl |
| 76 | 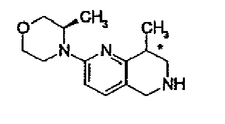 | HCl | | | | |

### Formulation Example 1

| | | |
|---|---|---|
| (1) | Compound of Example 1 | 10 mg |
| (2) | Lactose | 60 mg |
| (3) | Cornstarch | 35 mg |
| (4) | hydroxypropylmethylcellulose | 3 mg |
| (5) | Magnesium stearate | 2 mg |

A mixture of 10 mg of the compound obtained in Example 1, 60 mg of lactose and 35 mg of corn starch is granulated using 0.03 mL of a 10 wt% aqueous solution of hydroxypropylmethylcellulose (3 mg as hydroxypropylmethylcellulose), and then dried at 40°C and sieved. The obtained granules are mixed with 2 mg of magnesium stearate and compressed. The obtained uncoated tablets are sugar-coated with an aqueous suspension of sucrose, titanium dioxide, talc and gum arabic. The thus-coated tablets are glazed with beeswax to give finally-coated tablets.

### Formulation Example 2

| | |
|---|---|
| (1) Compound of Example 1 | 10 mg |
| (2) Lactose | 70 mg |
| (3) Cornstarch | 50 mg |
| (4) Soluble starch | 7 mg |
| (5) Magnesium stearate | 3 mg |

The compound (10 mg) obtained in Example 1 and 3 mg of magnesium stearate are granulated with 0.07 mL of an aqueous solution of soluble starch (7 mg as soluble starch), dried, and mixed with 70 mg of lactose and 50 mg of corn starch. The mixture is compressed to give tablets.

### Reference Formulation Example 1

| | | |
|---|---|---|
| (1) | Rofecoxib | 5.0 mg |
| (2) | Sodium chloride | 20.0 mg |
| (3) | Distilled water | amount to make total volume 2.0 mL |

Rofecoxib (5.0 mg) and 20.0 mg of Sodium chloride are dissolved in distilled water, and water is added to make the total volume 2.0 mL. The solution is filtered, and filled into 2 mL of ampoule under sterile condition. The ampoule is sterilized, and then sealed to give a solution for injection.

### Reference Formulation Example 2

| | | |
|---|---|---|
| (1) | Rofecoxib | 50 mg |
| (2) | Lactose | 34 mg |
| (3) | Cornstarch | 10.6 mg |
| (4) | Cornstarch (paste) | 5 mg |
| (5) | Magnesium stearate | 0.4 mg |
| (6) | Calcium carboxymethylcellulose | 20 mg |
| total | | 120 mg |

The above-mentioned (1) to (6) are mixed according to a conventional method and the mixture is tableted by a tableting machine to give a tablet.

### Formulation Example 3

The formulation prepared in Formulation Example 1 or 2, and the formulation prepared in Reference Formulation Example 1 or 2 are combined.

### Experimental Example 1

The serotonin 5-HT_{2C} receptor agonist activity of the Example compounds was evaluated based on the changes in the intracellular calcium concentration by the following method. After transcription, 5-HT_{2C} undergoes RNA editing of the second intracellular loop, which results in the change of three amino acids and 14 receptor isoforms. 5-HT_{2C} stably expressing CHO cell that expresses isoform type VSV stably was purchased from Euroscreen S.A., and cultured in an UltraCHO (BioWhittaker) medium containing 1% dialyzed bovine serum and 400 µg/ml G418. The cells were plated in a 384-well black clear bottom plate (PE Biosystems) at 5000 cells/well, cultured for 24 hr in a CO₂ incubator, and changes in the intracellular calcium concentration mediated by the 5-HT_{2C} receptor were evaluated using Calcium Kit-Fluo 3 (Dojindo Laboratories). A calcium kit buffer containing 2.5 mM probenecid, 0.04% Pluronic F-127 and 2.5 µg Fluo-3 AM (calcium indicator fluorescent dye) was prepared and used as a Fluo-3 loading solution (contained in Dojindo Laboratories Calcium Kit). The loading solution was incubated at 37°C, the medium in the wells of the cell culture plate was removed, and the loading solution was added to each well by 40 µL. The cells were reacted at 37°C for 1 hr to allow uptake of Fluo-3 AM into the cells and washed. The Example compound was diluted with a calcium kit buffer, and dispensed to each well of the 384-well plate (REMP) by 40 µL to give a Example compound plate. The cell culture plate and test compound plate were set on a Fluometric Imaging Plate Reader (FLIPR, Molecular Devices), and changes in the intracellular calcium concentration were measured. An increase in the fluorescence intensity of Fluo-3 matches with an increase in the intracellular calcium concentration mediated by a receptor. The changes in the intracellular fluorescence intensity were measured every second with a CCD camera of FLIPR and, after measurement for 5 seconds before addition of the compound, a diluted solution of the Example compound was added by 20 µL to each well of the cell culture plate using an automatic dispenser in FLIPR.
The agonist activity was evaluated based on the difference in the fluorescence level obtained by subtracting the fluorescence intensity before addition of the compound from the maximum fluorescence intensity after the addition thereof. The activity of the test compound is shown by the ratio relative to the maximum response by 5-HT (Table 6).

**[Table 6]**

| Ex. No. | ratio relative to maximum response by 5-HT (1 µM) | Ex. No. | ratio relative to maximum response by 5-HT (1 µM) |
|---|---|---|---|
| 2 | 92.8 | 29 | 97 |
| 4 | 101.2 | 30 | 97.8 |
| 5 | 100.7 | 31 | 88.4 |
| 6 | 100.2 | 43 | 97 |
| 7 | 95.6 | 48 | 95.5 |
| 8 | 90.8 | 53 | 91 |
| 15 | 101.8 | 73 | 101 |
| 17 | 100.2 | 74 | 103 |
| 24 | 101.4 | 75 | 103 |
| 26 | 98.2 | 76 | 105 |
| 27 | 91.7 | 79 | 106 |
| 28 | 101.8 | 80 | 102 |

### Experimental Example 2

### [Experiment method]

SD female rats (body weight 180-350 g) were anesthetized with urethane (Wako Pure Chemical Industries, Ltd.), and the spinal cord was cut at T8-9 level to eliminate the micturition reflex. During the operation, halothane (Takeda Pharmaceutical Company Limited) anesthesia was added as necessary. The rats were fixed at a dorsal position, and two catheters (PE-100; Clay Adams) were indwelled in the bladder. One of the catheters was filled with saline stained with Evans Blue dye (Merck), and connected to a 50 ml syringe (TERUMO CORPORATION) fixed on an infusion pump (KD Scientific) via a three-way cock. The other catheter was connected to a pressure transducer (DX-100; NIHON KOHDEN CORPORATION), and signals of the transducer were transmitted to computer via an amplifier (blood pressure amplification unit AP-641G; NIHON KOHDEN CORPORATION) and a data analyzer (BIOPAC; MP100), and recorded on a hard disk. The data was analyzed on the computer using a software (BIOPAC; AcqKnowledge). Saline was injected into the bladder using the infusion pump at a rate of 360 ml/hr, infusion was stopped on the moment fluid leakage from the urethral meatus was observed, and the solution in the bladder was discharged by opening the three-way cock. The maximum intravesical pressure during infusion was taken as a leak point pressure. The leak point pressure was measured at least 3 times until the value was stabilized, the average value of the last three measures was taken as the data, which was compared before and after administration of duloxetine. Duloxetine was dissolved in N,N-dimethylformamide (DMA)/polyethylene glycol 400 (PEG400) (1:1) and intravenously administered at 0.5 ml/kg.

### [Results]

When saline was injected into the bladder of spinal cord transected rat under urethane anesthesia at 360 ml/hr, the intravesical pressure started to rise immediately after injection or several seconds thereafter, and the rise rate was about 10 cmH₂O/sec and incontinence was observed in about 5-10 seconds after the start of the injection. The leak point pressure measured by infusion was 65.7 cmH₂O on average of 10 cases. When duloxetine which is a therapeutic drug for stress urinary incontinence and has been reported to increase the leak point pressure in experiment animals (for example, American Journal of Physiology-Renal Physiology, 2007, vol. 293, p. F920-F926 and the like) was intravenously administered and the leak point pressure was compared before and after the administration, it significantly increased the leak point pressure (Table 7).

Table 7: Action of intravenous administration of duloxetine on leak point pressure in spinal cord-transected female rats under urethane anesthesia by high speed infusion method

**[Table 7]**

| | dose (mg/kg, i.v.) | n | change of leak point pressure (cmH₂O) |
|---|---|---|---|
| solvent | - | 5 | 5.0±2.0 |
| duloxetine | 1 | 5 | 11.4±2.4* |

The data shows mean±SEM of each group. * P<D.05, vs solvent administration group (Student's t-test).

### Experimental Example 3

### [Experiment method]

SD female rats (body weight 180-350 g) were anesthetized with urethane (Wako Pure Chemical Industries, Ltd.), and the spinal cord was cut at T8-9 level to eliminate the micturition reflex. During the operation, halothane (Takeda Pharmaceutical Company Limited) anesthesia was added as necessary. The rats were fixed at a dorsal position, and two catheters (PE-100; Clay Adams) were indwelled in the bladder. One of the catheters was filled with saline stained with Evans Blue dye (Merck), and connected to a 50 ml syringe (TERUMO CORPORATION) fixed on an infusion pump (KD Scientific) via a three-way cock. The other catheter was connected to a pressure transducer (DX-100; NIHON KOHDEN CORPORATION), and signals of the transducer were transmitted to computer via an amplifier (blood pressure amplification unit AP-641G; NIHON KOHDEN CORPORATION) and a data analyzer (BIOPAC; MP100), and recorded on a hard disk. The data was analyzed on the computer using a software (BIOPAC; AcqKnowledge). Saline was injected into the bladder using the infusion pump at a rate of 360 ml/hr, infusion was stopped on the moment fluid leakage from the urethral meatus was observed, and the solution in the bladder was discharged by opening the three-way cock. The maximum intravesical pressure during infusion was taken as a leak point pressure, which was measured repeatedly until the value was stabilized, and the average value of the last three measures was taken as the data. Serotonin 5-HT_{2C} receptor agonist, WAY-161503, WAY-163909, APD-356 and compound A were intravenously administered, and the leak point pressure was compared before and after the administration. All the drugs were dissolved in DMA/PEG400 (1:1) and intravenously administered at 0.5 ml/kg. [Results]

Intravenous administration of all the tested serotonin 5-HT_{2C} receptor agonists, i.e., WAY-161503, WAY-163909, APD-356 and compound A, increased the leak point pressure in a dose-dependent manner (Table 8).

Table 8: Action of intravenous administration of serotonin 5-HT_{2C} receptor agonist on leak point pressure in spinal cord-transected female rats under urethane anesthesia by high speed infusion method

**[Table 8]**

| | dose (mg/kg, i.v.) | n | change of leak point pressure (cmH₂O) |
|---|---|---|---|
| solvent | - | 5 | 5.0±2.0 |
| WAY-161503 | 0.03 | 4 | 7.4±3.8 |
| | 0.3 | 6 | 12.8±1.0* |
| WAY-163909 | 0.1 | 5 | 4.4±1.6 |
| | 1 | 5 | 12.9±4.2 |
| | 3 | 3 | 30.1±2.6* |
| APD-356 | 0.1 | 5 | 5.2±2.6 |
| | 1 | 5 | 19.2±3.1* |
| | 3 | 3 | 28.9±3.3* |
| compound A | 0.1 | 4 | 8.4±5.0 |
| | 1 | 4 | 10.3±2.4 |
| | 3 | 3 | 25.7±8.7* |

The data shows mean+SEM. * P<0.025, increase in the leak point pressure was compared with solvent administration group (one-sided test, Williams' test).

### Experimental Example 4

### [Experiment method]

Female SD rats (body weight 190-310 g) were anesthetized with urethane (Wako Pure Chemical Industries, Ltd.), and the spinal cord was transected at T8-9 level to eliminate the micturition reflex. During the operation, halothane (Takeda Pharmaceutical Company Limited) anesthesia was added when necessary. After abdominal section, bladder neck was ligated with a suture thread, and then the hypogastric nerve and pudendal nerve were bilaterally transected. In some animals, the nerves advancing toward the iliococcygeus muscle and pubococcygeus muscle were also bilaterally transected. A catheter (PE-90, Clay Adams) was placed in the bladder, and the other end of the bladder catheter was connected to a pressure transducer and a water reservoir (60 ml syringe) of saline via a three-way cock. A microtip transducer catheter (SPR-524, Millar Instruments Inc.) was inserted toward the bladder from the urethral orifice, and adjusted using a scale on the catheter surface so that the transducer part is positioned in the urethra at 10.0 - 15.0 mm from the urethral orifice.
The changes in the topical pressure within the urethra (hereinafter conveniently indicated as urethral pressure) as measured by the microtip transducer was transmitted to a computer via an amplifier (blood pressure amplification unit AP-641G; NIHON KOHDEN) and a data analyzer (MP-100; biopack; sampled at 500 Hz), and recorded on a hard disc. The intravesical pressure was rapidly increased to 50 cmH₂O for 30 sec by setting the position of the water reservoir of saline at 50 cm higher, and changes in the urethral pressure were observed. The reaction of the urethra induced by an increased intravesical pressure was measured 3 times, and the average of the last 2 measures was taken as the value before drug administration. The evaluation item was reflex urethral closure response, and the recorded values were subjected to smoothing process at 500 points to calculate an average urethral pressure per 1 sec, after which the value immediately before increase in the intravesical pressure was subtracted from the maximum value on increase in the intravesical pressure and taken as the urethral closure response.

### [Results]

Using rats in which the spinal cord was transected to eliminate the micturition reflex, and the hypogastric nerve and the pudendal nerve controlling the internal urethral sphincter, external urethral sphincter and coccygeus muscle were bilaterally transected, the intravesical pressure was rapidly increased from 0 cmH₂O to 50 cmH₂O. As a result, a urethral pressure-increasing response (urethral closure response) was observed (Fig. 1). The mean±SEM of the urethral closure responses in 20 rats subjected to the experiment was 5.6±0.7 cmH₂O.
In rats in which the nerve advancing toward the iliococcygeus muscle and pubococcygeus muscle was bilaterally transected in addition to the hypogastric nerve and pudendal nerve, the mean±SEM of the urethral closure responses due to an increased intravesical pressure was 1.3±0.4 cmH₂O (4 rats), which was significantly low (P<0.05, two-sided test, Student's t-test) as compared to the rats free of transection of the nerve advancing toward the iliococcygeus muscle and pubococcygeus muscle. The results reveal that the urethral closure response obtained by rapidly increasing the intravesical pressure from 0 cmH₂O to 50 cmH₂O in the rats with bilaterally transected hypogastric nerve and pudendal nerve is attributable to a contractile response mainly by the iliococcygeus muscle and pubococcygeus muscle.

### Experimental Example 5

### [Experiment method]

Female SD rats (body weight 200-310 g) were anesthetized with urethane (Wako Pure Chemical Industries, Ltd.), and the spinal cord was transected at T8-9 level to eliminate the micturition reflex. During the operation, halothane (Takeda Pharmaceutical Company Limited) anesthesia was added when necessary. After abdominal section, bladder neck was ligated with a suture thread, and then the hypogastric nerve and pudendal nerve were bilaterally transected. A catheter (PE-90, Clay Adams) was placed in the bladder, and the other end of the bladder catheter was connected to a pressure transducer and a water reservoir (60 ml syringe) of saline via a three-way cock. A microtip transducer catheter (SPR-524, Millar Instruments Inc.) was inserted toward the bladder from the urethral orifice, and adjusted using a scale on the catheter surface so that the transducer part is positioned in the urethra at 10.0 - 15.0 mm from the urethral orifice.
The changes in the topical pressure within the urinary tract (hereinafter conveniently indicated as urethral pressure) as measured by the microtip transducer was transmitted to a computer via an amplifier (blood pressure amplification unit AP-641G; NIHON KOHDEN) and a data analyzer (MP-100; biopack; sampled at 500 Hz), and recorded on a hard disc. The intravesical pressure was rapidly increased to 50 cmH₂O for 30 sec by setting the position of the water reservoir of saline at 50 cm higher, and changes in the urethral pressure were observed. The reaction of the urinary tract induced by an increased intravesical pressure was measured 3 times, and the average of the last 2 measures was taken as the value before drug administration. The evaluation item was reflex urethral closure response, and the recorded values were subjected to smoothing process at 500 points to calculate an average urethral pressure per 1 sec, after which the value immediately before increase in the intravesical pressure was subtracted from the maximum value on increase in the intravesical pressure and taken as the urethral closure response. After measurement of the value before drug administration, WAY-163909, APD-356 or compound A, which are 5-HT_{2c} receptor agonists, was intravenously administered, and the urethral closure response was evaluated again 10 min later. The drugs were all dissolved in N,N-dimethylformamide/polyethylene glycol 400 (1:1) and administered intravenously at a volume of 0.5 ml/kg.

### [Results]

Using female spinal cord-transected rats in which the hypogastric nerve and pudendal nerve were bilaterally transected, the intravesical pressure was rapidly increased from 0 cmH₂O to 50 cm₂O, and the action of various 5-HT_{2C} receptor agonists (WAY-163909, APD-356 and compound A) on the urethral pressure-increasing response (urethral closure response) induced thereby was investigated. As a result, all of these three kinds of 5-HT_{2C} receptor agonists with different chemotype (a basic skeleton on the chemical structure) enhanced the urethral closure response induced by the increased intravesical pressure (Table 9, Fig. 1). Particularly, WAY-163909 and compound A, which are selective 5-HT_{2C} receptor agonists, enhanced the reflex urethral closure response. The results clarified that the urethral closure response by the iliococcygeus muscle and pubococcygeus muscle, which are pelvic floor muscles, is enhanced by stimulation of a 5-HT_{2C} receptor (Table 9).

Table 9: Action of 5-HT_{2C} receptor agonist on urethral closure response (cmH₂O) induced by increased intravesical pressure in spinal cord transected female rats whose hypogastric nerve and pudendal nerves were bilaterally cut

**[Table 9]**

| | dose (mg/kg, i.v.) | urethral closure response (cmH₂O) | |
|---|---|---|---|
| | | before drug administration | after drug administration |
| solvent administration group | - | 7.9±1.7 | 5.1±2.2 |
| WAY-163909 | 3 | 4.4±0.8 | 10.2±1.6*** |
| APD-356 | 1 | 5.3±1.7 | 8.6±2.7* |
| Compound A | 1 | 4.6±0.8 | 7.5±1.6* |

The data shows mean±SEM of each group (n=5). * P<0.05, *** P<0.001, difference in the urethral closure response before and after drug administration was compared with solvent administration group (two-sided test, Dunnet's t-test).

### Industrial Applicability

Since the compound of the present invention has a superior serotonin 5-HT_{2C} receptor activating action, it is useful as a safe prophylactic or therapeutic drug for any serotonin 5-HT_{2C} associated disease, for example, a lower urinary tract symptom, obesity and/or organ prolapse and the like.
In the screening method for a prophylactic or therapeutic drug for stress urinary incontinence of the present invention, since the urethral resistance upon a rapid and transient rise in the intravesical pressure is measured as a leak point pressure conveniently by a stable procedure, it is superior as an in vivo evaluation system, and can be applied beneficially and efficiently to screening for a substance used for the prophylaxis or treatment of stress urinary incontinence. On the other hand, it is also useful as an evaluation system to verify that a substance used for the prophylaxis or treatment of other diseases does not induce stress urinary incontinence. Moreover, using the screening method of the present invention, various pathological and physiological studies aiming at elucidation of the pathology mechanism of stress urinary incontinence, such as identification of a gene with expression that varies along with pathology and elucidation of behavior thereof, analysis of protein expression variation, study of treatment effect of gene introduction on stress urinary incontinence and the like, can be efficiently conducted with high precision.
Moreover, a substance usable for the screening method of the present invention, for example, a substance that increases leak point pressure upon a rise in the intravesical pressure can be used as a prophylactic or therapeutic agent for stress urinary incontinence.
In the screening method for a prophylactic or therapeutic drug for cystoceles or enteroceles of the present invention, since contraction force of pelvic floor muscles is measured, the method is superior as an in vivo evaluation system for pathology, and can be applied beneficially and efficiently to screening for a substance used for the prophylaxis or treatment of cystoceles or enteroceles. On the other hand, it is also useful as an evaluation system to verify that a substance used for the prophylaxis or treatment of other diseases does not induce cystoceles and enteroceles. Moreover, using the screening method of the present invention, various pathological and physiological studies aiming at elucidation of the pathology mechanism of cystoceles and enteroceles, such as identification of a gene with expression that varies along with pathology and elucidation of behavior thereof, analysis of protein expression variation, study of treatment effect of gene introduction on cystoceles and enteroceles and the like, can be efficiently conducted with high precision. Furthermore, a substance obtained by the screening method of the present invention, for example, a medicament comprising a serotonin 5-HT_{2C} receptor activator can be used as a prophylactic or therapeutic drug for cystoceles or rectocele.

This application is based on a patent application No. 2007-297170 filed in Japan, the contents of which are incorporated in full herein.

## Claims

1. A serotonin 5-HT_{2C} receptor activator comprising a compound represented by the formula 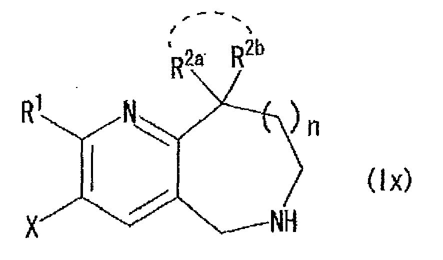 wherein
X is a hydrogen atom or a halogen atom,
R¹ is
(1) a group represented by the formula 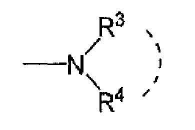 wherein
R³ and R⁴ are the same or different and each is a hydrogen atom, a cycloalkyl group or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ wherein R⁵ is a hydrogen atom, a cycloalkyl group, an alkyl group optionally substituted by a cycloalkyl group, an aryl group or a heteroaryl group,
(3) a group represented by -SR⁶ wherein R⁶ is an alkyl group, an aryl group or a heteroaryl group,
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof.

2. A serotonin 5-HT_{2C} receptor activator comprising a compound represented by the formula 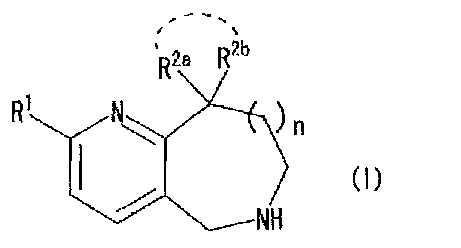 wherein
R¹ is
(1) a group represented by the formula 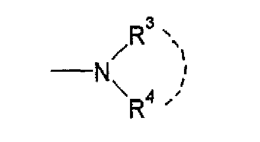 wherein
R³ and R⁴ are the same or different and each is a hydrogen atom or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ wherein R⁵ is an alkyl group optionally substituted by a cycloalkyl group, an aryl group or a heteroaryl group,
(3) a group represented by -SR⁶ wherein R⁶ is an alkyl group, an aryl group or a heteroaryl group,
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof.

3. The agent according to claim 1, wherein R¹ is a group represented by the formula 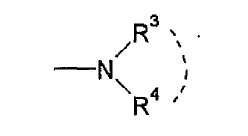 wherein
R³ and R⁴ are the same or different and each is a hydrogen atom, a cycloalkyl group or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group.

4. The agent according to claim 1, wherein n is 0.

5. The agent according to claim 1, which is an agent for the prophylaxis or treatment of a lower urinary tract symptom, obesity and/or organ prolapse.

6. A method for the prophylaxis or treatment of a lower urinary tract symptom, obesity and/or organ prolapse, comprising administering, to a mammal, an effective amount of a compound represented by the formula 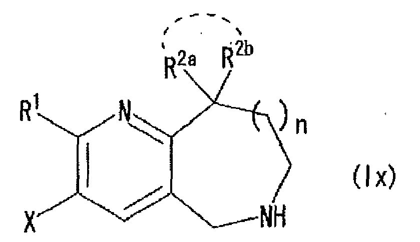 wherein
X is a hydrogen atom or a halogen atom, R¹ is
(1) a group represented by the formula 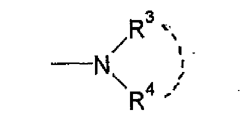 wherein
R³ and R⁴ are the same or different and each is a hydrogen atom, a cycloalkyl group or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ wherein R⁵ is a hydrogen atom, a cycloalkyl group, an alkyl group optionally substituted by a cycloalkyl group, an aryl group or a heteroaryl group,
(3) a group represented by -SR⁶ wherein R⁶ is an alkyl group, an aryl group or a heteroaryl group,
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof.

7. A method for the prophylaxis or treatment of a lower urinary tract symptom, obesity and/or organ prolapse, comprising administering, to a mammal, an effective amount of a compound represented by the formula 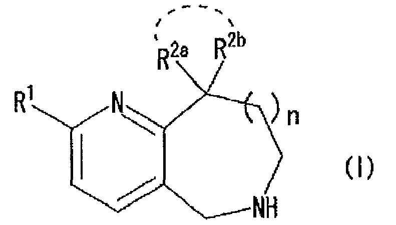 wherein
R¹ is
(1) a group represented by the formula 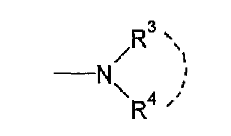 wherein R³ and R⁴ are the same or different and each is a hydrogen atom or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ (R⁵ is an alkyl group optionally substituted by a cycloalkyl group, an aryl group or a heteroaryl group),
(3) a group represented by -SR⁶ (R⁶ is an alkyl group, an aryl group or a heteroaryl group,
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof.

8. Use of a compound represented by the formula 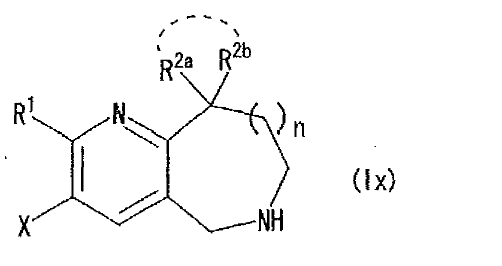 wherein
X is a hydrogen atom or a halogen atom, R¹ is
(1) a group represented by the formula 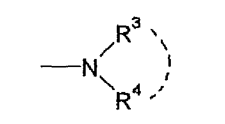 wherein
R³ and R⁴ are the same or different and each is a hydrogen atom, a cycloalkyl group or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ (R⁵ is a hydrogen atom, a cycloalkyl group, an alkyl group optionally substituted by a cycloalkyl group, an aryl group or a heteroaryl group,
(3) a group represented by -SR⁶ (R⁶ is an alkyl group, an aryl group or a heteroaryl group),
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof for the production of a prophylactic or therapeutic agent for a lower urinary tract symptom, obesity and/or organ prolapse.

9. Use of a compound represented by the formula 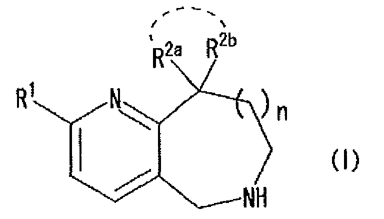 wherein
R¹ is
(1) a group represented by the formula 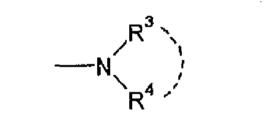 wherein
R³ and R⁴ are the same or different and each is a hydrogen atom or an alkyl group optionally substituted by a cycloalkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ (R⁵ is an alkyl group optionally substituted by a cycloalkyl group, an aryl group or a heteroaryl group),
(3) a group represented by -SR⁶ (R⁶ is an alkyl group, an aryl group or a heteroaryl group),
(4) an alkyl group,
(5) a cycloalkyl group,
(6) an aryl group optionally substituted by substituent(s) selected from a halogen atom, an alkyl group optionally substituted by a halogen atom, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
n is 0 or 1,
or a salt thereof for the production of a prophylactic or therapeutic agent for a lower urinary tract symptom, obesity and/or organ prolapse.

10. A compound represented by the formula 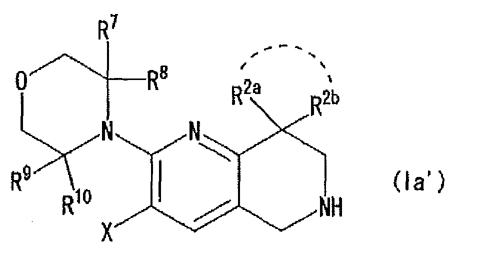 wherein
X is a hydrogen atom or a halogen atom,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
R⁷, R⁸, R⁹ and R¹⁰ are the same or different and each is a hydrogen atom or a substituent,
provided that
(1) one or each of R^{2a} and R^{2b} is an optionally substituted alkyl group,
(2) R⁷, R⁸, R⁹ and R¹⁰ are the same or different and each is a hydrogen atom or a substituent, and R^{2a} and R^{2b} form, together with the adjacent carbon atom, an alicyclic hydrocarbon group optionally substituted by substituent(s) selected from a halogen atom, an alkoxy group and an alkyl group, and
(3) at least one group of R⁷, R⁸, R⁹ and R¹⁰ is a substituent, or a salt thereof.

11. A compound represented by the formula 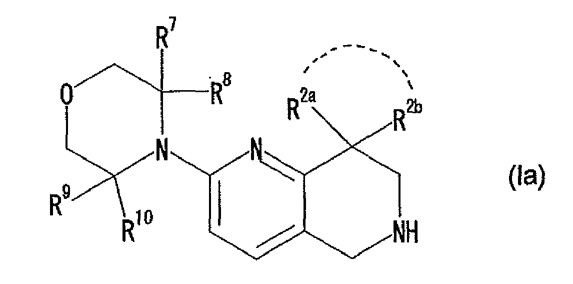 wherein
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, and
R⁷, R⁸, R⁹ and R¹⁰ are the same or different and each is a hydrogen atom or a substituent,
provided that
(1) one or each of R^{2a} and R^{2b} is an optionally substituted alkyl group,
(2) R⁷, R⁸, R⁹ and R¹⁰ are the same or different and each is a hydrogen atom or a substituent, and R^{2a} and R^{2b} form, together with the adjacent carbon atom, an alicyclic hydrocarbon group optionally substituted by substituent(s) selected from a halogen atom, an alkoxy group and an alkyl group, or
(3) at least one group of R⁷, R⁸, R⁹ and R¹⁰ is a substituent,
or a salt thereof.

12. The compound according to claim 10, wherein at least one group of R^{2a}, R^{2b}, R⁷, R⁸, R⁹ and R¹⁰ is an optionally substituted alkyl group.

13. A compound represented by the formula 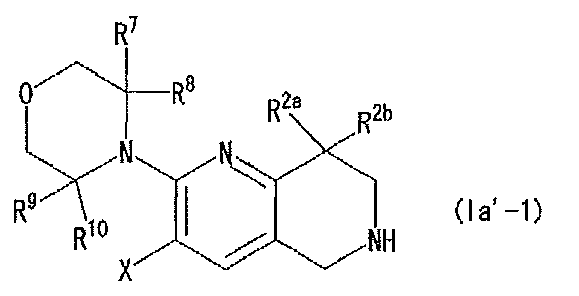 wherein
(1)
X is a hydrogen atom,
R^{2a} and R^{2b} are the same or different and each is a hydrogen atom or a C₁₋₆ alkyl group (provided that one or each of R^{2a} and R^{2b} is a C₁₋₆ alkyl group),
R⁷ and R⁸ are the same or different and each is a hydrogen atom or a C₁₋₆ alkyl group (provided that at least one group of R⁷ and R⁸ is a hydrogen atom), and
R⁹ and R¹⁰ are each a hydrogen atom, or
(2)
X is a hydrogen atom or a halogen atom,
R^{2a} and R^{2b} are each a hydrogen atom,
R⁷, R⁹ and R¹⁰ are each a hydrogen atom, and
R⁸ is a C₁₋₆ alkyl group,
or a salt thereof.

14. A compound represented by the formula 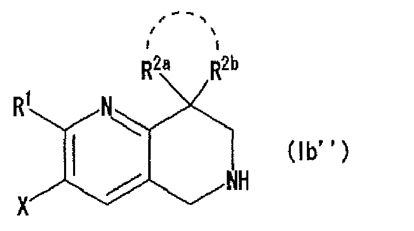 wherein
X is a hydrogen atom or a halogen atom, R¹ is a group represented by the formula 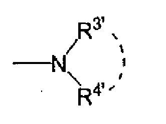 wherein
R³' is a hydrogen atom or an alkyl group optionally substituted by a cycloalkyl group,
R^{4'} is a cycloalkyl group or an alkyl group optionally substituted by a cycloalkyl group, or
R^{3'} and R^{4'} are crosslinked to each other via a carbon-carbon bond to form, together with the adjacent nitrogen atom, a non-aromatic cyclic amino group optionally substituted by substituent(s) selected from a halogen atom, an alkyl group, an alkoxy group and an aryl group,
excluding a methylamino group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group,
or a salt thereof.

15. A compound represented by the formula 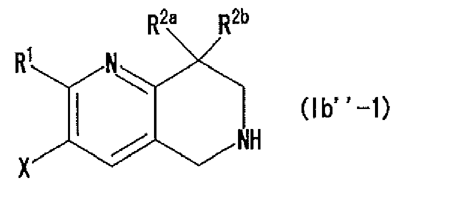 wherein
X is a hydrogen atom or a halogen atom, R¹ is
(1) a group represented by the formula 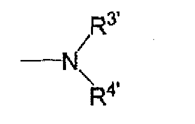 wherein
R^{3'} is a hydrogen atom or a C₁₋₆ alkyl group, and R^{4'} is a C₃₋₆ cycloalkyl group or a C₁₋₆ alkyl group optionally substituted by a C₃₋₆ cycloalkyl group, excluding a methylamino group, or
(2) a group represented by the formula 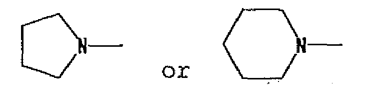 optionally substituted by 1 to 3 C₁₋₆ alkyl group, and
R^{2a} and R^{2b} are the same or different and each is a hydrogen atom or a C₁₋₆ alkyl group,
or a salt thereof.

16. A compound represented by the formula 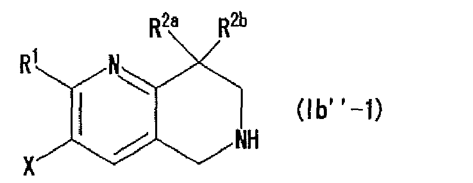 wherein
X is a hydrogen atom or a halogen atom, R¹ is
(1) a group represented by the formula 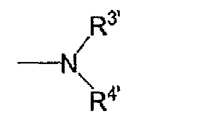 wherein
R^{3'} is a hydrogen atom or a C₁₋₆ alkyl group, and R^{4'} is C₃₋₆ cycloalkyl or a C₁₋₆ alkyl group optionally substituted by a C₃₋₆ cycloalkyl group,
excluding a methylamino group, or
(2) a group represented by the formula 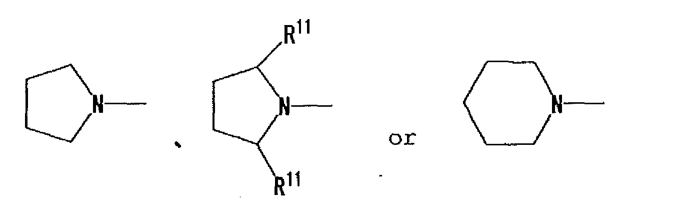 wherein R¹¹ is a hydrogen atom or a C₁₋₆ alkyl group, and
R^{2a} and R^{2b} are the same or different and each is a hydrogen atom or a C₁₋₆ alkyl group (provided that at least one group of R^{2a} and R^{2b} is a hydrogen atom),
or a salt thereof.

17. A compound represented by the formula 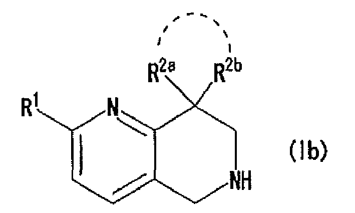 wherein
R¹ is a group represented by the formula 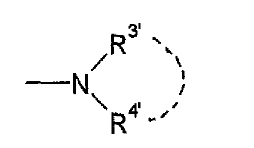 wherein
R^{3'} is a hydrogen atom or an alkyl group optionally substituted by a cycloalkyl group,
R^{4'} is an alkyl group optionally substituted by a cycloalkyl group, or
R^{3'} and R^{4'} are crosslinked to each other via a carbon-carbon bond to form, together with the adjacent nitrogen atom, a non-aromatic cyclic amino group optionally substituted by substituent(s) selected from a halogen atom, an alkyl group, an alkoxy group and an aryl group,
excluding a methylamino group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group,
or a salt thereof.

18. A compound represented by the formula 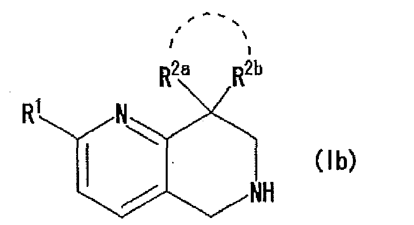 wherein
R¹ is
(1) a group represented by -OR⁵ (R⁵ is a hydrogen atom, a cycloalkyl group, an alkyl group optionally substituted by a cycloalkyl group, an aryl group or a heteroaryl group),
(2) a C₃₋₅ alkyl group (alkyl group having a carbon number of 3 to 5),
(3) an aryl group optionally substituted by substituent(s) selected from a halogen atom, an alkyl group optionally substituted by a halogen atom, an alkoxy group, an alkylthio group and an aryl group,
(4) a heteroaryl group, or
(5) a cycloalkyl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group, (excluding a compound wherein R¹ is a methoxy group and R^{2a} and R^{2b} are each a hydrogen atom),
or a salt thereof.

19. A compound represented by the formula 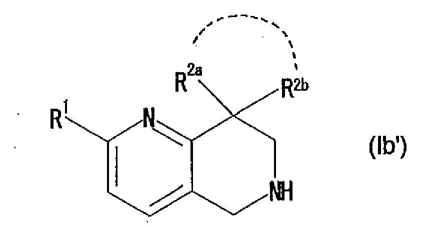 wherein
R¹ is
(1) a group represented by -OR⁵ (R⁵ is an alkyl group optionally substituted by a cycloalkyl group, an aryl group or a heteroaryl group),
(2) a C₃ alkyl group (alkyl group having a carbon number of 3),
(3) an aryl group optionally substituted by substituent(s) selected from a halogen atom, an alkyl group optionally substituted by a halogen atom, an alkoxy group, an alkylthio group and an aryl group, or
(4) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group,
(excluding a compound wherein R¹ is a methoxy group and R^{2a} and R^{2b} are each a hydrogen atom),
or a salt thereof.

20. A compound represented by the formula 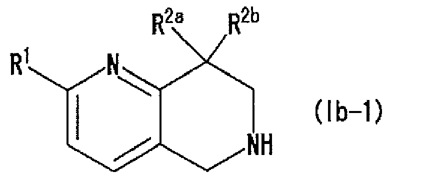 wherein
R¹ is
(1) a group represented by -OR⁵ (R⁵ is a hydrogen atom, a C₃₋₆ cycloalkyl group, or a C₁₋₆ alkyl group optionally substituted by a C₃₋₆ cycloalkyl group),
(2) a C₃₋₅ alkyl group,
(3) a phenyl group optionally substituted by substituent(s) selected from a halogen atom, a C₁₋₆ alkyl group optionally substituted by a halogen atom, and a C₁₋₆ alkylthio group,
(4) a group represented by the formula 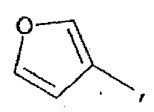 or
(5) a C₃₋₆ cycloalkyl group,
R^{2a} and R^{2b} are the same or different and each is a hydrogen atom or an optionally substituted alkyl group, (excluding a compound wherein R¹ is a methoxy group and R^{2a} and R^{2b} are each a hydrogen atom),
or a salt thereof.

21. The compound according to claim 18, wherein R¹ is a phenyl group substituted by substituent(s) selected from a halogen atom, a C₁₋₆ alkyl group substituted by a halogen atom and a C₁₋₆ alkylthio group.

22. A compound represented by the formula 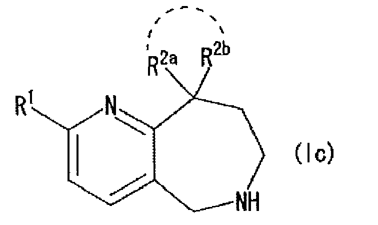 wherein
R¹ is
(1) a group represented by the formula 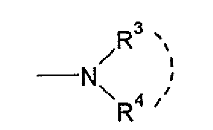 wherein
R³ and R⁴ are the same or different and each is a hydrogen atom or an alkyl group, or
R³ and R⁴ form, together with the adjacent nitrogen atom, a non-aromatic cyclic amino group optionally substituted by substituent(s) selected from a halogen atom, an alkyl group, an alkoxy group and an aryl group,
(2) a group represented by -OR⁵ (R⁵ is an alkyl group, an aryl group or a heteroaryl group),
(3) a group represented by -SR⁶ (R⁶ is an alkyl group, an aryl group or a heteroaryl group,
(4) an alkyl group,
(5) a cycloalkyl group,
(6) an aryl group optionally substituted by substituent(s) selected from a halogen atom, an alkyl group, an alkoxy group, an alkylthio group and an aryl group, or
(7) a heteroaryl group,
R^{2a} and R^{2b} are the same or different and each is a hydrogen atom or an optionally substituted alkyl group, or
R^{2a} and R^{2b} form, together with the adjacent carbon atom, an alicyclic hydrocarbon group optionally substituted by substituent(s) selected from a halogen atom, an alkyl group and an alkoxy group,
or a salt thereof.

23. A compound represented by the formula 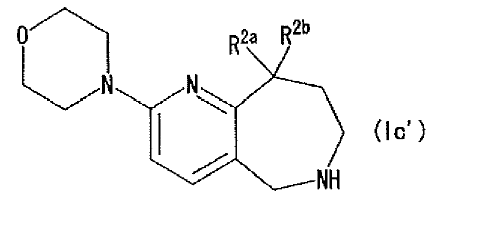 wherein one of R^{2a} and R^{2b} is a hydrogen atom, and the other is a C₁₋₆ alkyl group,
or a salt thereof.

24. 2-Isopropyl-5,6,7,8-tetrahydro-1,6-naphthyridine or a salt thereof.

25. 2-Isopropoxy-5,6,7,8-tetrahydro-1,6-naphthyridine or a salt thereof.

26. 2-Cyclopropyl-5,6,7,8-tetrahydro-1,6-naphthyridine or a salt thereof.

27. 8-Methyl-2-morpholin-4-yl-5,6,7,8-tetrahydro-1,6-naphthyridine or a salt thereof.

28. 8-Methyl-2-[(3R)-3-methylmorpholin-9-yl]-5,6,7,8-tetrahydro-1,6-naphthyridine or a salt thereof.

29. 9-Methyl-2-morpholin-4-yl-6,7,8,9-tetrahydro-5H-pyrido[3,2-c]azepine or a salt thereof.

30. N-Isopropyl-N,8-dimethyl-5,6,7,8-tetrahydro-1,6-naphthyridin-2-amine or a salt thereof.

31. A prodrug of the compound according to any one of claims 10 to 30.

32. A medicament comprising the compound according to any one of claims 10 to 30 or a prodrug thereof.

33. A method of screening for a substance that increases a leak point pressure upon a rise in the intravesical pressure, which comprises measuring a leak point pressure when an intravesical pressure is increased by injection of saline rapidly and transiently into the bladder of an animal showing no micturition reflex but showing a functional bladder-spinal cord-urethra reflex.

34. A method of screening for a prophylactic or therapeutic drug for stress urinary incontinence, which comprises measuring a leak point pressure when an intravesical pressure is increased by injection of saline rapidly and transiently into the bladder of an animal showing no micturition reflex but showing a functional bladder-spinal cord- urethra reflex.

35. The method according to claim 33 or 34, wherein the animal used shows a decreased leak point pressure upon a rise in the intravesical pressure.

36. The method according to claim 35, wherein the decreased leak point pressure upon a rise in the intravesical pressure is based on the transection or injury of the nerve involved in the reflex contraction of the pelvic floor muscles or the external urethral sphincter, childbirth, ovariectomy, a mechanical vaginal expansion treatment, diabetes, a drug administration or a combination of these.

37. The method according to claim 33 or 34, wherein the animal is female.

38. A drug for the prophylaxis or treatment of cystoceles or enteroceles, comprising a serotonin 5-HT_{2C} receptor activator.

39. A method for the prophylaxis or treatment of cystoceles or enteroceles, comprising administering an effective amount of a serotonin 5-HT_{2C} receptor activator to a mammal.

40. Use of a serotonin 5-HT_{2C} receptor activator for the production of an agent for the prophylaxis or treatment of cystoceles or enteroceles.

41. A method of screening for a drug for the prophylaxis or treatment of cystoceles or enteroceles, comprising increasing an intravesical pressure after bilateral transection of the hypogastric nerve and pudendal nerve of an animal, and measuring a closure response in the urethra, the rectum or the vagina observed at that time.

42. A method of screening for a drug for the prophylaxis or treatment of cystoceles or enteroceles, comprising increasing an intravesical pressure after bilateral transection of the hypogastric nerve and pudendal nerve of an animal, and measuring the leak point pressure at that time.

43. The method according to claim 41 or 42, wherein the closure response in the urethra, the rectum or the vagina or urethral resistance (decreased leak point pressure) upon increase in the intravesical pressure after bilateral transection of the hypogastric nerve and pudendal nerve of an animal is based on the unilateral transection or injury of the nerve involved in the reflex contraction of the pelvic floor muscles, childbirth, ovariectomy, a mechanical vaginal expansion treatment, diabetes, a drug administration or a combination of these.

44. The method according to any one of claims 41 to 43, wherein the animal is female.
